# EUROPÄISCHE PATENTSCHRIFT

(11) **EP 0 871 630 B1**
(45) Veröffentlichungstag und Bekanntmachung des Hinweises auf die Patenterteilung: **02.01.2002**
(21) Anmeldenummer: 96934560.2
(22) Anmeldetag: 07.10.1996
(51) Int. Cl.: C07D 491/147, A61K 31/435, C07D 491/14

(54) **4a,5a,8a,8b-TETRAHYDRO-6H-PYRROLO 3',4':4,5]FURO 3,2-b]PYRIDIN-6,8(7H)-DION DERIVATE ZUR BEKÄMPFUNG VON ENDOPARASITEN UND VERFAHREN ZU IHRER HERSTELLUNG**
4a,5a,8a,8b-TETRAHYDRO-6H-PYRROLO 3,4':4,5]FURO(3,2-b)PYRIDINE-6,8 7H]-DIONE DERIVATIVES FOR USE IN CONTROLLING ENDOPARASITES AND A METHOD OF PRODUCING SAID DERIVATIVES
DERIVES DE 4a,5a,8a,8b-TETRAHYDRO-6H-PYRROLO(3',4':4,5)FURO(3,2-b)PYRIDINE-6,8(7H)-DIONE POUR LUTTER CONTRE LES ENDOPARASITES ET PROCEDE DE PRODUCTION DESDITS DERIVES

(30) Priorität: 19.10.1995 DE 19538960
(43) Veröffentlichungstag der Anmeldung: 21.10.1998
(73) Patentinhaber: BAYER AG, 51368 Leverkusen (DE)
(72) Erfinder: JESCHKE, Peter, D-51373 Leverkusen (DE); HARDER, Achim, D-51109 Köln (DE); MENCKE, Norbert, D-51381 Leverkusen (DE)
(86) Internationale Anmeldenummer: EP9604346
(87) Internationale Veröffentlichungsnummer: WO9714695

(56) Entgegenhaltungen:
- DE-A- 4 121 365
- US-A- 5 225 434
- CHEM. PHARM. BULL., Bd. 35, Nr. 3, 1987, Seiten 1049-1057, XP002022281 TAZUKO HISANO ET AL.: "Reaction of aromatic n-oxides with dipolarophiles. XII. Stereoselective exo cycloaddition of 3,5-lutidine N-oxide with N-substituted maleimides and a frontier molecular orbital and mechanistic study" in der Anmeldung erwähnt
- CHEM. PHARM. BULL. , Bd. 39, Nr. 1, 1991, Seiten 10-17, XP002022282 TOSHIKAZU MATSUOKA ET AL.: "Reaction of aromatic N-oxides with dipolarophiles. XV. Formation of the 1,5-sigmatropy products and their double ene reaction products" in der Anmeldung erwähnt

## Beschreibung

Die vorliegende Erfindung betrifft die Verwendung von 4a, 5a, 8a, 8b-Tetrahydro-6H-pyrrolo[3',4':4,5]furo[3,2-b]pyridin-6,8(7H)-dion Derivaten zur Bekämpfung von Endo-parasiten, neue 4a, 5a, 8a, 8b-Tetrahydro-6H-pyrrolo[3',4':4,5]furo[3,2-b]pyridin-6,8 (7H)-dion Derivate und Verfahren zu ihrer Herstellung.

Einige 4a, 5a, 8a, 8b-Tetrahydro-6H-pyrrolo[3',4':4,5]furo[3,2-b]pyridin-6,8(7H)-dion Derivate sind bereits bekannt. Es ist jedoch nichts über ihre Verwendung gegen Endoparasiten bekannt (vgl. Z. B.: T. Hisano et al. Chem. Pharm. Bull. 35 (3), (1987) S. 1049-1057; Heterocycles 29 (6), (1989) S. 1029-1032; Chem. Pharm. Bull. 38 (3), (1990) S. 605-611; Chem. Pharm. Bull. 39 (1), (1991) S. 10-17).

Die vorliegende Erfindung betrifft:
1. Die Verwendung von 4a, 5a, 8a, 8b-Tetrahydro-6H-pyrrolo[3',4':4,5]furo[3,2-b]pyridin-6,8(7H)-dion Derivaten der allgemeinen Formel ( I ) und deren Salze, 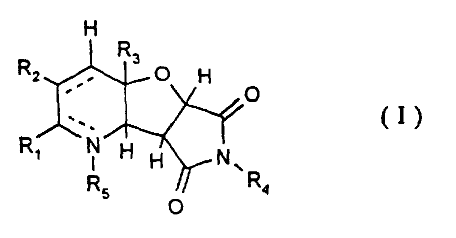 in welcher
   - R¹: für Wasserstoff, geradkettiges oder verzweigtes Alkyl, Cycloalkyl, Arylalkyl, Aryl, Heteroaryl, Heteroarylalkyl, die gegebenenfalls substituiert sind, steht,
   - R²: für Wasserstoff, geradkettiges oder verzweigtes Alkyl, Cycloalkyl, Alkoxycarbonyl, steht, die gegebenenfalls substituiert sind,
   - R¹ und R²: gemeinsam mit den Atomen, an die sie gebunden sind, für einen 5- oder 6-gliedrigen Ring stehen, der gegebenfalls duch Sauerstoff, Schwefel, Sulfoxyl oder Sulfonyl unterbrochen sein kann und gegebenenfalls substituiert ist,
   - R³: für Wasserstoff, geradkettiges oder verzweigtes Alkyl, Cycloalkyl, Alkoxycarbonyl, steht, die gegebenenfalls substituiert sind,
   - R⁴: für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl, Amino, Alkylamino, Dialkylamino, Cycloalkylamino, die gegebenenfalls substituiert sind, steht,
   - R⁵: für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl, die gegebenenfalls substituiert sind, Formyl, Alkoxydicarbonyl oder gegebenenfalls für einen Rest aus der Gruppe G¹, G², G³ und G⁴ steht 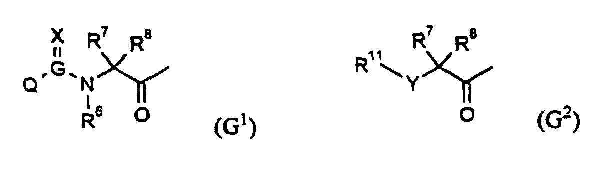 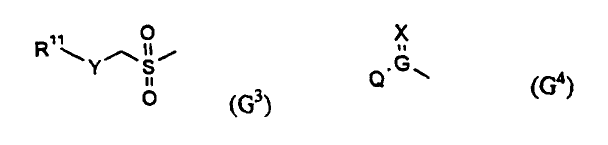 worin
   - R⁶: für Wasserstoff, geradkettiges oder verzweigtes Alkyl, Cycloalkyl, Aryl, Arylalkyl, Heteroaryl, Heteroarylalkyl, die gegebenenfalls substituiert sind, steht,
   - R⁶ und R⁷: gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
   - R⁷ und R⁸: unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
   - R⁷ und R⁸: gemeinsam für einen spirocyclischen Ring stehen, der gegebenenfalls substituiert ist, 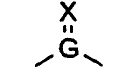 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeuten kann,
   - R⁹: für Wasserstoff, Hydroxy, Alkoxy, Alkylcarbonyl, Halogenalkylcarbonyl, Alkylsulfonyl, Nitro oder Cyan steht, und
   - R¹⁰: für Wasserstoff oder Alkyl steht, und
   - Q: für geradkettiges oder verzweigtes Alkyl, Alkenyl, Alkinyl, Cycloalkyl, Aryl, Arylalkyl, Hetaryl oder Hetarylalkyl, die gegebenenfalls substituiert sind, oder gegebenefalls für einen Rest aus der Gruppe G⁵ und G⁶ 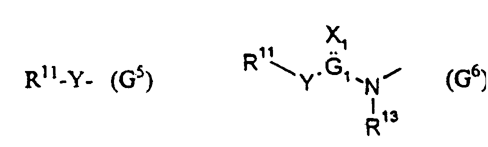 steht,
   worin 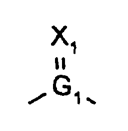 Carboxy, Thiocarboxy oder Sulfonyl bedeuten kann,
   - Y: für Sauerstoff, Schwefel oder -NR¹² steht,
   - R¹¹: für den Fall, daß Y für Stickstoff steht, eine über ein Stickstoffatom verknüpfte cyclische Aminogruppe bedeuten kann,
   - R¹¹ und R¹²: unabhängig voneinander für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl stehen, die gegebenenfalls substituiert sind, oder
   - R¹¹ und R¹²: gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls substituiert ist,
   - R¹³: für Wasserstoff oder Alkyl steht,
   - R¹⁴: für Wasserstoff, geradkettiges oder verzweigtes Alkyl, Alkenyl, Alkinyl, Cycloalkyl, Cycloalkylalkyl, Alkoxycarbonyl, Alkylcarbonyl, Cycloalkylcarbonyl, Cyan, Aryl, Arylalkyl, Heteroaryl, Heteroarylalkyl stehen, die gegebenenfalls substituiert sind, steht,
zur Bekämpfung von Endoparasiten in der Medizin und Tiermedizin.

Die Verbindungen der Formel (I) können in Abhängigkeit von der Art der Substituenten als geometrische und/oder optische Isomere oder Isomerengemische unterschiedlicher Zusammensetzung auftreten. Die Erfindung betrifft sowohl die reinen Isomeren als auch die Isomerengemische.

In der Formel (I) kann die gestrichelte Linie eine Einfachbindung bedeuten oder für eine oder zwei Doppelbindungen zwischen dem Kohlenstoffatom, das den Substituenten R² trägt, und dem benachbarten Kohlenstoffatom und/oder zwischen dem Kohlenstoffatom und dem benachbarten Stickstoffatom, die die Substituenten R¹ und R⁵ tragen, stehen.

Im Falle einer vorhandenen Doppelbindung zwischen dem Kohlenstoffatom, das den Substituenten R¹ trägt, und dem benachbarten Stickstoffatom mit einem Substituenten R⁵, liegen die Verbindungen der Formel ( I ) in Form ihrer Salze vor.

Bevorzugt verwendet werden die 4a, 5a, 8a, 8b-Tetrahydro-6H-pyrrolo[3',4':4,5]furo[3,2-b]pyridin-6,8(7H)-dion Derivate der allgemeinen Formel (I) und deren Salze, 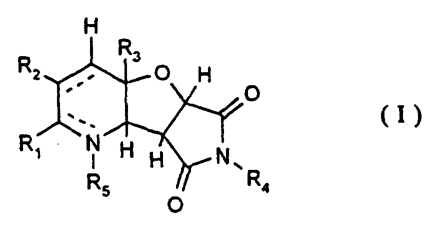 in welcher
- R¹: für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
- R¹ und R²: gemeinsam mit den Atomen, an die sie gebunden sind, für einen 5- oder 6-gliedrigen Ring stehen, der gegebenfalls substituiert ist,
- R² und R³: unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
- R⁴: für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxykarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁₋₄-Alkylamino, C₁₋₄-Dialkylamino, C₃₋₇-Cycloalkylamino, die gegebenenfalls substituiert sind, steht,
- R⁵: für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, Nitro-C₁₋₄-alkyl, Cyano-C₁₋₄-alkyl, C₁₋₄-Alkoxycarbonyl-C₁₋₄-alkyl, Carbamoyl-C₁₋₄-alkyl, Carboxyl-C₁₋₄-alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Formyl, C₁₋₄-Alkoxydicarbonyl oder für einen Rest der Gruppe G¹, G², G³ und G⁴ 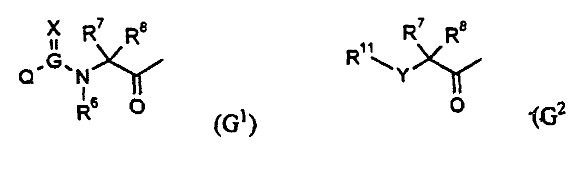 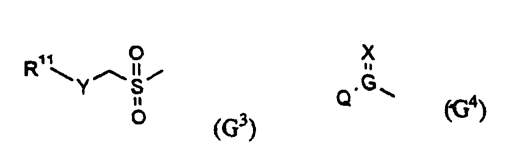 worin
- R⁶: für Wasserstoff, geradkettiges oder verzweigtes C₁₋₆-Alkyl, C₃₋₆-Cycloalkyl, Aryl-C₁₋₂-alkyl, Heteroaryl-C₁₋₂-alkyl, die gegebenenfalls substituiert sind, steht,
- R⁶ und R⁷: gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
- R⁷ und R⁸: unabhängig voneinander für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxy-C₁₋₆-alkyl, C₁₋₂-Alkoxy-C₁₋₆-alkyl, Mercapto-C₁₋₆-alkyl, C₁₋₂-Alkylthio-C₁₋₆-alkyl, C₁₋₂-Alkylsulfinyl-C₁₋₆-alkyl, C₁₋₂-Alkylsulfonyl-C₁₋₆-alkyl, Carboxy-C₁₋₆-alkyl, Carbamoyl-C₁₋₆-alkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste oder durch einen, zwei, drei oder vier C₁₋₂-Alkylreste substituiert sein kann, C₁₋₄-Alkoxycarbonylamino-C₁₋₆-alkyl, C₂₋₆-Alkenyl, C₃₋₆-Cyclo-C₁₋₂-alkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl stehen, oder
- R⁷ und R⁸: gemeinsam für einen spirocyclischen Ring stehen, 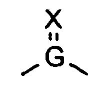 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeutet,
- R⁹: für Wasserstoff, Hydroxy, C₁₋₄-Alkoxy, C₁₋₄-Alkylcarbonyl, Halogen-C₁₋₄-alkylcarbonyl, C₁₋₄-Alkylsulfonyl, Nitro oder Cyan steht, und
- R¹⁰: für Wasserstoff oder C₁₋₄-Alkyl steht, und
- Q: für geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste, tert-Butyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₂₋₆-Alkinyl, C₃₋₆-Cycloalkyl, Aryl, Aryl-C₁₋₂-alkyl, Heteroaryl oder Heteroaryl-C₁₋₂-alkyl, die gegebenenfalls substituiert sind, oder gegebenefalls für einen Rest aus der Gruppe G⁵ und G⁶ 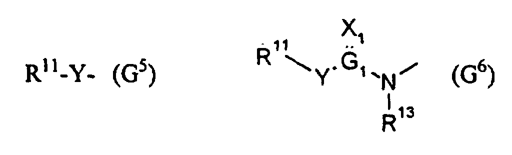 steht,
worin 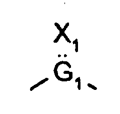 Carboxy, Thiocarboxy oder Sulfonyl bedeuten kann,
- Y: für Sauerstoff, Schwefel oder -NR¹² steht,
- R¹¹: für den Fall, daß Y für Stickstoff steht, eine über ein Stickstoffatom verknüpfte cyclische Aminogruppe bedeuten kann,
- R¹¹ und R¹²: unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl stehen, die gegebenenfalls substituiert sind, oder
- R¹¹ und R¹²: gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O,-N=, -NR¹⁴- oder durch quaternisierten Stickstoff unterbrochen sein kann und gebenenfalls substituiert ist,
- R¹³: für Wasserstoff oder C₁₋₄-Alkyl steht,
- R¹⁴: für Wasserstoff, geradkettiges oder verzweigtes C₁₋₆-Alkyl, C₂₋₆-Alkenyl, C₂₋₆-Alkinyl, C₃₋₈-Cycloalkyl, C₃₋₈-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Heteroaryl, Heteroaryl-C₁₋₂-alkyl stehen, die gegebenenfalls substituiert sind, steht,
sowie deren optische Isomere und Racemate,
zur Bekämpfung von Endoparasiten in der Medizin und Tiermedizin.

Die Verbindungen der allgemeinen Formel ( I ) sind teilweise bekannt und lassen sich analog zu bekannten Verfahren herstellen.

Die Erfindung betrifft ferner:
2. Neue 4a, 5a, 8a, 8b-Tetrahydro-6H-pyrrolo[3',4':4,5]furo[3,2-b]pyridin-6,8(7H)-dion Derivate der allgemeinen Formel ( Ia ) und deren Salze, 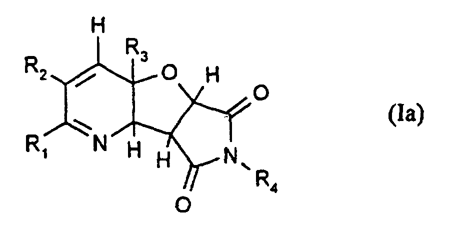 in welcher
   - R¹: für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
   - R² und R³: unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
   - R⁴: für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxycarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alklnyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁₋₄-Alkylamino, C₁₋₄-Dialkylamino, C₃₋₇-Cycloalkylamino, die gegebenenfalls substituiert sind, steht,
   mit der Maßgabe für den Fall, daß
   - R¹: für Wasserstoff steht und
   - R² und R³: für Methyl stehen,
   - R⁴: nicht für als Methyl, n-Butyl, Phenyl, 2-, 3- oder 4-Methylphenyl, 2-, 3- oder 4-Chlor-phenyl, 2-, 3- oder 4-Fluorphenyl, 2-Chlor, 4-fluor-phenyl, 2-Fluor, 4-chlor-phenyl, 3-Chlor, 4-fluorphenyl, 2-, 3- oder 4-Nitro-phenyl, 4-Methoxy-phenyl oder α-Naphthyl steht,
   sowie deren optische Isomere und Racemate.
3. Verfahren zur Herstellung der neuen 4a, 5a, 8a, 8b-Tetrahydro-6H-pyrrolo[3',4':4,5]furo[3,2-b]pyridin-6,8(7H)-dion Derivate der allgemeinen Formel ( Ia ) und deren Salze, 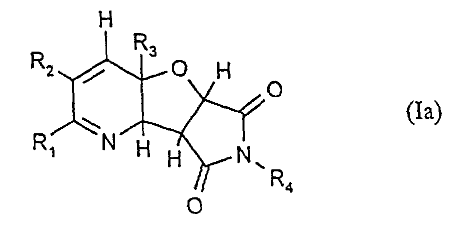 in welcher
   R¹, R², R³ und R⁴ die unter Punkt 2 angegebene Bedeutung besitzen,
   dadurch gekennzeichnet, daß man
   a) Pyridin-N-oxide der allgemeinen Formel ( II ) 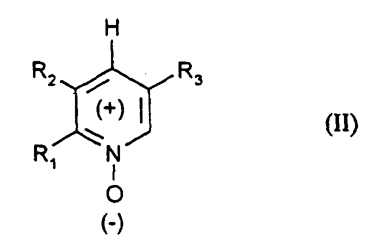
   in welcher
   R¹, R² und R³ die unter Punkt 2 angegebene Bedeutung besitzen, mit Maleinsäureimiden der allgemeinen Formel (III) 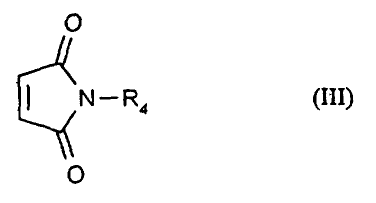 in welcher
   R⁴ die unter Punkt 2 angegebene Bedeutung hat, gegebenenfalls in Gegenwart eines Vedünnungsmittels umsetzt.
   Die Erfindung betrifft ferner:
4. Neue 1, 2, 3, 4, 4a, 5a, 8a, 8b-Oktahydro-6H-pyrrolo[3',4':4,5]furo[3,2-b]pyridin-6,8(7H)-dion- und 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) sowie deren Salze, 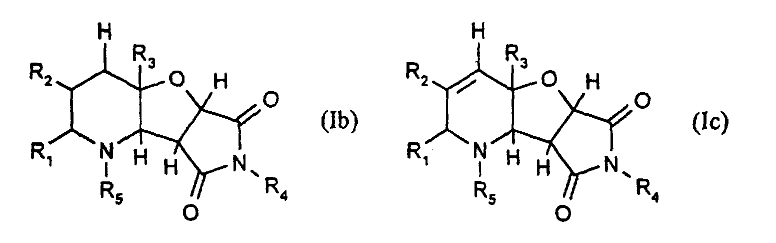 in welchen
   - R¹: für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
   - R² und R³: unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
   - R⁴: für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxykarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁₋₄-Alkylamino, C₁₋₄-Dialkylamino, C₃₋₇-Cycloalkylalmino, die gegebenenfalls substituiert sind, steht,
   - R⁵: für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, Nitro-C₁₋₄-alkyl, Cyano-C₁₋₄-alkyl, C₁₋₄-Alkoxycarbonyl-C₁₋₄-alkyl, Carbamoyl-C₁₋₄-alkyl, Carboxyl-C₁₋₄-alkyl,C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Formyl, C₁₋₄-Alkoxydicarbonyl oder für einen Rest aus der Gruppe G¹, G², G³ und G⁴ 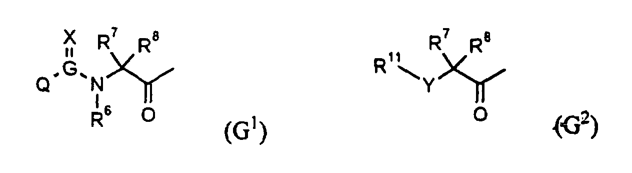 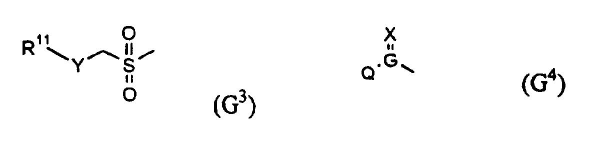 worin
   - R⁶: für Wasserstoff, geradkettiges oder verzweigtes C₁₋₄-Alkyl, C₃₋₆-Cycloalkyl, Aryl-C₁₋₂-alkyl oder Heteroaryl-C₁₋₂-alkyl, die gegebenenfalls substituiert sind, steht,
   - R⁶ und R⁷: gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
   - R⁷: für Wasserstoff, geradkettiges oder verzweigtes C₁₋₆-Alkyl, C₂₋₄-Alkenyl, C₃₋₆-Cycloalkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl stehen, oder
   - R⁸: für Wasserstoff oder Methyl steht, 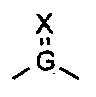 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeuten kann,
   - R⁹: für Wasserstoff, Hydroxy, C₁₋₄-Alkoxy, C₁₋₄-Alkylcarbonyl, Halogen-C₁₋₄-alkylcarbonyl, C₁₋₄-Alkylsulfonyl, Nitro oder Cyan steht, und
   - R¹⁰: für Wasserstoff oder C₁₋₄-Alkyl steht, und
   - Q: für geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste, tert-Butyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₂₋₆-Alkinyl, C₃₋₆-Cycloalkyl, Aryl, Aryl-C₁₋₂-alkyl, Heteroaryl oder Hetaryl-C₁₋₂-alkyl, die -gegebenenfalls substituiert sind, oder gegebenefalls für einen Rest aus der Gruppe G⁵ und G⁶ 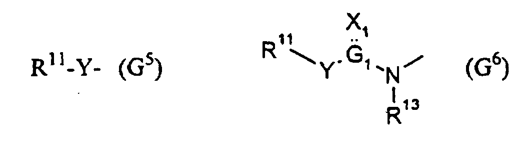 steht,
   worin 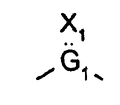 Carboxy, Thiocarboxy oder Sulfonyl bedeuten kann,
   - Y: für Sauerstoff, Schwefel oder -NR¹² steht,
   - R¹¹: für den Fall, daß Y für Stickstoff steht, eine über ein Stickstoffatom verknüpfte cyclische Aminogruppe bedeuten kann,
   - R¹¹ und R¹²: unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl stehen, die gegebenenfalls substituiert sind, oder
   - R¹¹ und R¹²: gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls substituiert ist,
   - R¹³: für Wasserstoff oder C₁₋₄-Alkyl steht,
   - R¹⁴: für Wasserstoff, geradkettiges oder verzweigtes C₁₋₆-Alkyl, C₂₋₆-Alkenyl, C₂₋₆-Alkinyl, C₃₋₈-Cycloalkyl, C₃₋₈-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Heteroaryl, Heteroaryl-C₁₋₂-alkyl stehen, die gegebenenfalls substituiert sind, steht,
   sowie deren optische Isomere und Racemate.
5. Verfahren zur Herstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) sowie deren Salze, 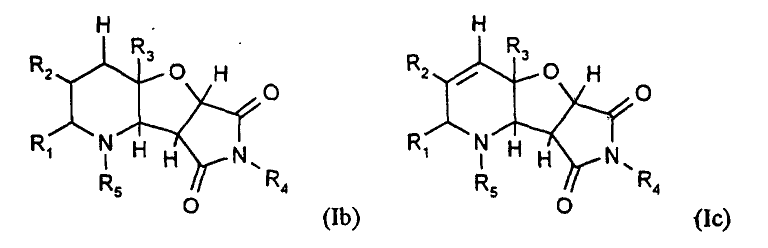 in welchen
   R¹, R², R³, R⁴ und R⁵ die unter Punkt 4 angegebene Bedeutung besitzen, dadurch gekennzeichnet, daß man
   die z. B. gemäß Verfahren 3 erhältlichen 4a, 5a, 8a, 8b-Tetrahydro-6H-pyrrolo[3',4':4,5]furo[3,2-b] pyridin-6,8(7H)-dion Derivate der allgemeinen Formel (Ia) 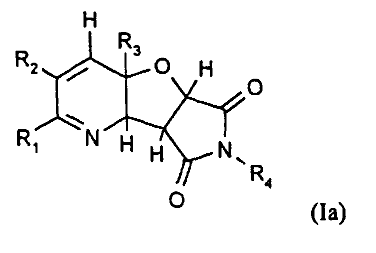 in welcher
   R¹, R², R³ und R⁴ die unter Punkt 2 angegebene Bedeutung besitzen,
   in einem ersten Reaktionsschritt in Gegenwart von geeigneten Katalysatoren zu 1,2, 3, 4, 4a, 5a, 8a, 8b-Oktahydro-6H-pyrrolo[3',4':4,5]furo[3,2-b]pyridin-6,8(7H)dion- und/oder 1, 2, 4a, 5a, 8a, 8b-Hexahydro-6H-pyrrolo[3',4':4,5]furo[3,2-b]pyridin-6,8(7H)-dion Derivaten der allgemeinen Formeln ( Id ) und ( Ie ) 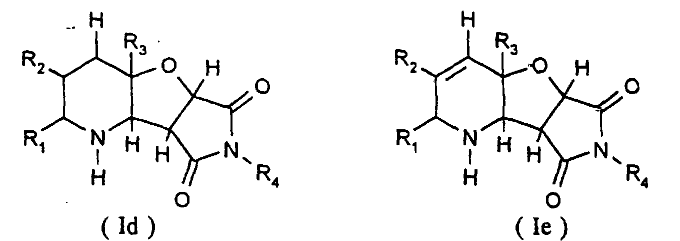 in welchen
   R¹, R², R³ und R⁴ die weiter oben angegebene Bedeutung besitzen,
   hydriert, oder
   indem man zur selektiven Darstellung der neuen Derivate der allgemeinen Formel (Ie) und deren Salze die Derivate der allgemeinen Formel (Ia) zunächst mit Methylhalogeniden der allgemeinen Formel (IV)

   Me-Hal (IV)

   in welcher
   - Hal: für Halogen, insbesondere Fluor, Chlor, Brom oder Iod steht, gegebenenfalls in Gegenwart von Verdünnungsmitteln umsetzt, danach die gebildeten N¹-Methylammoniumhalogenide der allgemeinen Formel (V) 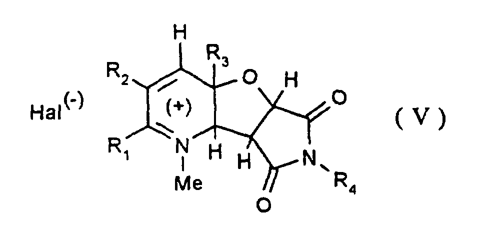
   in Gegenwart eines geeigneten Verdünnungsmittels hydriert, anschließend das resultierende N¹-Methylderivat der allgemeinen Formel (VI) 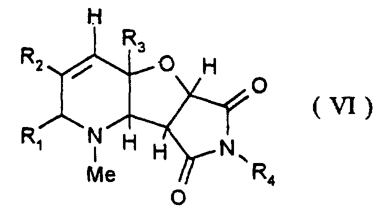 in welcher
   R¹, R², R³ und R⁴ die weiter oben angegebene Bedeutung besitzen,
   am N¹-Atom unter Ausbildung von ( Ie ) demethyliert, und nachfolgend
   in einem zweiten Reaktionsschritt die auf diese Weise erhaltenen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexa-hydro-6H-pyrrolo[3',4':4,5]furo[3,2-b]pyridin-6,8(7H)-dion Derivate der allgemeinen Formeln (Id) und (Ie) 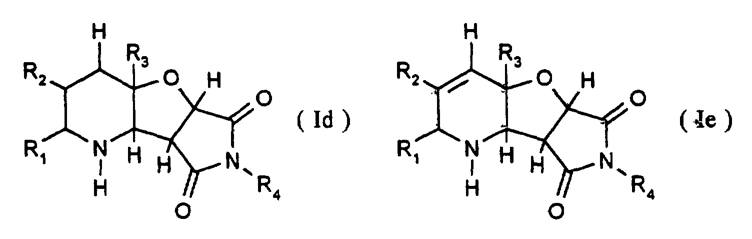 in welchen
   R¹, R², R³ und R⁴ die weiter oben angegebene Bedeutung besitzen,
   a) mit Verbindungen der allgemeinen Formel (VII)

      R⁵-E (VII)

      in welcher
      - R⁵: die weiter oben angegebene Bedeutung hat, und
      - E: für einen elektronenziehende Abgangsgruppe steht,
      gegebenenfalls in Gegenwart von Verdunnungsmitteln und gegebenenfalls in Gegenwart eines basischen Reaktionshilfsmittels umsetzt, oder
   b) mit Verbindungen der allgemeinen Formel (VIII) 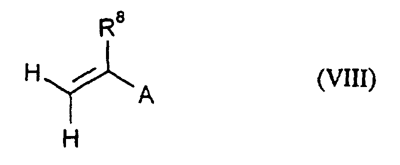 in welcher
      - A: für eine geeignete Akzeptorgruppe, wie beispielsweise Nitro, Nitril, Carbamoyl oder R¹³-O-CO- steht, und
      R⁸ und R¹³ die unter Punkt 4 angegebene Bedeutung haben, oder indem man
   c) mit einem Epoxid der allgemeinen Formel (IX) 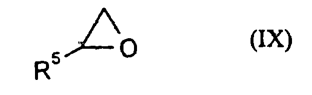 in welcher
      - R⁵: die unter Punkt 4 angegebene Bedeutung hat, gegebenenfalls in Gegenwart eines Katalysators oder in Gegenwart eines basischen Reaktionshilfsmittels, gegebenenfalls in Gegenwart von Verdünnungsmitteln umsetzt, oder
   d) mit Verbindungen der allgemeinen Formel (X) 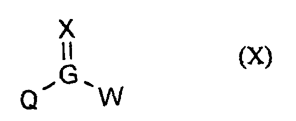 in welcher
      - G, Q und X: die unter Punkt 4 angegebene Bedeutung haben, und
      - W: für eine geeignete Abgangsgruppe, wie beispielsweise Halogen, Alkoxy, Alkylthio oder Aryloxy steht, gegebenenfalls in Gegenwart eines Katalysators, gegebenenfalls in Gegenwart eines Säurebindemittels und gegebenfalls in Gegenwart von Verdünnungsmitteln umsetzt, oder indem man
      zur Darstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) und deren Salze, in der die Gruppe 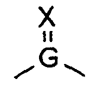 für Carboxy steht,
   e) mit einem Carbonsäureanhydrid der allgemeinen Formel (XI)

      (Q-C=O)₂O (XI)

      in welcher
      - Q: die unter Punkt 4 angegebene Bedeutung hat, gegebenenfalls in Gegenwart eines Katalysators,
      gegebenenfalls in Gegenwart von Verdünnungsmitteln umsetzt, oder
   f) mit Aminosäuederivaten der allgemeinen Formel (XII) 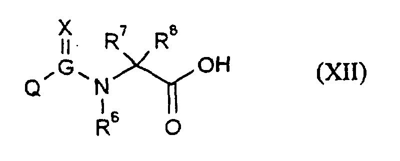 in welcher
      G, Q, X , R⁶, R⁷ und R⁸ die unter Punkt 4 angegebene Bedeutung haben, bzw. deren carboxyaktivierten Derivate,
      gegebenenfalls in Gegenwart eines Katalysators, gegebenenfalls in Gegenwart eines Säurebindemittels und gegebenenfalls in Gegenwart von Verdünnungsmitteln umsetzt, oder
   g) mit Verbindungen der allgemeinen Formeln (XIII) oder (XIV) 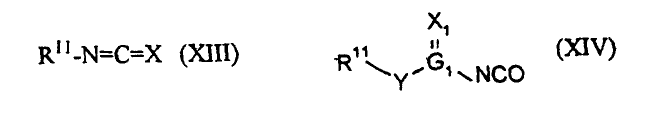 in welchen
      die Reste R¹¹, G¹, X, X¹ und Y die unter Punkt 1 angegebene Bedeutung haben,
      gegebenenfalls in Gegenwart eines Katalysators, gegebenenfalls in Gegenwart eines Säurebindemittels und gegebenfalls in Gegenwart von Verdünnungsmitteln umsetzt, oder indem man
      zur Darstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib) und ( Ic ) und deren Salze, in der die Gruppe 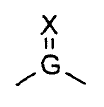 für Carboxy und Y für Sauerstoff steht,
   h) in einem dritten Reaktionsschritt mit Kohlendioxid und einem Alkalimetallcarbonat der allgemeinen Formel (XV)

      M₂CO₃ (XV)

      in welcher
      - M: für ein einwertiges Alkalimetallkation, vorzugsweise Lithium, Natrium, Kalium oder Caesium, insbesondere Kalium oder Caesium, steht,
      dann in einem vierten Reaktionsschritt die resultierenden Alkalimetallsalze von Verbindungen der allgemeinen Formeln (XVI) und (XVII) 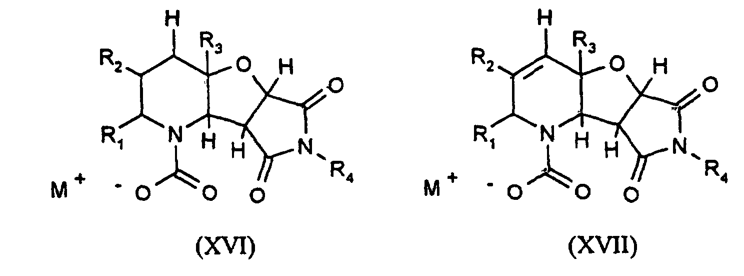
      in welchen
      die Reste R¹, R², R³ und R⁴ die unter Punkt 4 angegebene Bedeutung haben,
      - M: für ein salzartig gebundenes Metallkationenäquivalent steht,
      mit einem Alkylierungsmittel der Formel (VIIa)

      R¹¹-Hal (VIIa)

      in welcher
      - R¹¹: die unter Punkt 4 angegebene Bedeutung besitzt, und
      - Hal: für Halogen wie Fluor, Chlor, Brom oder lod steht,
      gegebenenfalls in Gegenwart eines basischen Reaktionshilfsmittels und gegebenenfalls in Gegenwart von Verdünnungsmitteln umsetzt, oder indem man
   i) 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo [3',4':4,5] furo [3,2-b]pyridin-6,8(7H)-dion Derivate der allgemeinen Formeln ( Id ) und ( Ie ) 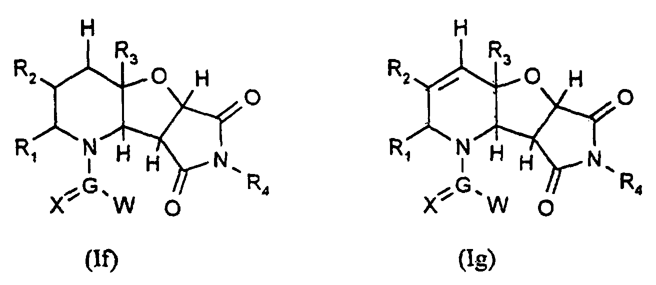 in welchen
      die Reste R¹, R², R³, R⁴, G, W und X die unter Punkt 4 angegebene Bedeutung haben,
      gegebenenfalls in Gegenwart eines Katalysators, gegebenenfalls in Gegenwart eines Säurebindemittels und gegebenenfalls in Gegenwart von Verdünnungsmitteln, mit Verbindungen der allgemeinen Formel (XVIII)

      R¹¹-Y-H (XVIII)

      in welcher
      die Reste R¹¹ und Y die weiter oben unter Punkt 4 angegebene Bedeutung haben, umsetzt.

Die erfindungsgemäßen 4a, 5a, 8a, 8b-Tetrahydro-6H-pyrrolo[3',4':4,5]furo[3,2-b]py-ridin-6,8(7H)-dion Derivate und deren Salze sind durch die allgemeine Formel ( I ) allgemein definiert.

Die erfindungsgemäßen 4a, 5a, 8a, 8b-Tetrahydro-6H-pyrrolo[3',4':4,5]furo[3,2-b]py-ridin-6,8(7H)-dion Derivate und deren Säureadditionssalze und Metallsalz-Komplexe besitzen sehr gute endoparasitizide, insbesondere anthelmintische Wirkung und können bevorzugt im Bereich der Veterinärmedizin eingesetzt werden.

Gegebenenfalls substituiertes Alkyl allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl genannt. Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines Restes in den allgemeinen Formeln bedeutet geradkettiges oder verzweigtes Alkenyl mit vor-zugsweise 1 bis 6, insbesondere 1 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-bute-nyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl genannt. Vorzugsweise seien gegebenenfalls substituiertes Ethenyl, 2-Propenyl, 2-Butenyl oder 1-Methyl-2-propenyl genannt.

Gegebenenfalls substituiertes Alkinyl allein oder als Bestandteil eines Restes in den allgemeinen Formeln bedeutet geradkettiges oder verzweigtes Alkinyl mit vor-zugsweise 1 bis 6, insbesondere 1 bis 4 Kohlenstoffatomen. Beispielhaft seien gegebenenfalls substituiertes Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Me-thyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-bu-tinyl, 1-Ethyl-3-butinyl, 2-Ethyl-3-butinyl und 1-Ethyl-1-methyl-2-propinyl genannt.

Vorzugsweise seien gegebenenfalls substituiertes Ethinyl, 2-Propinyl oder 2-Butinyl genannt.

Gegebenenfalls substituiertes Cycloalkyl allein oder als Bestandteil eines Restes in den allgemeinen Formeln bedeutet mono-, bi- und tricyclisches Cycloalkyl, vorzugsweise mit 3 bis 10, insbesondere mit 3, 5 oder 7 Kohlenstoffatomen. Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]oktyl und Adamantyl genannt.

Halogenalkyl allein oder als Bestandteil eines Restes in den allgemeinen Formeln enthält 1 bis 4, insbesondere 1 oder 2 Kohlenstoffatome und vorzugsweise 1 bis 9, insbesondere 1 bis 5 gleiche oder verschiedene Halogenatome vorzugsweise Fluor, Chlor und Brom, insbesondere Fluor und Chlor. Beispielhaft seien Trifluormethyl, Trichlormethyl, Chlordifluormethyl, Dichlorfluormethyl, Chlormethyl, Brommethyl, 1-Fluorethyl, 2-Fluorethyl, 2, 2-Difluorethyl, 2,2,2-Trifluorethyl, 2,2,2-Trichlorethyl, 2-Chlor-2,2-difluorethyl, Pentafluorethyl und Pentafluor-tert-butyl genannt.

Gegebenenfalls substituiertes Alkoxy allein oder als Bestandteil eines Restes in den allgemeinen Formeln bedeutet geradkettiges oder verzweigtes Alkoxy mit vor-zugsweise 1 bis 6, insbesondere 1 bis 4 Kohlenstoffatomen. Beispielhaft seien gegebenenfalls substituiertes Methoxy, Ethoxy, n-Propoxy, Isopropoxy, n-Butoxy, Iso-butoxy, sec-Butoxy und tert-Butoxy genannt.

Gegebenenfalls substituiertes Halogenalkoxy allein oder als Bestandteil eines Restes in den allgemeinen Formeln bedeutet geradkettiges oder verzweigtes Halogenalkoxy mit vorzugsweise 1 bis 6, insbesondere 1 bis 4 Kohlenstoffatomen. Beispielhaft seien gegebenenfalls substituiertes Difluormethoxy, Trifluormethoxy, Trichlormethoxy, Chlordifluormethoxy, 1-Fluorethoxy, 2-Fluorethoxy, 2,2-Difluorethoxy, 1,1,2,2,-Tetrafluorethoxy, 2,2,2-Trifluorethoxy und 2-Chlor-1,1,2-trifluorethoxy genannt.

Gegebenenfalls substituiertes Alkylthio allein oder als Bestandteil eines Restes in den allgemeinen Formeln bedeutet geradkettiges oder verzweigtes Alkylthio mit vor-zugsweise 1 bis 6, insbesondere 1 bis 4 Kohlenstoffatomen. Beispielhaft seien gegebenenfalls substituiertes Methylthio, Ethylthio, n-Propylthio, Isopropylthio, n-Butylthio, Isobutylthio, sec-Butylthio und tert-Butylthio genannt.

Gegebenenfalls substituiertes Halogenalkylthio allein oder als Bestandteil eines Restes in den allgemeinen Formeln bedeutet geradkettiges oder verzweigtes Halogenalkylthio mit vorzugsweise 1 bis 6, insbesondere 1 bis 4 Kohlenstoffatomen. Beispielhaft seien gegebenenfalls substituiertes Difluormethylthio, Trifluormethylthio, Trichlormethylthio, Chlordifluormethylthio, 1-Fluorethylthio, 2-Fluorethylthio, 2,2-Difluorethylthio, 1,1,2,2,-Tetrafluorethylthio, 2,2,2-Trifluorethylthio und 2-Chlor-1,1,2-trifluorethylthio genannt.

Gegebenenfalls substituiertes Alkylcarbonyl allein oder als Bestandteil eines Restes in den allgemeinen Formeln bedeutet geradkettiges oder verzweigtes Alkylcarbonyl mit vorzugsweise 1 bis 6, insbesondere 1 bis 4 Kohlenstoffatomen. Beispielhaft seien gegebenenfalls substituiertes Methylcarbonyl, Ethylcarbonyl, n-Propylcarbonyl, Isopropylcarbonyl, n-Butylcarbonyl, Isobutylcarbonyl, sec-Butylcarbonyl und tert-Butylcarbonyl genannt.

Gegebenenfalls substituiertes Cycloalkylcarbonyl allein oder als Bestandteil eines Restes in den allgemeinen Formeln bedeutet mono-, bi- und tricyclisches Cycloalkylcarbonyl, vorzugsweise mit 3 bis 10, insbesondere mit 3, 5 oder 7 Kohlenstoffatomen. Beispielhaft seien gegebenenfalls substituiertes Cyclopropylcarbonyl, Cyclobutylcarbonyl, Cyclopentylcarbonyl, Cyclohexylcarbonyl, Cycloheptylcarbonyl, Cyclooktylcarbonyl, Bicyclo[2.2.1]heptylcarbonyl, Bicyclo[2.2.2]oktylcarbonyl und Adamantylcarbonyl genannt.

Gegebenenfalls substituiertes Alkoxycarbonyl allein oder als Bestandteil eines Restes in den allgemeinen Formeln bedeutet geradkettiges oder verzweigtes Alkoxycarbonyl mit vorzugsweise 1 bis 6, insbesondere 1 bis 4 Kohlenstoffatomen. Beispielhaft seien gegebenenfalls substituiertes Methoxycarbonyl, Ethoxycarbonyl, n-Propoxycarbonyl, Isopropoxycarbonyl, n-Butoxycarbonyl, Isobutoxycarbonyl, sec-Butoxycarbonyl und tert-Butoxycarbonyl genannt.

Aryl ist beispielsweise ein ein-, zwei- oder mehrkerniger aromatischer Rest wie Phenyl, Naphthyl, Tetrahydronaphthyl, Indanyl, Fluorenyl und ähnliches, vorzugsweise Phenyl oder Naphthyl.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet vor-zugsweise gegebenenfalls im Arylteil und/oder Alkyl substituiertes Arylalkyl mit vorzugsweise 6 oder 10, insbesondere 8 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls substituiertes Hetaryl allein oder als Bestandteil eines Restes in den allgemeinen Formeln bedeutet 5- bis 7-gliedrige Ringe mit vorzugsweise 1 bis 3, insbesondere 1 oder 2 gleichen oder verschiedenen Heteroaromaten. Als Heteroatome in den Heteroaromaten stehen Sauerstoff, Schwefel oder Stickstoff. Beispielhaft und vorzugsweise seien gegebenenfalls substituiertes Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio und tert-Butylthio; Halogenalkyl mit vor-zugsweise 1 bis 4, insbesondere 1 bis 2 Kohlenstoffatomen und vorzugsweise 1 bis 5, insbesondere 1 bis 3 Halogen-atomen, wobei die Halogenatome gleich oder verschieden sind und als Halogenatome, vorzugsweise Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethylamino, n-Propylamino, Isopropylamino, Methyl-n-butylamino; Alkylcarbonylreste wie Methylcarbonyl; Alkoxycarbonyl mit vorzugsweise 2 bis 4, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogenalkylsulfinyl mit 1 bis 4, insbesondere 1 bis 2 Kohlenstoffatomen und 1 bis 5 Halogenatomen wie Trifluormethylsilfinyl; Sulfonyl (-SO₂-OH); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluor-isobutylsulfonyl; Arylsulfonyl mit vorzugsweise 6 oder 10 Aryl kohlenstoffatomen wie Phenylsulfonyl; Acyl, Aryl, Aryloxy, Hetaryl, Hetaryloxy, die ihrerseits einen der oben genannten Substituenten tragen können sowie den Formiminorest (-HC=N-O-Alkyl).

Als geeignete cyclische Aminogruppen kommen heteroaromatische oder aliphatische Ringsysteme mit einem oder mehreren Stickstoffatomen als Heteroatom in Frage, bei denen die Heterocyclen gesättigt oder ungesättigt, ein Ringsystem oder mehrere kondensierte Ringsysteme sein können, und .gegebenenfalls weitere Heteroatome wie Stickstoff, Sauerstoff und Schwefel usw. enthalten. Außerdem können cyclische Aminogruppen auch ein Spiroring oder ein verbrücktes Ringsystem bedeuten. Die Anzahl der Atome, die cyclische Aminogruppen bilden, ist nicht beschränkt, beispielsweise bestehen sie im Falle eines Einringsystems aus 3 bis 8 Atomen und im Falle eines Dreiringsystems aus 7 bis 11 Atomen.

Beispielhaft für cyclischen Aminogruppen mit gesättigten und ungesättigten monocyclischen Gruppen mit einem Stickstoffatom als Heteroatom seien 1-Azetidinyl, Pyr-rolidino, 2-Pyrrolin-1-yl, 1-Pyrrolyl, Piperidino, 1,4-Dihydropyridin-1-yl, 1,2,5,6-Tetrahydropyridin-1-yl, Homopiperidino genannt; beispielhaft für cyclischen Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-pyridazin-1-yl, 1,2-Dihydropyrimidin-1-yl, Perhydropyrimidin-1-yl und 1,4-Diazacyclo-heptan-1-yl genannt; beispielhaft für cyclischen Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino oder Dioxothiomorpholino genannt; beispielhaft für cyclischen Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclischen Aminogruppen mit spirocyclischen Gruppen sei das 2-Azaspiro[4,5]decan-2-yl genannt; beispielhaft für cyclischen Aminogruppen verbrückten heterocyclischen Gruppen sei das 2-Azabicyclo[2,2,1]heptan-7-yl genannt.

Bevorzugt sind Verbindungen der Formel ( I ) und deren Salze 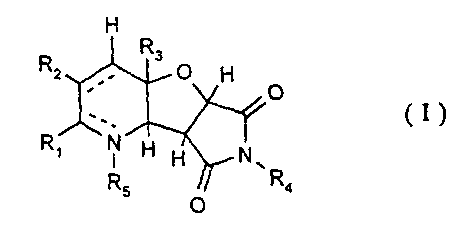 in welcher
- R¹: für Wasserstoff, geradkettiges oder verzweigtes C₁₋₄-Alkyl, insbesondere Methyl, Ethyl, Propyl, Isopropyl, Isobutyl, sec-Butyl, tert-Butyl, C₃₋₆-Cycloalkyl, insbesondere Cyclopropyl, Cyclobutyl, Cyclopentyl, Cyclohexyl, Aryl, Heteroaryl, die gegebenenfalls substituiert sind, stehen,
- R¹ und R²: gemeinsam mit den Atomen, an die sie gebunden sind, für einen 5-oder 6-gliedrigen Ring stehen, der gegebenfalls substituiert ist,
- R² und R³: unabhängig voneinander für Wasserstoff, geradkettiges oder verzweigtes verzweigtes C₁₋₄-Alkyl, insbesondere Methyl, Ethyl, Propyl, Isopropyl, Isobutyl, sec-Butyl, tert-Butyl, Halogen-C₁₋₄-alkyl, insbesondere Chlormethyl, Brommethyl, Difluormethyl, Trifluormethyl oder Trichlormethyl, Hydroxy-C₁₋₄-alkyl, insbesondere Hydroxymethyl, C₁₋₄-Alkanoyloxy-C₁₋₄-alkyl, insbesondere Acetoxymethyl, C₁₋₂-Alkoxyalkyl, insbesondere Methoxymethyl, C₁₋₂-Alkylthioalkyl, insbesondere Methylthiomethyl, C₁₋₂-Alkylsulfinyl-C₁₋₄-alkyl, insbesondere Methylsulfinylmethyl, C₁₋₂-Alkylsulfonyl-C₁₋₄-alkyl, insbesondere Methylsulfonylmethyl, Amino-C₁₋₄-alkyl, insbesondere Aminomethyl, C₁₋₆-Alkylamino-C₁₋₄-alkyl, insbesondere Methylaminomethyl, C₁₋₆-Dialkylamino-C₁₋₄-alkyl, insbesondere Dimethylaminomethyl, C₃₋₆-Cycloalkylamino-C₁₋₂-alkyl, insbesondere Morpholinomethyl, Thiomorpholinomethyl, C₃₋₆-Cycloalkyl, insbesondere Cyclopropyl, Cyclobutyl, C₁₋₄-Alkoxycarbonyl, insbesondere Methoxycarbonyl, Ethoxycarbonyl stehen,
- R⁴: für Wasserstoff, geradkettiges oder verzweigtes C₁₋₆-Alkyl, insbesondere Methyl, Ethyl, Propyl, Isopropyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, Halogen-C₁₋₆-alkyl, insbesondere Fluormethyl, Difluormethyl, Difluorchlormethyl, Trifluormethyl, Trichlormethyl, 1-Fluorethyl, Trifluorethyl, 2-Chlor-1,1,2-trifluorethyl, Hydroxy-C₁₋₆-alkyl, insbesondere Hydroxymethyl, Hydroxyethyl, 2-Hydroxypropyl, C₁₋₄-Alkanoyloxy-C₁₋₄-alkyl, insbesondere Acetoxymethyl, Acetoxyethyl, 2-Acetoxypropyl, C₁₋₂-Alkoxy-C₁₋₄-alkyl, insbesondere Methoxymethyl, Methoxyethyl, 2-Methoxypropyl, C₁₋₄-Alkoxykarbonyl-C₁₋₄-alkyl, insbesondere Methoxycarbonylmethyl, Ethoxycarbonylmethyl, tert-Butoxycarbonylmethyl, Methoxycarbonylethyl, Ethoxycarbonylethyl, Amino-C₁₋₆-alkyl, insbesondere Aminomethyl, Aminoethyl, 2-Aminopropyl, C₁₋₆-Alkylamino-C₁₋₆-alkyl, insbesondere Methylaminomethyl, Methylaminoethyl, C₁₋₆-Dialkylamino-C₁₋₆-alkyl, insbesondere Dimethylaminomethyl, Dimethylaminoethyl, C₁₋₆-Trialkylammonium-C₁₋₆-alkylhalogenid, insbesondere Trimethylaminomethylen-iodid, Trimethylaminoethylen-iodid, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methylpiperazino)-ethyl, C₂₋₆-Alkenyl, insbesondere Vinyl, 2-Propenyl, 2-Butenyl, 3-Butenyl, 1-Methyl-2-propenyl, 2-Methyl-2-propenyl, 2-Pentenyl, 1-Methyl-2-butenyl, 2-Methyl-2-butenyl, 3-Methyl-2-butenyl, 1-Methyl-3-butenyl, 1,1-Dimethyl-2-propenyl, 1,2-Dimethyl-2-propenyl, 1-Ethyl-2-propenyl, 2-Hexenyl, 1,1-Dimethyl-2-butenyl, 1,2-Dimethyl-2-butenyl, 1-Ethyl-2-butenyl, C₂₋₆-Alkinyl, insbesondere Ethinyl, 2-Propinyl, 2-Butinyl, 3-butinyl, 1-Methyl-2-propinyl, 2-Pentinyl, 1-Methyl-3-butinyl, 2-Methyl-3-butinyl, 1,1-Dimethyl-2-propinyl, 1-Ethyl-2-propinyl, C₃₋₆-Cycloalkyl insbesondere Cyclopropyl, Cyclobutyl, Cyclopentyl, Cyclohexyl, C₃₋₆-Cycloalkyl-C₁₋₂-alkyl, insbesondere Cyclopropylmethyl, Cyclobutylmethyl, Cyclopentylmethyl, Cyclohexylmethyl, Aryl, insbesondere Phenyl, Aryl-C₁₋₂-alkyl, insbesondere Phenylmethyl, 1-Phenylethyl, 2-Phenylethyl, Heteroaryl, insbesondere Pyridyl oder Thiazolyl, N-Morpholinyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
- R⁵: für Wasserstoff, geradkettiges oder verzweigtes C₁₋₆-Alkyl, insbesondere Methyl, Ethyl, Propyl, Isopropyl, Isobutyl, sec-Butyl, tert-Butyl, Pentyl, 1-methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, Halogen-C₁₋₆-alkyl, insbesondere Fluormethyl, Difluormethyl, Difluorchlormethyl, Trifluormethyl, Trichlormethyl, 1-Fluorethyl, Trifluorethyl, 2-Chlor-1,1,2-trifluorethyl, Hydroxy-C₁₋₆-alkyl, insbesondere Hydroxymethyl, Hydroxyethyl, 2-Hydroxypropyl, C₁₋₄-Alkanoyloxy-C₁₋₄-alkyl, insbesondere Acetoxymethyl, Acetoxyethyl, 2-Acetoxypropyl, C₁₋₂-Alkoxy-C₁₋₄-alkyl, insbesondere Methoxymethyl, Methoxyethyl, 2-Methoxypropyl, Amino-C₁₋₆-alkyl, insbesondere Aminomethyl, Aminoethyl, 2-Aminopropyl, C₁₋₆-Alkylamino-C₁₋₆-alkyl, insbesondere Methylaminomethyl, Methylaminoethyl, C₁₋₆-Dialkylamino-C₁₋₆-alkyl, insbesondere Dimethylaminomethyl, Dimethylaminoethyl, C₁₋₆-Trialkylammonium-C₁₋₆-alkyl-halogenid, insbesondere Trimethylammoniummethylen- oder Trimethylammoniumethylen-iodid, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methylpiperazino)-ethyl, Nitro-C₁₋₄-alkyl, insbesondere Nitro-methyl, 2-Nitroethyl, 2-Nitropropyl, Cyano-C₁₋₄-alkyl, insbesondere Cyanomethyl, 2-Cyanoethyl, 2-Cyanopropyl, C₁₋₄-Alkoxycarbonyl-C₁₋₄-alkyl, insbesondere Methoxycarbonylmethyl, 2-Methoxycarbonylethyl, 2-Ethoxycarbonylethyl, Carbamoyl-C₁₋₄-alkyl, insbesondere 2-Carbamoylethyl, Carboxyl-C₁₋₄-alkyl, insbesondere 2-Carboxylethyl, C₂₋₆-Alkenyl, insbesondere Vinyl, 2-Propenyl, 2-Butenyl, 3-Butenyl, 1-Methyl-2-propenyl, 2-Methyl-2-propenyl, 2-Pentenyl, 1-Methyl-2-butenyl, 2-Methyl-2-butenyl, 3-Methyl-2-butenyt, 1-Methyl-3-butenyl, 1,1-Dimethyl-2-propenyl, 1,2-Dimethyl-2-propenyl, 1-Ethyl-2-propenyl, 2-Hexenyl, 1,1-Dimethyl-2-butenyl, 1,2-Dimethyl-2-butenyl, 1-Ethyl-2-butenyl, C₂₋₆-Alkinyl, insbesondere Ethinyl, 2-Propinyl, 2-Butinyl, 3-Butinyl, 1-Methyl-2-propinyl, 2-Pentinyl, 1-Methyl-3-butinyl, 2-Methyl-3-butinyl, 1,1-Dimethyl-2-propinyl, 1-Ethyl-2-propinyl, C₃₋₆-Cycloalkyl, insbesondere Cyclopropyl, Cyclobutyl, Cyclopentyl, Cyclohexyl, C₃₋₆-Cycloalkyl-C₁₋₂-alkyl, insbesondere Cyclopropylmethyl, Cyclobutylmethyl, Cyclopentylmethyl, Cyclohexylmethyl, C₁₋₄-Alkoxydicarbonyl, insbesondere Methoxydicarbonyl oder Ethoxydicarbonyl, Aryl-C₁₋₂-alkyl, insbesondere Phenylmethyl, Heteroaryl, insbesondere Pyridyl, Pyrimidyl, Pyrazinyl oder Thiazolyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methykarbonyl, C₁₋₄-Alkoxycarbonyl, insbesondere Methoxycarbonyl, substituiert sein können, Methoxycarbonyl, substituiert sein können oder für einen Rest aus der Gruppe G¹, G², G³ und G⁴ 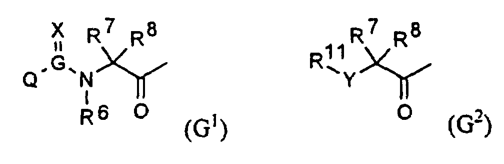 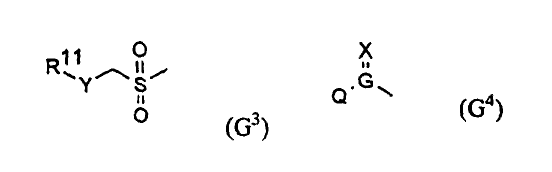 worin
- R⁶: für Wasserstoff, geradkettiges oder verzweigtes C₁₋₆-Alkyl, insbesondere Methyl, Ethyl, Propyl, Isopropyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dime thylpropyl, 1-Ethylpropyl, Hexyl, 1-Methylpentyl, C₃₋₆-Cycloalkyl, insbesondere Cyclopropyl, Cyclobutyl, Cyclopentyl, Cyclohexyl, Aryl-C₁₋₂-alkyl, insbesondere für Phenylmethyl, Heteroaryl-C₁₋₂-alkyl, insbesondere für Pyridylmethyl und Thiazolylmethyl, die gegebenenfalls substituiert sind, stehen,
- R⁶ und R⁷: gemeinsam mit den Atomen, an die sie gebunden sind, für einen 5-oder 6-gliedrigen Ring stehen, der gegebenenfalls Schwefel, unterbrochen sein kann und gegebenenfalls durch Hydroxy substituiert ist, stehen,
- R⁷: für Wasserstoff, geradkettiges oder verzweigtes Alkyl mit bis zu 6 Kohlenstoffatomen, insbesondere Methyl, Ethyl, Propyl, Isopropyl, sec-Butyl, tert-Butyl, Hydroxy-C₁₋₂-alkyl, insbesondere Hydroxymethyl, 1-Hydroxyethyl, C₁₋₄-Alkanoyloxy-C₁₋₄-alkyl, insbesondere Acetoxymethyl, 1-Acetoxyethyl, C₁₋₄-Alkoxy-C₁₋₄-alkyl, insbesondere Methoxymethyl, 1-Methoxyethyl, Aryl-C₁₋₄-alkoxy-C₁₋₄-alkyl, insbesondere Benzyloxymethyl, 1-Benzyloxymethyl, Mercapto-C₁₋₄-alkyl, insbesondere Mercaptomethyl, C₁₋₄-Alkyithio-C₁₋₄-alkyl, insbesondere Methylsulfinylmethyl, C₁₋₄-Alkylsulfonyl-C₁₋₄-alkyl, insbesondere Methylsulfonylethyl, Carboxy-C₁₋₄-alkyl, insbesondere Carboxymethyl, Carboxyethyl, C₁₋₄-Alkoxycarbonyl-C₁₋₄-alkyl, insbesondere Methoxycarbonylmethyl, Ethoxycarbonylmethyl, Aryl-C₁₋₄-alkoxycarbonyl-C₁₋₄-alkyl, insbesondere Benzyloxycarbonylmethyl, Carbamoyl-C₁₋₄-alkyl, insbesondere Carbamoylmethyl, Carbamoylethyl, Amino-C₁₋₄-alkyl, insbesondere Aminopropyl, Aminobutyl, C₁₋₄-Alkylamino-C₁₋₄-alkyl, insbesondere Methylaminopropyl, Methylaminobutyl, C₁₋₄-Dialkylamino-C₁₋₄-alkyl, insbesondere Dimethylaminopropyl, Dimethyl-aminobutyl, Guanido-C₁₋₄-alkyl, insbesondere Guanidopropyl, C₁₋₄-Alkoxycarbonylamino-C₁₋₄-alkyl, insbesondere tert-Butylcarbonylaminopropyl, tert-Butylcarbonylaminobutyl, Alkenyl mit bis zu 6 Kohlenstoffatomen, insbesondere Vinyl, 2-Propenyl, 2-Butenyl, 1-Methyl-2-propenyl, C₃₋₆-Cycloalkyl-C₁₋₂-alkyl, insbesondere Cyclopropylmethyl, Cyclobutylmethyl, Cyclohexylmethyl, Hetaryl-C₁₋₂-alkyl, insbesondere Benzo[b]thien-1-yl-methyl, Benzo[b]thien-3-yl, Pyrid-2-yl-methyl, Pyrid-3-yl-methyl, Pyrid-4-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methylimidazol-4-yl-methyl, Aryl-C₁₋₂-alkyl, insbesondere Benzyl steht, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Halo-genalkyl, insbesondere Trifluormethylethyl, Difluormethyl oder Trichlormethyl, C₁₋₄-Alkoxy, insbesondere Methoxy, Ethoxy oder tert-Butyloxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₂-Alkylendioxy, insbesondere Methylendioxy oder Ethylendioxy, Nitro, Amino, C₁₋₄-Alkylamino, insbesondere Methylamino, C₁₋₄-Di-alkylamino, insbesondere Dimethylamino, C₃₋₆-Cycloalkylamino, insbesondere Pyrrolidino, Piperidino, C₃₋₆-Cycloalkylthioamino, insbesondere Thiomorpholino und Dioxothiomorpholino, C₃₋₆-Cycloalkyldiamino, insbesondere N-Methylpiperazino, substituiert sein können, und
- R⁸: für Wasserstoff oder Methyl steht, 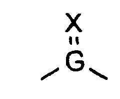 für Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ steht,
- R⁹: für Wasserstoff, Hydroxy, C₁₋₄-Alkoxy, insbesondere Methoxy, Ethoxy, sec-Butyloxy, C₁₋₄-Alkylcarbonyl, insbesondere Methylcarbonyl, Ethylcarbonyl, C₁₋₄-Halogenalkylcarbonyl, insbesondere Trifluormethylcarbonyl, Trichlormethylcarbonyl, C₁₋₄-Alkylsulfonyl, insbesondere Methylsulfonyl, Ethylsulfonyl, Propylsulfonyl, Nitro oder Cyan steht, und
- R¹⁰: für Wasserstoff oder C₁₋₄-Alkyl, insbesondere Methyl oder Ethyl steht, und
- Q: für geradkettiges oder verzweigtes C₁₋₄-Alkyl, insbesondere Methyl, Ethyl, Propyl, Isopropyl, sec-Butyl, tert-Butyl, C₁₋₄-Halogenalkyl, insbesondere Trifluormethyl, Trichlormethyl, Chlordifluormethyl, Dichlorfluormethyl, 1-Fluorethyl, Chlormethyl, Brommethyl, 1-Fluorethyl, 2,2,2-Trifluorethyl, 2,2,2-Trichlorethyl, C₂₋₆-Alkenyl, insbesondere Vinyl, 2-Propenyl, 1-Methyl-2-propenyl und 2-Butenyl, C₂₋₆-Halogenalkenyl, insbesondere Difluorvinyl, Dichlorvinyl, 2-Chlor-2-propenyl, 2,3,3-Trifluor-2-propenyl, 2,3,3-Trichlor-2-propenyl, 4,4-Difluor-3-butenyl, C₃₋₆-Cyclo-alkyl, insbesondere Cyclopropyl, Cyclobutyl, Cyclopentyl und Cyclohexyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Halogenalkyl, insbesondere Trifluormethyl, Difluormethyl oder Trichlormethyl, C₁₋₄-Alkoxy, insbesondere Methoxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, Nitro, Amino, C₁₋₄-Alkylamino, insbesondere Methylamino, C₁₋₄-Di-alkylamino, insbesondere Dimethylamino, substituiert sein können,
oder gegebenefalls für einen Rest aus der Gruppe G⁵ und G⁶ 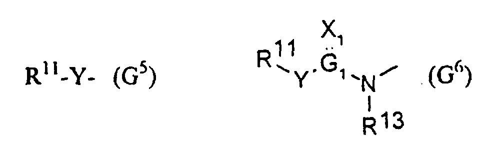 steht,
worin 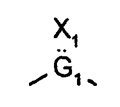 Carboxy, Thiocarboxy oder Sulfonyl bedeuten kann,
- Y: für Sauerstoff, Schwefel oder -NR¹² steht,
- R¹¹: für den Fall, daß Y für Stickstoff steht, eine über ein Stickstoffatom verknüpfte cyclische Aminogruppe, insbesondere 1-Azetidinyl, Pyrrolidino, 2-Pyrrolin-1-yl, 1-Pyrrolyl, Piperidino, 1,4-Dihydropyridin-1-yl, 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-pyridazin-1-yl, 1,2-Dihydropyrimidin-1-yl, Perhydropyrimidin-1-yl, 1,4-Diazacyclo-heptan-1-yl, Thiazolidin-3-yl, Isothiazolin-2-yl, Morpholino, Thiomorpholino, Dioxothiomorpholino, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können,
- R¹¹ und R¹²: unabhängig voneinander für Wasserstoff, geradkettiges oder verzweigtes C₁₋₄-Alkyl, insbesondere Methyl, Ethyl, Propyl, Isopropyl, sec-Butyl, tert-Butyl, Carboxyl-C₁₋₄-alkyl, insbesondere Carboxylmethyl, C₂₋₄-Alkenyl, insbesondere Vinyl, 2-Propenyl, l-Methyl-2-propenyl und 2-Butenyl, C₂₋₄-Alkinyl, insbesondere Ethinyl, 2-Propinyl und 2-Butinyl, C₃₋₆-Cycloalkyl, insbesondere Cyclopropyl, Cyclobutyl, Cyclopentyl und Cyclohexyl, C₃₋₆-Cycloalkyl-C₁₋₂-alkyl, insbesondere Cyclopropylmethyl, Cyclobutylmethyl, Cyclopentylmethyl und Cyclohexylmethyl, Hetaryl, insbesondere Pyridyl und Thiazolyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, stehen, oder
- R¹¹ und R¹²: gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls durch C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,
oder einen Rest aus den Gruppen G⁷, G⁸, G⁹, G¹⁰ und G¹¹ 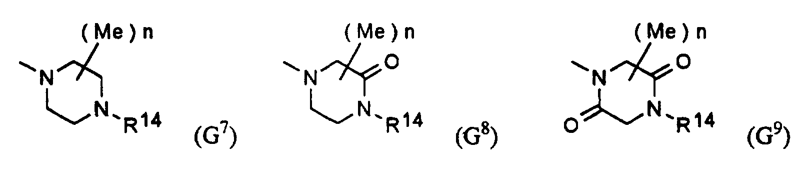 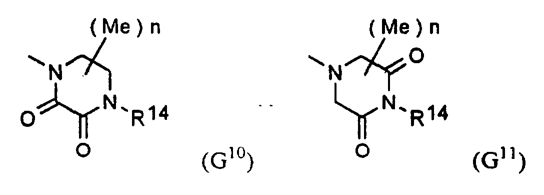 worin
n die Zahlen 0, 1, 2, 3 oder 4 bedeuten kann,
- R¹³: für Wasserstoff oder C₁₋₄-Alkyl, insbesondere Methyl, Ethyl, Propyl, Isopropyl, sec-Butyl, tert-Butyl, steht, und
- R¹⁴: für Wasserstoff, geradkettiges oder verzweigtes C₁₋₆-Alkyl, insbesondere Methyl, Ethyl, Propyl, Isopropyl, sec-Butyl, tert-Butyl, C₂₋₆-Alkenyl, insbesondere Vinyl, 2-Propenyl, 1-Methyl-2-propenyl und 2-Butenyl, C₂₋₆-Alkinyl, insbesondere Ethinyl, 2-Propinyl und 2-Butinyl, C₃₋₆-Cycloalkyl, insbesondere Cyclopropyl, Cyclobutyl, Cyclopentyl und Cyclohexyl, C₃₋₆-Cycloalkyl-C₁₋₂-alkyl, insbesondere Cyclopropylmethyl, Cyclobutylmethyl, Cyclopentylmethyl und Cyclohexylmethyl, C₁₋₄-Alkylcarbonyl, insbesondere Methylcarbonyl, Ethylcarbonyl, n-Propylcarbonyl, Isopropylcarbonyl, n-Butylcarbonyl, Isobutylcarbonyl, sec-Butylcarbonyl, tert-Butylcarbonyl, C₃₋₆-Cycloalkylcarbonyl, insbesondere Cyclopropylcarbonyl, Cyclobutylcarbonyl, Cyclopentylcarbonyl und Cyclohexylcarbonyl, C₁₋₄-Alkoxycarbonyl, insbesondere Methoxycarbonyl, Ethoxycarbonyl, n-Propoxycarbonyl, Isopropoxycarbonyl, n-Butoxycarbonyl, Isobutoxycarbonyl, sec-Butoxycarbonyl, tert-Butoxycarbonyl, Hydoxy-C₁₋₄-alkyl, insbesondere Hydroxymethyl, Hydroxyethyl, Hydroxyethylsulfonylethyl, C₁₋₄-Alkylamino, insbesondere Methylamino, Ethylamino, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Dimethylaminopropyl, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methyl-piperazino)-ethyl, C₃₋₆-Cycloalkylaminocarbonyl-C₁₋₂-alkyl, insbesondere N-Morpholinocarbonylmethyl, Cyan, Aryl, insbesondere Phenyl, Aryl-C₁₋₂-alkyl, insbesondere Phenylmethyl, Hetaryl, insbesondere Pyridyl oder Thiazolyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
mit der Maßgabe für die Verbindungen der allgemeinen Formel ( Ia ) für den Fall, daß
- R¹: für Wasserstoff steht,
- R² und R³: für Methyl stehen,
- R⁴: für andere Reste als Methyl, n-Butyl, Phenyl, 2-, 3- oder 4-Methylphenyl, 2-, 3- oder 4-Chlor-phenyl, 2-, 3- oder 4-Fluor-phenyl, 2-Chlor, 4-fluorphenyl, 2-Fluor, 4-chlor-phenyl, 3-Chlor, 4-fluorphenyl, 2-, 3- oder 4-Nitrophenyl, 4-Methoxy-phenyl steht,
sowie deren optische Isomere und Racemate.

Besonders bevorzugt sind Verbindungen der Formel ( I ) und deren Salze 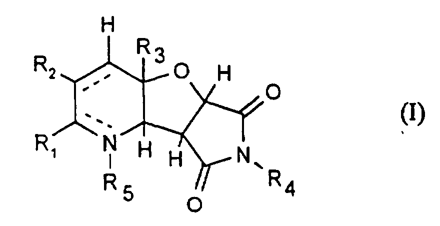 in welcher
- R¹: für Wasserstoff, geradkettiges oder verzweigtes C₁₋₄-Alkyl, insbesondere Methyl, Ethyl, Propyl, Isopropyl, Isobutyl, sec-Butyl, C₃₋₆-Cycloalkyl, insbesondere Cyclopropyl, stehen,
- R²: für Wasserstoff, geradkettiges oder verzweigtes verzweigtes C₁₋₄-Alkyl, insbesondere Methyl, Ethyl, Propyl, Isopropyl, lsobutyl, sec-Butyl, tert-Butyl, Halogen-C₁₋₄-alkyl, insbesondere Chlormethyl, Brommethyl, Hydroxy-C₁₋₄-alkyl, insbesondere Hydroxymethyl, C₁₋₄-Alkanoyloxy-C₁₋₄-alkyl, insbesondere Acetoxymethyl, C₁₋₂-Alkoxyalkyl, insbesondere Methoxymethyl, C₁₋₂-Alkylthioalkyl, insbesondere Methylthiomethyl, C₁₋₂-Alkylsulfinyl-C₁₋₄-alkyl, insbesondere Methylsulfinylmethyl, C₁₋₂-Alkylsulfonyl-C₁₋₄-alkyl, insbesondere Methylsulfonylmethyl, C₁₋₆-Alkylamino-C₁₋₄-alkyl, insbesondere Methylaminomethyl, C₁₋₆-Dialkylamino-C₁₋₄-alkyl, insbesondere Dimethylaminomethyl, C₃₋₆-Cycloalkylamin-C₁₋₂-alkyl, insbesondere Morpholinomethyl, Thiomorpholinomethyl, C₁₋₄-Alkoxycarbonyl, insbesondere Methoxycarbonyl, Ethoxycarbonyl steht,
- R³: für Wasserstoff, geradkettiges oder verzweigtes verzweigtes C₁₋₄-Alkyl, insbesondere Methyl, Ethyl, Propyl, Isopropyl, Isobutyl, sec-Butyl, tert-Butyl, steht,
- R⁴: für geradkettiges oder verzweigtes C₁₋₆-Alkyl, insbesondere Methyl, Ethyl, Propyl, Isopropyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, Halogen-C₁₋₆-alkyl, insbesondere Fluormethyl, Difluormethyl, Fluorethyl, C₁₋₄-Alkoxykarbonyl-C₁₋₄-alkyl, insbesondere Methoxycarbonylmethyl, Ethoxycarbonylmethyl, tert-Butoxycarbonylmethyl, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, C₂₋₆-Alkenyl, insbesondere 2-Propenyl, 2-Butenyl, C₃₋₆-Cycloalkyl insbesondere Cyclopropyl, Cyclobutyl, Cyclopentyl, Cyclohexyl, C₃₋₆-Cycloalkyl-C₁₋₂-alkyl, insbesondere Cyclopropylmethyl, Cyclobutylmethyl, Aryl-C₁₋₂-alkyl, insbesondere Phenylmethyl, 1-Phenylethyl, 2-Phenylethyl, Heteroaryl, insbesondere Pyridyl oder Thiazolyl, N-Morpholinyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
- R⁵: für Wasserstoff, geradkettiges oder verzweigtes C₁₋₆-Alkyl, insbesondere Methyl, Ethyl, Propyl, Isopropyl, Isobutyl, sec-Butyl, C₁₋₄-Alkanoyloxy-C₁₋₄-alkyl, insbesondere Methoxycarbonylmethyl, Ethoxycarbonylmethyl, Methoxycarbonylethyl, Ethoxycarbonylethyl, tert-Butoxycarbonylmethyl, Amino-C₁₋₆-alkyl, insbesondere Aminomethyl, Aminoethyl, C₁₋₆-Alkylamino-C₁₋₆-alkyl, insbesondere Methylaminomethyl, Methylaminoethyl, C₁₋₆-Dialkylamino-C₁₋₆-alkyl, insbesondere Dimethylaminomethyl, Dimethylaminoethyl, C₁₋₆-Trialkylammonium-C₁₋₆-alkyl-halogenid, insbesondere Trimethylammonummethylen- oder Trimethylammoniumethyleniodid, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methyl-piperazino)-ethyl, Nitro-C₁₋₄-alkyl, 2-Nitroethyl, Cyano-C₁₋₄-alkyl, insbesondere 2-Cyanoethyl, C₁₋₄-Alkoxycarbonyl-C₁₋₄-alkyl, insbesondere 2-Methoxycarbonylethyl, C₂₋₆-Alkenyl, insbesondere 2-Propenyl, C₂₋₆-Alkinyl, insbesondere 2-Propinyl, C₁₋₄-Alkoxydicarbonyl, insbesondere Methoxydicarbonyl oder Ethoxydicarbonyl, Heteroaryl, insbesondere Pyridyl, Pyrimidyl, Pyrazinyl oder Thiazolyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, Methoxycarbonyl, substituiert sein können
. oder für einen Rest aus der Gruppe G¹, G², G³ und G⁴ 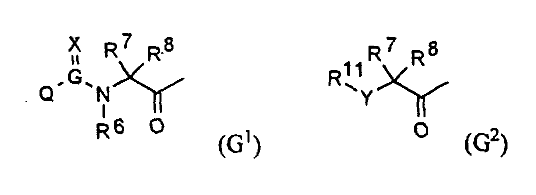 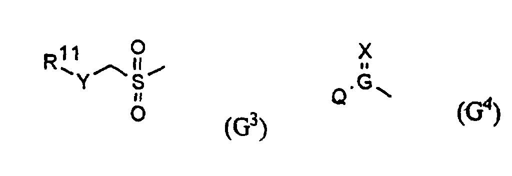 worin
- R⁶: für Wasserstoff, geradkettiges oder verzweigtes C₁₋₆-Alkyl, insbesondere Methyl, Ethyl, C₃₋₆-Cycloalkyl, insbesondere Cyclopropyl, stehen,
- R⁶ und R⁷: gemeinsam mit den Atomen, an die sie gebunden sind, für einen 5-oder 6-gliedrigen Ring stehen, der gegebenenfalls Schwefel, unterbrochen sein kann und gegebenenfalls durch Hydroxy substituiert ist, stehen,
- R⁷: für Wasserstoff, geradkettiges oder verzweigtes Alkyl mit bis zu 6 Kohlenstoffatomen, insbesondere Methyl, Ethyl, Propyl, Isopropyl, sec-Butyl,
- R⁸: für Wasserstoff oder Methyl steht, 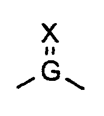 für Carboxy, -C=CH-NO₂, -C=CH-CN, -C=N-R⁹, Sulfonyl, steht,
- R⁹: für C₁₋₄-Halogenalkylcarbonyl, insbesondere Trifluormethylcarbonyl, Trichlormethylcarbonyl, C₁₋₄-Alkylsulfonyl, insbesondere Methylsulfonyl, Ethylsulfonyl, Nitro oder Cyan steht, und
- Q: für einen Rest aus der Gruppe G⁵ und G⁶ 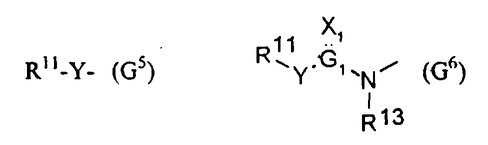 steht,
worin 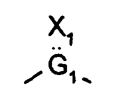 Carboxy oder Sulfonyl bedeuten kann,
- Y: für Sauerstoff oder -NR¹² steht,
- R¹¹: für den Fall, daß Y für Stickstoff steht, eine über ein Stickstoffatom verknüpfte cyclische Aminogruppe, insbesondere Pyrrolidino, 2-Pyrrolin-1-yl, 1-Pyrrolyl, Piperidino, 1,4-Dihydropyridin-1-yl, 1-Piperazinyl, 1-Homopiperazinyl, Morpholino, Thiomorpholino, Dioxothiomorpholino, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-MonoalkylaminoC₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können,
- R¹¹ und R¹²: unabhängig voneinander für Wasserstoff, geradkettiges oder verzweigtes C₁₋₄-Alkyl, insbesondere Methyl, Ethyl, Propyl, Isopropyl, sec-Butyl, Carboxyl-C₁₋₄-alkyl, insbesondere Carboxylmethyl, C₂₋₄-Alkenyl, insbesondere Vinyl, 2-Propenyl, 1-Methyl-2-propenyl C₂₋₄-Alkinyl, insbesondere Ethinyl, 2-Propinyl, C₃₋₆-Cycloalkyl, insbesondere Cyclopropyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-DialkylaminoC₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, stehen, oder
- R¹¹ und R¹²: gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls durch C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,
oder einen Rest aus den Gruppen G⁷, G⁸, G⁹, G¹⁰ und G¹¹ 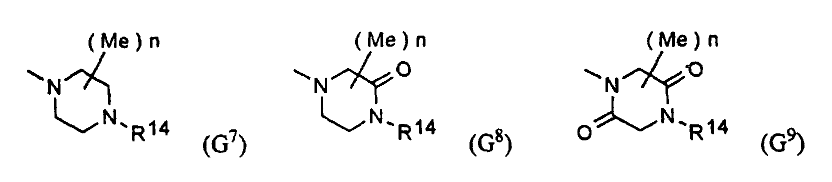 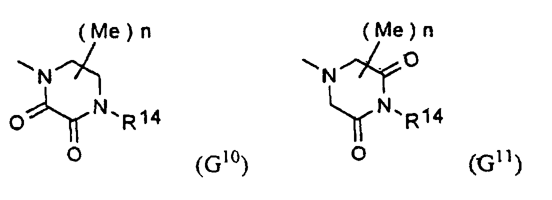 worin
- n: die Zahlen 0, 1, 2 oder 3 bedeuten kann,
- R¹³: für C₁₋₄-Alkyl, insbesondere Methyl oder Ethyl, steht, und
- R¹⁴: für Wasserstoff, geradkettiges oder verzweigtes C₁₋₆-Alkyl, insbesondere Methyl, Ethyl, C₂₋₆-Alkenyl, insbesondere Vinyl, 2-Propenyl, C₂₋₆-Alkinyl, insbesondere 2-Propinyl, C₃₋₆-Cycloalkyl, insbesondere Cyclopropyl, C₁₋₄-Alkoxycarbonyl, insbesondere Methoxycarbonyl, Ethoxycarbonyl, tert-Butoxycarbonyl, Hydoxy-C₁₋₄-alkyl, insbesondere Hydroxymethyl, Hydroxyethyl, Hydroxyethylsulfonylethyl, C₁₋₄-Alkylamino, insbesondere Methylamino, Ethylamino, C₁₋₄-Dialkylamino-C₁₋₄-alkyl, insbesondere Dimethylaminomethyl, Dimethylaminoethyl, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methyl-piperazino)-ethyl, C₃₋₆-Cycloalkylaminocarbonyl-C₁₋₂-alkyl, insbesondere N-Morpholinocarbonylmethyl, Cyan, Aryl, insbesondere Phenyl, Aryl-C₁₋₂-alkyl, insbesondere Phenylmethyl, Hetaryl, insbesondere Pyridyl oder Thiazolyl, Heteroaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
mit der Maßgabe für die Verbindungen der allgemeinen Formel ( Ia ) für den Fall, daß
- R¹: für Wasserstoff steht,
- R² und R³: ür Methyl stehen,
- R⁴: für andere Reste als Methyl oder n-Butyl steht,
sowie deren optische Isomere und Racemate.

Ganz besonders bevorzugt sind Verbindungen der Formel ( I ) und deren Salze 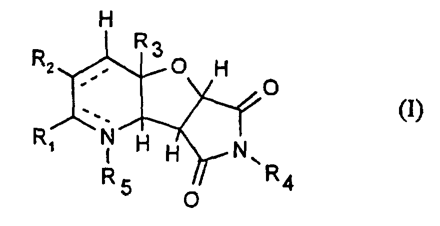 in welcher
- R¹: für Wasserstoff steht,
- R²: geradkettiges oder verzweigtes verzweigtes C₁₋₄-Alkyl, insbesondere Methyl, Ethyl steht,
- R³: für Methyl steht,
- R⁴: für geradkettiges oder verzweigtes C₁₋₆-Alkyl, insbesondere Methyl, Ethyl, Propyl, Isopropyl, Isobutyl, sec-Butyl, n-Butyl, C₃₋₆-Cycloalkyl, nsbesondere Cyclopropyl, Cyclobutyl, Cyclopentyl, C₃₋₆-Cycloalkyl-C₁₋₂-alkyl, insbesondere Cyclopropylmethyl, Phenyl-C₁₋₂-alkyl, insbesondere Phenylmethyl, 2-Phenylethyl, Phenyl, N-Morpholinyl, Hetero-C₁₋₂-alkyl, insbesondere 2-Chlor-pyrid-5-yl-methyl, Morpholinylethyl und Chlorthiazol-5-yl-methyl, stehen, wobei die Phenyl oder Hetarylreste durch Halogen, Methyl, Halogenmethyl substituiert sein können, Phenyl, Phenoxy, C₁₋₄-Alkylphenyl, C₁₋₄-Halogenalkylphenoxy, C₁₋₄-Alkylphenoxy,
- R⁵: für Wasserstoff, geradkettiges oder verzweigtes C₁₋₆-Alkyl, insbesondere Methyl, Ethyl, Cyano-C₁₋₄-alkyl, insbesondere 2-Cyanoethyl, C₁₋₄-Alkanoyloxy-C₁₋₄-alkyl, insbesondere Methoxycarbonylmethyl, C₂₋₆-Alkenyl, insbesondere 2-Propenyl, C₂₋₆-Alkinyl, insbesondere 2-Propinyl, C₁₋₄-Alkoxydicarbonyl, insbesondere Methoxydicarbonyl oder Ethoxydicarbonyl, Heteroaryl-C₁₋₂-alkyl, insbesondere 5-Chlorpyrid-2-yl-methyl und Chlor-thiazol-5-yl-methyl, stehen,
oder für einen Rest aus der Gruppe G¹, G², G³ und G⁴ 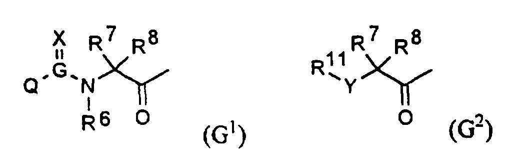 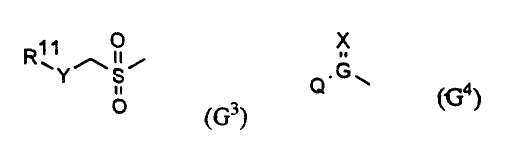 worin
- R⁶: für Wasserstoff oder Methyl stehen,
- R⁷: für Wasserstoff, geradkettiges oder verzweigtes Alkyl mit bis zu -6 Kohlenstoffatomen, insbesondere Methyl oder Ethyl, stehen,
- R⁸: für Wasserstoff, 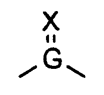 für Carboxy oder Sulfonyl steht,
- Q: für einen Rest aus der Gruppe G⁵ und G⁶ 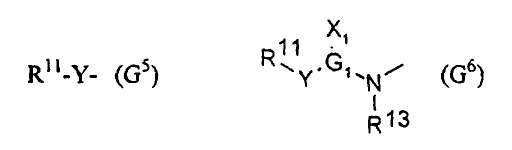 steht,
worin 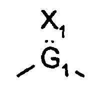 Carboxy oder Sulfonyl bedeuten kann,
- Y: für Sauerstoff oder -NR¹² steht,
- R¹¹: für den Fall, daß Y für Stickstoff steht, eine über ein Stickstoffatom verknüpfte cyclische Aminogruppe, insbesondere Pyrrolidino, 1-Pyrrolyl, Piperidino, Morpholino, Thiomorpholino, Dioxothiomorpholino, bedeutet,
- R¹¹ und R¹²: unabhängig für geradkettiges oder verzweigtes C₁₋₄-Alkyl, ins-besondere Methyl, Ethyl, Propyl, Isopropyl, sec-Butyl, C₂₋₄-Alkenyl, insbesondere Vinyl, 2-Propenyl, 1-Methyl-2-propenyl, C₂₋₄-Alkinyl, insbesondere Ethinyl, 2-Propinyl, C₃₋₆-Cycloalkyl, insbesondere Cyclopropyl, Heteroaryl-C₁₋₂-alkyl, insbesondere 5-Chlor-pyrid-2-yl-methyl und Chlorthiazol-5-yl-methyl, stehen, oder
- R¹¹ und R¹²: gemeinsam mit dem angrenzenden N-Atom für ein carbocyclisches 5-, 6- oder 7-gliedriges Ringsystem, das gegebenenfalls auch durch Sauerstoff, Schwefel, Sulfonyl, Carbonyl, -N=, -NR¹⁴- oder durch quaternisierten Stickstoff unterbrochen sein kann und gebenenfalls durch C₁₋₄-Alkyl, insbesondere Methyl, thyl, Hydoxy-C₁₋₄-Alkyl, insbesondere Hydroxymethyl, Amino-C₁₋₄-Dialkylamino-C₁₋₄-alkyl, insbesondere Dimethylaminomethyl, Hydroxy, C₁₋₄-Alkoxycarbonyl, insbesondere Methoxycarbonyl, stehen,
oder einen Rest aus den Gruppen G⁷, G⁸, G⁹, G¹⁰ und G¹¹ 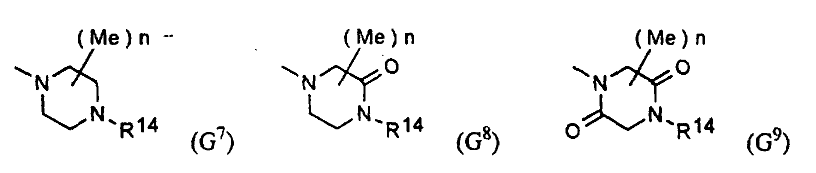 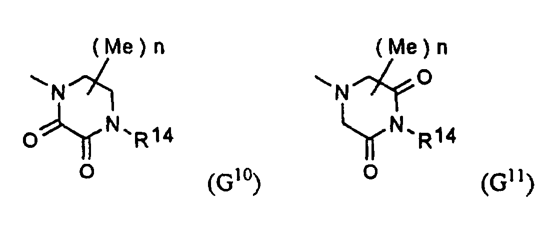 worin
- n: die Zahlen 0, 1 oder 2 bedeuten kann,
- R¹³: für C₁₋₄-Alkyl, insbesondere Methyl, steht, und
- R¹⁴: für geradkettiges oder verzweigtes C₁₋₆-Alkyl, insbesondere Methyl, C₂₋₆-Alkenyl, insbesondere Vinyl, 2-Propenyl, C₂₋₆-Alkinyl, insbesondere 2-Propinyl, C₃₋₆-Cycloalkyl, insbesondere Cyclopropyl, C₁₋₄-Alkoxycarbonyl, insbesondere Methoxycarbonyl, Hydoxy-C₁₋₄-alkyl, insbesondere Hydroxyethyl, Hydroxyethylsulfonylethyl, C₁₋₄-Alkylamino, insbesondere Methylamino, Ethylamino, C₁₋₄-Dialkylamino-C₁₋₄-alkyl, insbesondere Dimethylaminoethyl, C₃₋₇-Cycloalkylamino-C₁₋₄-alkyl, insbesondere N-Morpholinoethyl, N-Piperidinoethyl, C₃₋₆-Cycloalkylaminocarbonyl-C₁₋₂-alkyl, insbesondere N-Morpholinocarbonylmethyl, steht,
mit der Maßgabe für die Verbindungen der allgemeinen Formel ( Ia ) für den Fall, daß
- R¹: für Wasserstoff steht,
- R² und R³: für Methyl stehen,
- R⁴: für andere Reste als Methyl oder n-Butyl steht,
sowie deren optische Isomere und Racemate.

Die erfindungsgemäß zu verwendenden Verbindung der allgemeinen Formel (I) und deren Salze enthalten außerdem ein oder mehrere Chiralitätszentren und können somit in reinen Stereoisomeren oder in Form verschiedener Enantiomeren- und Diastereoisomerengemische vorliegen, die erforderlichenfalls in an sich bekannter Weise getrennt werden können. Die Erfindung betrifft daher sowohl die reinen Enantiomeren und Diastereomeren, als auch deren Gemische zur Bekämpfung von Endoparasiten, besonders auf dem Gebiet der Medizin und Veterinärmedizin.

Vorzugsweise werden jedoch die optisch aktiven, sterioisomeren Formen der Verbindungen der allgemeinen Formel (I) und deren Salze erfindungsgemäß verwendet.

Als geeignete Salze der Verbindungen der allgemeinen Formel (I) können übliche nicht toxische Salze, d. h. Salze mit verschiedenen Basen und Salze mit zugesetzten Säuren, genannt werden. Vorzugsweise sind Salze mit anorganischen, Basen wie Alkalimetallsalze, beispielsweise Natrium-, Kalium oder Cäsiumsalze, Erdalkali-metallsalze, beispielsweise Calzium- oder Magnesiumsalze, Ammoniumsalze, Salze mit organischen Basen sowie mit organischen Aminen, beispielsweise Triethylam-monium-, Pyridinium-, Picolinium-, Ethanolammonium-, Triethanolammonium-, Dicyclohexylammonium- oder N,N'-Dibenzylethylendiammoniumsalze, Salze mit anorganischen Säuren, beispielsweise Hydrochloride, Hydrobromide, Dihydrosulfate oder Trihydrophosphate, Salze mit organischen Carbonsäuren oder organischen Sulfosäuren, beispielsweise Formiate, Acetate, Trifluoracetate, Maleate, Tartrate, Methansulfonate, Benzolsulfonate oder para-Toluolsulfonate, Salze mit basischen Aminosäuren oder sauren Aminosäuren, beispielsweise Arginate, Aspartate oder Glutamate, zu nennen.

Beispiele für die neuen erfindungsgemäßen Verbindungen sind in den Tabellen 1 bis 52 aufgeführt.

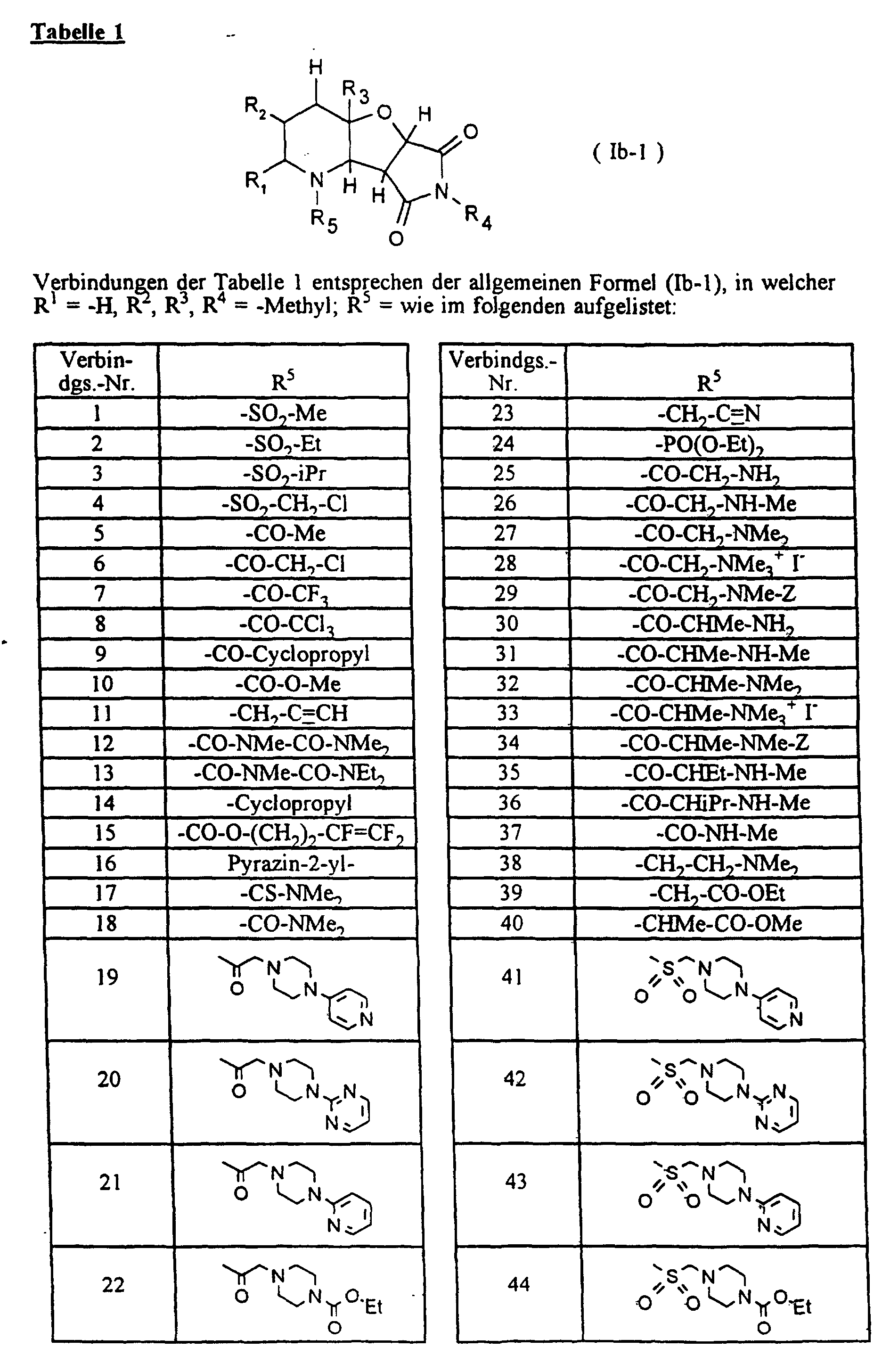

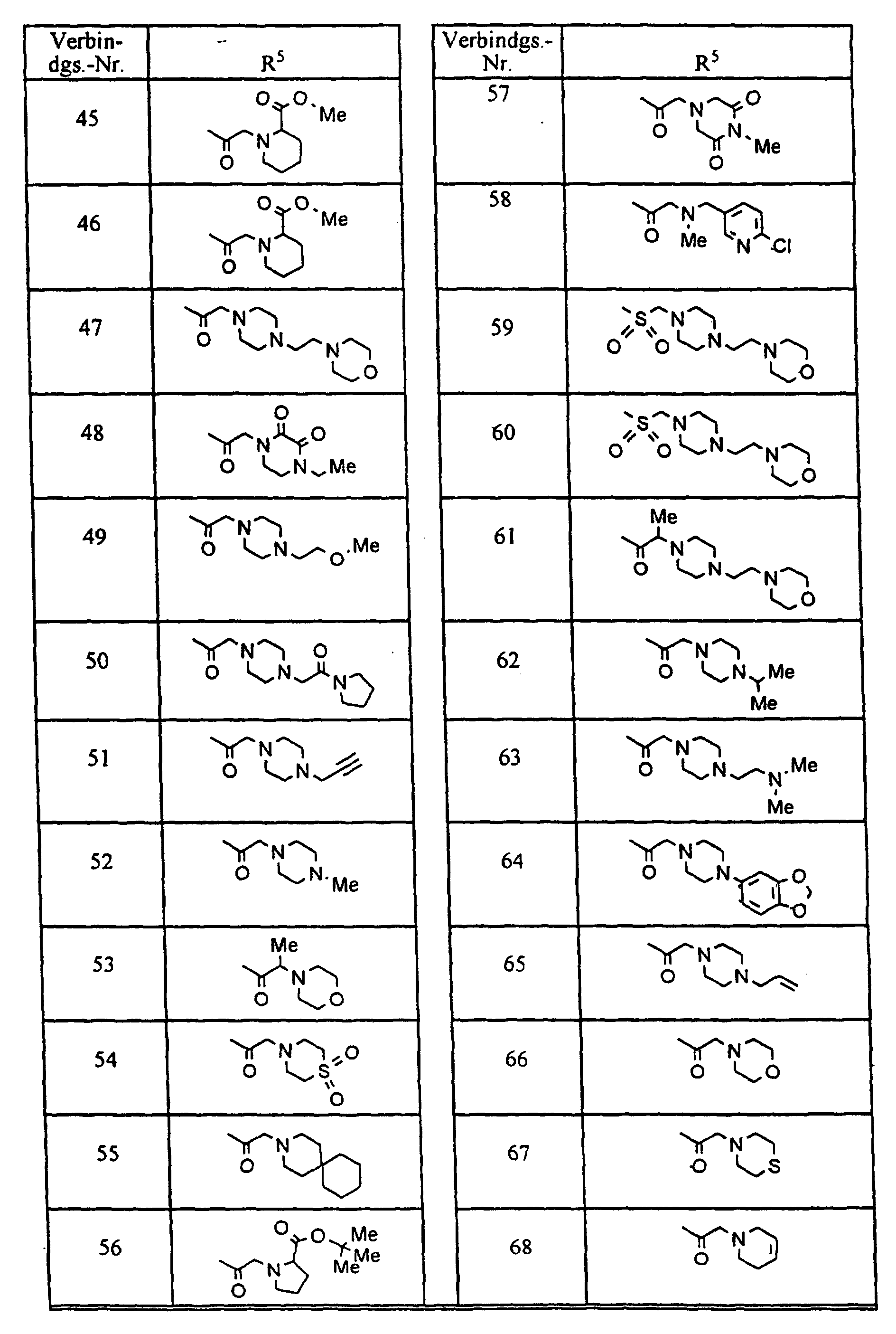

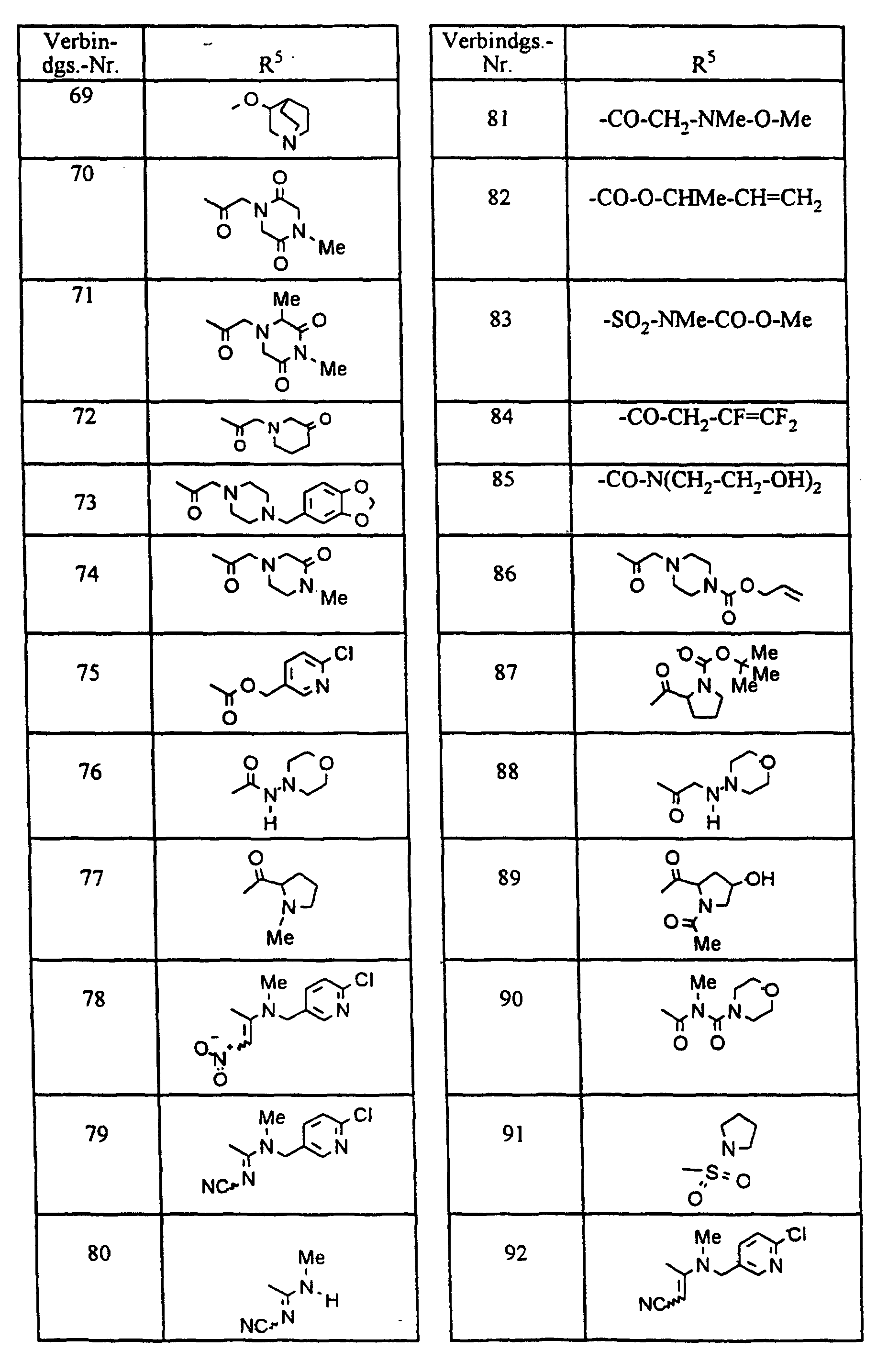

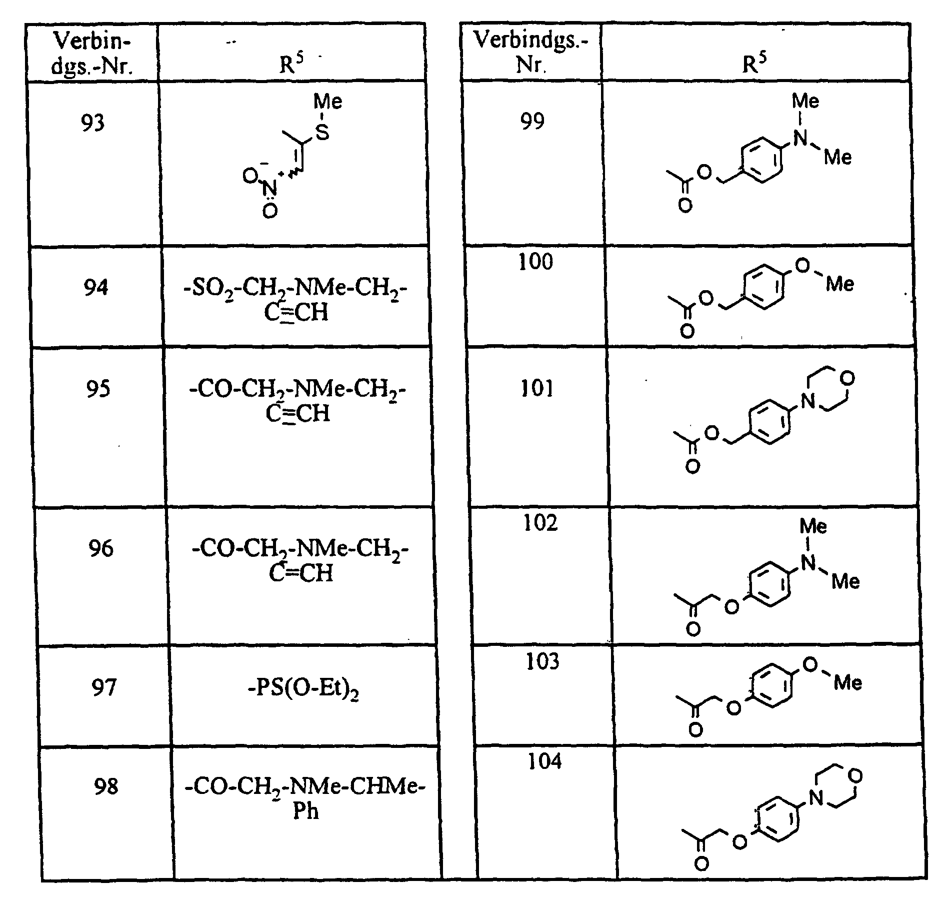

**Tabelle 2**

| |
|---|
| Tabelle 2 enthält die Verbindungen der allgemeinen Formel ( Ib-1 ), in welcher R¹ = -H; R², R³ = -Methyl; R⁴ = -Ethyl; R⁵ die in Tabelle 1 aufgelisteten Bedeutungen besitzt. |

**Tabelle 3**

| |
|---|
| Tabelle 3 enthält die Verbindungen der allgemeinen Formel ( Ib-1 ), in welcher R¹ = -H; R², R³ = -Methyl; R⁴ = -n-Propyl; R⁵ die in Tabelle 1 aufgelisteten Bedeutungen besitzt. |

**Tabelle 4**

| |
|---|
| Tabelle 4 enthält die Verbindungen der allgemeinen Formel ( Ib-1 ), in welcher R¹ = -H; R², R³ = -Methyl; R⁴ = -Isopropyl; R⁵ die in Tabelle 1 aufgelisteten Bedeutungen besitzt. |

**Tabelle 5**

| |
|---|
| Tabelle 5 enthält die Verbindungen der allgemeinen Formel ( Ib-1 ), in welcher R¹ = -H; R², R³ = -Methyl; R⁴ = -Cyclopropyl; R⁵ die in Tabelle 1 aufgelisteten Bedeutungen besitzt. |

**Tabelle 6**

| |
|---|
| Tabelle 6 enthält die Verbindungen der allgemeinen Formel ( Ib-1 ), in welcher R¹ = -H; R², R³ = -Methyl; R⁴ = -n-Butyl; R⁵ die in Tabelle 1 aufgelisteten Bedeutungen besitzt. |

**Tabelle 7**

| |
|---|
| Tabelle 7 enthält die Verbindungen der allgemeinen Formel ( Ib-1 ), in welcher R¹ = -H; R², R³ = -Methyl; R⁴ = -sec-Butyl; R⁵ die in Tabelle 1 aufgelisteten Bedeutungen besitzt. |

**Tabelle 8**

| |
|---|
| Tabelle 8 enthält die Verbindungen der allgemeinen Formel ( Ib-1 ), in welcher R¹ = -H; R², R³ = -Methyl; R⁴ = -Isobutyl; R⁵ die in Tabelle 1 aufgelisteten Bedeutungen besitzt. |

**Tabelle 9**

| |
|---|
| Tabelle 9 enthält die Verbindungen der allgemeinen Formel ( Ib-1 ), in welcher R¹ = -H; R², R³ = -Methyl; R⁴ = -Cyclobutyl; R⁵ die in Tabelle 1 aufgelisteten Bedeutungen besitzt. |

**Tabelle 10**

| |
|---|
| Tabelle 10 enthält die Verbindungen der allgemeinen Formel ( Ib-1 ), in welcher R¹ = -H; R², R³ = -Methyl; R⁴ = -Cyclopropylmethyl; R⁵ die in Tabelle 1 aufgelisteten Bedeutungen besitzt. |

**Tabelle 11**

| |
|---|
| Tabelle 11 enthält die Verbindungen der allgemeinen Formel ( Ib-1 ), in welcher R¹ = -H; R², R³ = -Methyl; R⁴ = -Cyclopentyl; R⁵ die in Tabelle 1 aufgelisteten Bedeutungen besitzt. |

**Tabelle 12**

| |
|---|
| Tabelle 12 enthält die Verbindungen der allgemeinen Formel ( Ib-1 ), in welcher R¹ = -H; R², R³ = -Methyl; R⁴ = 2-Phenylethyl; R⁵ die in Tabelle 1 aufelisteten Bedeutungen besitzt. |

**Tabelle 13**

| |
|---|
| Tabelle 13 enthält die Verbindungen der allgemeinen Formel ( Ib-1 ), in welcher R¹ = -H; R², R³ = -Methyl; R⁴ = -Allyl; R⁵ die in Tabelle 1 aufgelisteten Bedeutungen besitzt. |

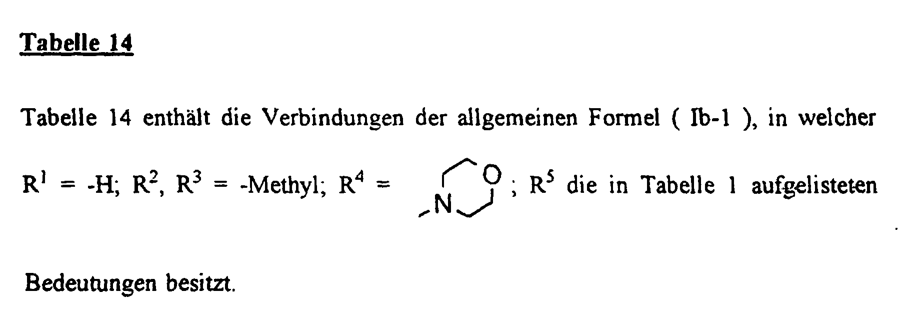

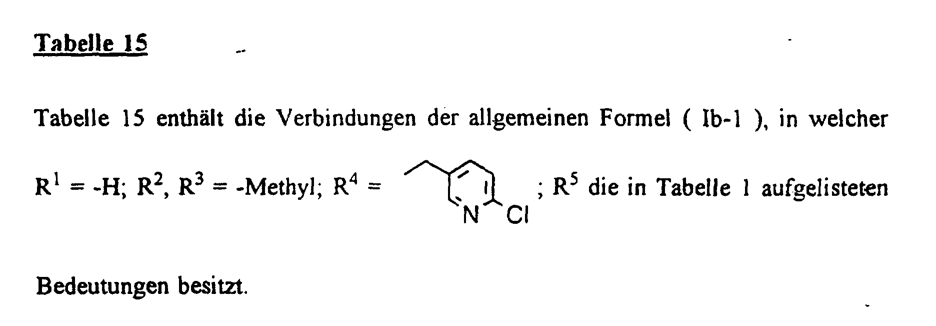

**Tabelle 16**

| |
|---|
| Tabelle 16 enthält die Verbindungen der allgemeinen Formel ( Ib-1 ), in welcher R¹ = -H; R² = -CH₂-Br , R³, R⁴ = -Methyl ; R⁵ die in Tabelle 1 aufgelisteten Bedeutungen besitzt. |

**Tabelle 17**

| |
|---|
| Tabelle 17 enthält die Verbindungen der allgemeinen Formel ( Ib-1 ), in welcher R¹ = -H; R² = -CH₂-Br , R³ = -Methyl; R⁴ = -Ethyl ; R⁵ die in Tabelle 1 aufgelisteten Bedeutungen besitzt. |

**Tabelle 18**

| |
|---|
| Tabelle 18 enthält die Verbindungen der allgemeinen Formel ( Ib-1 ), in welcher R¹ = -H; R² = -CH₂-Br , R³ = -Methyl; R⁴ = -Cyclopropyl ; R⁵ die in Tabelle 1 aufgelisteten Bedeutungen besitzt. |

**Tabelle 19**

| |
|---|
| Tabelle 19 enthält die Verbindungen der allgemeinen Formel ( Ib-1 ), in welcher R¹ = -H; R² = -CH₂-Br , R³ = -Methyl; R⁴ = -n-Butyl ; R⁵ die in Tabelle 1 aufgelisteten Bedeutungen besitzt. |

**Tabelle 20**

| |
|---|
| Tabelle 20 enthält die Verbindungen der allgemeinen Formel ( Ib-1 ), in welcher R¹ = -H; R² = -CH₂-Br , R³ = -Methyl; R⁴ = -Cyclobutyl ; R⁵ die in Tabelle 1 aufgelisteten Bedeutungen besitzt. |

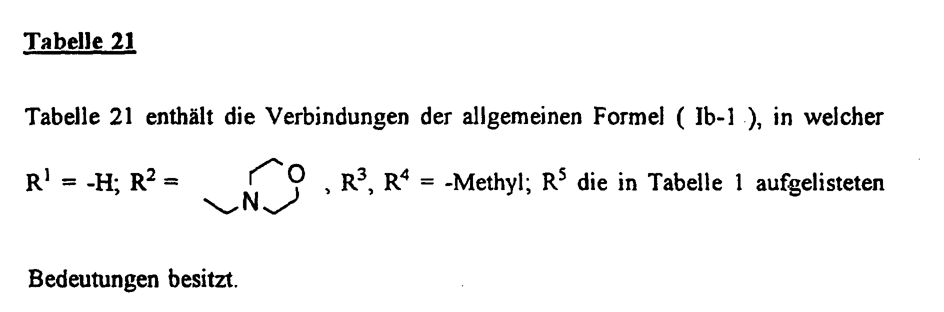

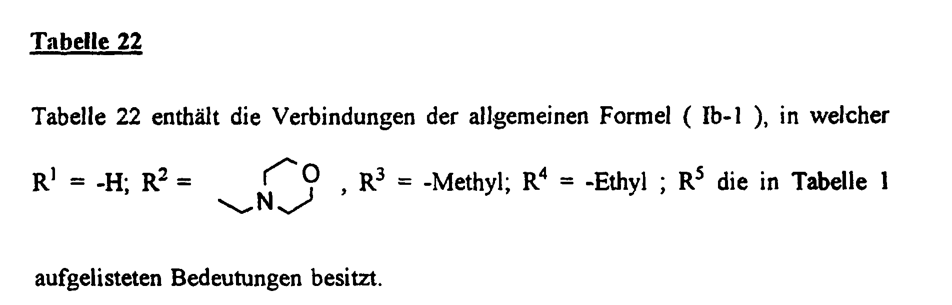

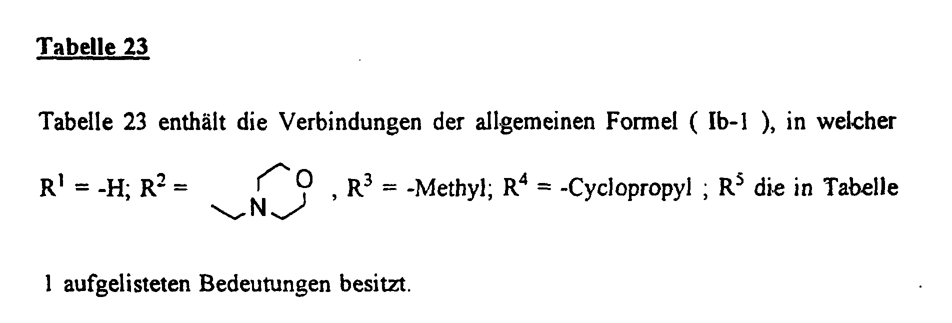

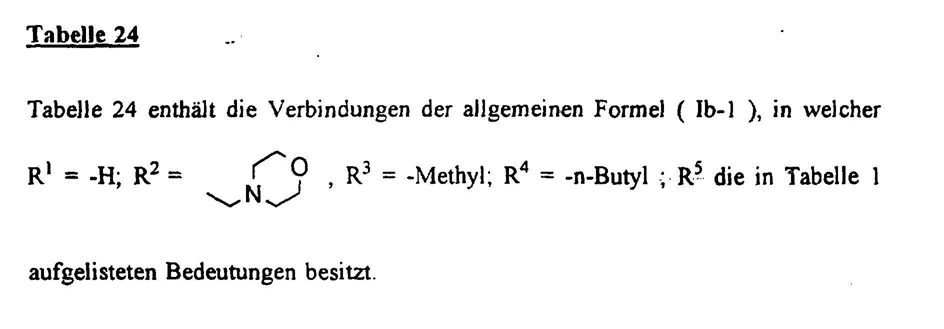

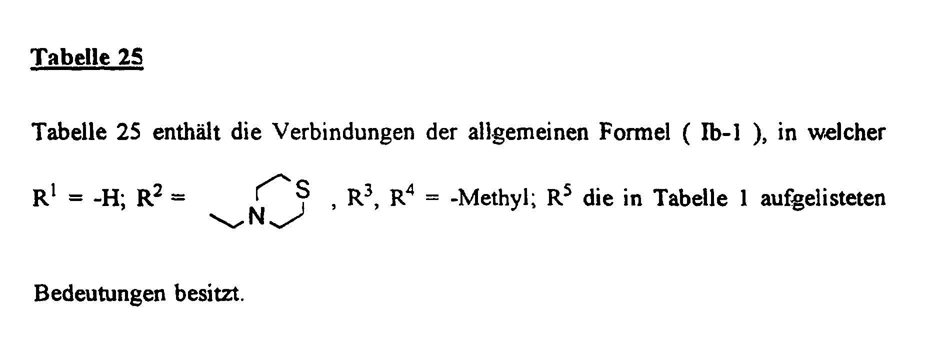

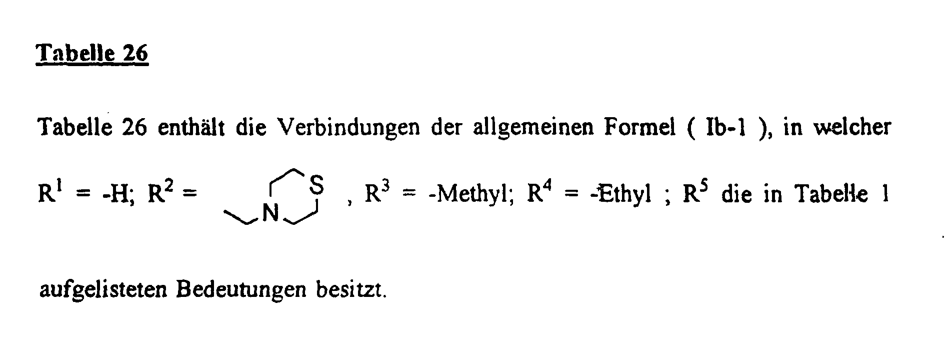

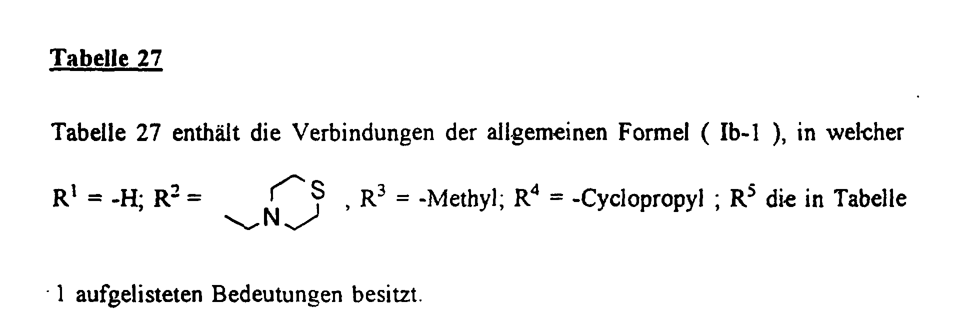

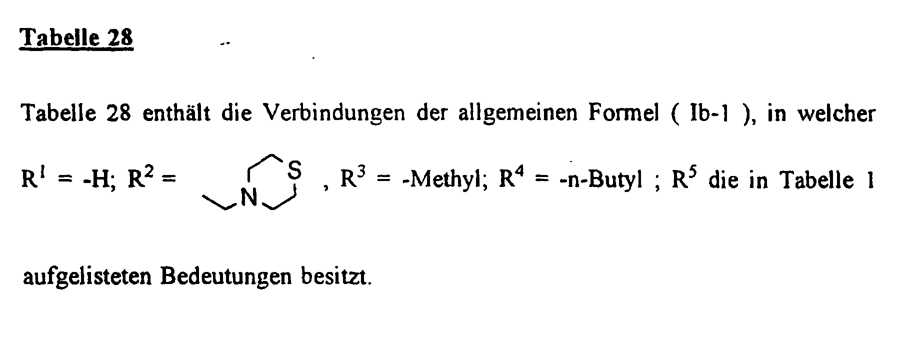

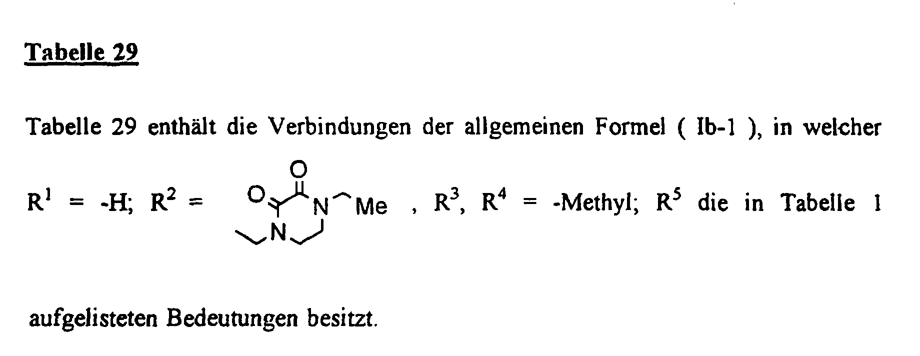

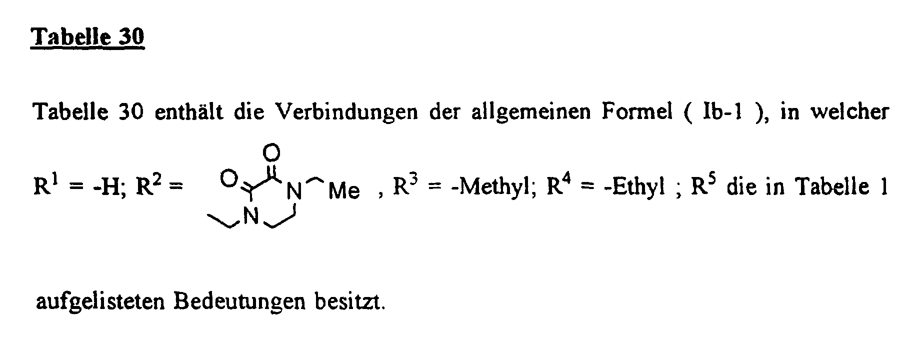

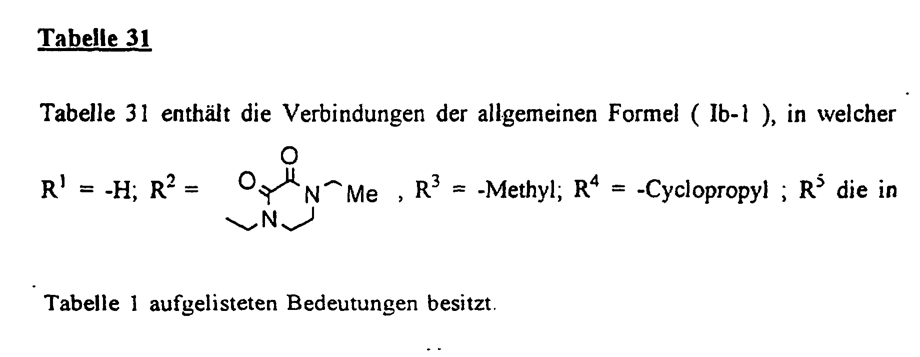

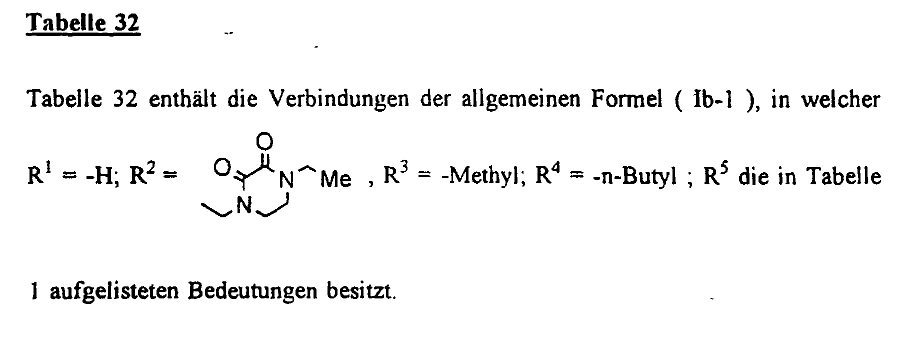

**Tabelle 33**

| |
|---|
| Tabelle 33 enthält die Verbindungen der allgemeinen Formel ( Ib-1 ), in welcher R¹ = -H; R² = -CO-O-Me, R³, R⁴ = -Methyl; R⁵ die in Tabelle 1 aufgelisteten Bedeutungen besitzt. |

**Tabelle 34**

| |
|---|
| Tabelle 34 enthält die Verbindungen der allgemeinen Formel ( Ib-1 ), in welcher R¹ = -H; R² = -CO-O-Me, R³ = -Methyl; R⁴ = -Ethyl ; R⁵ die in Tabelle 1 aufgelisteten Bedeutungen besitzt. |

**Tabelle 35**

| |
|---|
| Tabelle 35 enthält die Verbindungen der allgemeinen Formel ( Ib-1 ), in welcher R¹ = -H; R² = -CO-O-Me, R³ = -Methyl; R⁴ = -Cyclopropyl ; R⁵ die in Tabelle 1 aufgelisteten Bedeutungen besitzt. |

**Tabelle 36**

| |
|---|
| Tabelle 36 enthält die Verbindungen der allgemeinen Formel ( Ib-1 ), in welcher R¹ = -H; R² = -CO-O-Me, R³ = -Methyl; R⁴ = -n-Butyl ; R⁵ die in Tabelle 1 aufgelisteten Bedeutungen besitzt. |

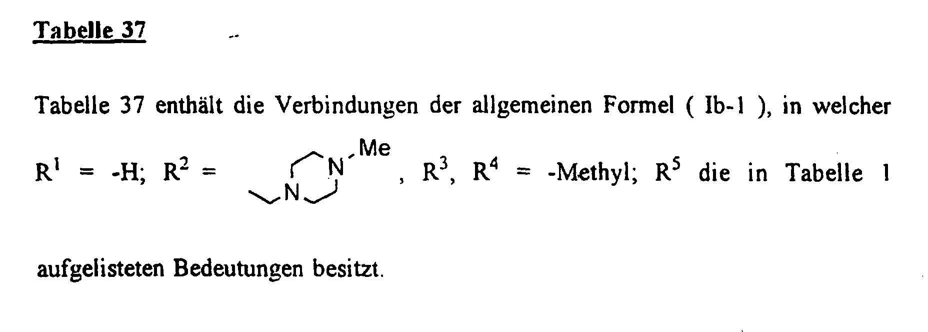

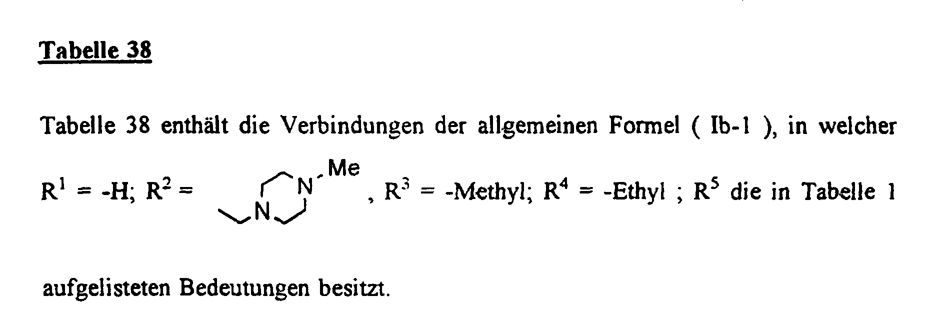

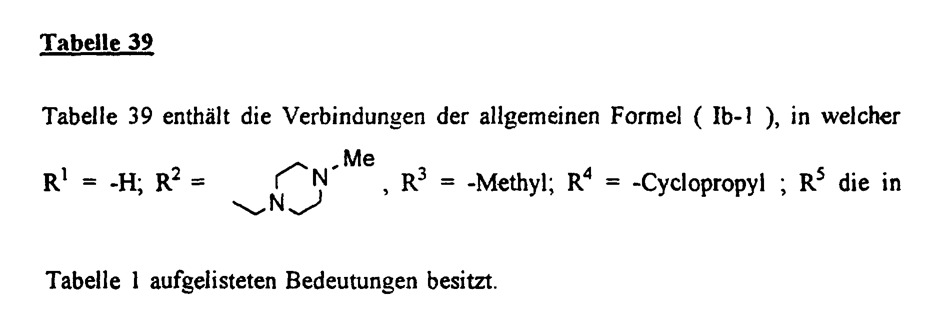

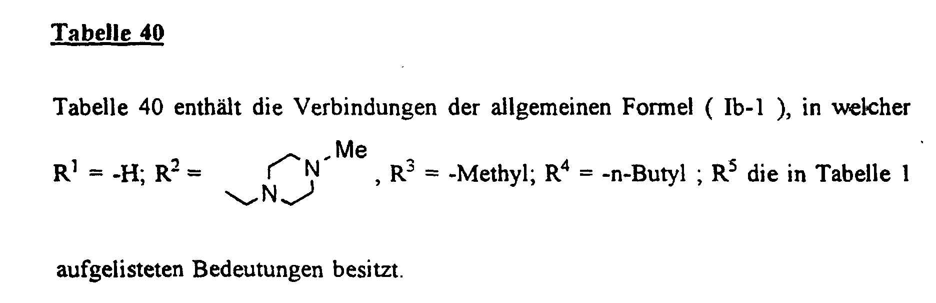

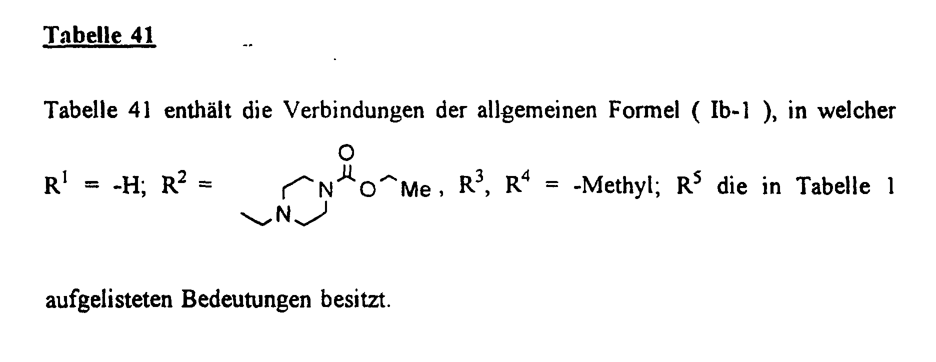

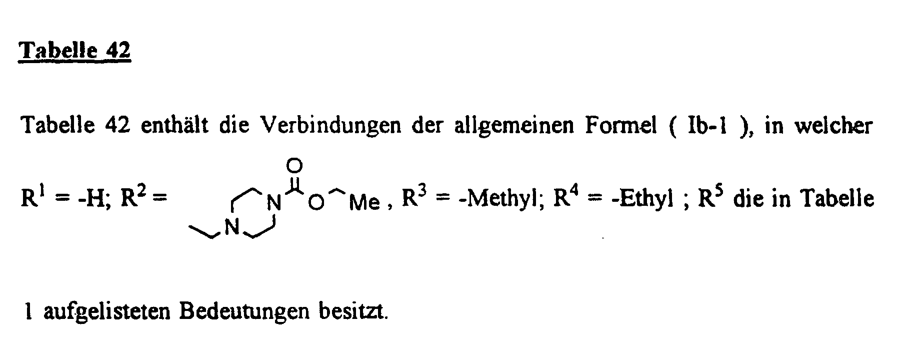

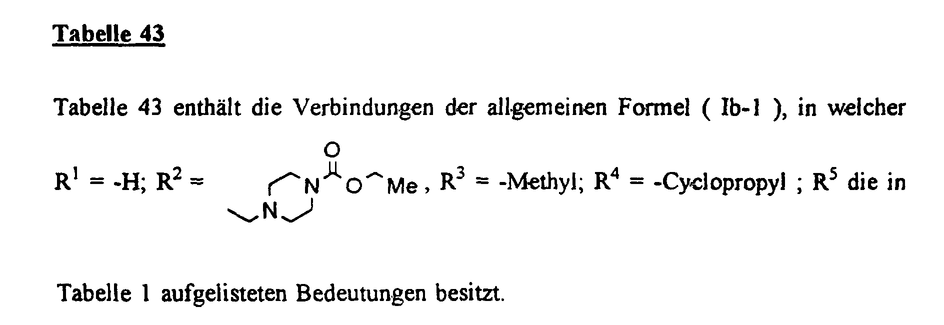

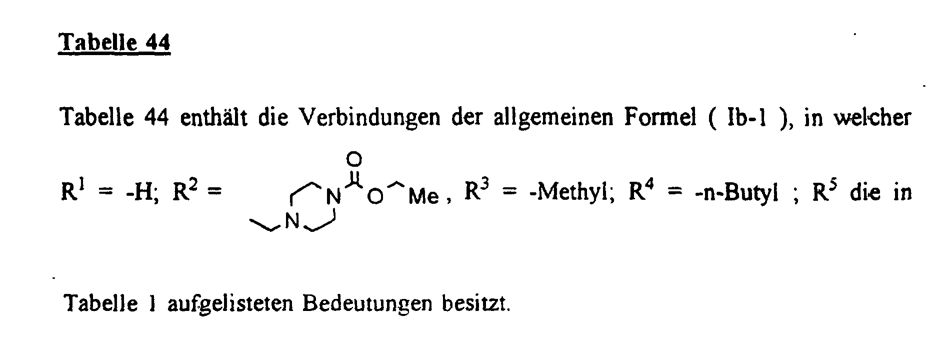

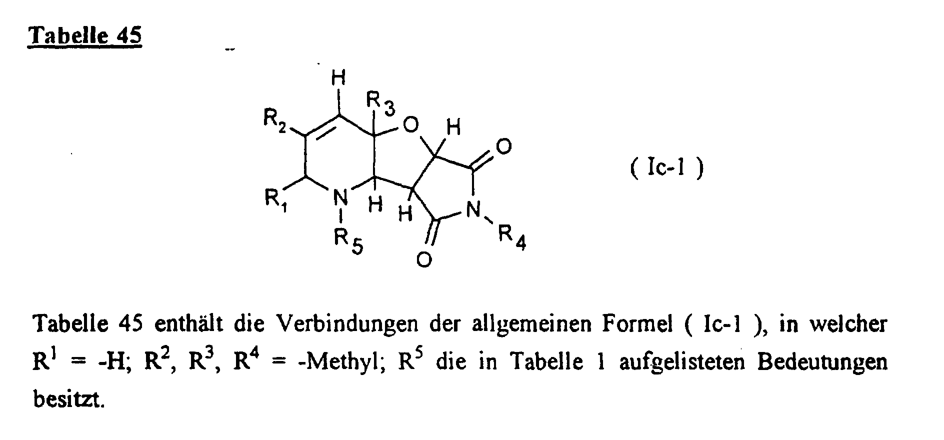

**Tabelle 46**

| |
|---|
| Tabelle 46 enthält die Verbindungen der allgemeinen Formel ( Ic-1 ), in welcher R¹ = -H; R², R³ = -Methyl; R⁴ = -Ethyl; R⁵ die in Tabelle 1 aufgelisteten Bedeutungen besitzt. |

**Tabelle 47**

| |
|---|
| Tabelle 47 enthält die Verbindungen der allgemeinen Formel ( Ic-1 ), in welcher R¹ = -H; R², R³ = -Methyl; R⁴ = -n-Propyl; R⁵ die in Tabelle 1 aufgelisteten Bedeutungen besitzt. |

**Tabelle 48**

| |
|---|
| Tabelle 48 enthält die Verbindungen der allgemeinen Formel ( Ic-1 ), in welcher R¹ = -H; R², R³ = -Methyl; R⁴ = -Isopropyl; R⁵ die in Tabelle 1 aufgelisteten Bedeutungen besitzt. |

**Tabelle 49**

| |
|---|
| Tabelle 49 enthält die Verbindungen der allgemeinen Formel ( Ic-1 ), in welcher R¹ = -H; R², R³ = -Methyl; R⁴ = -Cyclopropyl; R⁵ die in Tabelle 1 aufgelisteten Bedeutungen besitzt. |

**Tabelle 50**

| |
|---|
| Tabelle 50 enthält die Verbindungen der allgemeinen Formel ( Ic-1 ), in welcher R¹ = -H; R², R³ = -Methyl; R⁴ = -n-Butyl; R⁵ die in Tabelle 1 aufgelisteten Bedeutungen besitzt. |

**Tabelle 51**

| |
|---|
| Tabelle 51 enthält die Verbindungen der allgemeinen Formel ( lc-1 ), in welcher R¹ = -H; R², R³ = -Methyl; R⁴ = -Cyclopropylmethyl; R⁵ die in Tabelle 1 aufgelisteten Bedeutungen besitzt. |

**Tabelle 52**

| |
|---|
| Tabelle 52 enthält die Verbindungen der allgemeinen Formel ( Ic-1 ), in welcher R¹ = -H; R², R³ = -Methyl; R⁴ = 2-Phenylethyl; R⁵ die in Tabelle 1 aufgelisteten Bedeutungen besitzt. |

Die Verbindungen der Formel ( Ia ) sind teilweise bekannt, sie lassen sich nach dem oben unter Punkt 3 angegebenen Verfahren a) herstellen (vgl. Z. B.: T. Hisano et al. Chem. Pharm. Bull. 35 (3), (1987) S. 1049-1057; Heterocycles 29 (6), (1989) S. 1029-1032; Chem. Pharm. Bull. 38 (3), (1990) S. 605-611; Chem. Pharm. Bull. 39 (1), (1991) S. 10-17).

Setzt man bei Verfahren 3a zur Herstellung der neuen 4a, 5a, 8a, 8b-Tetrahydro-6H-pyrrolo[3',4':4,5]furo[3,2-b]pyridin-6,8(7H)-dion Derivate der allgemeinen Formel (Ia) als Verbindungen der allgemeinen Formel (II) 3,5-Dimethyl-pyridin-N-oxid und als Verbindungen der Formel (III) N-Benzyl-maleinimid ein, so läßt sich das Verfahren durch folgendes Reaktionsschema wiedergeben:

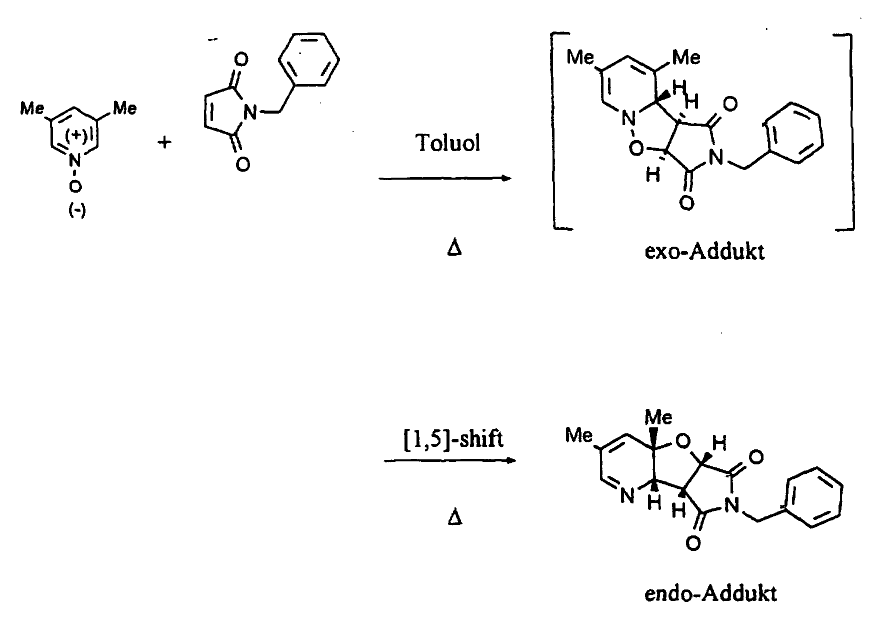

Die zur Durchführung des erfindungsgemäßen Verfahrens 3a als Ausgangsstoffe benötigten substituierten Pyridin-N-oxide sind durch die Formel (II) allgemein definiert. In dieser Formel stehen R¹, R², R³ vorzugsweise für diejenigen Reste, die bereits im Zusammenhang mit der Beschreibung der erfindungsgemäßen Stoffe der Formel ( I ) als bevorzugt für diese Substituenten genannt wurden.

Die als Ausgangsmaterialien verwendeten substituierten Pyridin-N-oxide sind teilweise bekannt, können z. T. kommerziell oder nach literaturbekannten Methoden erhalten werden (z. B. J. M. Essery und K. Schofield J. Chem. Soc. (1960) S. 49539).

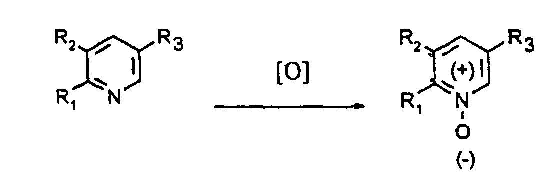

Für die Oxidation der Pyridinderivate zu den N-Oxiden der allgemeinen Formel (II) kommen die verschiedensten Peroxide wie Wasserstoffperoxid, tert.-Butylperoxid, organische oder anorganische Peroxide oder deren Salze wie 3-Chlorperbenzoesäure, Peressigsäure, Perameisensäure, Dibenzoylperoxid usw. in Frage.

Man kann das Peroxid auch in situ aus einem anderen Peroxid, beispielsweise Peressigsäure aus Essigsäure und Wasserstoffperoxid herstellen.

Das Peroxid wird vorzugsweise in gleichen Äquivalenten wie das Ausgangspyridin eingesetzt. Man kann auch einen Überschuß verwenden, um die Reaktion zu vervollständigen. Geeignet sind alle Lösungsmittel, die selbst nicht mit den Oxidationsmitteln störende Nebenreaktionen eingehen, wie z. B. Wasser, Alkylalkohole, Carbonsäuren wie Essigsäure oder Ameisensäure oder halogenierte Kohlenwasserstoffe wie u. a. Methylenchlorid. Die Reaktionstemperaturen liegen zwischen -30°C und +130°C vorzugsweise zwischen -10°C und +80°C.

Die außerdem für die Durchführung des erfindungsgemäßen Verfahrens 3a als Ausgangsstoffe benötigten N-substituierten Maleinimide sind durch die Formel (III) allgemein definiert. In dieser Formel steht R⁴ vorzugsweise für denjenigen Rest, der bereits im Zusammenhang mit der Beschreibung der erfindungsgemäßen Stoffe der allgemeinen Formel (I) als bevorzugt für diesen Substituenten genannt wird.

Die als Ausgangsmaterialien verwendeten N-substituierten Maleinimide der allgemeinen Formel (III) sind teilweise bekannt, können z. T. kommerziell oder nach literaturbekannten Methoden erhalten werden (vgl. z. B.: EP 0608 445 Al, N. B. Mehta et al. J. Org. Chem. 25 (1960) S. 1012-1015; T. F. Braish et al. Synlett (1992) S. 979-980).

Im allgemeinen ist es vorteilhaft das erfindungsgemäße Verfahren 3a in Gegenwart von Verdünnungssmitteln durchzuführen. Verdünnungsmittel werden vorteilhafterweise in einer solchen Menge eingesetzt, daß das Reaktionsgemisch während des ganzen Verfahrens gut rührbar bleibt. Als Verdünnungsmittel zur Durchführung des erfindungsgemäßen Verfahrens 3a kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Anisol, Phenetol, Cyclohexylmethylether, Dimethylether, Diethylether, Di-propylesther, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methyl-morpholin, Pyridin und Tetramethylendiamin, Nitrokohlenwasserstoffe wie Nitromethan, Nitroethan, Nitropropan, Nitrobenzol, Chlomitrobenzol, o-Nitrotoluol; Nitrile wie Acetonitril, Propionitril, Butyronitril, Isobutyronitril, Benzonitril, m-Chlor-benzonitril sowie Verbindungen wie Tetrahydro-thiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloalipha-tische oder aromatische Kohlenwasserstoffe wie Pentan, Hexan, Heptan, Oktan, Nonan und technische Kohlenwasserstoffe, beispielsweise sogenannte White Spirits mit Komponenten mit Siedepunkten im Bereich beilspielsweise 40 bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methylformamid, N,N-Dimethylformamid, N,N-Dipropylformamid, N,N-Dibutylformamid, N-Methyl-pyrrolidon, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin; Ketone. wie Aceton, Acetophenon, Methylethylketon, Methylbutylketon.

Selbstverständlich kann man in das erfindungsgemäße Verfahren auch Gemische der genannten Lösungs- und Verdünnungsmittel einsetzen.

Bevorzugte Verdünnungsmittel sind aromatische Halogenkohlenwasserstoffe, insbesondere Chlorbenzol und Brombenzol, aromatische Nitrokohlenwasserstoffe, wie Nitrobenzol, aromatische Kohlenwasserstoffe, insbesondere Benzol und Toluol, Ketone wie Acetophenon, Amide wie N,N-Dimethylformamid, Sulfoxide wie Tetramethylensulfoxid und oder Gemische von diesen mit anderen genannten Verdünnungsmitteln.

Das Verfahren 3a wird durchgeführt, indem man substituierte Pyridin-N-oxide der allgemeinen Formel (II) mit N-substituierten Maleinimiden der allgemeinen Formel (III) in einem der angegebenen Verdünnungsmittel umsetzt.

Die Reaktionsdauer beträgt 4 bis 72 Stunden. Die Umsetzung erfolgt bei Temperaturen zwischen -10°C und +250°C, bevorzugt zwischen 0°C und +200°C, besonders bevorzugt bei Raumtemperatur bis +150°C. Vorzugseise arbeitet man bei dem Druck, der sich beim Erhitzen auf die erforderliche Reaktionstemperatur unter den Reaktionsbedingungen einstellt.

Zur Durchführung des erfindungsgemäßen Verfahrens 3a setzt man pro Mol an Verbindung der Formel (II) im allgemeinen 1,0 bis 3,0 Mol, vorzugsweise 1,0 bis 2,0 Mol N-substituiertes Maleinimid, ein.

Nach vollendeter Umsetzung wird die Reaktionslösung im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Im einzelnen seien folgende Verbindungen der allgemeinen Formel (Ia) genannt, in welcher die Reste R¹ bis R⁴ die folgende Bedeutung haben:

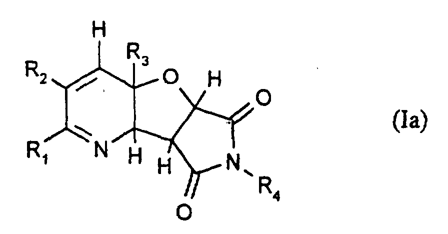

| R¹ | R² | R³ | R⁴ |
|---|---|---|---|
| -H | -CH₃ | -CH₃ | -CH₂-CF₃ |
| -H | -CH₃ | -CH₃ | -CH₂-CH₂-F |
| -H | -CH₃ | -CH₃ | -CH₂-CH₂-CF₃ |
| -H | -CH₃ | -CH₃ | -CHMe₂ |
| -H | -CH₃ | -CH₃ | -CHMe-CF₃ |
| -H | -CH₃ | -CH₃ | -CH₂-C≡CH |
| -H | -CH₃ | -CH₃ | -(R)-CHMe-Phenyl |
| -H | -CH₃ | -CH₃ | -CH₂-CO-O-Me |
| -H | -CH₃ | -CH₃ | -CH₂-CO-O-tBu |
| -H | -CH₃ | -CH₃ | -(R)-CHMe-CO-O-tBu |
| -H | -CH₃ | -CH₃ | -(R)-CHMe-CO-O-Me |
| -H | -CH₃ | -CH₃ | -(S)-CHMe-CO-O-tBu |
| -H | -CH₃ | -CH₃ | -(S)-CHMe-CO-O-Me |
| -H | -CH₂-Br | -CH₃ | -Me |
| -H | -CH₂-Br | -CH₃ | -Et |
| -H | -CH₂-Br | -CH₃ | -Cyclopropyl |
| -H | -CH₂-Br | -CH₃ | -Cyclobutyl |
| -H | -CH₂-Br | -CH₃ | -n-Butyl |
| -H | -CH₂-Br | -CH₃ | -sec-Butyl |
| -H | -CH₂-Br | -CH₃ | -(S)-CHMe-Phenyl |
| -H | -CH₂-Br | -CH₃ | -Benzyl |
| -H | -CH₂-NMe₂ | -CH₃ | -Me |
| -H | -CH₂-NMe₂ | -CH₃ | -Et |
| -H | -CH₂-NMe₂ | -CH₃ | -Cyclopropyl |
| -H | -CH₂-NMe₂ | -CH₃ | -Cyclobutyl |
| -H | -CH₂-NMe₂ | -CH₃ | -n-Butyl |
| -H | -CH₂-NMe₂ | -CH₃ | -sec-Butyl |
| -H | -CH₂-NMe₂ | -CH₃ | -(S)-CHMe-Phenyl |
| -H | -CH₂-NMe₂ | -CH₃ | -Benzyl |

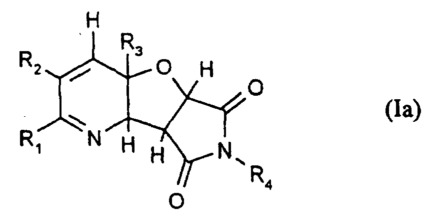

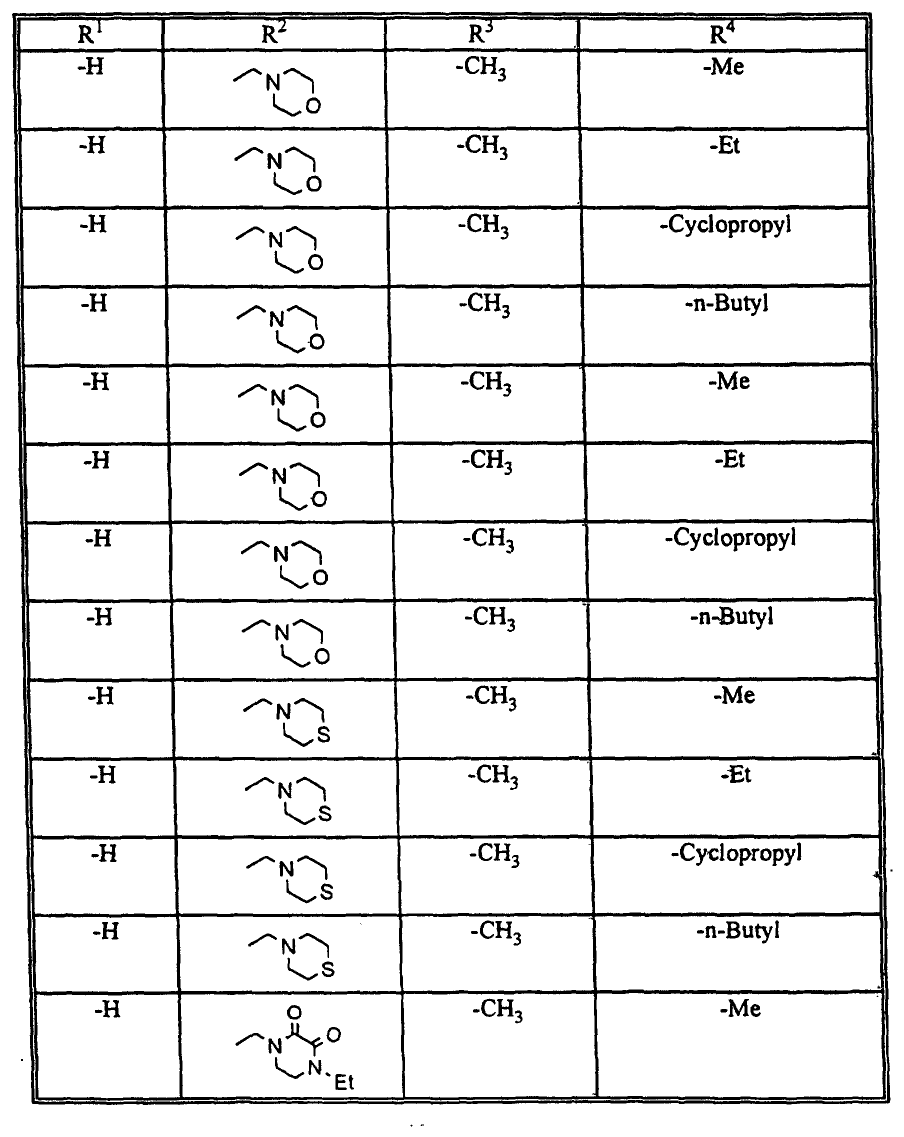

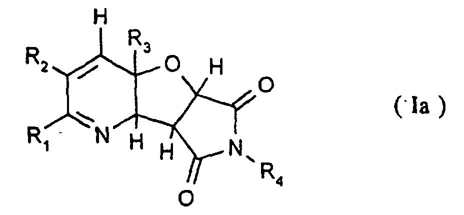

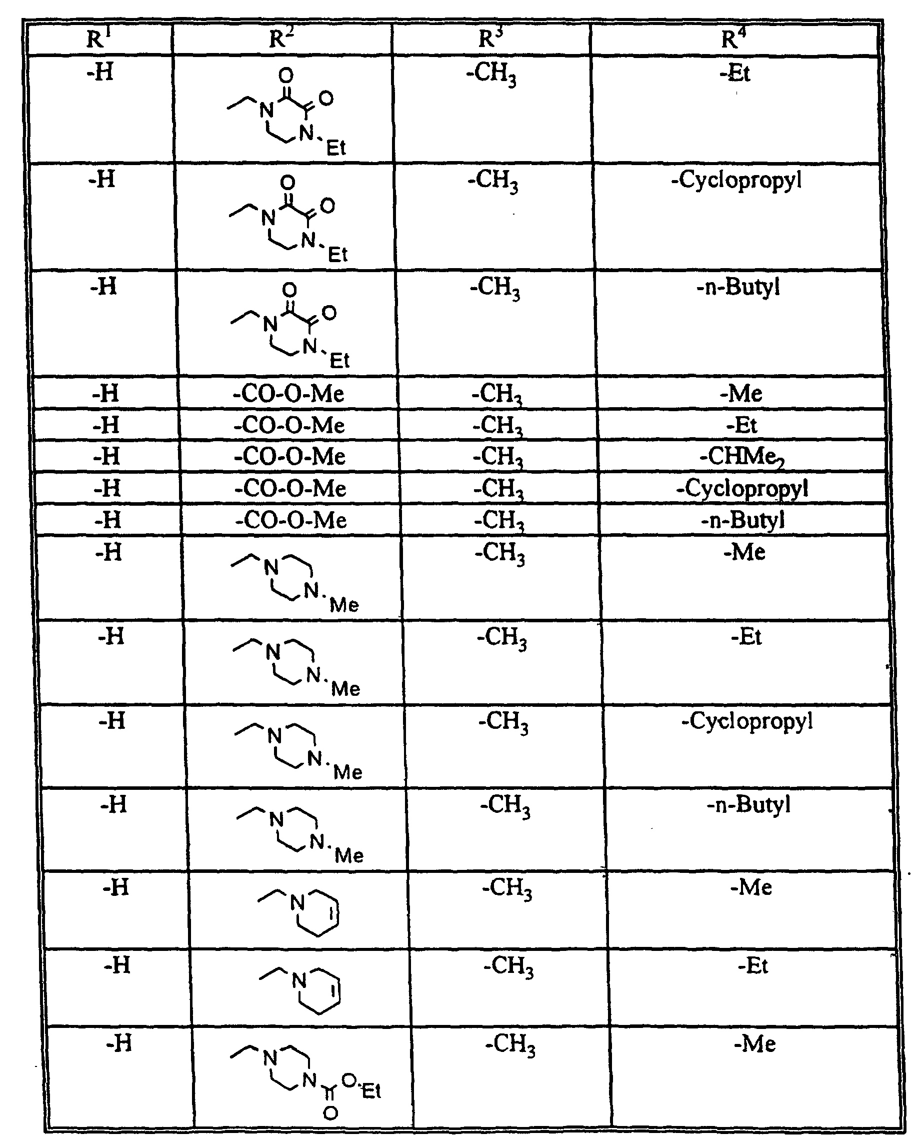

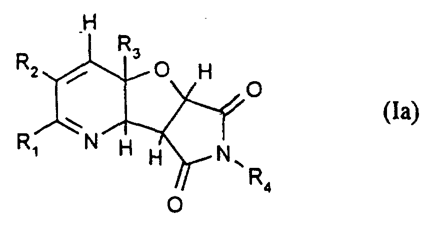

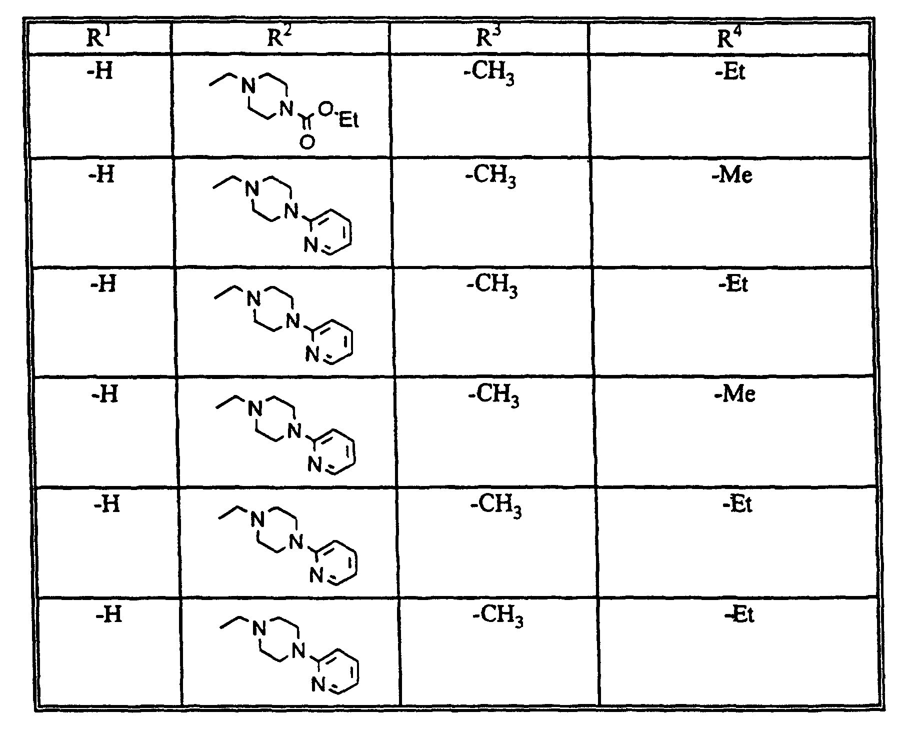

Die vorliegende Erfindung betrifft auch die Verbindungen der allgemeinen Formel (I) in Form eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Weinsäure, Ölsäure, Methansulfonsäure, Benzolsulfonsäure oder Toluolsulfonsäure.

Als Beispiele zur Herstellung geeigneter Salze von Verbindungen der allgemeinen Formel (Ia) seien die Hydrochloridbildung und die Darstellung der entsprechenden N¹-Methylammoniumhalogenide der allgemeinen Formel (V), wie beispielsweise das N¹-Methylammoniumiodid vom 7-Ethyl-4a, 5a, 8a, 8b-tetrahydro-3,4a-dimethyl-6H-pyrrolo[3',4':4,5]furo[3,2-b]pyridin-6,8(7H)-dion gezeigt:

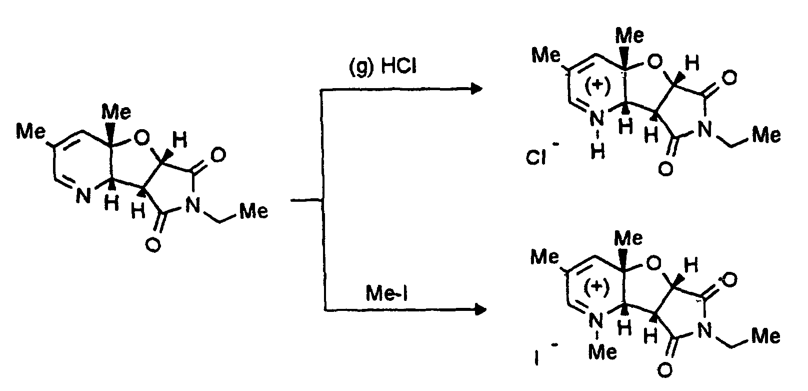

Die Salzbildung wird durchgeführt, indem man Verbindungen der allgemeinen Formel (Ia) beispielsweise in Gegenwart von anorganischen Säuren, wie gasförmiger Chlorwasserstoffsäure, oder mit einem Alkylierungsmittel, wie Methyliodid, in einem der bei Verfahren 3a angegebenen Verdünnungsmittel umsetzt.

Die Reaktionsdauer beträgt 10 Minuten bis 24 Stunden. Die Umsetzung erfolgt bei Temperaturen zwischen -60°C und +150°C, bevorzugt zwischen -10°C und +80°C, besonders bevorzugt bei 0°C bis Raumtemperatur. Es wird unter Normaldruck gearbeitet. Zur Salzbildung setzt man pro Mol an Verbindung der Formel ( Ia ) im allgemeinen einen Überschuß an Säure oder Alkylierungsmittel ein.

Nach vollendeter Umsetzung wird das zumeist ausgefallene Salz abgetrennt, gewaschen und im Vakuum getrocknet (vgl. auch die Herstellungsbeispiele).

Überraschender Weise können die neuen 4a, 5a, 8a, 8b-Tetrahydro-6H-pyrrolo [3',4':4,5]furo[3,2-b]pyridin-6,8(7H)-dion Derivate der allgemeinen Formel (Ia) via Hydrierung einer bzw. beider Doppelbindungen im Dihydropyridinteil in die neuen 1, 2, 3, 4, 4a, 5a, 8a, 8b-Oktahydro-6H-pyrrolo[3',4':4,5]furo[3,2-b]pyridin-6,8(7H)-dion- und 1, 2, 4a, 5a, 8a, 8b-Hexahydro-6H-pyrrolo[3',4':4,5]furo[3,2-b]pyridin-6,8(7H)-dion Derivate der allgemeinen Formeln (Id) und (Ie) überführt und für Folgereaktionen gemäß dem Verfahren 5 genutzt werden.

Im allgemeinen geht man bei dem Verfahren 5 so vor, daß man zunächst die neuen 4a, 5a, 8a, 8b-Tetrahydro-6H-pyrrolo[3',4':4,5]furo[3,2-b]pyridin-6,8(7H)-dion Derivate der allgemeinen Formel (Ia) in Gegenwart eines Katalysators und in Gegenwart eines Verdünnungsmittels hydriert, wobei sich die entsprechenden neuen 1, 2, 3, 4, 4a, 5a, 8a, 8b-Oktahydro-6H-pyrrolo[3',4':4,5]furo[3,2-b]pyridin-6,8 (7H)-dion-und/oder 1, 2, 4a, 5a, 8a, 8b-Hexahydro-6H-pyrrolo[3',4':4,5]furo[3,2-b]pyridin-6,8 (7H)dion Derivate der allgemeinen Formeln (Id) und (Ie) bilden.

Erwartungsgemäß liegen die Verbindungen der allgemeinen Formel (Id) in Form eines Isomerengemisches, bestehend aus einem 3α-Isomer und einem 3β-Isomer, vor.

Setzt man beispielsweise zur Hydrierung das 7-Benzyl-4a, 5a, 8a, 8b-Tetrahydro-6H-pyrrolo[3',4':4,5]furo[3,2-b]pyridin-6,8(7H)-dion als Verbindung der allgemeinen Formel ( Ia ) ein, so ensteht ein Isomerengemisch aus 7-Benzyl-1, 2, 3, 4, 4aα, 5aα, 8aα, 8bα-(±)-oktahydro-3α, 4α-dimethyl-6H-pyrrolo[3',4':4,5]furo[3,2-b]pyridin-6,8(7H)-dion (3α-Isomer) und 7-Benzyl- 1, 2, 3, 4, 4aα, 5aα, 8aα, 8bα-(±)-oktahydro-3β, 4a-dimethyl-6H-pyrrolo[3',4':4,5]furo[3,2-b]pyridin-6,8(7H)-dion (3β-Isomer).

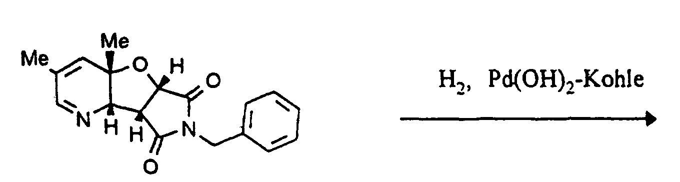

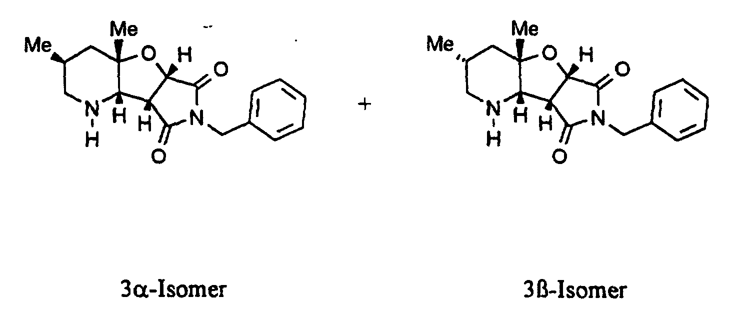

Setzt man beispielsweise zur Hydrierung das 7-Methyl-4a, 5a, 8a, 8b-Tetrahydro-6H-pyrrolo[3',4':4,5]furo[3,2-b]pyridin-6,8(7H)-dion als Verbindung der allgemeinen Formel (Ia) ein, so kann neben dem Isomerengemisch aus 7-Methyl-1, 2, 3, 4, 4aα, 5aα, 8aα, 8ba-(±)-oktahydro-3α, 4a-dimethyl-6H-pyrrolo[3',4':4,5]furo[3,2-b]pyridin-6,8-(7H)-dion (3α-Isomer) und 7-Methyl- 1, 2, 3, 4, 4aα, 5aα, 8aα, 8bα-(±)-oktahydro-3β, 4a-dimethyl-6H-pyrrolo[3',4':4,5]furo[3,2-b]pyridin-6,8(7H)-dion (3β-Isomer) der allgemeinen Formel (Id) auch das 7-Methyl-1, 2, 4a, 5a, 8a, 8b-Hexahydro-6H-pyrrolo[3',4':4,5]furo[3,2-b]pyridin-6,8(7H)-dion der allgemeinen Formel ( Ie ) erhalten werden.

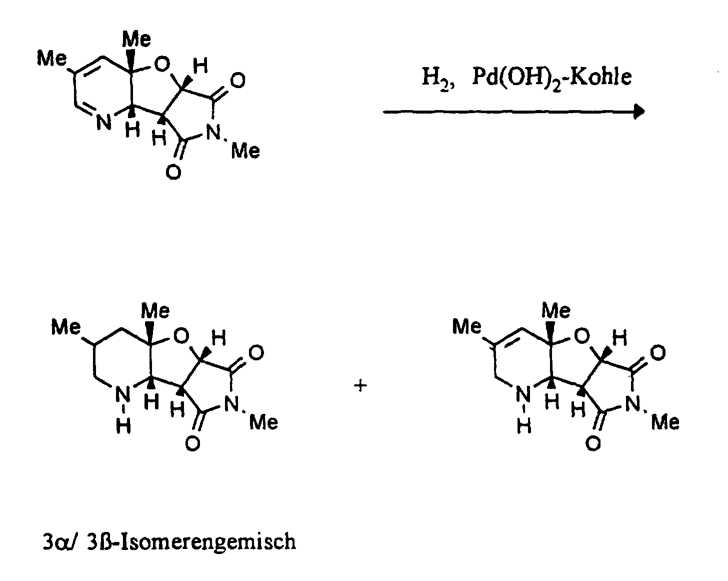

Als Verdünnungsmittel zur Hydrierung finden die bei Verfahren 3a genannten, inerten organischen Lösungsmittel, wie z. B. Alkohole, insbesondere Ethanol, Verwendung.

Die Bildung der erfindungsgemäßen 1, 2, 4a, 5a, 8a, 8b-Hexahydro-6H-pyrrolo [3',4': 4,5]furo[3,2-b]pyridin-6,8(7H)dion Derivate der allgemeinen Formel (Ie) kann von den weiter unten genannten Hydrierungsbedingungen beeinflußt werden.

Als geeignete Katalysatoren zur Durchführung der katalytische Hydrierung kommen alle üblichen Hydrierkatalysatoren, wie beispielsweise Platin-Katalysatoren (Platin-Platte, Platin-Schwamm, Platin-Schwarz, kolloidales Platin, Platinoxid, Platindraht usw.), Palladium-Katalysatoren (beispielsweis Palladium-Schwamm, Palladium-Schwarz, Palladiumoxid, Palladium-Kohle, kolloidales Palladium, Palladium-Barium-sulfat, Palladium-Bariumkarbonat, Palladium-Hydroxid usw.), Nickel-Katalysatoren (beispielsweise reduziertes Nickel, Nickeloxid, Raney-Nickel usw.), Ruthenium-Katalysatoren, Cobalt-Katalysatoren (beispielsweise reduziertes Cobalt, Raney-Cobalt usw.), Eisen-Katalysatoren (beispielsweise reduziertes Eisen, Raney-Eisen usw.), Kupfer-Katalysatoren (beispielsweise reduziertes Kupfer, Raney-Kupfer, Ullman-Kupfer usw.) in Frage. Vorzugsweise verwendet man jedoch Edelmetall-Katalysatoren, wie beispielsweise Platin- und Palladium- oder Ruthenium-Katalysatoren gegebenenfalls auf einem geeigneten Träger wie beispielsweise Kohlenstoff oder Siliziumdioxid.

Zur Durchführung der Hydrierung wird eine alkoholische Lösung der Verbindungen der Formel (Ia) in Gegenwart eines geeigneten Hydrierkatalysators, beispielsweise Palladium-Hydroxyd/Kohle, umgesetzt. Die Reaktionsdauer beträgt 1 bis 20 Stunden. Die Hydrierung wird bei Temperaturen zwischen +10°C und +150°C, bevorzugt zwischen +15°C und +100°C durchgeführt.

Die so erhaltenen Isomerengemische (3α-Isomere und 3ß-Isomere) werden in üblicher Weise, beispielsweise durch chromatographische Reinigung, aufgearbeitet. (vgl. auch die Herstellungsbeispiele). Sie können aber auch direkt (ohne weitere Auftrennung) entsprechend den Verfahren 5a bis 5i umgesetzt werden.

Selbstverständlich kann durch Verwendung eines "chiralen Hydrierkatalysators" z. B. mit chiralen Diphosphinliganden, beispielsweise (2S,3S)-(-)-2,3-Bis-(diphenylphosphino)-butan [(S,S)-Chiraphos] (N. K. Roberts in "Catalytic Aspects of Metal Phosphine Complexes" (1982) S. 337 ACS Washington) oder R(+)-2,2'- bzw. S(-)-2,2'-Bis-(diphenyl-phosphino)-1,1'-binaphthalin [R(+)-BINAP bzw. S(-)-BINAP] (vgl. A. Miyashita et al. Tetrahedron 40 (1984) S. 1245) auch der Anteil eines Isomers (3α-Isomer oder 3β-Isomer) im Isomerengemisch deutlich erhöht werden bzw. läßt sich das Entstehen eines weiteren Isomers (3α-Isomer oder 3β-Isomer) sogar vollständig unterdrücken.

Die selektive Darstellung der erfindungsgemäßen 1,2,4a,5a,8a,8b-Hexahydro-6H pyrrolo[3',4':4,5]furo[3,2-b]pyridin-6,8(7H)dion-Derivate der allgemeinen Formel (Ie) kann aus den entsprechenden N¹-Methyl-ammoniumhalogeniden der allgemeinen Formel (V), wie beispielsweise dem N¹-Methylammoniumiodid, und nachfolgender N¹-Demethylierung erfolgen (vgl. B. Frøilund et al., J. Med. Chem. 38, 1995, S. 3287).

Als Beispiel zur selektiven Herstellung der erfindungsgemäßen 1,2,4a,5a,8a,8b-Hexahydro-6H-pyrrolo[3',4':4,5]furo[3,2-b]pyridin-6,8(7H)dion-Derivate der allgemeinen Formel (Ie) sei die Hydrierung des entsprechenden N¹-Methylammonium-iodids vom 7-Methyl-4aα,5aα,8aα,8bα-tetrahydro-3,4a-dimethyl-6H-pyrrolo[3',4':4,5]furo[3,2-b]pyridin-6,8(7H)dion gezeigt.

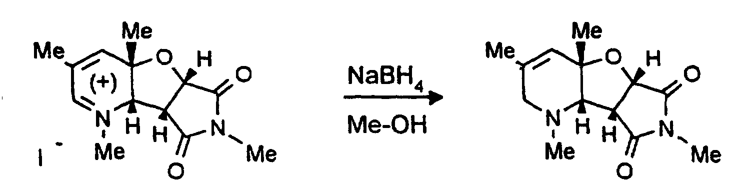

Die Hydrierung des entsprechenden N¹-Methylammoniumiodids wird durchgeführt, indem man Verbindungen der allgemeinen Formel ( V ) beispielsweise das N¹-Methylammoniumiodid vom 7-Methyl-4aα,5aα,8aα,8bα-tetrahydro-3,4a-dimethyl-6H-pyrrolo[3',4':4,5]furo[3,2-b]pyridin-6,8(7H)dion in Gegenwart von Natriumborhydrid in einem der beim nachfolgenden Verfahren 3a angegebenen Verdünnungsmittel umsetzt.

Als Verdünnungsmittel zur Hydrierung werden bevorzugt inerte organische Lösungsmittel, wie z. B. Alkohole, insbesondere Methanol oder Ethanol verwendet.

Die Reaktionsdauer beträgt 10 Minuten bis 48 Stunden. Die Umsetzung erfolgt bei Temperaturen zwischen -60°C und +100°C, bevorzugt zwischen -30°C und +80°C, besonders bevorzugt bei -10°C bis Raumtemperatur. Es wird unter Normaldruck gearbeitet. Zur Hydrierung setzt man pro Mol an N¹-Methylammonhalogenid der Formel (V) im allgemeinen einen geringen Überschuß an Hydrierungsmittel ein.

Nach vollendeter Umsetzung wird in üblicher Weise, beispielsweise durch chromatographische Reinigung, aufgearbeitet (vgl. auch die Herstellungsbeispiele).

Im allgemeinen geht man bei der nachfolgenden N¹-Demethylierung so vor, daß man zunächst die neuen 1,2,4a,5a,8a,8b-Hexahydro-6H-pyrrolo[3',4':4,5]furo[3,2-b]pyri-din-6,8(7H)-dione der allgemeinen Formel (VI) nach R. F. Olofson et al. (J. Org. Chem. 49, 1984, S. 2081) in Gegenwart eines geeigneten Chlorameisensäureesters umsetzt, wobei sich die entsprechenden Carbamate der 1,2,4a,5a,8a,8b-Hexahydro-6H-pyrrolo[3',4':4,5]furo[3,2-b]pyridin-6,8(7H)-dione der allgemeinen Formel (Ig) bilden (vgl. auch Verfahren 5i).

Als Beispiel zur N¹-Demethylierung der erfindungsgemäßen 1,2,4a,5a,8a,8b-Hexahydro-6H-pyrrolo[3',4':4,5]furo[3,2-b]pyridin-6,8(7H)-dione der allgemeinen Formel (VI) sei die Abspaltung des entsprechenden N¹-Methylrestes vom 7-Methyl-4aα,5aα,8aα,8bα-tetrahydro-3,4a-dimethyl-6H-pyrrolo[3',4': 4,5]furo[3,2-b]pyridin-6,8(7H)dion mittels 1-Chlorethoxycarbonylchlorid gezeigt.

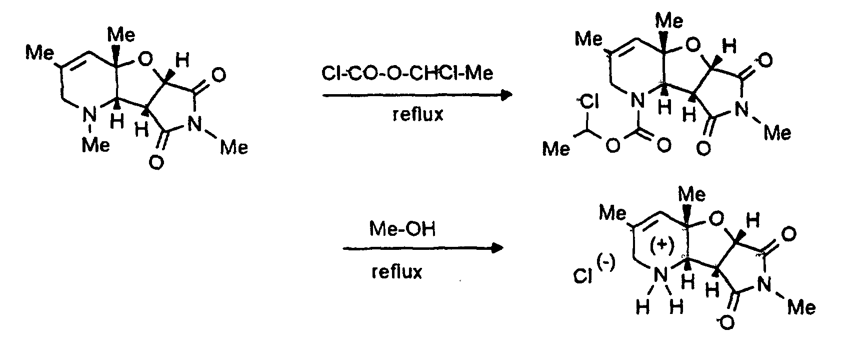

Zur Durchführung der N¹-Demethylierung werden die erfindungsgemäßen 1,2,4a, 5a,8a,8b-Hexahydro-6H-pyrrolo[3',4':4,5]furo[3,2-b]pyridin-6,8(7H)-dione der allgemeinen Formel (VI) in Gegenwart eines Überschusses an 1-Chlorethoxycarbonylchlorid erhitzt. Die Reaktionsdauer beträgt 1 bis 24 Stunden. Die N¹-Demethylierung wind bei Temperaturen zwischen 0°C und +200°C, bevorzugt bei Temperaturen zwischen +5°C bis +150°C, besonders bevorzugt bei Temperaturen zwischen +50°C und +130°C durchgeführt.

Die auf diese Weise erhaltenen 1-Chlorethoxycarbonyl-1,2,4a,5a,8a,8b-hexahydro-6H-pyrrolo[3',4':4,5]furo[3,2-b]pyridin-6,8(7H)-dione werden in üblicher Weise, beispielsweise durch chromatographische Reinigung, aufgearbeitet (vgl. auch die Herstellungsbeispiele).

Anschließend werden die 1-Chlorethoxycarbonyl-1,2,4a,5a,8a,8b-hexahydro-6H-pyrrolo[3',4':4,5]furo[3,2-b]pyridin-6,8(7H)-dione in einem der beim nachfolgenden Verfahren 3a angegebenen Verdünnungsmittel umsetzt.

Als Verdünnungsmittel zur N¹-Demethylierung werden bevorzugt inerte organische Lösungsmittel, wie z. B. Alkohole, insbesondere Methanol oder Ethanol verwendet.

Die Reaktionsdauer beträgt 10 Minuten bis 24 Stunden. Die Umsetzung erfolgt bei Temperaturen zwischen 0°C und +100°C, bevorzugt bei Temperaturen zwischen + 10 °C bis +80 °C. Es kann grundsätzlich unter Normaldruck gearbeitet werden.

Nach vollendeter Umsetzung wird das entstandene Hydrochlorid in üblicher Weise, aufgearbeitet (vgl. auch die Herstellungsbeispiele).

Setzt man bei Verfahren 5a zur Herstellung der neuen 1-substituierten 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Ethyl-1, 2, 3, 4, 4aα, 5aα, 8aα,8bα-(±)-oktahydro-3α,4a-dimethyl-6H-pyrrolo [3',4':4,5]furo[3,2-b]pyridin-6,8(7H)dion und als Verbindungen der allgemeinen Formel (VII) Methyliodid ein, so läßt sich das Verfahren durch folgendes Reaktionsschema wiedergeben:

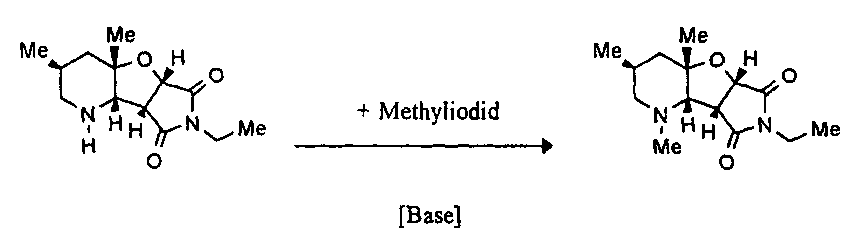

Die zur Durchführung des erfindungsgemäßen Verfahrens 5a als Ausgangsstoffe benötigten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione sind durch die Formel (Id) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel (Ib) als bevorzugt für diese Substituenten genannt werden.

Die als Ausgangsmaterialien verwendeten 1, 2, 3, 4, 4aα, 5aα, 8aα, 8bα-(±)Oktahydro-3α,4a-dialkyl-6H-pyrrolo[3',4':4,5]furo[3,2-b]pyridin-6,8(7H)-dione der allgemeinen Formel (Id) sind neu und können nach dem weiter oben beschriebenen Hydrierungsverfahren aus den 4a, 5a, 8a, 8b-Tetrahydro-3,4a-dialkyl-6H-pyrrolo[3',4': 4,5]furo[3,2-b]pyridin-6,8(7H)-dion Derivaten (Ia) erhalten werden.

Die außerdem für die Durchführung des erfindungsgemäßen Verfahrens 5a als Ausgangsstoffe zu verwendenden Alkylierungs- oder Heteroarylierungsmittel (R⁵ = Heteroaryl) sind allgemein durch die Formel (VII) definiert.

In der Formel (VII) hat R⁵ die Bedeutung die bereits im Zusammenhang mit der Beschreibung der erfindungsgemäßen Stoffe der allgemeinen Formel (Ib) als bevorzugt für diese Substituenten genannt wurde und E hat die Bedeutung einer elektronenziehenden Abgangsgruppe.

Geeignete Abgangsgruppen sind z. B. Halogen wie Fluor, Chlor, Brom und Iod, Sulfonat wie Aryl- und Perfluoralkylsulfonat, monosubstituiertes Diazo und monosubstituiertes Nitrato, sowie die zusätzlich in J. March, Advanced Organic Chemistry, 3rd ed., John Wiley & Sons, New York 1985, s. 310-316 aufgeführten.

Alkylierungs- oder Heteroarylierungsmittel der Formel (VII) sind allgemein bekannte Verbindungen der organischen Chemie bzw. können kommerziell oder nach literaturbekannten Methoden erhalten werden (z.B.: Houben-Weyl, Methoden der organischen Chemie, Band V/3, S. 830, 862; Band V/4 , S. 361, 610).

Im allgemeinen ist es vorteilhaft das erfindungsgemäße Verfahren 5a in Gegenwart von Verdünnungssmitteln und gegebenenfalls in Gegenwart eines basischen Reaktionshilfsmittels durchzuführen.

Als Verdünnungsmittel zur Durchführung des erfindungsgemäßen Verfahrens 3a kommen alle inerten organischen Lösungsmittel in Frage.

Bevorzugte Verdünnungsmittel sind Ketone, insbesondere Aceton, Methylethylketon oder Methylbutylketon, Amide, insbesondere N,N-Dimethylformamid, N,N-Dimethylacetamid oder N-Methyl-pyrrolidon, Benzinfraktionen innerhalb eines Siedepunktintervalles von 70°C bis 190°C, insbesondere Benzol, Toluol, Chlorbenzol, Brombenzol, Nitrobenzol oder Xylol sowie Gemische von diesen mit anderen genannten Verdünnungsmitteln.

Selbstverständlich kann man in das erfindungsgemäße Verfahren 5a auch Gemische der genannten Lösungs- und Verdünnungsmittel einsetzen.

Als basische Reaktionshilfsmittel zur Durchführung der effindungsgemäßen Verfahrens 5a können alle geeigneten Säurebindemittel eingesetzt werden wie Amine, insbesondere tertiäre Amine sowie Alkali- und Erdalkaliverbindungen.

Beispielhaft seien dafür erwähnt die Hydride, Hydroxide, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triazabicyclo(4.4.0)dec-5-en (MTBD); Diazabicyclo(4.3.0)nonen (DBN), Diazabicyclo(2.2.2)-octan (DABCO), 1,8-Diazabicyclo(5.4.0)undecen (DBU) Cyclohexyltetrabutylguanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyltoluidin, N,N-Dimethyl-p-aminopyridin, N-Methyl-pyrrolidin, N-Methylpiperidin, N-Methylimidazol, N-Methylpyrrol, N-Methyl-morpholin, N-Methylhexamethylenimin, Pyridin, 4-Pyrrolidinopyridin, 4-Dimethylamino-pyridin, Chinolin, α-Picolin, β-Picolin, Isochinolin, Pyrimidin, Acridin, N,N,N',N'-Tetramethylendiamin, N,N',N'-Tetraethylendiamin, Chinoxalin, N-Propyl-diisopropylamin, N-Ethyl-diisopropylamin, N,N'-Dimethylcyclohexylamin, 2,6-Lutidin, 2,4-Lutidin oder Triethylendiamin.

Vorzugsweise finden tertiären Amine, insbesondere Trialkylamine wie Triethylamin, N,N-Diisopropylethylamin, N-Propyl-diisopropylamin, N,N'-Dimethyl-cyclohexylamin oder N-Methylmorpholin sowie Alkalimetallcarbonate, insbesondere Natriumhydrogencarbonat, Natriumcarbonat, Kaliumcarbonat oder Kaliumhydrogencarbonat Verwendung.

Das Verfahren 5a wird durchgeführt, indem man Verbindungen der allgemeinen Formel (Id) in Gegenwart eines basischen Reaktionshilfsmittels mit Verbindungen der allgemeinen Formel (VII) in einem der angegebenen Verdünnungsmittel umsetzt.

Die Reaktionsdauer beträgt 4 bis 72 Stunden. Die Umsetzung erfolgt bei Temperaturen zwischen -10°C und +250°C, bevorzugt zwischen 0°C und +200°C, besonders bevorzugt bei 0°C bis 150°C. Es wird unter Normaldruck gearbeitet. Zur Durchführung des erfindungsgemäßen Verfahrens 5a setzt man pro Mol an Verbindung der Formel (Id) im allgemeinen 1,0 bis 6,0 Mol, vorzugsweise 1,0 bis 4,0 Mol basischen Reaktionshilfsmittels und 1,0 bis 4,0 Mol, vorzugsweise 1,0 bis 2,0 Mol Alkylierungsmittel (VII), ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Gegebenenfalls kann zur Darstellung von Verbindungen der allgemeinen Formel (Ib) und (Ic) für das erfindungsgemäße Verfahren 5a auch die literaturbekannte Aminoalkylierung oder reduktive Aminoalkylierung (vgl. Houben-Weyl, Methoden der Organischen Chemie, Band XI/I, S. 648; W. S. Emerson, Org. Reactions 4 (1948), S. 174) in Frage kommen.

Setzt man bei Verfahren 5b zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Ethyl-1, 2, 3, 4, 4aα, 5aα, 8aα, 8bα-(±)-oktahydro-3α,4a-dimethyl-6H-pyrrolo[3',4':4,5]furo[3,2-b] pyridin-6,8(7H)dion und als Verbindung der allgemeinen Formel (VIII) Acrylnitril ein, so läßt sich das Verfahren durch folgendes Reaktionsschema wiedergeben:

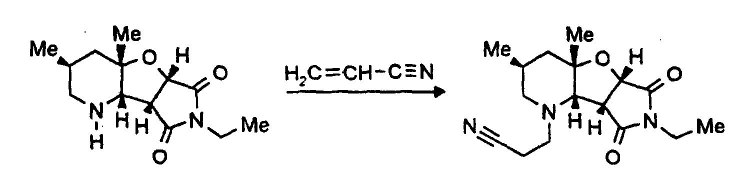

Die zur Durchführung des erfindungsgemäßen Verfahrens 5b als Ausgangsstoffe benötigten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione sind durch die Formel ( Id ) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel ( Ib ) als bevorzugt für diese Substituenten genannt werden.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione der allgemeinen Formel ( Id ) sind neu und können nach dem weiter oben beschriebenen Hydrierungsverfahren aus den 4a, 5a, 8a, 8b-Tetrahydro-3,4a-dialkyl-6H-pyrrolo [3',4': 4,5]furo [3,2-b]pyridin-6,8(7H)-dion Derivaten ( Ia ) erhalten werden.

Die außerdem für die Durchführung des erfindungsgemäßen Verfahrens 5b als Ausgangsstoffe zu verwendenden Verbindungen sind allgemein durch die Formel. (VIII) definiert.

In der Formel (VIII) hat R⁸ die Bedeutung die bereits im Zusammenhang mit der Beschreibung der erfindungsgemäßen Stoffe der allgemeinen Formel (Ib) als bevorzugt für diese Substituenten genannt wurden und A steht für eine geeignete Akzeptorgruppe, beispielsweise für Nitro, Nitril, Carbamoyl, Carboxyl oder C₁₋₄-Alkoxycarbonyl .

Die Verbindungen der Formel (VIII) sind allgemein bekannte Verbindungen der organischen Chemie bzw. können z. T. kommerziell oder nach literaturbekannten Methoden erhalten werden (z. B.: Houben-Weyl, Methoden der Organischen Chemie, Band VIII, S. 265).

Die Umsetzung der Verbindungen (Id) mit (VIII) führt man gegebenenfalls in Gegenwart eines Katalysators unter Verwendung von Verdünnungsmitteln durch.

Als Verdünnungsmittel zur Durchführung des erfindungsgemäßen Verfahrens 5b finden die bei Verfahren 3a genannten inerten Lösungsmittel wie z. B. Alkohole, insbesondere Methanol oder Ethanol, Verwendung.

Das Verfahren 5b wird durchgeführt, indem man Verbindungen der allgemeinen Formel (Id) gegebenenfalls in Gegenwart eines geeigneten Katalysators mit einer Verbindung allgemeinen Formel (VIII) in einem der angegebenen Verdünnungsmittel umsetzt.

Die Reaktionsdauer beträgt 4 bis 72 Stunden. Die Umsetzung erfolgt bei Temperaturen zwischen -10°C und +200°C, bevorzugt zwischen -5°C und +150°C, besonders bevorzugt bei 0°C bis 100. Es kann grundsätzlich unter Normaldruck gearbeitet, aber auch bei erhöhtem oder emiedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Zur Durchführung des erfindungsgemäßen Verfahrens 5b setzt man pro Mol an Verbindung der Formel (Id) im allgemeinen 1,0 bis 3,0 Mol, vorzugsweise 1,0 bis 2,0 Mol an Verbindung der Formel (VIII), ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 5c zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Ethyl-1, 2, 3, 4, 4aα, 5aα, 8aα, 8bα-(±)-oktahydro-3α,4a-dimethyl-6H-pyrrolo[3',4':4,5]furo[3,2-b]pyridin-6, 8(7H)dion und als Epoxid der allgemeinen Formel (IX) (±)-2,3-Epoxypropylisopropylether ein, so läßt sich das Verfahren durch folgendes Reaktionsschema wiedergeben:

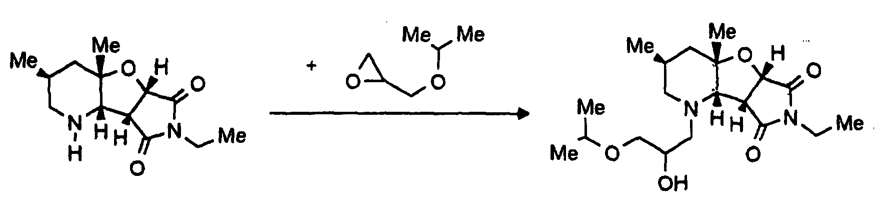

Die zur Durchführung des erfindungsgemäßen Verfahrens 5c als Ausgangsstoffe benötigten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione sind durch die Formel (Id) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel (Ib) als bevorzugt für diese Substituenten genannt werden.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione der allgemeinen Formel (Id) sind neu und können nach dem weiter oben beschriebenen Hydrierungsverfahren aus den 4a, 5a, 8a, 8b-Tetrahydro-3,4a-dialkyl-6H-pyrrolo[3',4': 4,5]furo [3,2-b]pyridin-6,8(7H)-dion Derivaten (Ia) erhalten werden.

Die außerdem für die Durchführung des erfindungsgemäßen Verfahrens 5c als Ausgangsstoffe zu verwendenden Epoxide sind allgemein durch die Formel (IX) definiert.

In der Formel (IX) hat R⁵ die Bedeutung die bereits im Zusammenhang mit der Beschreibung der erfindungsgemäßen Stoffe der allgemeinen Formel (Ib) als bevorzugt für diese Substituenten genannt wurden.

Die Epoxide der Formel (IX) sind allgemein bekannte Verbindungen der organischen Chemie bzw. können z. T. kommerziell oder nach literaturbekannten Methoden erhalten werden (z. B.: Houben-Weyl, Methoden der Organischen Chemie, Band VI/3, S. 371; D. Swern, Org. Reactions 7 (1953), S. 378).

Die Umsetzung der Verbindungen (Id) mit (IX) führt man gegebenenfalls in Gegenwart eines Katalysators oder in Gegenwart eines basischen Reaktionshilfsmittels unter Verwendung von Verdünnungsmitteln durch.

Als Verdünnungsmittel zur Durchführung des erfindungsgemäßen Verfahrens 5c finden die bei Verfahren 3a genannten inerten, aprotischen Lösungsmittel wie z.B. Ether, insbesondere Methyl-tert.-butylether, Tetrahydrofuran oder Dioxan, Alkohole, insbesondere Methanol, Verwendung.

Als gegebenenfalls eingesetztes Reaktionshilfsmittel zur Durchführung des erfindungsgemäßen Verfahrens Sc können alle bei Verfahren 5a genannten basischen Reaktionshilfsmittel, insbesondere Alkalimetallhydride, Verwendung finden.

Das Verfahren 5c wird durchgeführt, indem man Verbindungen der allgemeinen Formel (Id) gegebenenfalls in Gegenwart eines Reaktionshilfsmittels mit einem Epoxid der allgemeinen Formel (IX) in einem der angegebenen Verdünnungsmittel umsetzt.

Die Reaktionsdauer beträgt 4 bis 72 Stunden. Die Umsetzung erfolgt bei Temperaturen zwischen -10°C und +200°C, bevorzugt zwischen -5°C und +150°C, besonders bevorzugt bei 0°C bis 100°C. Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Zur Durchführung des erfindungsgemäßen Verfahrens Sc setzt man pro Mol an Verbindung der Formel (Id) im allgemeinen 0,5 bis 2,0 Mol, vorzugsweise 0,5 bis 1,5 Mol Epoxid, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 5d zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Methyl-1, 2, 3, 4, 4aα, 5aα, 8aα, 8bα-(±)-oktahydro-3α,4a-dimethyl-6H-pyrrolo[3',4':4,5]furo[3,2-b]pyri-din-6,8(7H)dion und als Verbindungen der allgemeinen Formel (X) Chlorameisensäureallylester ein, so läßt sich das Verfahren durch folgendes Reaktionsschema wiedergeben:

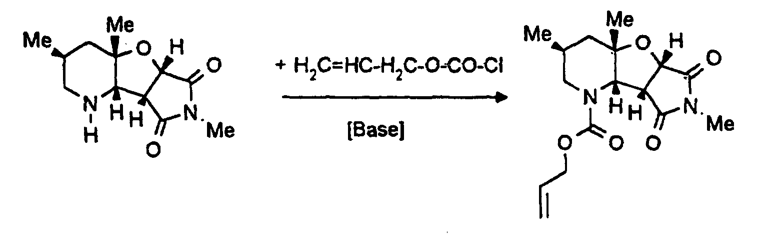

Die zur Durchführung des erfindungsgemäßen Verfahrens 5d als Ausgangsstoffe benötigten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione sind durch die Formel (Id) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel (Ib) als bevorzugt für diese Substituenten genannt werden.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione der allgemeinen Formel (Id) sind neu und können nach dem weiter oben beschriebenen Hydrierungsverfahren aus den 4a, 5a, 8a, 8b-Tetrahydro-3,4a-dialkyl-6H-pyrrolo [3', 4': 4,5]furo [3,2-b]pyridin-6,8(7H)-dion Derivaten (Ia) erhalten werden.

Die außerdem für die Durchführung des erfindungsgemäßen Verfahrens 5d als Ausgangsstoffe zu verwendenden Verbindungen sind allgemein durch die Formel (X) definiert.

In der Formel (X) haben G, X, Y, und W die Bedeutung die bereits im Zusammenhang mit der Beschreibung der erfindungsgemäßen Stoffe der allgemeinen Formel (Ib) als bevorzugt für diese Substituenten genannt wurden.

Die Verbindungen der Formel (X) sind allgemein bekannte Verbindungen der organischen Chemie bzw. können z. T. kommerziell oder nach literaturbekannten Methoden erhalten werden (z.B.: persubstituierte Allophansäurehalogenide: DE-OS 2 008 116; Carbamoylchloride: Liebigs Ann. 299, S. 85; Carbamate: Houben-Weyl, Methoden der organischen Chemie, Band E 4).

Die Umsetzung der Verbindungen (Id) mit (X) führt man vorzugsweise in Gegenwart eines basischen Reaktionshilfsmittels unter Verwendung von Verdünnungsmitteln durch.

Als Verdünnungsmittel zur Durchführung des erfindungsgemäßen Verfahrens 5d finden die bei Verfahren 3a genannten inerten, aprotischen Lösungsmittel wie z.B. Dioxan, Acetonitril oder Tetrahydrofuran aber auch Halogenkohlenwasserstoffe, insbesondere Chlorkohlenwasserstoffe, wie Methylenchlorid, Verwendung.

Als basische Reaktionshilfsmittel zur Durchführung des erfindungsgemäßen Verfahrens 5d können alle bei Verfahren 5a genannten Säurebindemittel, vorzugsweise jedoch tertiären Amine, insbesondere Trialkylamine wie Triethylamin, N,N-Diisopropylethylamin, N-Propyldiisopropylamin, N,N'-Dimethylcyclohexylamin oder N-Methylmorpholin, Verwendung finden.

Das Verfahren 5d wird durchgeführt, indem man Verbindungen der allgemeinen Formel (Id) in Gegenwart eines basischen Reaktionshilfsmittels mit Verbindungen der allgemeinen Formel (X) in einem der angegebenen Verdünnungsmittel umsetzt.

Die Reaktionsdauer beträgt 4 bis 72 Stunden. Die Umsetzung erfolgt bei Temperaturen zwischen -10°C und +150°C, bevorzugt zwischen -5°C und +80°C, besonders bevorzugt bei 0°C bis Raumtemperatur. Es wird unter Normaldruck gearbeitet. Zur Durchführung des erfindungsgemäßen Verfahrens 5b setzt man pro Mol an Verbindung der Formel (Id) im allgemeinen 1,0 bis 3,0 Mol, vorzugsweise 1,0 bis 1,5 Mol Acylierungsagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Bei den nach Verfahren 5d dargestellten Verbindungen mit einem Halogenacyl-(G=X für Carbonyl) oder Halogensulfonylrest (G=X für Sulfonyl), kann der Halogenrest nach üblichen Verfahren in einer Folgereaktion mit geeigneten Nucleophilen der allgemeinen Formel (XVIII) (R¹¹-YH), beispielsweise mit Amino-, Hydroxy- oder Mercaptoverbindungen, substituiert werden.

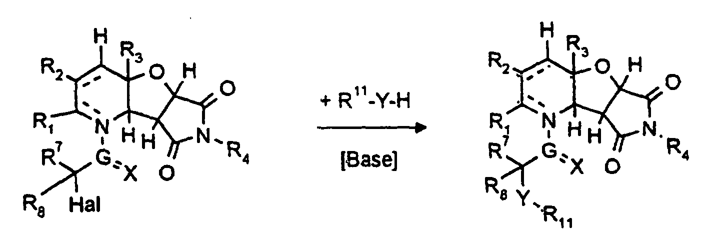

Der Halogenaustausch erfolgt dabei unter den dafür üblichen Reaktionsbedingungen zumeist in Gegenwart basischer Reaktionshilfsmittel und kann gegebenenfalls mittels Katalysatoren beschleunigt werden (vgl. auch Herstellungsbeispiele).

Setzt man bei Verfahren 5e zur Herstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln (Ib) und (Ie) als Verbindungen der allgemeinen Formeln (Id) und (Ie) 7-Methyl-1, 2, 3, 4, 4aα, 5aα, 8aα, 8bα-(±)-oktahydro-3a,4a-dimethyl-6H-pyrrolo[3',4':4,5]furo[3,2-b]pyridin-6,8(7H)dion bzw. 7-Methyl-1, 2, 4aα, 5aα, 8aα, 8bα-(±)-Hexahydro-3,4a-dimethyl-6H-pyrrolo[3',4':4,5]furo[3,2-b]pyridin-6,8(7H)dion und als Carbonsäureanhydrid der allgemeinen Formel (XI) Diglycolsäureanhydrid ein, so läßt sich das Verfahren durch folgende Reaktionsschemen wiedergeben: 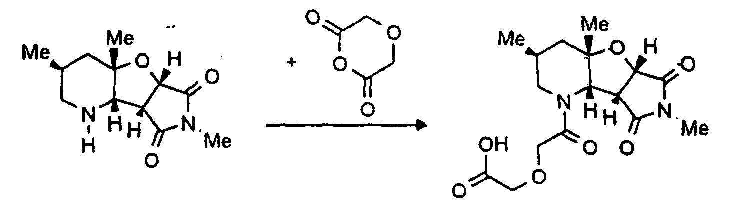 und/oder

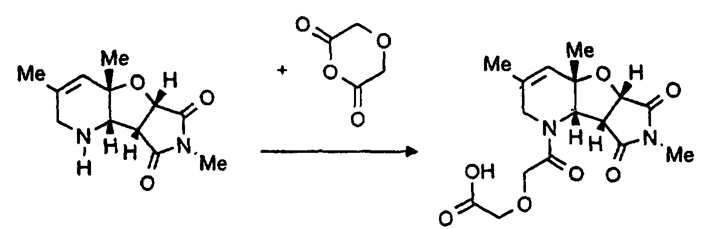

Die zur Durchführung des erfindungsgemäßen Verfahrens 5e als Ausgangsstoffe benötigten 1, 2, 3, 4, 4aα, 5aα, 8aα, 8bα-(±)-oktahydro-3α,4a-dialkyl-6H-pyrrolo[3',4':4,5]furo[3,2-b]pyridin-6,8(7H)-dione und 1, 2, 4aα, 5aα, 8aα, 8bα-(±)-Hexahydro-3,4a-dialkyl-6H-pyrrolo[3',4':4,5]furo[3,2-b]pyridin-6,8(7H)dion sind durch die Formel (Id) und (Ie) allgemein definiert. In diesen Formeln stehen R¹, R², R³ und R⁴ vorzugsweise für diejenigen Reste, die bereits im Zusammenhang mit der Beschreibung der erfindungsgemäßen Stoffe der allgemeinen Formeln (Ib) und (Ic) als bevorzugt für diese Substituenten genannt werden.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione und 1, 2, 4aα, 5aα, 8aα, 8bα-(±)-Hexahydro-3,4a-dialkyl-6H-pyrrolo[3',4':4,5]furo[3,2-b]pyridin-6,8(7H)dion der allgemeinen Formeln (Id) und (Ie) sind neu und können nach den weiter oben beschriebenen Hydrierungsverfahren aus den 4a, 5a, 8a, 8b-Tetrahydro-3,4-dialkyl-6H-pyrrolo[3',4':4,5]furo[3,2-b]pyridin-6,8(7H)-dion Derivaten (Ia) erhalten werden.

Die außerdem für die Durchführung des erfindungsgemäßen Verfahrens 5e als Ausgangsstoffe zu verwendenden Carbonsäureanhydride sind allgemein durch die Formel (XI) definiert.

In der Formel (XI) bat Q die Bedeutung die bereits im Zusammenhang mit der Beschreibung der erfindungsgemäßen Stoffe der allgemeinen Formel (Ib) als bevorzugt für diese Substituenten genannt wurden.

Die Carbonsäureanhydride der Formel (XI) sind allgemein bekannte Verbindungen der organischen Chemie bzw. können z. T. kommerziell oder nach literaturbekannten Methoden erhalten werden (z.B.: Houben-Weyl, Methoden der organischen Chemie, Band VII/4, S. 120; Band VIII, S. 477).

Die Umsetzung der Verbindungen (Id) und/oder (Ie) mit (XI) führt man gegebenenfalls in Gegenwart eines Reaktionshilfsmittels unter Verwendung von Verdünnungsmitteln durch.

Als Verdünnungsmittel zur Durchführung des erfindungsgemäßen Verfahrens 5e finden die bei Verfahren 3a genannten inerten, aprotischen Lösungsmittel wie z.B. Ether, insbesondere Methyl-tert.-butylether, Tetrahydrofuran oder Dioxan, Verwendung.

Als gegebenenfalls eingesetztes Reaktionshilfsmittel zur Durchführung des erfindungsgemäßen Verfahrens 5e können alle bei Verfahren 5a genannten Säurebindemittel, vorzugsweise jedoch tertiären Amine, insbesondere Trialkylamine wie Triethylamin, N,N-Diisopropylethylamin, N-Propyldiisopropylamin, N,N'-Dimethyl-cyclohexylamin oder N-Methylmorpholin, Verwendung finden.

Das Verfahren 5e wird durchgeführt, indem man Verbindungen der allgemeinen Formeln (Id) und/oder (Ie) in gegebenenfalls Gegenwart eines Katalysators mit einem Carbonsäureanhydrid der allgemeinen Formel (XI) in einem der angegebenen Verdünnungsmittel umsetzt.

Als Katalysatoren können dabei sowohl die bei Verfahren 5a genannten basischen Reaktionshilfsmittel als auch saure Katalysatoren in Frage kommen. Als solche seien praktisch alle Mineralsäuren oder Lewis-Säuren genannt. Zu den Mineralsäuren gehören gehören vorzugsweise Halogenwasserstoffsäuren wie Fluorwasserstoffsäure, Bromwasserstoffsäure oder Iodwasserstoffsäure sowie Schwefelsäure, Phosphorsäure, Phosphorige Säure, Salpetersäure und zu den Lewis-Säuren gehören vorzugsweise Aluminiumchlorid, Bortrifluorid oder sein Etherat, Titan(IV)-chlorid, Zinn(IV)-chlorid.

Die Reaktionsdauer beträgt 4 bis 72 Stunden. Die Umsetzung erfolgt bei Temperaturen zwischen -10°C und +250°C, bevorzugt zwischen -5°C und +200°C, besonders bevorzugt bei 0°C bis 150°C. Es wird unter Normaldruck gearbeitet. Zur Durchführung des erfindungsgemäßen Verfahrens 5e setzt man pro Mol an Verbindungen der Formeln (Id) und/oder (Ie) im allgemeinen 1,0 bis 3,0 Mol, vorzugsweise 1,0 bis 1,5 Mol Carbonsäureanhydrid, ein.

Alternativ kann das Verfahren 5e auch mit überschüssigem Carbonsäureanhydrid der Formel (XI) ohne ein Verdünnungsmittel durchgeführt werden, sofern das Reaktionsgemisch gut rührbar bleibt.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 5f zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Methyl-1, 2, 3, 4, 4aα, 5aα, 8aα, 8bα-(±)-oktahydro-3α,4a-dimethyl-6H-pyrrolo[3',4':4,5]furo[3,2-b]pyri-din-6,8(7H)dion und als Aminosäurederivate der allgemeinen Formel (XII) N-Benzyl-oxycarbonylsarkosin (Z-Sar-OH) ein, so läßt sich das Verfahren durch folgendes Reaktionsschema wiedergeben:

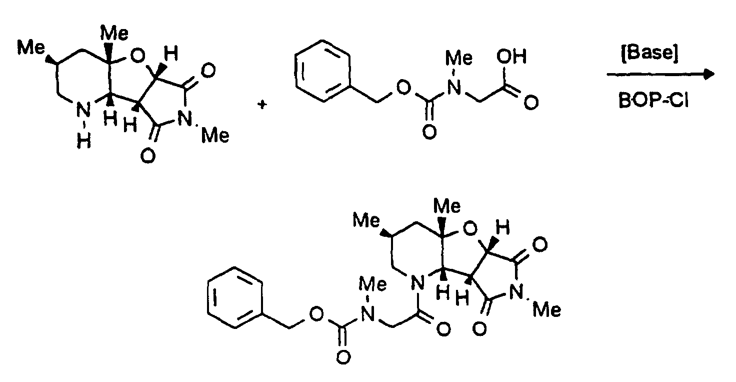

Die zur Durchführung des erfindungsgemäßen Verfahrens 5f als Ausgangsstoffe benötigten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione sind durch die Formel ( Id ) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel ( Ib ) als bevorzugt für diese Substituenten genannt werden.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione der allgemeinen Formel ( Id ) sind neu und können nach dem weiter oben beschriebenen Hydrierungsverfahren aus den 4a, 5a, 8a, 8b-Tetrahydro-3,4a-dialkyl-6H-pyrrolo[3',4': 4,5]furo[3,2-b]pyridin-6,8(7H)-dion Derivaten ( Ia ) erhalten werden.

Die außerdem für die Durchführung des erfindungsgemäßen Verfahrens 5f als Ausgangsstoffe zu verwendenden Aminosäurederivate sind allgemein durch die Formel (XII) definiert.

In der Formel (XII) haben G, Q, X, R⁶, R⁷ und R⁸ die Bedeutung die bereits im Zusammenhang mit der Beschreibung der erfindungsgemäßen Stoffe der allgemeinen Formel (Ib) als bevorzugt für diesen Substituenten genannt wurden.

Die als Ausgangsstoffe verwendeten natürlichen oder synthetischen Aminosäuren können, falls chiral, in der (S)- oder (R)-Form (bzw. L- oder D-Form) vorliegen.

Beispielsweise seien genannt:
Aad, Abu, jAbu, ABz, 2ABz, εAca, Ach, Acp, Adpd, Ahb, Aib, ßAib, Ala, ßAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, HyI, Hyp, 3Hyp, Ile, Ise, Iva, Kyn, Lant, Lcn, Leu, Lsg, Lys, ßLys, ΔLys, Met, Mim, Min, nArg, Nle, Nva, Oly, Orn, Pan, Pec, Pen, Phe, Phg, Pic, Pro, ΔPro, Pse, Pse, Pya, Pyr, Pza, Qin, Ros, Sar, Sec, Sem, Ser, Thi, ßThi, Thr, Thy, Thx, Tia, Tle, Tly, Trp, Trta, Tyr, Val, Nal, Tbg, Npg, Chg, Thia (vgl. z. B. Houben-Weyl, Methoden der Organischen Chemie, Band XV/1 und 2, Stuttgart, 1974).

Die Verbindungen der Formel (XII) können z. T. kommerziell oder nach literaturbekannten Methoden erhalten werden (vgl. z. B.: N-Methylaminosäuren: R. Bowmann et al. J. Chem. Soc. (1950) S. 1346; J. R. McDermott et al Can J. Chem. 51 (1973) S. 1915; H. Wurziger et al. Kontakte (Merck, Darmstadt) 3 (1987) S. 8).

Die Umsetzung der 1, 2, 3, 4, 4aα, 5aα, 8aα, 8bα-(±)-Oktahydro-3α,,4a-dialkyl-6H-pyrrolo[3',4':4,5]furo[3,2-b]pyridin-6,8(7H)-dione der allgemeinen Formel ( Ic ) mit Aminosäurederivaten der Formel (XII) führt man vorzugsweise in Gegenwart von Kupplungsreagenzien und in Gegenwart eines basischen Reaktionshilfsmittels unter Verwendung von Verdünnungsmitteln durch.

Als Kupplungsreagenzien zur Durchführung des Verfahrens 5f finden alle, die zur Herstellung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis Synthesis, Biology (Academic Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid, N-Hydroxy-5-norbornen-2,3-dicarbox-amid (HONB), 1-Hydroxy-benzotriazol (HOBt) oder 3-Hydroxy-4-oxo-3,4-dihydro-1,2,3-benzotriazin als Alkoholkomponente, Kupplung mit Carbodiimiden wie Dicyclohexylcarbodiimid (DCC) nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutyl-chlorformiat (IIDQ) oder Kupplung mit Phosphoniumreagenzien, wie Benzotriazol-1-yl-oxy-tris(dimethylamino-phosphonium)-hexafluorophosphat (BOP), Bis(2-oxo-3-oxazolidinyl)phosphoniumsäurechlorid (BOP-Cl), oder mit Phosphonsäureesterreagenzien, wie Cyanphosphonsäurediethylester (DEPC) und Diphenylphosphorylazid (DPPA) oder Uroniumreagenzien, wie 2-(lH-Benzotriazol-1-yl)-1,1,3,3-tetra-methyluronium-tetrafluoroborat (TBTU).

Bevorzugt ist die Kupplung mit Phosphoniumreagenzien wie Bis(2-oxo-3-oxazolidinyl)-phosphoniumsäurechlorid (BOP-Cl), Benzotriazol-1-yl-oxy-tris(dimethylamino-phosphonium)-hexafluorophosphat (BOP) und Phosphonsäureesterreagenzien, wie Cyanphosphonsäurediethylester (DEPC) oder Diphenylphosphorylazid (DPPA).

Als basische Reaktionshilfsmittel zur Durchführung der erfindungsgemäßen Verfahrens 5f können ebenso alle für das Verfahren 5a geeigneten Säurebindemittel eingesetzt werden.

Vorzugsweise kommen tertiären Amine, insbesondere Trialkylamine wie Triethylamin, N,N-Diisopropylethylamin, N-Propyldiisopropylamin, N,N'-Dimethylcyclohexylamin oder N-Methylmorpholin in Frage.

Als Verdünnungsmittel zur Durchführung des erfindungsgemäßen Verfahrens 5f finden die bei Verfahren 3a genannten Lösungsmittel wie z. B. Halogenkohlenwasserstoffe, insbesondere Chlorkohlenwasserstoffe, wie Methylenchlorid oder 1,2-Dichlorethan und Gemische von diesen mit anderen genannten Verdünnungsmitteln, Verwendung.

Das Verfahren 5f wird im allgemeinen so durchgeführt, indem man Verbindungen der Formel (Id) in Gegenwart eines der angegebenen Kupplungsreagenzien und in Gegenwart eines der angegebenen basischen Reaktionshilfsmittel mit Verbindungen der allgemeinen Formel (XII) in einem der angegebenen Verdünnungsmittel umsetzt. Die Reaktionsdauer beträgt 4 bis 72 Stunden. Die Umsetzung erfolgt bei Temperaturen zwischen -10°C und +120°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 5f setzt man pro Mol an 1, 2, 3, 4, 4aα, 5aα, 8aα, 8bα-(±)-Oktahydro-3α,4a-dialkyl-6H-pyrrolo[3',4':4,5]furo[3,2-b] pyridin-6,8(7H)-dione der Formel ( Id ) im allgemeinen 1,0 bis 3,0 Mol, vorzugsweise 1,0 bis 1,5 Mol Kupplungsreagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 5g zur Herstellung der der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Methyl-1, 2, 3, 4, 4aα, 5aα, 8aα, 8bα-(±)-Oktahydro-3α,4a-dimethyl-6H-pyrrolo[3',4':4,5]furo[3,2-b]pyridin-6,8(7H)dion und als Verbindungen der allgemeinen Formel (XIII) Trichloracetylisocyanat ein, so läßt sich das Verfahren durch folgendes Reaktionsschema wiedergeben:

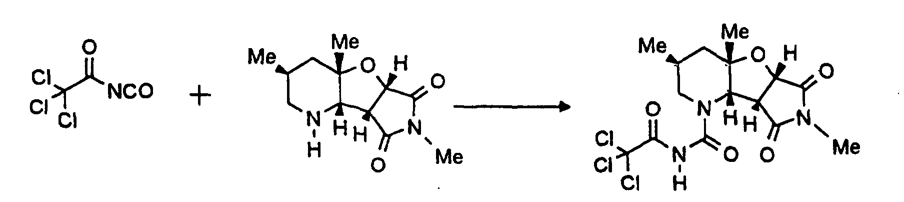

Die zur Durchführung des erfindungsgemäßen Verfahrens 5g als Ausgangsstoffe benötigten 1, 2, 3, 4, 4aα, 5aα, 8aα, 8bα-(±)-Oktahydro-3α, 4a-dialkyl-6H-pyrrolo[3',4':4,5]furo[3,2-b]pyridin-6,8(7H)-dione sind durch die Formel (Id) allgemein definiert. In dieser Formel stehen R¹, R², R³ und R⁴ vorzugsweise für diejenigen Reste, die bereits im Zusammenhang mit der Beschreibung der erfindungsgemäßen Stoffe der allgemeinen Formel (Ib) als bevorzugt für diese Substituenten genannt werden.

Die als Ausgangsmaterialien verwendeten 1, 2, 3, 4, 4aα, 5aα, 8aα, 8bα-(±)-Oktahydro-3α, 4a-dialkyl-6H-pyrrolo[3',4':4,5]furo[3,2-b]pyridin-6,8(7H)-dione der allgemeinen Formel (Id) sind neu und können nach dem weiter oben beschriebenen Hydrierungsverfahren aus den 4a, 5a, 8a, 8b-Tetrahydro-3,4a-dialkyl-6H-pyrrolo[3',4': 4,5]furo[3,2-b]pyridin-6,8(7H)-dion Derivaten ( Ia ) erhalten werden.

Die außerdem für die Durchführung des erfindungsgemäßen Verfahrens 5g als Ausgangsstoffe zu verwendenden Verbindungen sind allgemein durch die Formeln (XIII) und (XIV) definiert.

In den Formeln (XIII) und (XIV) haben R¹¹, Y, G¹, X¹ und X die Bedeutung die be-reits im Zusammenhang mit der Beschreibung der erfindungsgemäßen Stoffe der allgemeinen Formel (Ib) als bevorzugt für diesen Substituenten genannt wurden.

Die Verbindungen der Formeln (XIII) und (XIV) sind allgemein bekannte Verbindungen der organischen Chemie, können z. T. kommerziell oder nach literaturbekannten Methoden erhalten werden (Houben-Weyl, Methoden der organischen Chemie, Band E 4).

Die Umsetzung der Verbindungen (Id) mit (XIII) oder (XIV) nach dem erfindungsgemäßen Verfahren 5f führt man vorzugsweise in Gegenwart von Verdünnungsmitteln, gegebenenfalls in Gegenwart eines basischen Reaktionshilfsmittels, durch.

Als Verdünnungsmittel zur Durchführung des erfindungsgemäßen Verfahrens 5g finden die bei Verfahren 3a genannten Lösungsmittel wie z. B. Nitrile wie Acetonitril, Propionitril, Butyronitril, insbesondere Acetonitril, und Ether wie Ethylpropylether, n-Butylether, Diethylether, Dipropylesther, Diisopropylether, Di-n-butylether, Diisobutylether, Diisoamylether, Tetrahydrofuran, Dioxan, insbesondere Terahydrofuran und Dioxan, Verwendung.

Das Verfahren 5g kann auch in Gegenwart von basischen Reaktionshilfsmitteln durchgeführt werden. Als solche basischen Reaktionshilfsmittel zur Durchführung des erfindungsgemäßen Verfahrens 5g können alle bei Verfahren 5a genannten Säurebindemittel, vorzugsweise jedoch tertiären Amine, insbesondere Trialkylamine wie Triethylamin, N,N-Diisopropylethylamin oder N-Methylmorpholin, und Amidinbasen oder Guanidinbasen wie Diazabicyclo-(4.3.0)nonen (DBN), Diazabicyclo (2.2.2)-octan (DABCO), 1,8-Diaza-bicyclo(5.4.0)-undecen (DBU) insbesondere 1,8-Diazabicyclo(5.4.0)-undecen (DBU), Verwendung.

Das Verfahren 5g wird durchgeführt, indem Verbindungen der allgemeinen Formel (Id) mit äquimolaren Mengen einer Verbindung der Formeln (XIII) oder (XIV) in einem der weiter oben angegebenen Verdünnungsmittel, gegebenenfalls in Gegenwart eines basischen Reaktionshilfsmittels, zusammengegeben werden. Die Reaktionsdauer beträgt 1 bis 72 Stunden. Die Reaktion wird bei Temperaturen zwischen -50°C bis +200°C, bevorzugt in einem Temperaturbereich zwischen -20°C und +150°C, insbesondere in einem Temperaturbereich zwischen -10°C und +120°C durchgeführt.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Nach vollendeter Umsetzung wird das Reaktionsgemisch nach allgemein üblichen Methoden aufgearbeitet (vgl. auch die Herstellungsbeispiele).

Verfahren zur Herstellung von organischen Carbamaten aus einem basisch reagierenden Amin, Kohlendioxid und einem Alkylierungsmittel in Gegenwart von basischen Alkalimetall-, Erdalkalimetall- oder Ammoniumsalzen sind bekannt (vgl. EP-OS 511 948, EP-OS 628 542 und dort zitierte Lit.).

Es wurde nun gefunden, daß auch die basischen, erfindungsgemäßen, 1, 2, 3, 4,4a,5a,8a,8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion-Derivate der allgemeinen Formeln (Id) und (Ie) als sekundäre Aminoverbindungen mit Kohlendioxid und einem Alkylierungsmittel in Gegenwart von Metallcarbonaten zu Carbamaten der allgemeinen Formel (Ib) und (Ic) reagieren.

Setzt man bei Verfahren 5h zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (Id) 7-Methyl-1, 2, 3, 4, 4aα, 5aα, 8aα, 8bα-(±)-Oktahydro-3α, 4a-dimethyl-6H-pyrrolo[3',4':4,5]furo[3,2-b]pyridin-6,8(7H)-dion, Kohlendioxid, Kaliumkarbonat und als Verbindungen der allgemeinen Formel (VIIa) Propargylbromid ein, so läßt sich das Verfahren durch folgendes Reaktionsschema wiedergeben:

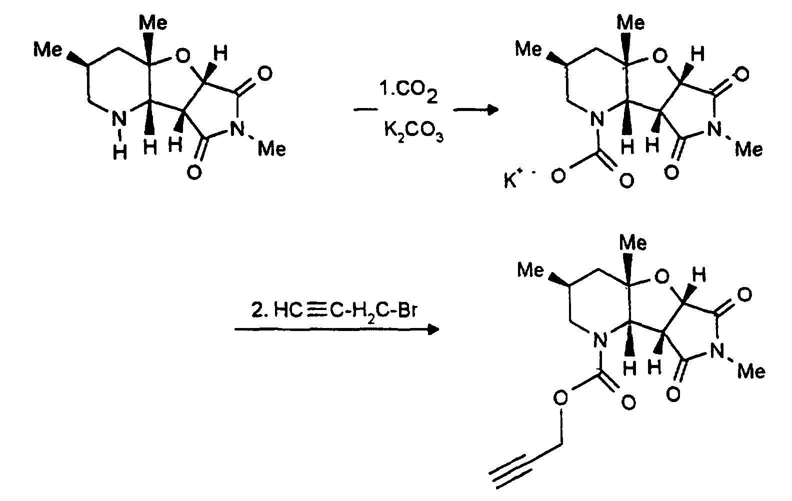

Bevorzugt werden bei Verfahren 5h die 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Id) eingesetzt, bei denen die Reste R¹, R², R³ und R⁴ die bei den Verbindungen der allgemeinen Formel (Ib) bevorzugten und besonders bevorzugten Bedeutungen besitzen.

Die als Ausgangsmaterialien verwendeten 1, 2, 3, 4, 4aα, 5aα, 8aα, 8bα-(±)-Oktahydro-3α,4a-dialkyl-6H-pyrrolo[3',4':4,5]furo[3,2-b]pyridin-6,8(7H)-dione der allgemeinen Formel (Id) sind neu und können nach dem weiter oben beschriebenen Hydrierungsverfahren aus den 4a, 5a, 8a, 8b-Tetrahydro-3,4-dialkyl-6H-pyrrolo[3',4' :4,5]furo [3,2-b]pyridin-6,8(7H)-dion Derivaten (Ia) erhalten werden.

Als Kohlendioxid kann in das erfindungsgemäße Verfahren das übliche Handelsprodukt, gegebenenfalls auch sogenanntes "Trockeneis", eingesetzt werden.

Die außerdem für die Durchführung des erfindungsgemäßen Verfahrens 5h als Ausgangsstoffe zu verwendenden Alkylierungsmittel der Formel (VIIa) sind allgemein bekannte Verbindungen der organischen Chemie.

In der Formel (VIIa) hat R¹¹ die Bedeutung die bereits im Zusammenhang mit der Beschreibung der erfindungsgemäßen Stoffe der allgemeinen Formel (Ib) als bevorzugt für diesen Substituenten genannt wurde und Hal hat die Bedeutung einer elektronenziehenden Abgangsgruppe.

Geeignete Abgangsgruppen sind z. B. Halogen wie Fluor, Chlor, Brom und Iod, Sulfonat wie Aryl- und Perfluoralkylsulfonat, monosubstituiertes Diazo und monosubstituiertes Nitrato, sowie die zusätzlich in J. March, Advanced Organic Chemistry, 3rd ed., John Wiley & Sons, New York 1985, s. 310-316 aufgeführten.

Als basisch reagierende Verbindungen können in der vorliegenden Erfindung eine oder mehrere basische Verbindungen der Elemente Lithium, Natrium, Magnesium, Kalium, Kalzium, Rubidium, Strontium, Cäsium, Barium, und/oder des Ammoniums Verwendung finden. Als basische Verbindungen kommen z.B. basisch reagierende Salze, Oxide, Hydride und Hydroxide in Frage. Beispielsweise seien genannt: Lithiumhydrid, Natriumhydrid, Kaliumhydrid, Kalziumhydrid, Lithiumhydroxid, Natriumhydroxid, Kaliumhydroxid, Rubidiumhydroxid, Magnesiumhydroxid, Kalziumhydroxid, Strontiumhydroxid, Bariumhydroxid, Lithiumoxid, Natriumperoxid, Kaliumoxid, Kaliumperoxid, Kalziumoxid, Bariumoxid, Magnesiumoxid, Strontiumoxid, Lithiumcarbonat, Lithiumhydrogencarbonat, Rubidiumcarbonat, Rubidiumhydrogencarbonat, Cäsiumhydrogencarbonat, Cäsiumcarbonat, Lithiumcyanid, Natriumcyanid, Kaliumcyanid, Rubidiumcyanid, Ammoniumhydrogencarbonat, Cäsiumcarbonat, Ammoni-umcarbamat, Kaliumsulfid, Kaliumhydrogensulfid, Natriumsulfid, Natriumhydrogensulfid und/oder deren natürlich vorkommende oder synthetisch erhältliche Gemische, wie beispielsweise Dolomit oder Magnesiumoxidcarbonat und/oder Verbindungen, die Natrium- oder Kaliummetall auf den entsprechenden Carbonaten in dispergierter Form enthalten.

Bevorzugt sind jedoch Alkalicarbonate und/oder -hydrogencarbonate, ganz besonders bevorzugt Cäsiumcarbonat oder Kaliumcarbonat.

Die basisch reagierenden Verbindungen können in wasserfreier Form oder, soweit es sich um Salze handelt, die mit Hydratwasser kristallisieren, auch in hydratisierter Form eingesetzt werden. Bevorzugt werden jedoch wasserfreie Verbindungen verwendet.

Als Verdünnungsmittel zur Durchführung des erfindungsgemäßen Verfahrens 5h finden die bei Verfahren 3a genannten Lösungsmittel wie z. B. Amide wie Hexamethylenphosphortriamid, N,N-Dimethylformamid, N,N-Dipropylformamid, N,N-Dibutylformamid, N-Methyl-pyrrolidon oder N-Methyl-caprolactam, insbesondere N,N-Dialkylformamide, wie N,N-Dimethylformamid, und Sulfoxide wie Dimethylsulfoxid, Tetramethylensulfoxid, Dipropylsulfoxid, Benzylmethylsulfoxid, Diisobutylsulfoxid, Dibutylsulfoxid, Diisoamylsulfoxid, insbesondere Dimethylsulfoxid, Verwendung.

Alternativ kann man das Verfahren 5h auch in Gegenwart von basischen Reaktionshilfsmitteln durchführen, d. h. in Gegenwart von weiteren Basen, beispielsweise in einer Menge von weniger als 0,5 Mol, bezogen auf die eingesetzte Base.

Als solche basischen Reaktionshilfsmittel zur Durchführung des erfindungsgemäßen Verfahrens 5h können alle bei Verfahren 5a genannten Säurebindemittel, vorzugsweise jedoch tertiären Amine, insbesondere Trialkylamine wie Triethylamin, N,N-Diisopropylethylamin oder N-Methylmorpholin, und Amidinbasen oder Guanidinbasen wie 7-Methyl-1,5,7-triazabicyclo(4.4.0)dec-5-en (MTBD); Diazabicyclo-(4.3.0)nonen (DBN), Diazabicyclo(2.2.2)-octan (DABCO), 1,8-Diazabicyclo(5.4.0)-undecen (DBU) Cyclohexyltetrabutylguanidin (CyTBG), Cyclohexyltetramethylguanidin (CyTMG), Cyclohexyltetrabutylguanidin, N,N,N,N-Tetramethyl-1,8-naphthalindiamin, insbesondere Cyclohexyltetramethylguanidin (CyTMG) und Cyclohexyltetra-butylguanidin (CyTBG), Verwendung.

Das Verfahren 5h wird durchgeführt, indem Verbindungen der allgemeinen Formel (Id) in Gegenwart von Kohlendioxid, einem 2- bis 3-fachen Überschuß an Alkalimetallcarbonat der Formel (XV) und einem Alkylierungsmittel der Formel (VIIa) in einem der weiter oben bei Verfahren 3a angegebenen Verdünnungsmittel, gegebenenfalls in Gegenwart eines basischen Reaktionshilfsmittels, bei Raumtemperatur zusammengegeben werden. In einem zweiten Reaktionsschritt erfolgt die Alkylierung der in situ gebildeten Alkalimetallsalze der Formeln (XIV) und (XVII) mit Verbindungen der Formel (VIIa) während einer Reaktionszeit von 1 bis 72 Stunden und einer Reaktionstemperatur zwischen -50°C und +180°C, bevorzugt sind Temperaturen im Bereich zwischen -30°C und +150°C, insbesondere solche im Bereich -10°C bis +100°C.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Die Aufarbeitung und Isolierung der Reaktionsprodukte wird nach allgemein üblichen Methoden durchgeführt (vgl. auch die Herstellungsbeispiele).

Setzt man bei Verfahren 5i zur Herstellung der neuen 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (Ib) als Verbindungen der allgemeinen Formel (If) 1-(4-Nitrophenoxy-carbonyl)-7-methyl-1, 2, 3, 4, 4aα, 5aα, 8aα, 8bα-(±)-Oktahydro-3α, 4a-dimethyl-6H-pyrrolo[3',4':4,5]furo[3,2-b]pyridin-6,8(7H)-dion und als Nucleophil der allgemeinen Formel (XVIII) Morpholin ein, so läßt sich das Verfahren durch folgendes Reaktionsschema wiedergeben:

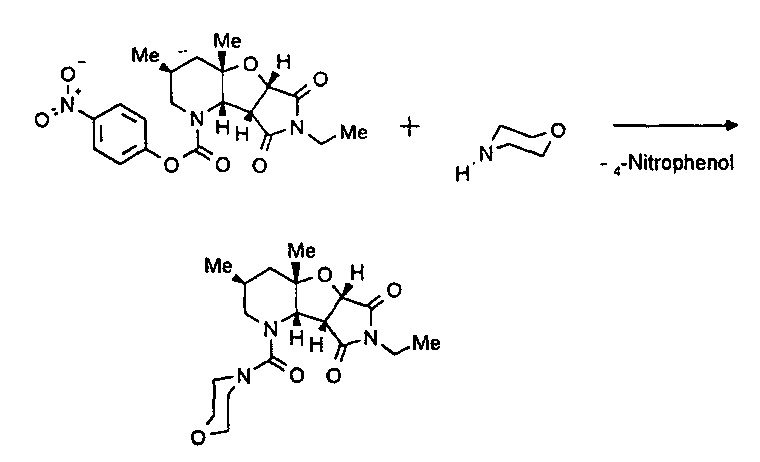

Die zur Durchführung des erfindungsgemäßen Verfahrens 5i als Ausgangsstoffe benötigten 1, 2, 3, 4, 4a, 5a, 8a, 8b-Oktahydro-6H-pyrrolo[3',4':4,5]furo[3,2-b]pyridin-6,8(7H)-dion Derivate sind durch die allgemeine Formel (If) allgemein definiert. In dieser Formel stehen R¹, R², R³, R⁴, R⁵, G, W und X vorzugsweise für diejenigen Reste, die bereits im Zusammenhang mit der Beschreibung der erfindungsgemäßen Stoffe der allgemeinen Formel (If) als bevorzugt für diese Substituenten genannt werden.

Die als Ausgangsmaterialien verwendeten 1, 2, 3, 4, 4a, 5a, 8a, 8b-Oktahydro-6H-pyrrolo[3',4':4,5]furo[3,2-b]pyridin-6,8(7H)-dion Derivate der allgemeinen Formel (If) können nach dem weiter oben bereits aufgezeigten erfindungsgemäßen Verfahren 5d dargestellt werden.

Die außerdem für die Durchführung des erfindungsgemäßen Verfahrens 5i als Ausgangsstoffe zu verwendenden nucleophilen Agenzien der Formel (XVIII) sind allgemein bekannte Verbindungen der organischen Chemie bzw. können z. T. kommerziell erhalten werden. In der Formel (XVIII) haben R¹¹ und Y die Bedeutung die bereits im Zusammenhang mit der Beschreibung der erfindungsgemäßen Stoffe der allgemeinen Formel (Ib) als bevorzugt genannt wurden.

Das Verfahren 5i wird durchgeführt, indem Verbindungen der allgemeinen Formel (If) in Gegenwart eines nucleophilen Agenz der Formel (XV) in einem der weiter oben angegebenen Verdünnungsmittel umsetzt. Die Reaktionsdauer beträgt 4 bis 72 Stunden. Die Umsetzung erfolgt bei Temperaturen zwischen +10°C und +200°C, bevorzugt zwischen +20°C und +150°C, besonders bevorzugt bei Siedetemperatur des Verdünnungsmittels.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Mit den erfindungsgemäßen Verfahren 5a bis 5i sind, aus den einzelnen Bausteinen mit sowohl (S)- als auch (R)-Konfiguration (bzw. L- und D-Konfiguration), erfindungsgemäße Verbindungen unter Beibehaltung der ursprünglichen Konfiguration der Ausgangsstoffe erhältlich.

Mit den in den vorstehenden Verfahrensvarianten 5a bis 5i bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: PHysiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittel.gemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

### Beispiel A

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus den nachfolgenden Tabellen ersichtlich.

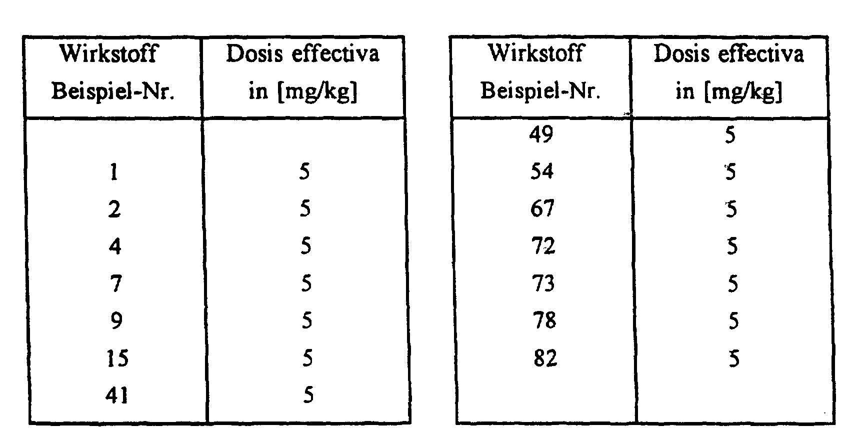

### Beispiel B

### In vivo Nematodentest

### Trichostrongylus colubriformis / Schaf

Experimentell mit *Trichostrongylus colubriformis* infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

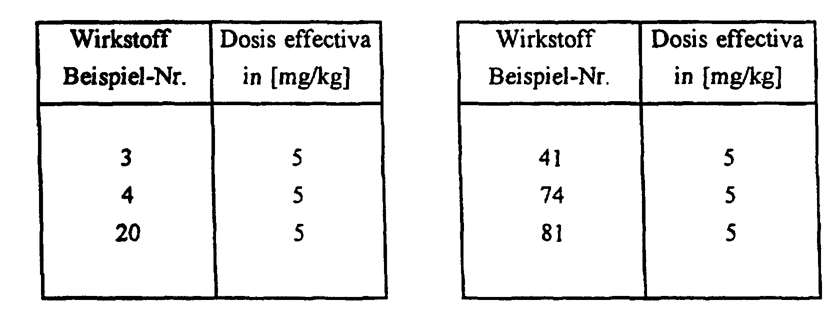

### Herstellungsbeispiele

### Beispiel 1

### 7-Benzyl-4aα, 5aα, 8aα, 8bα-(±)-tetrahydro-3, 4a-dimethyl-6H-pyrrolo[3',4':4,5]furo[3,2-b]pyridin-6,8(7H)-dion

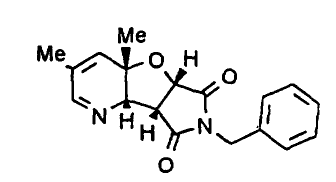

7,4 g (0,04 Mol) N-Benzylmaleinimid und 2,4 g (0,02 Mol) 3,5-Dimethyl-pyridin-N-oxid werden in 30 ml Toluol 10 Stunden unter Rückflußtemperatur gerührt. Anschließend wird das Reaktionsgemisch im Vakuum eingeengt und das zurückbleibende Rohprodukt über eine Kieselgelsäule (Kieselgel 60 - Merck, Korngröße : 0,04 bis 0,063 mm) mit dem Fließmittel Cyclohexan : Essigsäureethylester (1:1) chromatographiert. Man erhält 3,6 g (58,0 % der Theorie) 7-Benzyl-4aα, 5aα, 8aα, 8bα-(±)-tetrahydro-3, 4a-dimethyl-6H-pyrrolo[3',4':4,5]furo[3,2-b] pyridin-6,8(7H)-dion.

Fp.: 123 - 124°C

¹H-NMR (400 MHz, CDCl₃, δ): 1,27 (s, 3H, C-CH₃); 1,56 (d, 3H; =C-CH₃; J = 1,6 Hz); 3,94 (dd, 1H, -CH-; J = 9,6; 7,9 ); 4,24 (dd, 1H, J = 9,6; 2,4 Hz); 4,57 (s, 2H, -CH₂-Phenyl); 4,76 (d, 1H, -O-CH-; J = 7,9 Hz); 5,74 (dd, 1H, =CH-; J = 2,4; 1,2 Hz) 7,26-7,41 (2m, 5H, Phenyl); 7,53 (t, 1H, =CH-; J = 2,4 Hz) ppm.

¹³C-NMR (100 MHz, CDCl₃, δ): 17,8; 24,8 (-CH₃); 42,0 (-CH₂); 50,1; 65,7 (-CH-) 74,4 (-O-CH-); 79,2 (-C-Me); 128,4 (=CMe); 131,1; 159,1 (=CH-; Hetaryl); 127,5; 128,1; 128,7; 135,0 (=CH-; Phenyl); 172,7; 173,7 (-C=O) ppm.

EI-MS m/z (%): 310 (M⁺,100); 271 (38); 214 (62).

Analog können die in der nachstehenden Tabelle 53 aufgeführten Verbindungen der allgemeinen Formel ( Ia ) hergestellt werden.

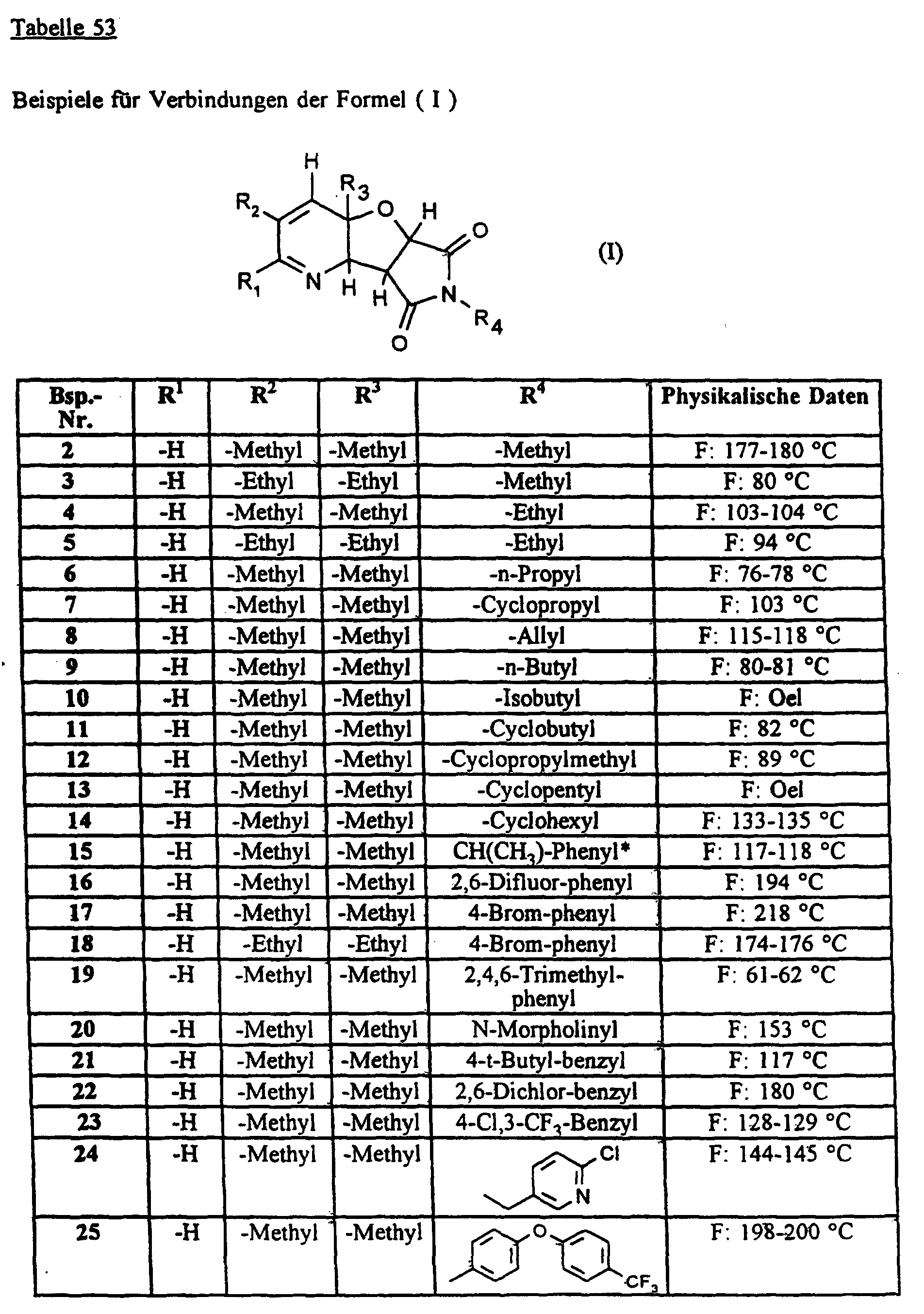

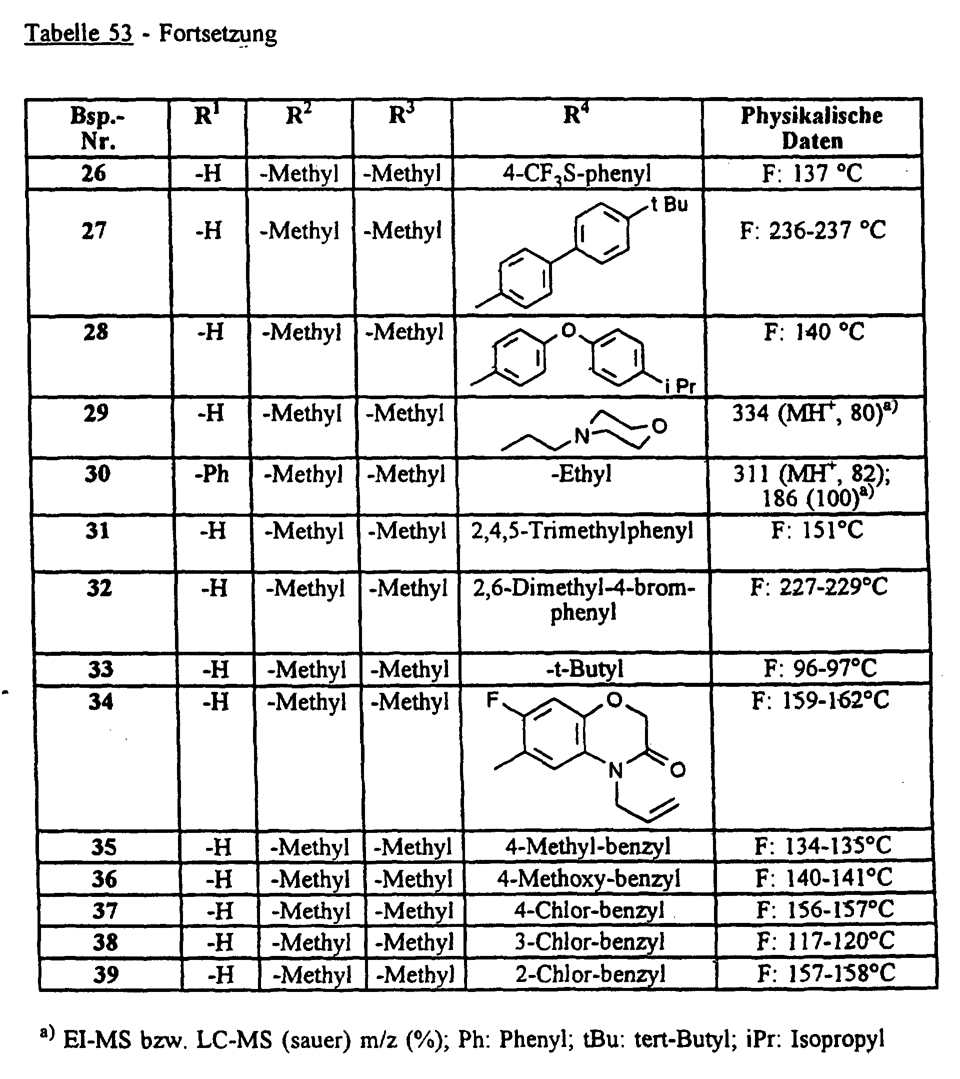

### Beispiel 40

### Hydrochlorid des 7-Ethyl-4aα, 5aα, 8aα, 8bα-(±)-tetrahydro-3, 4a-dimethyl-6H-pyrrolo[3',4':4,5]furo[3,2-b]pyridin-6,8(7H)-dions ( 4 )

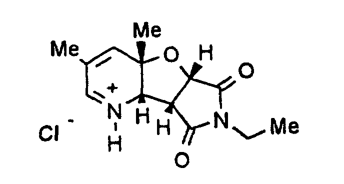

In eine Lösung aus 1,5 g (6,0 mMol) 7-Ethyl-4aα, 5aα, 8aα, 8bα-tetrahydro-3, 4a-dimethyl-6H-pyrrolo[3',4':4,5]furo[3,2-b]pyridin-6,8(7H)-dion ( 4 ) und 10 ml Aceton wird unter Rühren trockenes Chlorwasserstoffgas eingeleitet. Nach ca. 20 Minuten Reaktionszeit trennt man das ausgefallene farblose Hydrochlorid ab und wäscht mit viel Aceton.

Man erhält 1,6 g (93,6 % der Theorie) des Hydrochlorids vom 7-Ethyl-4aα, 5aα, 8aα, 8bα-(±)-tetrahydro-3, 4a-dimethyl-6H-pyrrolo[3',4':4,5]furo[3,2-b]pyridin-6,8(7H)-dion.

Fp.: 134 - 135 °C

¹H-NMR (300 MHz, CDCl₃, 100 \f "Symbol"): 0,94 (t, 3H, -CH₃; J = 7,2 Hz); 1,34 (s, 3H, C-CH₃); 1,84 (d, 3H; =C-CH₃; J = 1,6 Hz); 3,25-3,32 (q, 2H, -CH₂; 7,2 Hz); 3,98 (dd, 1H, -CH-; J = 9,6; 7,9 ); 4,64 (dd, 1H, -CH-; J = 9,6; 2,4 Hz); 4,84 (d, 1H, -O-CH-; J = 7,9 Hz); 6,46 (dd, 1H, =CH-; J = 2,4; 1,2 Hz); 8,55 (t, 1H, =CH-; J = 2,4 Hz) ppm.

### Beispiel 41

### N¹-Methylammoniumiodid des 7-Ethyl-4aα, 5aα, 8aα, 8bα-(±)-tetrahydro-3, 4a-dimethyl-6H-pyrrolo[3',4':4,5]furo[3,2-b]pyridin-6,8(7H)-dions ( 4 )

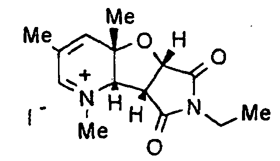

1,0 g (4,0 mMol) 7-Ethyl-4aα, 5aα, 8aα, 8bα-tetrahydro-3, 4a-dimethyl-6H-pyrrolo [3',4':4,5]furo[3,2-b]pyridin-6,8(7H)-dion ( 4 ) werden in 5 ml Aceton gelöst und bei Raumtemperatur mit 1,4 g (9,8 mMol) Methyliodid versetzt. Nach 20 Minuten wird der ausgefallene Feststoff abgetrennt, mit viel Aceton gewaschen und getrocknet.

Man erhält 0,81 g (51,9 % der Theorie) des N¹-Methylammoniumiodids.

¹H-NMR (300 MHz, CDCl₃, δ): 0,94 (t, 3H, -CH₃; J = 7,2 Hz); 1,39 (s, 3H, CCH₃); 1,84 (d, 3H; =C-CH₃; J = 1,6 Hz); 3,28-3,31 (q, 2H, -CH₂; 7,2 Hz); 3,88 (s, 3H, =N⁺-CH₃); 4,21 (dd, 1H, -CH-; J = 9,6; 7,9 Hz); 4,68 (dd, 1H, -CH-; J = 9,6; 2,4 Hz); 4,91 (d, 1H, -O-CH-; J = 7,9 Hz); 6,58 (dd, 1H, =CH-; J = 2,4; 1,2 Hz); 8,86 (t, 1H, =CH-; J = 2,4 Hz) ppm.

EI-MS m/z (%): 263 (M⁺- I⁻,100); 271 (38); 122 (75).

### Beispiel 42

Die Darstellung des N¹-Methylammoniumiodids vom 7-Methyl-4aα, 5aα, 8aα, 8bα-(+/-)-tetrahydro-3,4-a-dimethyl-6H-pyrrolo[3',4':4,5]furo[3,2-b]pyridin-6,8(7H)-dion erfolgt analog aus:

| | |
|---|---|
| 5,0 g (0,021 Mol) | 7-Methyl-4aα, 5aα, 8aα, 8bα-(+/-)-tetrahydro-3,4-a-dimethyl-6H-pyrrolo[3',4':4,5]furo[3,2-b]pyridin-6,8(7H)-dion (2) |
| 4,2 g (0,030 Mol) | Methyliodid |
| 125 ml | Aceton |

Man erhält 5,6 g (70,4 % der Theorie) N¹-Methylammoniumiodid des 7-Methyl-4aα, 5aα, 8aα, 8bα-(+/-)-tetrahydro-3,4-a-dimethyl-6H-pyrrolo[3',4':4,5]furo[3,2-b]pyridin-6,8(7H)-dions. F.: 234°C Zers.

### Beispiel 43

2,0 g (6,4 mMol) 7-Benzyl-4aα, 5aα, 8aα, 8bα-(±)-tetrahydro-3, 4a-dimethyl-6H-pyr-rolo[3',4':4,5]furo[3,2-b]pyridin-6,8(7H)-dion werden in 70 ml Ethanol in Gegenwart von 0,3 g Pd(OH)₂-Kohle [20% Pd Gehalt] bis zur Beendigung der Wasserstoffaufnahme hydriert (ca. 4 Stunden). Nach Abfiltrieren des Katalysators wird die gesamte Reaktionslösung im Vakuum eingeengt. Man erhält 1,8 g (99,1 % der Theorie) eines (3:1) Isomerengemisches, das sich über eine Kieselgelsäule (Kieselgel 60 - Merck, Korngröße : 0,04 bis 0,063 mm) mit dem Fließmittel Essigsäureethylester chromatographisch auftrennen läßt.

### 7-Benzyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

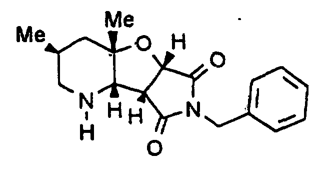

Fp.: 129 -130 °C

¹³C-NMR (100 MHz, CDCl₃, δ): 18,1; 25,3 (-CH₃); 39,2; 48,9; 42,0 (-CH₂); 26,8; 61,5; 50,3 (-CH-); 75,7 (-O-CH-); 84,8 (-C-Me); 127,5; 128,1; 128,5; 134,8 (=CH-; Phenyl); 174,5; 175,0 (-C=O) ppm.

### Beispiel 44

### 7-Benzyl-1, 2, 3, 4, 4aα, 5aα, 8aα, 8bα-(±)-oktahydro-3β, 4a-dimethyl-6H-pyrrolo[3',4':4,5]furo[3,2-b] pyridin-6,8(7H)-dion

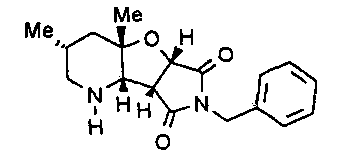

Fp.: 115 -117 °C

¹³C-NMR (100 MHz, CDCl₃, δ): 18,4; 25,0 (-CH₃); 41,7; 41,8; 51,2 (-CH₂); 26,1; 52,0; 59,7 (-CH-); 76,2 (-O-CH-); 86,6 (-C-Me); 127,3; 128,1; 135,2 (=CH-; Phenyl); 174,2; 174,6 (-C=O) ppm.

Bei der Hydrierung von Beispiel ( 2) wurde isoliert:

### Beispiel 45

### 7-Methyl-1, 2, 4aα, 5aα, 8aα, 8bα-(±)-hexahydro-3, 4a-dimethyl-6H-pyrrolo[3',4':4,5]furo[3,2-b] pyridin-6,8(7H)-dion

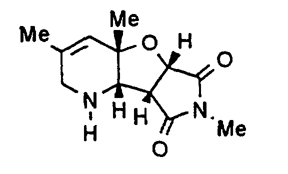

EI-MS m/z (%): 236 (M⁺, 61).

### Beispiel 46

### 7-Methyl-1,2,4aα,5aα,8aα,8bα-(+/-)-hexahydro-1,3,4a-trimethyl-6H-pyrrolo[3',4': 4,5]furo[3,2-b]pyridin-6,8(7H)-dion

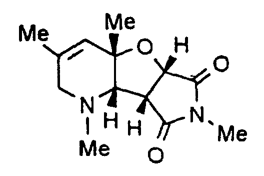

5,0 g (13,3 mMol) N¹-Methylammoniumiodid des 7-Methyl-4aα,5aα,8aα,8bα-(+/-)-Tetrahydro-3,4a-dimethyl-6H-pyrrolo[3',4':4,5]furo[3,2-b]pyridin-6,8(7H)-dions werden in 100 ml Methanol suspendiert und bei -5°C portionsweise mit 0,64 g (16,9 mMol) NaBH₄ versetzt. Nach zweistündigem Rühren bei 0°C wird ca. 18 Stunden bei Raumtemperatur gerührt. Anschließend engt man die gesamte Reaktionslösung im Vakuum ein, nimmt den verbleibenden Rückstand in Chloroform auf und schüttelt mehrmals mit Wasser. Die organische Phase wird abgetrennt, über Magnesiumsulfat getrocknet und im Vakuum -eingeengt. Man erhält 2,8 g (87,3 % der Theorie) 7-Methyl-1,2,4aα,5aα,8aα,8bα-(+/-)-hexahydro-1,3,4a-trimethyl-6H-pyrrolo[3',4' :4,5]furo[3,2-b]pyridin-6,8(7H)-dion.

F.: 124 - 125 °C

¹H-NMR (400 MHz, CDCl₃, 100δ): 1,37 (s, 3H, -C-CH₃); 1,57 (s, 3H; =C-CH₃); 2,85; 2,91 (2s, 6H, 2 x -N-CH₃); 2,97; 3,15 (2d, 2H, -C-CH^{a}H^{b}-; J = 9,8 Hz); 3,45 (dd, 1H, -CH-; J = 9,9; 7,7 Hz); 3,55 (d, 1H, -CH-; J = 9,9 Hz); 4,70 (d; 1H; -O-CH-; J = 7,7 Hz); 5,29 (m, 1H, =CH-) ppm.

EI-MS m/z (%): 250 (M^{+.},15); 122 (100).

### Beispiel 47

### 1-(1-Chlorethoxycarbonyl)-1,2,4aα, 5aα,8aα,8bα-(+/-)-hexahydro-3,4a,7-trimethyl-6H-pyrrolo[3',4' :4,5]furo[3,2-b]pyridin-6,8(7H)-dion

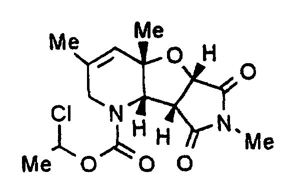

2,4 g (9,6 mMol) 7-Methyl-1,2,4aα,5aα,8aα,8bα-(+/-)-hexahydro-1,3;4a-trimethyl-6H-pyrrolo[3',4':4,5]furo[3,2-b]pyridin-6,8(7H)-dion werden in 15 ml 1,2-Dichlorethan verrührt, mit 2,7 g (19,2 mMol) 1-Chlorethoxycarbonylchlorid versetzt und 20 Minuten unter Rückflußtemperatur erhitzt. Nach jeweils 20 Minuten werden zweimal 1,3 g (9,6 mMol) 1-Chlorethoxycarbonylchlorid hinzugegeben und anschließend 5 Stunden unter Rückflußtemperatur gerührt. Der gesamte Reaktionsansatz wird im Vaku-um eingeengt und mittels einer Kieselgelsäule (Kieselgel 60 Merck, Korngröße : 0,04 bis 0,063 mm) mit dem Fließmittel Essigsäureethylester gereinigt. Man erhält 1,7 g (53,9 % der Theorie) 1-(1-Chlorethoxycarbonyl)-1,2,4aα,5aα,8aα,8bα-(+/-)-hexahydro-3,4a,7-trimethyl-6H-pyrrolo[3'4':4,5]furo[3,2-b]pyridin-6,8(7H)-dion, das gleich weiter umgesetzt werden kann.

### Beispiel 48

### Hydrochlorid des 1,2,4aα,5aα,8aα,8bα-(+/-)-Hexahydro-3,4a,7-trimethyl-6H-pyrrolo[3',4':4,5]furo[3,2-b]pyridin-6,8(7H)-dions (45)

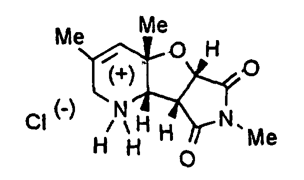

1,7 g (5,2 mMol) 1-(1-Chlorethoxycarbonyl)-1,2,4aα,5aα,8aα,8bα-(+/-)-Hexahydro-3,4a,7-trimethyl-6H-pyrrolo[3',4':4,5]furo[3,2-b]pyridin-6,8(7H)-dion werden in 4 Methanol verrührt und zunächst auf 50 - 60°C erwärmt; anschließend rührt man ca. 30 Minuten unter Rückflußtemperatur nach. Das ausgefallene Hydrochlorid wird mit wenig Ether gewaschen. Man erhält 1,1 g (77,6 % der Theorie) Hydrochlorid des 1, 2,4aα,5aα,8aα,8bα-(+/-)-Hexahydro-3,4a,7-trimethyl-6H-pyrrolo[3'4' :4,5]faro[3,2-b]pyridin-6,8(7H)-dions.

¹H-NMR (400 MHz, D₂O, δ): 1,42 (s, 3H, -C-CH₃); 1,78 (s, 3H; =C-CH₃); 2,98 (2s, 6H, 2 x -N-CH₃); 3,58; 3,67 (2d, 2H, -C-CH^{a}H^{b}-; J = 9,8 Hz); 4,11 (d, 1H,-CH-; J = 9,9 Hz); 4,35 (dd, 1H, -CH-; J = 9,9; 7,7 Hz); 5,03 (d, 1H, -O-CH-; J = 7,7 Hz); 5,73 (m, 1H, =CH ppm.

### Beispiel 49

### 1-Allyloxycarbonyl-7-ethyl-1,2,4aα, 5aα,8aα,8bα-(+/-)-hexahydro-3,4a,-dimethyl-6H-pyrrolo[3',4':4,5]furo[3,2-b]pyridin-6,8(7H)-dion

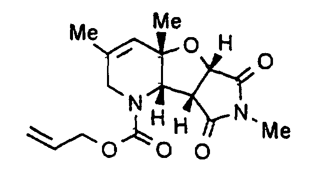

Zur Ausbildung der freien Base werden 2,6 (9,1 mMol) Hydrochlorid des 7-Ethyl-1,2, 4aα,5aα,8aα,8bα-(+/-)-hexahydro-3,4a,7-trimethyl-6H-pyrrolo[3',4' 4,5]furo[3,2-b]py-ridin-6,8(7H)-dions mit 1,4 g (10,9 mMol) N,N-Diisopropylethylamin ("Hünig's Base") in einem Gemisch aus 50 ml Wasser und 150 ml Methylenchlorid verrührt. Nach 10 Minuten wird die organische Phase abgetrennt und über Magnesiumsulfat getrocknet. Anschließend werden zur Methylenchloridphase bei 0°C 1,8 g (13,6 mMol) N,N-Diisopropylethylamin ("Hünig's Base") und 1,3 g (10,9 mMol) Chlorameisen-säureallylester gegeben, etwa 10 Minuten bei 0°C und 2 Stunden bei Raumtemperatur gerührt. Danach wird die organische Phase zweimal mit Wasser extrahiert, die organische Phase über Magnesiumsulfat getrocknet und im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Essigsäure-ethylester (1:1) chromatographiert. Man erhält 1,0 g (32,9 % der Theorie) 1-Allyl-oxycarbonyl-7-ethyl-1,2,4aα,5aα,8aα,8bα-(+/-)-hexahydro-3,4a-dimethyl-6H-pyrrolo [3',4':4,5]furo[3,2-b]pyridin-6,8 (7H)-dion.

F.: 87 - 90 °C

EI-MS m/z (%): 334 (M^{+.},2); 249 (M⁺ - [H₂C=CH-H₂C-O-CO], 100].

### Herstellung nach Verfahren 5a

### Beispiel 50

### 1-Methyl-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

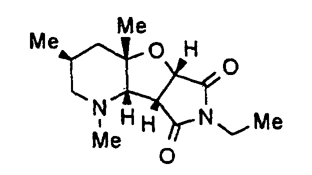

Zu einer Lösung von 2,0 g (8,4 mMol) 7-Ethyl-1, 2, 3, 4, 4aα, 5aα, 8aα, 8bα-(±)-oktahydro-3α,4a-dimethyl-6H-pyrrolo[3',4':4,5]furo[3,2-b]pyridin-6,8(7H)-dion und Natriumhydrogencarbonat in 100 ml Dimethylformamid wird unter Stickstoffatmosphäre 1,1g (9,6 mMol) Methyliodid zugegeben und ca. 5 Stunden bei 80°C gerührt. Die Reaktionslösung wird auf Wasser gegossen, mehrmals mit Essigsäureethylester extrahiert, die organisehe Phase abgetrennt und nach dem Trocknen über Natriumsulfat im Vakuum eingeengt. Das zurückbleibende Rohprodukt wird über eine Kieselgelsäule chromatographiert.

LC-MS (sauer) m/z (%): 283 (MH⁺, 100); 265 (12); 141 (37); 101 (66)

### Herstellung nach Verfahren 5b

### Beispiel 51

### 1-(-2-Cyanoethyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

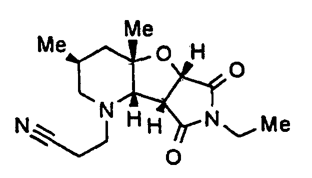

2,0 g (87,9 mMol) 7-Ethyl-1, 2, 3, 4, 4aα, 5aα, 8aα, 8bα-(±)-oktahydro-3α,4a-dime-thyl-6H-pyrrolo[3',4':4,5]furo[3,2-b]pyridin-6,8(7H)-dion und 0,5 g (10,0 mMol) Acrylnitril werden in 10 ml Ethanol 28 Stunden bei Rückfluß-temperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) chromatographiert.

LC-MS (sauer) m/z (%): 306 (MH⁺, 100); 253 (M⁺ - [NC-CH₂-CH₂-], 29).

### Herstellung nach Verfahren 5c

### Beispiel 52

### 1-((±)-2-Hydroxy-3-isopropoxyprop-1-yl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

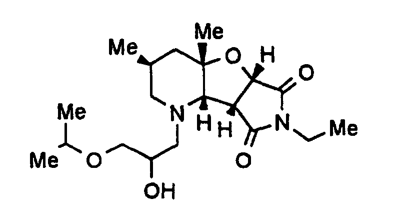

Zu einer Lösung von 5,0 g (19,2 mMol) 7-Ethyl-1, 2, 3, 4, 4aα, 5aα, 8aα, 8bα-(±)-oktahydro-3α,4a-dimethyl-6H-pyrrolo[3',4':4,5]furo[3,2-b]pyridin-6,8(7H)-dion und 0,9 g (8,0 mMol) (±)-2,3-Epoxypropylisopropylether in 40 ml Tetrahydofuran werden 24 Stunden unter Rückflußtemperatur erhitzt. Anschließend wird die gesamte Reaktionslösung im Vakuum eingeengt und das zurückbleibende Rohprodukt chromatographiert.

LC-MS (sauer) m/z (%): 369 (MH⁺, 100); 253 (16)

### Herstellung nach Verfahren 5d

### Beispiel 53

### 1-Allyloxycarbonyl-7-methyl-1, 2, 3, 4, 4aα, 5aα, 8aα, 8bα-(±)-oktahydro-3β, 4a-dimethyl-6H-pyrrolo[3',4':4,5]furo[3,2-b]pyridin-6,8(7H)-dion

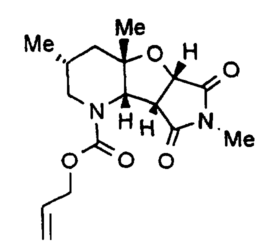

Zu einer Lösung von 2,0 g (8,4 mMol) 7-Methyl-1, 2, 3, 4, 4aα, 5aα, 8aα, 8bα-(±)-oktahydro-3β,4a-dimethyl-6H-pyrrolo[3',4':4,5]furo[3,2-b]pyridin-6,8(7H)-dion und 1,1g (9,6 mMol) Chlorameisensäureallylester in 100 ml Methylenchlorid werden bei 0°C 2,8 g (21,6 mMol) N,N-Diisopropylethylamin ("Hünig's Base") zugegeben und 2 Stunden bei 0 °C und anschließend 2 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Essigsäureethylester chromatographiert. Man erhält 1,9 g (73,9 % der Theorie) 1-Allyloxycarbonyl-7-methyl-1, 2, 3, 4, 4aα, 5aα, 8aα, 8bα-(±)-oktahydro-3β,4a-dimethyl-6H-pyrrolo[3',4':4,5]furo[3,2-b]pyridin-6,8(7H)-dion als öliges Produkt.

EI-MS m/z (%): 322 (M⁺,5); 237 (100); 108 (72).

### Beispiel 54

### 1-Allyloxycarbonyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

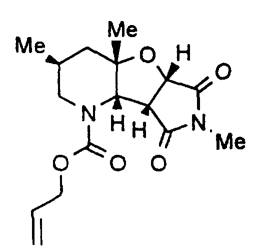

Die N-Acylierung erfolgt analog der Reaktionsvorschrift des Beispiels ( 53 ) inner-halb von 4 Stunden unter Verwendung von:

| | |
|---|---|
| 2,0 g (8,4 mMol) | 7-Methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion |
| 1,1 g (9,6 mMol) | Chlorameisensäureallylester |
| 2,8 g (21,6 mMol) | N,N-Diisopropylethylamin ("Hünig's Base") |
| 100 ml | Methylenchlorid |

Man erhält 1,6 g (64,6 % der Theorie) 1-Allyloxycarbonyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8 (7H)-dion.

F.: 94 - 96 °C

EI-MS m/z (%): 322 (M⁺, 6); 237 (100); 108 (50).

### Beispiel 55

### 1-Chlormethylcarbonyl-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

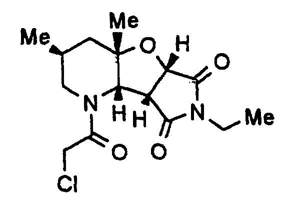

Die N-Acylierung erfolgt analog der Reaktionsvorschrift des Beispiels ( 53 ) innerhalb von 1 Stunde unter Verwendung von:

| | |
|---|---|
| 6,0 g (23,7 mMol) | 7-Ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion |
| 3,3 g (28,5 mMol) | Chloracetylchlorid |
| 6,6 g (65,1 mMol) | Triethylamin |
| 60 ml | Methylenchlorid |

EI-MS m/z (%): 328 (M^{+.}, 8); 251 (M^{+.} -[Cl-CH₂-CO], 100); 108 (44).

### Beispiel 56

### 1-(N-Morpholino-methylcarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

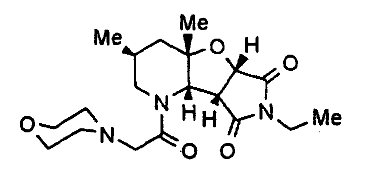

Zu einer Lösung von 0,5 g (1,5 mMol) 1-Chlormethylcarbonyl-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion ( 55 ) und 0,15 g (1,8 mMol) Morpholin in 5 ml Acetonitril werden 0,2 g (1,8 mMol) Triethylamin zugegeben und 1 Stunde bei Rückflußtemperatur erhitzt.

Die Reaktionslösung wird im Vakuum eingeengt, der Rückstand in Chloroform aufgenommen und mehrmals mit Wasser geschüttelt. Anschließend wird die organische Phase abgetrennt und nach dem Trocknen über Magnesiumsulfat im Vakuum eingeengt. Das zurückbleibende Rohprodukt über eine Kieselgelsäule (Kieselgel 60 - Merck, Korngröße : 0,04 bis 0,063 mm) mit dem Fließmittel Essigsäureethylester chromatographiert. Man erhält 0,25 g (43,3% der Theorie) 1-(N-Morpholino-methylcarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion.

¹H-NMR (300 MHz, CDCl₃, δ): 2,60 (m, br., 4H, -(CH₂)₂-N-); 3,77 (m, br., 4H, -(CH₂)₂-O) ppm.

EI-MS m/z (%): 379 (M^{+.}, 1); 251 (M^{+.} - [-CO-CH₂-NMorpholin], 1); 100([-CH₂-NMorpholin], 100).

### Herstellung nach Verfahren 5e

### Beispiel 57

### 1-(Carboxylmethyloxymethylcarbonyl)-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

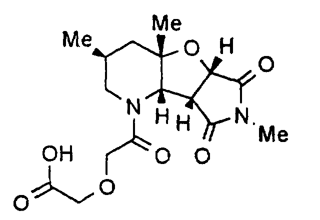

Zu einer Lösung von 1,4 g (12,0 mMol) Diglycolsäureanhydrid in 20 ml trockenem Tetrahydrofuran werden bei 50 °C tropfenweise 2,0 g (8,4 mMol) 7-Methyl-1, 2, 3, 4, 4aα, 5aα, 8aα, 8bα-(±)-oktahydro-3α,4a-dimethyl-6H-pyrrolo[3',4':4,5]furo[3,2-b]pyri-din-6,8(7H)-dion, gelöst in 10 ml Tetrahydofuran, getropft und anschließend 12 Stunden bei dieser Temperatur weitergerührt. Anschließend wird die gesamte Reaktionslösung im Vakuum eingeengt, die zurückbleibende Substanz abgetrennt und mehrmals mit Ether gewaschen.

LC-MS (sauer) m/z (%): 355 (MH⁺,100); 309 (12).

### Beispiel 58

### 1-(Carboxylmethyloxymethylcarbonyl)-7-methyl-1, 2, 4aα, 5aα, 8aα, 8bα-(±)-hexahydro-3, 4a-dimethyl-6H-pyrrolo[3',4':4,5]furo[3,2-b]pyridin-6,8(7H)-dion

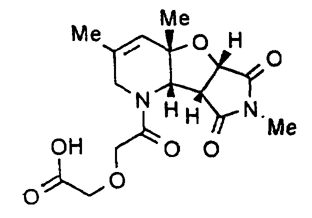

Die N-Acylierung erfolgt analog der Reaktionsvorschrift des Beispiels ( 57 ) innerhalb von 12 Stunden unter Verwendung von:

| | |
|---|---|
| 2,0 g (8,4 mMol) | 7-Methyl-1, 2, 4aα, 5aα, 8aα, 8bα-(±)-hexahydro-3, 4a-dimethyl-6H-pyrrolo[3',4':4,5]furo[3,2-b]pyridin-6,8(7H)-dion |
| 1,4 g (12,0 mMol) | Diglycolsäureanhydrid |
| 30 ml | Tetrahydrofuran |

LC-MS (sauer) m/z (%): 353 (M-H⁺,100); 237 (18).

### Herstellung nach Verfahren 5f

### Beispiel 59

### 1-(N-Benzyloxycarbonyl-N-methyl-glycyl)-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

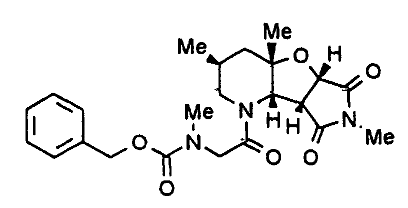

Zu einer Lösung von 2,0 g (8,4 mMol) 7-Methyl-1, 2, 3, 4, 4aα, 5aα, 8aα, 8bα-(±)-oktahydro-3α,4a-dimethyl-6H-pyrrolo[3',4':4,5]furo[3,2-b]pyridin-6,8(7H)-dion und 1,7 g (7,6 mMol) N-Benzyloxycarbonyl-sarkosin (Z-Sar-OH) in 100 ml Methylenchlorid werden bei 0 °C 3,2 g (25,2 mMol) N,N-Diisopropylethylamin ("Hünig's Base") sowie 2,1 g (8,4 mMol) Bis(2-oxo-3-oxazolidinyl)-phosphoniumsäurechlorid (BOP-Cl) zugegeben und 30 Minuten bei 0 °C, anschließend 18 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine LiChroprep RP-18 Säule (LiChroprep RP-18 - Merck, Korngröße : 0,4 bis 0.063 mm) mit dem Fließmittel Acetonitril - Wasser (4:6) chromatographiert.

¹H-NMR (300 MHz, CDCl₃, δ): 2,98; 3,08 (2s, 6H, 2 x N-CH₃) ppm.

EI-MS m/z (%): 444 (MH⁺; 4); 443 (M^{+.}, 15); 237 (M^{+.} - [-CO-CH₂-NMe-CO-O-Ben-yl]; 38); 209 (20); 91(Benzyl; 100).

### Herstellung nach Verfahren 5g

### Beispiel 60

### 1-Trichloracetylaminocarbonyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

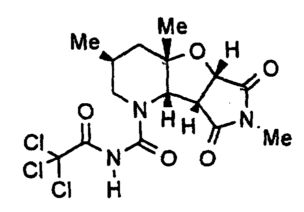

Zu einer Lösung von 2,0 g (8,4 mMol) 7-Methyl-1, 2, 3, 4, 4aα, 5aα, 8aα, 8bα-(±)-oktahydro-3α,4a-dimethyl-6H-pyrrolo[3',4':4,5]furo[3,2-b]pyridin-6,8(7H)-dion in 30 ml Acetonitril werden bei 0 °C 1,6 g (8,4 mMol) Trichloracetylisocyanat gegeben, und 30 Minuten bei 0 °C gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60- Merck, Korngröße : 0,04 bis 0,063 mm) mit dem Fließmittel Essigsäureethylester chromatographiert. Man erhält 1,2 g (34,6 g der Theorie) 1-Trichloracetylaminocarbonyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion.

EI-MS m/z (%): 391 (M^{+.} -Cl, 1); 355 (M⁺ - 2Cl, 8); 306 (M^{+.} - [Cl₃CH]; 19); 237 (100)

### Herstellung nach Verfahren 5h

### Beispiel 61

### l-Propargyloxycarbonyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

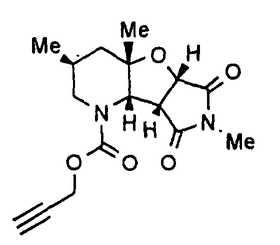

2,0 g (8,4 mMol) 7-Methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion und 30 ml Dimethylsulfoxid werden eine Stunde lang mit Kohlendioxid begast und anschließend mit 1,1 g (8,4 mmol) Propargylbromid versetzt. die Reaktionsmischung wurde 48 Stunden bei Raumtemperatur gerührt, danach vom Feststoff abgetrennt, unter vermindertem Druck die flüchtigen Komponenten abgezogen, mit Essigsäureethylester extrahiert und die organische Phase abgetrennt. Anschließend wird die organische Phase über Natriumsulfat getrocknet, im Vakuum eingeengt und das zurückbleibende Rohprodukt wird über eine LiChroprep RP-18 Säule (LiChroprep RP-18 - Merck, Korngröße : 0,4 bis 0.063 mm) mit dem Fließmittel Acetonitril - Wasser (4:6) chromatographiert.

EI-MS m/z (%): 320 (M^{+.}, 0,5); 237 (M^{+.} - [HC≡C-CH₂-O-CO-], 20).

Als Nebenprodukt wurde isoliert:

### Beispiel 62

### l-Propargyl-7-methyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

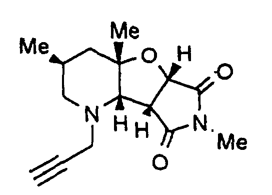

EI-MS m/z (%): 276 (M^{+.}, 5); 237 (M⁺ - [HC≡C-CH₂-], 100), 39 ([HC≡C-CH₂-], 46).

### Herstellung nach Verfahren 5i

### Beispiel 63

### 1-(4-Nitrophenoxycarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b] pyridin-6,8(7H)-dion

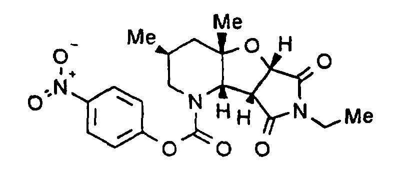

Zu einer Lösung von 4,0 g (15,8 mMol) 7-Ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion und 3,2g (15,8 mMol) Chlorameisensäure-(4-nitrophenyl)-ester in 100 ml Methylenchlorid werden bei 0 °C 4,4 g (34,8 mMol) N,N-Diisopropylethylamin ("Hünig's Base") zugegeben und 2 Stunden bei 0 °C und anschließend 18 Stunden bei Raumtemperatur gerührt. Die Reaktionslösung wird zweimal mit Wasser geschüttelt, die organische Phase abgetrennt und nach dem Trocknen über Magnesiumsulfat im Vakuum eingeengt. Das zurückbleibende Rohprodukt über eine Kieselgelsäule (Kieselgel 60 - Merck, Korngröße : 0,04 bis 0,063 mm) mit dem Fließmittel Cyclohexan : Essigsäureethylester (1:1) chromatographiert. Man erhält 4,0 g (62,4 % der Theorie) 1-(4-Nitrophenoxycarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b] pyridin-6,8(7H)-dion.

Fp.: 160-167 °C

¹H-NMR (400 MHz, CDCl₃, δ): 7,42; 8,29 (2d, 4H, 4-NO₂-Phenoxy; J_{H,H} = 9,1 Hz) ppm.

EI-MS m/z (%): 417 (M^{+.}, 0.29); 279 (33); 223 (100).

### Beispiel 64

### 1-(N-Morpholinocarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion

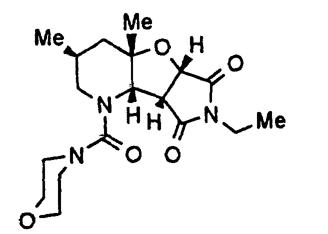

Zu einer Lösung von 2,0 g (4,8 mMol) 1-(4-Nitrophenoxycarbonyl)-7-ethyl-1, 2, 3, 4, 4aα, 5aα, 8aα, 8bα-(±)-oktahydro-3α, 4a-dimethyl-6H-pyrrolo[3',4':4,5]furo[3,2-b]pyridin-6,8(7H)-dion in 40 Dioxan werden bei Raumtemperatur 0,65 g (5,0 mMol) N,N-Diisopropylethylamin ("Hünig's Base") sowie 0,45 g (5,2 mMol) Morpholin zugegeben und ca. 48 Stunden unter Rückflußtemperatur gerührt. Die Reaktionslösung wird zweimal mit Wasser geschüttelt, die organische Phase abgetrennt und nach dem Trocknen über Magnesiumsulfat im Vakuum eingeengt. Das zurückbleibende Rohprodukt über eine Kieselgelsäule chromatographiert.

LC-MS (sauer) m/z (%): 366 (MH⁺, 100); 253 (3); 141 (20); 101 (33).

Analog zu den Verfahren können die in den nachstehenden Tabellen 54 und 55 aufgeführten Verbindungen der allgemeinen Formel (Ib), (Ic) und (Id; R⁵: -H) hergestellt werden.

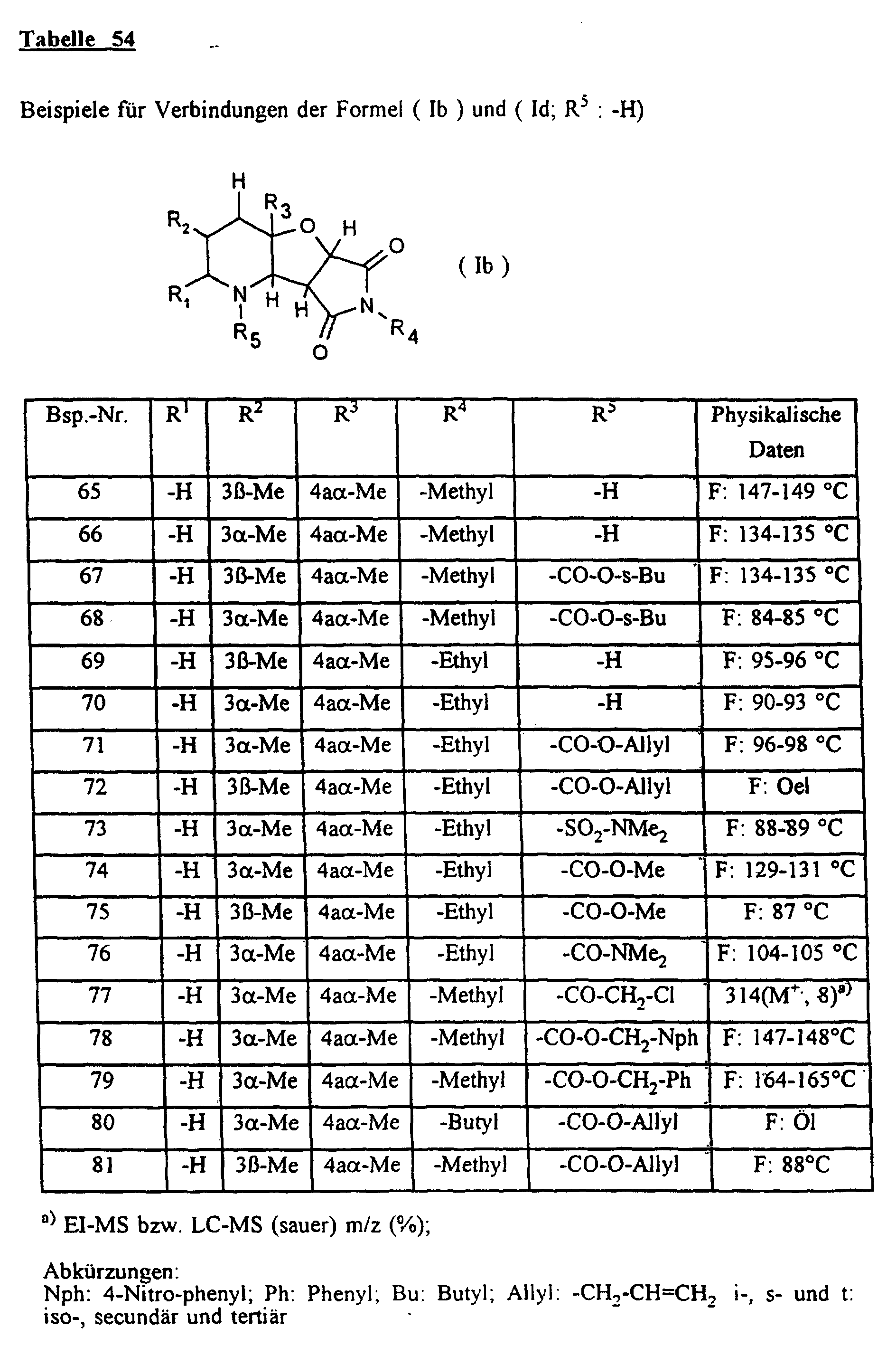

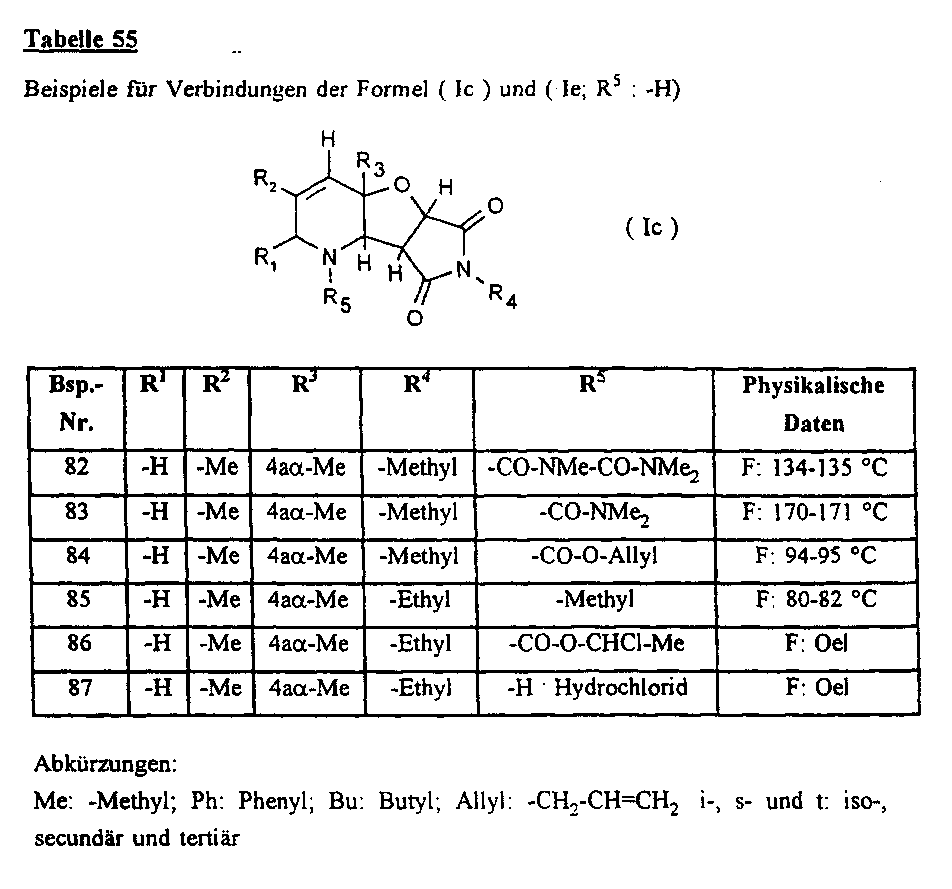

### Ausgangsstoffe der Formel ( II )

### Beispiel ( II-1 )

### 3,5-Dimethyl-pyridin-N-oxid

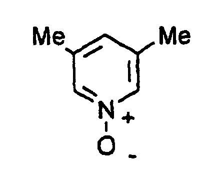

Zu einer Lösung von 73,5 g (0,68 Mol) 3,5-Dimethylpyridin in 410 ml Eisessig werden 68,6 ml Wasserstoffperoxid (30 %ig) getropft und 3 Stunden bei 70-80 °C gerührt. Anschließend gibt man nochmals 48 ml Wasserstoffperoxid (30 %ig) hinzu und rührt weitere 9 Stunden bei 70-80 °C. Zur Aufarbeitung wird der gesamte Reaktionsansatz mit konz. Natronlauge basisch gestellt, mit Chloroform ausgeschüttelt, die abgetrennte organische Phase über Natriumsulfat getrocknet und im Vakuum eingeengt. Das zurückbleibende Rohprodukt kristallisiert beim Verrühren mit einem Chloroform/Hexan-Gemisch.

Man erhält 53,6 g (63,4 % der Theorie) 3,5-Dimethyl-pyridin-N-oxid.

¹H-NMR (400 MHz, CDCl₃, δ): 2,27 (s, 6H, C-CH₃); 6,94; 7,92 (2 x t, 3H; Hetaryl) ppm.

EI-MS m/z (%): 123 (M^{+.},100); 107 (M⁺-0,5).

### Beispiel ( II-2 )

Die Darstellung von 3,5-Diethyl-pyridin-N-oxid erfolgt analog aus:
30,9 g (0,22 Mol) 3,5-Diethyl-pyridin
39 ml Wasserstoffperoxid (30 %ig)
137 ml Eisessig

Man erhält 33,8g (97,8 % der Theorie) 3,5-Diethyl-pyridin-N-oxid.

¹H-NMR (400 MHz, CDCl₃, ): 1,25 (t, 6H, 2 x -CH₃; J = 7,6 Hz); 2,60 (q, 4H, 2 x -CH₂-; J = 7,6 Hz); 6,98; 7,96 (2 x t, 3H; Hetaryl) ppm

### Beispiel ( II-3 )

Die Darstellung von 2-Phenyl-3-methyl-pyridin-N-oxid erfolgt analog aus:
30,9 g (0,18 Mol) 2-Phenyl-3-methyl-pyridin
32 ml Wasserstoffperoxid (30 %ig)
112 ml Eisessig

Man erhält 38,5g (85,9 % der Theorie) 2-Phenyl-3-methyl-pyridin-N-oxid.

¹H-NMR (300 MHz, CDCl₃, δ): 2,11 (s, 3H, Hetaryl-CH₃); 7,13-7,54 (3m, 7H, Phenyl-H / Hetaryl-H); 8,24 (m, 1H, Hetaryl-H) ppm.

### Ausgangsstoffe der Formel ( III )

### Beispiel ( III-1 )

### N-Morpholinyl-maleinimid

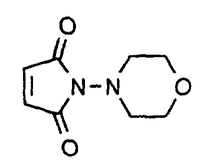

24,3 g (0,15 Mol) (E/Z)-N-Morpholino-maleinamidsäure werden mit 100 g Essigsäureanhydrid und 1,0 g wasserfr. Natriumacetat 1 Stunde bei 100 °C gerührt. Anschließend wird das gesamte Reaktionsgemisch auf Eiswasser gegeben, ca. 18 Stunden bei Raumtemperatur gerührt und mit Methylenchlorid extrahiert. Nach Abtrennen der organischen Phase und Trocknen über Natriumsulfat wird im Vakuum eingeengt und das zurückbleibende Rohprodukt über eine Kieselgelsäule (Kieselgel 60 - Merck, Korngröße : 0,04 bis 0,063 mm) mit dem Fließmittel Cyclohexan : Essigsäureethylester (1:1) chromatographiert. Man erhält 7,7 g (35,2 % der Theorie) N-Morpholino-maleinimid.

F: 104-105 °C

¹H-NMR (400 MHz, CDCl₃, δ): 3,30 (t, 4H, -CH₂-N-CH₂-; J = 4,4 Hz); 3,83 (t, 4H, -CH₂-O-CH₂-; J = 4,4 Hz); 6,64 (s, 2H, 2 x =CH-) ppm

EI-MS m/z (%): 182 (M^{+.}, 100).

### Beispiel ( III-2 )

### N-Cyclopropyl-maleinimid

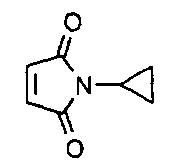

21,6 g (0,22 Mol) Maleinsäureanhydrid werden in 300 ml Methylenchlorid suspendiert und bei 0 °C innerhalb von 5 Minuten tropfenweise mit 16,0 g (0,22 Mol) Cyclopropylamin versetzt. Nach 16 stündigem Rühren bei Raumtemperatur wird das Reaktionsgemisch auf 0 °C abgekühlt und mit einigen Tropfen Dimethylformamid versetzt. Anschließend werden innerhalb von 30 Minuten 20 ml (0,24 Mol) Oxalylchlorid hinzugegeben.

Man rührt weitere 8 Stunden bei Raumtemperatur, zieht das überschüssige Oxalylchlorid im Vakuum ab, löst das zurückbleibende Rohprodukt erneut in 180 ml Methylenchlorid und versetzt mit 30,6 ml (0,22 Mol) Triethylamin. Das Reaktionsgemisch wird 1,5 Stunden bei Raumtemperatur gerührt, filtriert und das zurückbleibende Filtrat mit 1 N HCl gewaschen. Nach Abtrennen der organischen Phase und Trocknen über Natriumsulfat wird im Vakuum eingeengt und das verbleibende Rohprodukt über eine Kieselgelsäule (Kieselgel 60 - Merck, Korngröße : 0,04 bis 0,063 mm) mit dem Fließmittel Cyclohexan : Essigsäureethylester (1:1) chromatographiert Man erhält 11,3 g (37,7 % der Theorie) N-Cyclopropylmaleinimid.

¹H-NMR (400 MHz, CDCl₃, ): 0,84-0,98 (m, 4H, -CH₂-CH₂-); 2,49-2,56 (m, 1H,-CH-); 6,64 (s, 2H, 2 x =CH-) ppm

EI-MS m/z (%): 137 (M^{+.}, 19).

Analog können die in der nachstehenden Tabelle 56 aufgeführten Verbindungen hergestellt werden:

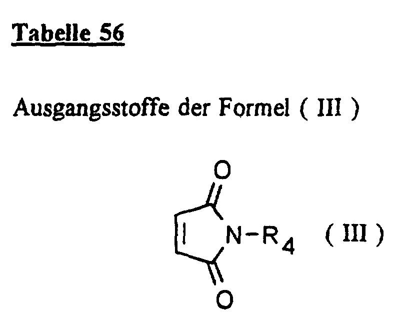

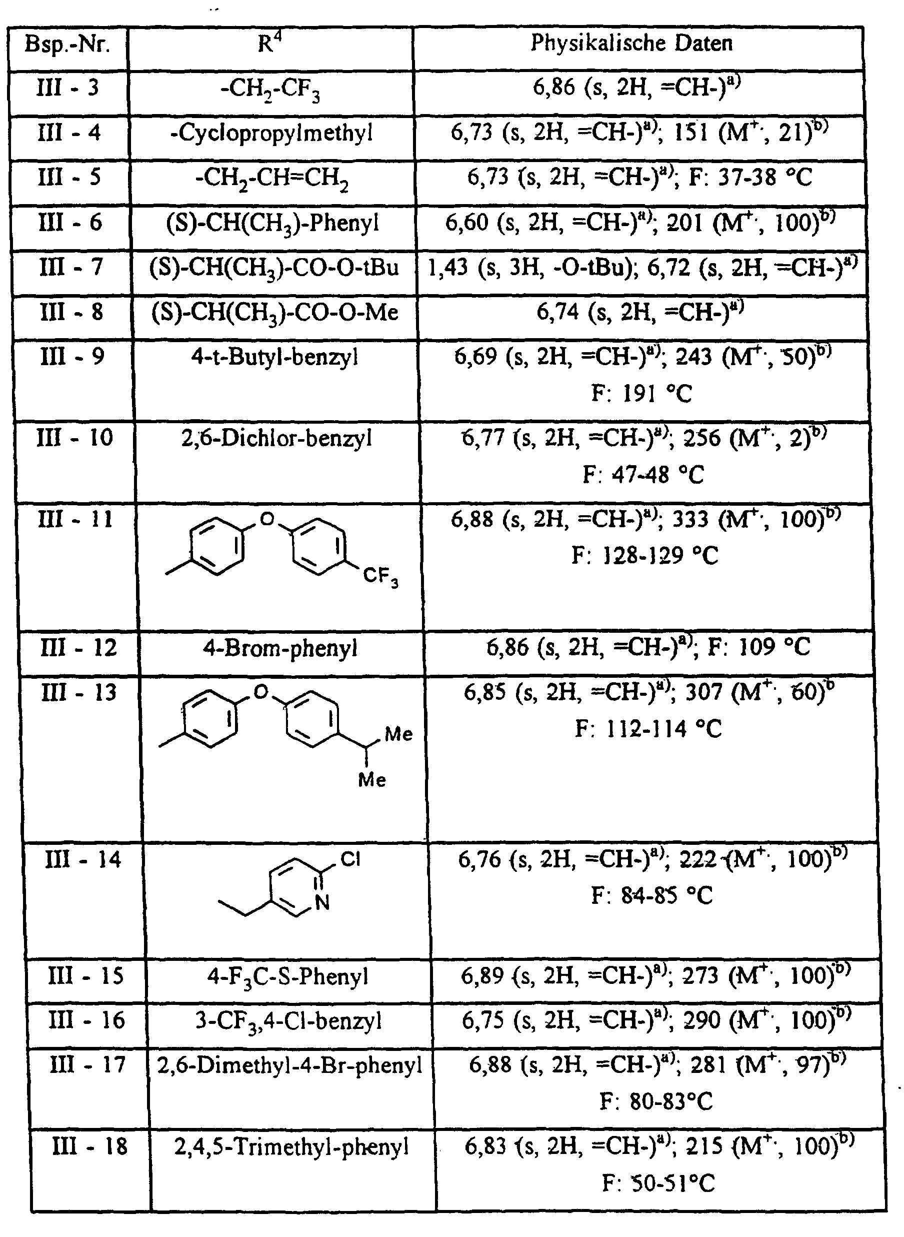

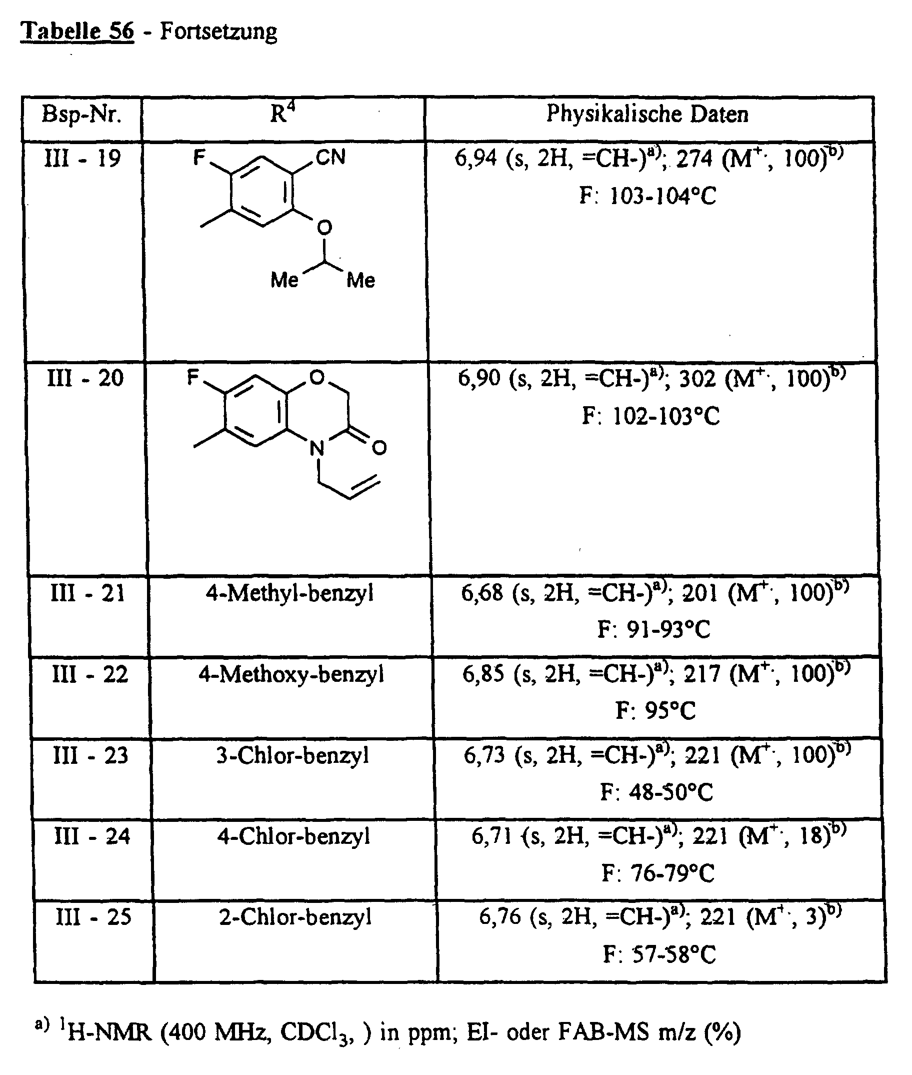

Beispiel zur Herstellung der als Vorstufe eingesetzten N-substituierten Maleinamidsäuren.

### (E/Z)-N-Morpholino-maleinamidsäure

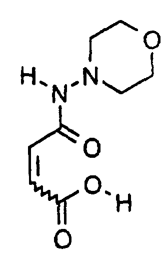

Zu einer Lösung von 14,8 g (0,15 Mol) Maleinsäureanhydrid in 290 ml Methylen-chlorid werden bei 0 °C N-Aminomorpholin, gelöst in 10 ml Methylenchlorid, getropft und anschließend ca. 18 Stunden bei Raumtemperatur gerührt. Die gesamte Reak-tionslösung wird im Vakuum eingeengt. Man erhält 25,3 g (83,1 % der Theorie) (E/Z)-N-Morpholino-maleinamidsäure, die ohne Reinigung weiter umgesetzt werden kann.

F.: > 177 °C (Zers.)

¹H-NMR (400 MHz, DMSO-d₆, δ): 2,72; 2,77 (2t, 4H, -CH₂-N-CH₂-; J = 4,4 Hz); 3,64; 3,66 (t, 4H, -CH₂-O-CH₂-; J = 4,4 Hz); 6,06; 6,20; 6,30; 6,80 (4d, 4H, 4 x =CH-); 9,12; 9,78 (2s, 2H, -NH-); 13,68 (br. s, 1H, -CO-OH) ppm

FAB-MS m/z (%): 201 (M⁺+H, 100), 154 (90); 136 (66).

Analog können die in der nachstehenden Tabelle 57 aufgeführten Verbindungen hergestellt werden:

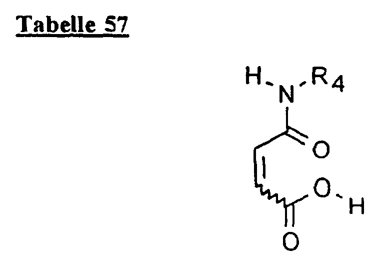

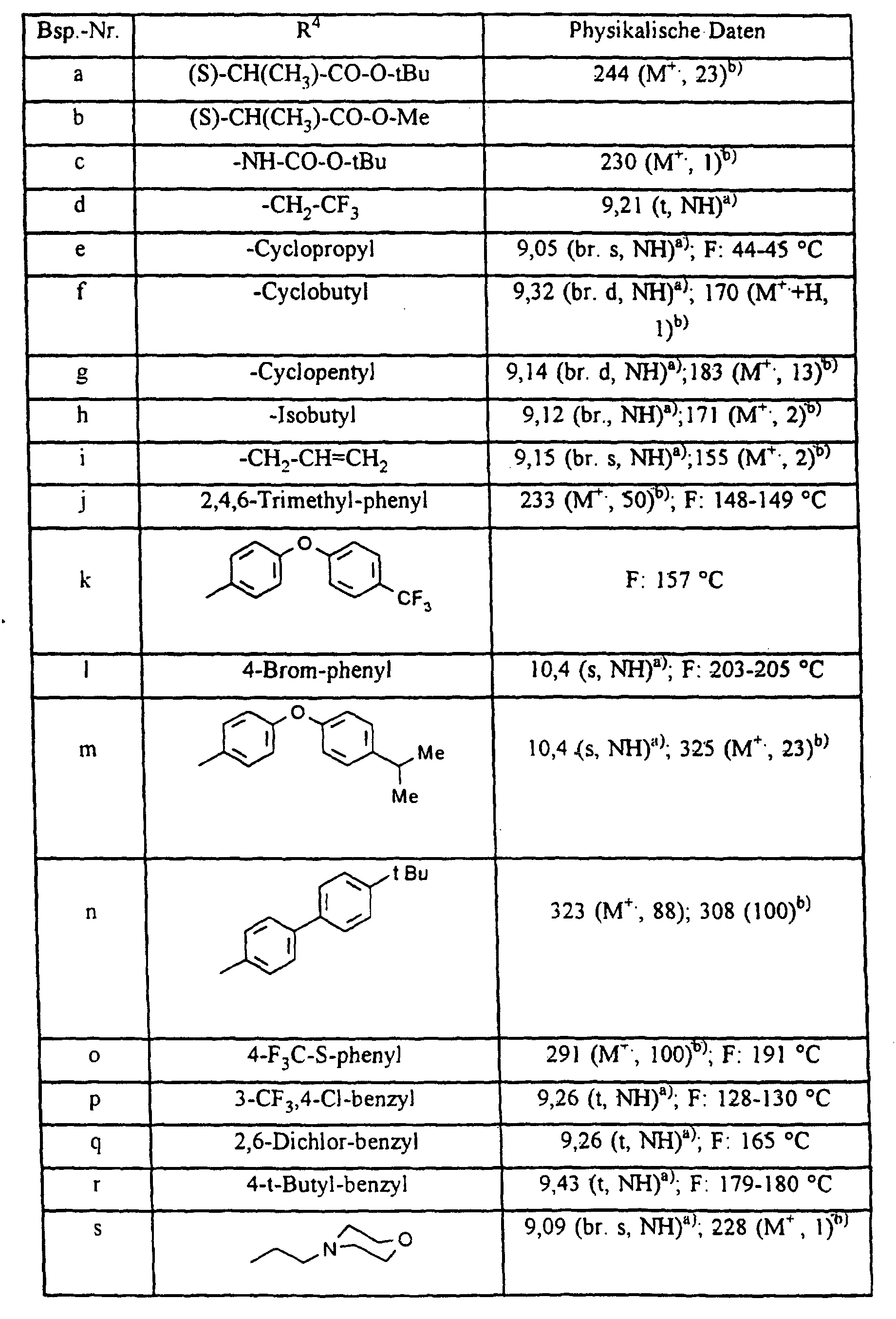

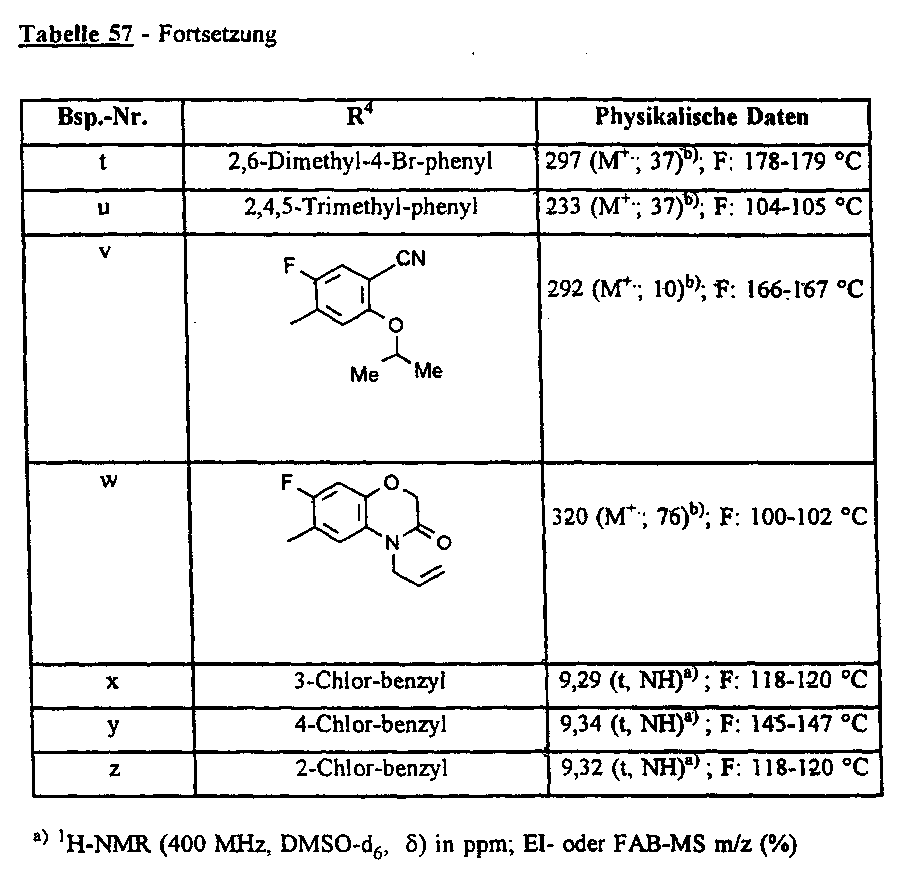

## Patentansprüche

1. Verwendung von 4a, 5a, 8a, 8b-Tetrahydro-6H-pyrrolo[3',4':4,5]furo[3,2-b]pyridin-6,8(7H)-dion Derivaten der allgemeinen Formel ( I ) und deren Salze, 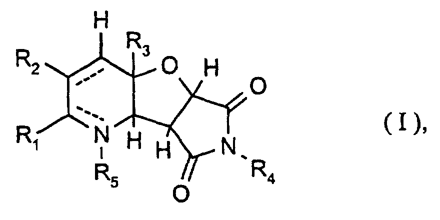 in welcher
R¹ für Wasserstoff, geradkettiges oder verzweigtes Alkyl, Cycloalkyl, Arylalkyl, Aryl, Heteroaryl, Heteroarylalkyl, die gegebenenfalls substituiert sind, steht,
R² für Wasserstoff, geradkettiges oder verzweigtes Alkyl, Cycloalkyl, Alkoxycarbonyl, steht, die gegebenenfalls substituiert sind,
R¹ und R² gemeinsam mit den Atomen, an die sie gebunden sind, für einen 5- oder 6-gliedrigen Ring stehen, der gegebenfalls duch Sauerstoff, Schwefel, Sulfoxyl oder Sulfonyl unterbrochen sein kann und gegebenenfalls substituiert ist,
R³ für Wasserstoff, geradkettiges oder verzweigtes Alkyl, Cycloalkyl, Alkoxycarbonyl, steht, die gegebenenfalls substituiert sind,
R⁴ für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl, Amino, Alkylamino, Dialkylamino, Cycloalkylamino, die gegebenenfalls substituiert sind, steht,
R⁵ für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl, die gegebenenfalls substituiert sind, Formyl, Alkoxydicarbonyl oder gegebenenfalls für einen Rest aus der Gruppe G¹, G², G³ und G⁴ steht 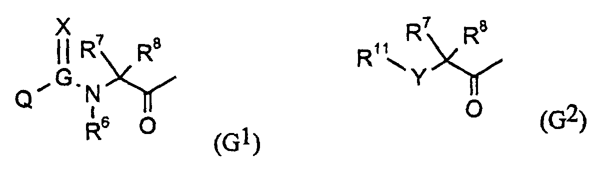 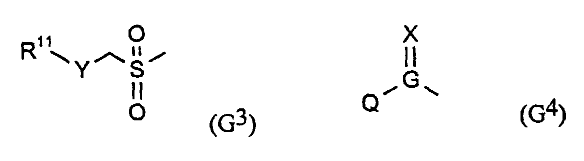 worin
R⁶ für Wasserstoff, geradkettiges oder verzweigtes Alkyl, Cycloalkyl, Aryl, Arylalkyl, Heteroaryl, Heteroarylalkyl, die gegebenenfalls substituiert sind, steht,
R⁶ und R⁷ gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
R⁷ und R⁸ unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
R⁷ und R⁸ gemeinsam für einen spirocyclischen Ring stehen, der gegebenenfalls substituiert ist, 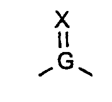 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeuten kann,
R⁹ für Wasserstoff, Hydroxy, Alkoxy, Alkylcarbonyl, Halogen-alkylcarbonyl, Alkylsulfonyl, Nitro oder Cyan steht, und
R¹⁰ für Wasserstoff oder Alkyl steht, und
Q für geradkettiges oder verzweigtes Alkyl, Alkenyl, Alkinyl, Cycloalkyl, Aryl, Arylalkyl, Hetaryl oder Hetarylalkyl, die gegebenenfalls substituiert sind, oder gegebenefalls für einen Rest aus der Gruppe G⁵ und G⁶ 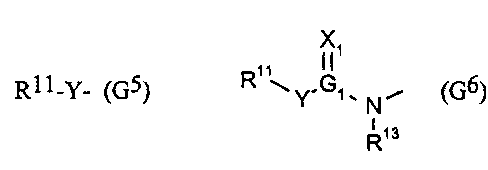 steht,
worin 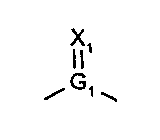 Carboxy, Thiocarboxy oder Sulfonyl bedeuten kann,
Y für Sauerstoff, Schwefel oder -NR¹² steht,
R¹¹ für den Fall, daß Y für Stickstoff steht, eine über ein Stickstoffatom verknüpfte cyclische Aminogruppe bedeuten kann,
R¹¹ und R¹² unabhängig voneinander für Wasserstoff, geradkettiges oder verzweigtes Alkyl, Alkenyl, Alkinyl, Cycloalkyl, Cycloalkylalkyl, Aryl, Arylalkyl, Heteroaryl, Heteroarylalkyl stehen, die gegebenenfalls substituiert sind, oder
R¹¹ und R¹² gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls substituiert ist,
R¹³ für Wasserstoff oder Alkyl steht,
R¹⁴ für Wasserstoff, geradkettiges oder verzweigtes Alkyl, Alkenyl, Alkinyl, Cycloalkyl, Cycloalkylalkyl, Alkoxycarbonyl, Alkylcarbonyl, Cycloalkylcarbonyl, Cyan, Aryl, Arylalkyl, Heteroaryl, Heteroarylalkyl stehen, die gegebenenfalls substituiert sind, steht, sowie deren optische Isomere und Racemate
zur Herstellung von Mitteln zur Bekämpfung von Endoparasiten in der Medizin und Tiermedizin,
wobei gegebenenfalls substituierte Reste einen oder mehrere der folgenden Substituenten tragen können:
Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen;
Hydroxy, Halogen, Cyano, Nitro, Amino, Monoalkyl- und Dialkylamino mit 1 bis 4 Kohlenstoffatomen je Alkylgruppe, Alkylcarbonyl mit 2 bis 4 Kohlenstoffatomen, Alkoxycarbonyl mit 2 bis 4 Kohlenstoffatomen, Alkylsulfinyl mit 1 bis 4 Kohlenstoffatomen, Halogenalkylsulfinyl und 1 bis 4 Kohlenstoffatomen und 1 bis 5 Halogenatomen,
Sulfonyl, Alkylsulfonyl mit 1 bis 4 Kohlenstoffatomen,
Halogenalkylsulfonyl mit 1 bis 4 Kohlenstoffatomen und 1 bis 5 Halogenatomen,
Arylsulfonyl mit 6 bis 10 Arylkohlenstoffatomen;
Acyl, Aryl, Aryloxy, Hetaryl, Hetaryloxy, die ihrerseits einen oder vorstehend genannten Rest tragen können;
Formiminorest,
und wobei in den obigen Definitionen gilt, dass Alkyl allein oder als Bestandteil eines Restes 1 bis 6 Kohlenstoffatome aufweist und geradkettig oder verzweigt sein kann,
Cycloalkyl allein oder als Bestandteil eines Restes 3 bis 10 Kohlenstoffatome aufweist und mono-, bi- oder tricyclisch sein kann,
Alkoxy allein oder als Bestandteil eines Restes 1 bis 6 Kohlenstoffatome aufweist und geradkettig oder verzweigt sein kann;
Arylalkyl im Arylteil 6 bis 10 Kohlenstoffatome und im Alkylteil 1 bis 4 Kohlenstoffatome aufweist,
Heteroaryl einen 5- bis 7-gliedrigen Ring mit 1 bis 3 gleichen oder verschiedenen Heteroatomen, ausgewählt aus Sauerstoff, Schwefel und Stickstoff bedeutet;
Alkoxycarbonyl 1 bis 6 Kohlenstoffatome aufweist und geradkettig oder verzweigt sein kann; Alkenyl und Alkinyl jeweils 1 bis 6 Kohlenstoffatome aufweisen und geradkettig oder verzweigt sein können;
Halogenalkyl allein oder als Bestandteil eines Restes 1 bis 4 Kohlenstoffatome und 1 bis 9 gleiche oder verschiedene Halogenatome enthält,
Alkylcarbonyl 1 bis 6 Kohlenstoffatome aufweist und geradkettig oder verzweigt sein kann;
Cycloalkylcarbonyl mono-, bi- oder tricyclisches Cycloalkylcarbonyl mit 3 bis 10 Kohlenstoffatomen bedeutet.

2. Verwendung von Verbindungen der Formel (I) gemäß Anspruch 1
in welcher
R¹ für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
R¹ und R² gemeinsam mit den Atomen, an die sie gebunden sind, für einen 5- oder 6-gliedrigen Ring stehen, der gegebenfalls substituiert ist,
R² und R³ unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
R⁴ für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxykarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁-C₄-Alkylamino, C₁-C₄-Dialkylamino, C₃-C₇-Cycloalkylamino, die gegebenenfalls substituiert sind, steht,
R⁵ für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, Nitro-C₁₋₄-alkyl, Cyano-C₁₋₄-alkyl, C₁₋₄-Alkoxycarbonyl-C₁₋₄-alkyl, Carbamoyl-C₁₋₄-alkyl, Carboxyl-C₁₋₄-alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Formyl, C₁₋₄-Alkoxydicarbonyl oder für einen Rest der Gruppe G¹, G², G³ und G⁴ 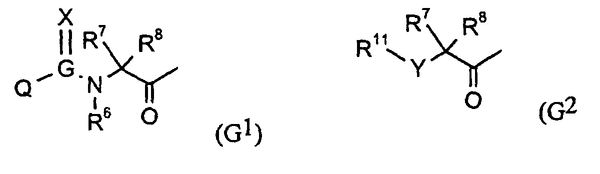 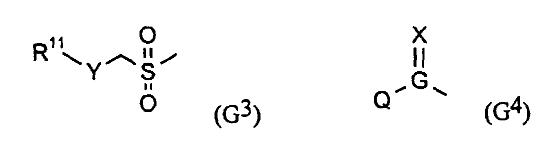 worin
R⁶ für Wasserstoff, geradkettiges oder verzweigtes C₁₋₆-Alkyl, C₃₋₆-Cycloalkyl, Aryl-C₁₋₂-alkyl, Heteroaryl-C₁₋₂-alkyl, die gegebenenfalls substituiert sind, steht,
R⁶ und R⁷ gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
R⁷ und R⁸ unabhängig voneinander für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxy-C₁₋₆-alkyl, C₁₋₂-Alkoxy-C₁₋₆-alkyl, Mercapto-C₁₋₆-alkyl, C₁₋₂-Alkylthio-C₁₋₆-alkyl, C₁₋₂-Alkylsulfinyl-C₁₋₆-alkyl, C₁₋₂-Alkylsulfonyl-C₁₋₆-alkyl, Carboxy-C₁₋₆-alkyl, Carbamoyl-C₁₋₆-alkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste oder durch einen, zwei, drei oder vier C₁₋₂-Alkylreste substituiert sein kann, C₁₋₄-Alkoxycarbonylamino-C₁₋₆-alkyl, C₂₋₆-Alkenyl, C₃₋₆-Cyclo-C₁₋₂-alkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl stehen, oder
R⁷ und R⁸ gemeinsam für einen spirocyclischen Ring stehen, 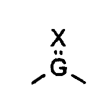 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeutet,
R⁹ für Wasserstoff, Hydroxy, C₁₋₄-Alkoxy, C₁₋₄-Alkylcarbonyl, Halogen-C₁₋₄-alkylcarbonyl, C₁₋₄-Alkylsulfonyl, Nitro oder Cyan steht, und
R¹⁰ für Wasserstoff oder C₁₋₄-Alkyl steht, und
Q für geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste, tert-Butyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₂₋₆-Alkinyl, C₃₋₆-Cycloalkyl, Aryl, Aryl-C₁₋₂-alkyl, Heteroaryl oder Heteroaryl-C₁₋₂-alkyl, die gegebenenfalls substituiert sind, oder gegebenefalls für einen Rest aus der Gruppe G⁵ und G⁶ 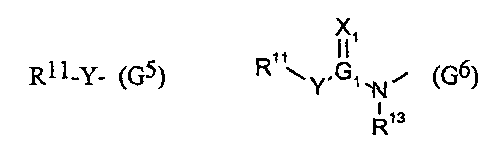 steht,
worin 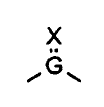 Carboxy, Thiocarboxy oder Sulfonyl bedeuten kann,
Y für Sauerstoff, Schwefel oder -NR¹² steht,
R¹¹ für den Fall, daß Y für Stickstoff steht, eine über ein Stickstoffatom verknüpfte cyclische Aminogruppe bedeuten kann,
R¹¹ und R¹² unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl stehen, die gegebenenfalls substituiert sind, oder
R¹¹ und R¹² gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls substituiert ist,
R¹³ für Wasserstoff oder C₁₋₄-Alkyl steht,
R¹⁴ für Wasserstoff, geradkettiges oder verzweigtes C₁₋₆-Alkyl, C₂₋₆-Alkenyl, C₂₋₆-Alkinyl, C₃₋₈-Cycloalkyl, C₃₋₈-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Heteroaryl, Heteroaryl-C₁₋₂-alkyl stehen, die gegebenenfalls substituiert sind, steht,
sowie deren optische Isomere und Racemate, zur Herstellung von Mitteln zur Bekämpfung von Endoparasiten in der Medizin und Tiermedizin,
wobei gegebenenfalls substituierte Reste einen oder mehrere der folgenden Substituenten tragen können:
Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen;
Hydroxy, Halogen, Cyano, Nitro, Amino, Monoalkyl- und Dialkylamino mit 1 bis 4 Kohlenstoffatomen je Alkylgruppe, Alkylcarbonyl mit 2 bis 4 Kohlenstoffatomen, Alkoxycarbonyl mit 2 bis 4 Kohlenstoffatomen, Alkylsulfinyl mit 1 bis 4 Kohlenstoffatomen, Halogenalkylsulfinyl und 1 bis 4 Kohlenstoffatomen und 1 bis 5 Halogenatomen,
Sulfonyl, Alkylsulfonyl mit 1 bis 4 Kohlenstoffatomen,
Halogenalkylsulfonyl mit 1 bis 4 Kohlenstoffatomen und 1 bis 5 Halogenatomen,
Arylsulfonyl mit 6 bis 10 Arylkohlenstoffatomen;
Acyl, Aryl, Aryloxy, Hetaryl, Hetaryloxy, die ihrerseits einen oder vorstehend genannten Rest tragen können;
Formiminorest,
und wobei in den obigen Definitionen gilt, dass Heteroaryl einen 5- bis 7-gliedrigen Ring mit 1 bis 3 gleichen oder verschiedenen Heteroatomen ausgewählt aus Sauerstoff, Schwefel und Stickstoff bedeutet.

3. 4a, 5a, 8a, 8b-Tetrahydro-6H-pyrrolo[3',4':4,5]furo[3,2-b]pyridin-6,8(7H)-dion Derivate der allgemeinen Formel ( Ia ) und deren Salze, 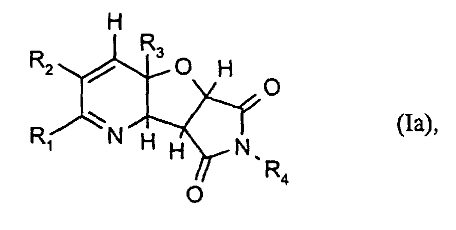 in welcher
R¹ für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
R² und R³ unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
R⁴ für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxycarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁-C₄-Alkylamino, C₁-C₄-Dialkylamino, C₃-C₇-Cycloalkylamino, die gegebenenfalls substituiert sind, steht,
mit der Maßgabe für den Fall, daß
R¹ für Wasserstoff steht, und
R² und R³ für Methyl stehen,
R⁴ nicht für Methyl, n-Butyl, Phenyl, 2-, 3- oder 4-Methylphenyl, 2-, 3- oder 4-Chlor-phenyl, 2-, 3- oder 4-Fluor-phenyl, 2-Chlor, 4-fluorphenyl, 2-Fluor, 4-chlor-phenyl, 3-Chlor, 4-fluorphenyl, 2-, 3- oder 4-Nitro-phenyl, 4-Methoxy-phenyl oder α-Naphthyl steht,
sowie deren optische Isomere und Racemate.

4. Verfahren zur Herstellung der neuen 4a, 5a, 8a, 8b-Tetrahydro-6H-pyrrolo[3',4':4,5]furo[3,2-b]pyridin-6,8(7H)-dion Derivate der allgemeinen Formel ( Ia ) gemäß Anspruch 3 und deren Salze, 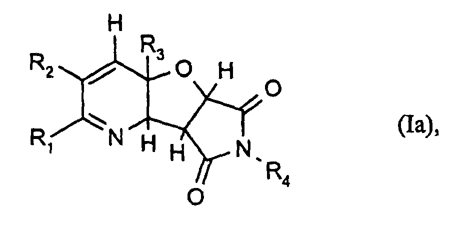 in welcher
R¹, R², R³ und R⁴ die in Anspruch 3 angegebene Bedeutung besitzen, **dadurch gekennzeichnet, daß** man
a) Pyridin-N-oxide der allgemeinen Formel ( II ) 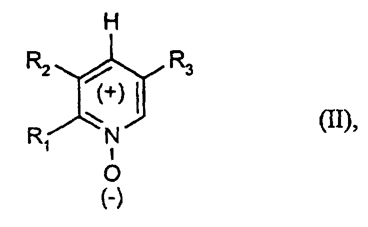
in welcher
R¹, R² und R³ die in Anspruch 3 angegebene Bedeutung besitzen, mit Maleinsäureimiden der allgemeinen Formel (III) 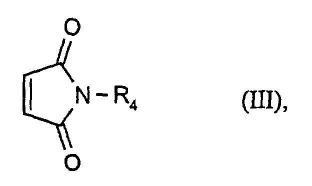 in welcher
R⁴ die in Anspruch 3 angegebene Bedeutung hat, gegebenenfalls in Gegenwart eines Vedünnungsmittels umsetzt.

5. Neue 1, 2, 3, 4, 4a, 5a, 8a, 8b-Oktahydro-6H-pyrrolo[3',4':4,5]furo[3,2-b]pyridin-6,8(7H)-dion- und 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) sowie deren Salze, 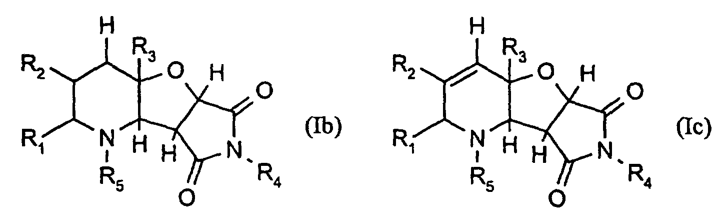 in welchen
R¹ für Wasserstoff, geradkettiges oder verzweigtes Alkyl mit bis zu 4 Kohlenstoffatomen, C₃₋₆-Cycloalkyl, Aryl-C₁₋₂-alkyl, Aryl, Heteroaryl, Heteroaryl-C₁₋₂-alkyl, die gegebenenfalls substituiert sind, steht,
R² und R³ unabhängig voneinander für Wasserstoff, geradkettiges oder verzweigtes Alkyl mit bis zu 4 Kohlenstoffatomen, Halogenalkyl, Hydroxyalkyl, C₁₋₄-Alkanoyloxyalkyl, C₁₋₂-Alkoxyalkyl, C₁₋₂-Mercaptoalkyl, C₁₋₂-Alkylthioalkyl, C₁₋₂-Alkylsulfinylalkyl, C₁₋₂-Alkylsulfonylalkyl, Aminoalkyl, C₁₋₆-Alkylaminoalkyl, C₁₋₆-Dialkylaminoalkyl, C₃₋₆-Cycloalkylaminoalkyl, C₃₋₆-Cycloalkyl, C₁₋₄-Alkoxycarbonyl, stehen,
R⁴ für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, C₁₋₄-Alkoxykarbonyl-C₁₋₄-alkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Amino, C₁-C₄-Alkylamino, C₁-C₄-Dialkylamino, C₃-C₇-Cycloalkylamino, die gegebenenfalls substituiert sind, steht,
R⁵ für Wasserstoff, geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Amino-C₁₋₆-alkyl, C₁₋₆-Alkylamino-C₁₋₆-alkyl, C₁₋₆-Dialkylamino-C₁₋₆-alkyl, C₁₋₆-Trialkylammonium-C₁₋₆-alkyl-halogenid, Nitro-C₁₋₄-alkyl, Cyano-C₁₋₄-alkyl, C₁₋₄-Alkoxycarbonyl-C₁₋₄-alkyl, Carbamoyl-C₁₋₄-alkyl, Carboxyl-C₁₋₄-alkyl,C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl, Formyl, C₁₋₄-Alkoxydicarbonyl oder für einen Rest aus der Gruppe G¹, G², G³ und G⁴ 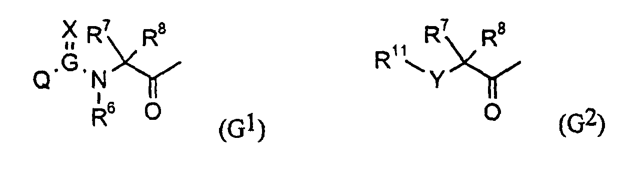 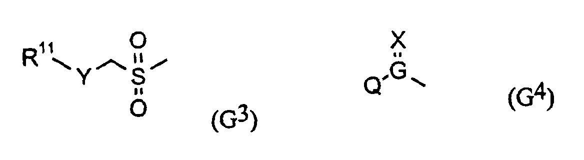 worin
R⁶ für Wasserstoff, geradkettiges oder verzweigtes C₁₋₄-Alkyl, C₃₋₆-Cycloalkyl, Aryl-C₁₋₂-alkyl oder Heteroaryl-C₁₋₂-alkyl, die gegebenenfalls substituiert sind, steht,
R⁶ und R⁷ gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist, stehen,
R⁷ für Wasserstoff, geradkettiges oder verzweigtes C₁₋₆-Alkyl, C₂₋₄-Alkenyl, C₃₋₆-Cycloalkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl steht, oder
R⁸ für Wasserstoff oder Methyl steht, 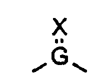 Carboxy, Thiocarboxy, -C=CH-NO₂, -C=CH-CN, -C=N-R⁹, Sulfoxyl, Sulfonyl, -P(O)-OR¹⁰ oder P(S)-OR¹⁰ bedeuten kann,
R⁹ für Wasserstoff, Hydroxy, C₁₋₄-Alkoxy, C₁₋₄-Alkylcarbonyl, Halogen-C₁₋₄-alkylcarbonyl, C₁₋₄-Alkylsulfonyl, Nitro oder Cyan steht, und
R¹⁰ für Wasserstoff oder C₁₋₄-Alkyl steht, und
Q für geradkettiges oder verzweigtes C₁₋₆-Alkyl, Halogen-C₁₋₆-alkyl, Hydroxy-C₁₋₆-alkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Amino-C₁₋₆-alkyl, C₁₋₆-Alkylamino-C₁₋₆-alkyl, C₁₋₆-Dialkylamino-C₁₋₆-alkyl, Guanidino-C₁₋₆-alkyl, das gegebenenfalls durch einen oder zwei Benzyloxycarbonylreste, tert-Butyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₂₋₆-Alkinyl, C₃₋₆-Cycloalkyl, Aryl, Aryl-C₁₋₂-alkyl, Heteroaryl oder Hetaryl-C₁₋₂-alkyl, die gegebenenfalls substituiert sind, oder gegebenenfalls für einen Rest aus der Gruppe G⁵ und G⁶ 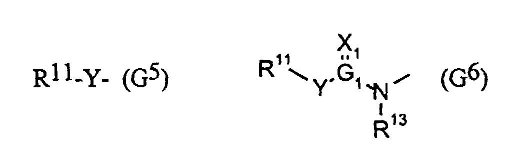 steht,
worin 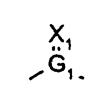 Carboxy, Thiocarboxy oder Sulfonyl bedeuten kann,
Y für Sauerstoff, Schwefel oder -NR¹² steht,
R¹¹ für den Fall, daß Y für Stickstoff steht, eine über ein Stickstoffatom verknüpfte cyclische Aminogruppe bedeuten kann,
R¹¹ und R¹² unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Aryl, Aryl-C₁₋₂-alkyl, Heteroaryl, Heteroaryl-C₁₋₂-alkyl stehen, die gegebenenfalls substituiert sind, oder
R¹¹ und R¹² gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹⁴- oder durch quaternisierten Stickstoff unterbrochen sein kann und gebenenfalls substituiert ist,
R¹³ für Wasserstoff oder C₁₋₄-Alkyl steht,
R¹⁴ für Wasserstoff, geradkettiges oder verzweigtes C₁₋₆-Alkyl, C₂₋₆-Alkenyl, C₂₋₆-Alkinyl, C₃₋₈-Cycloalkyl, C₃₋₈-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Heteroaryl, Heteroaryl-C₁₋₂-alkyl stehen, die gegebenenfalls substituiert sind, steht,
sowie deren optische Isomere und Racemate.

6. Verfahren zur Herstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) gemäß Anspruch 5 sowie deren Salze, 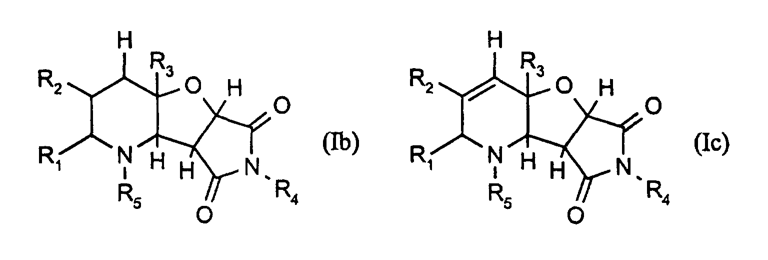 in welchen
R¹, R², R³, R⁴ und R⁵ die in Anspruch 5 angegebene Bedeutung besitzen, **dadurch gekennzeichnet, daß** man
die z. B. gemäß Verfahren 3 erhältlichen 4a, 5a, 8a, 8b-Tetrahydro-6H-pyrrolo[3',4':4,5]furo[3,2-b] pyridin-6,8(7H)-dion Derivate der allgemeinen Formel (Ia) 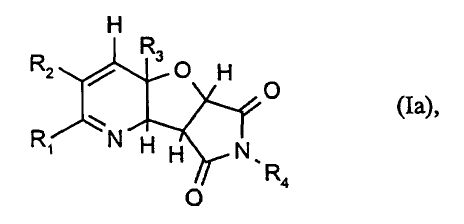 in welcher
R¹, R², R³ und R⁴ die in Anspruch 3 angegebene Bedeutung besitzen,
in einem ersten Reaktionsschritt in Gegenwart von geeigneten Katalysatoren zu 1, 2, 3, 4, 4a, 5a, 8a, 8b-Oktahydro-6H-pyrrolo[3',4':4,5]furo[3,2-b]pyridin-6,8(7H)dion- und/oder 1, 2, 4a, 5a, 8a, 8b-Hexahydro-6H-pyrrolo[3',4':4,5]furo[3,2-b]pyridin-6,8(7H)-dion Derivaten der allgemeinen Formeln ( Id ) und ( Ie ) 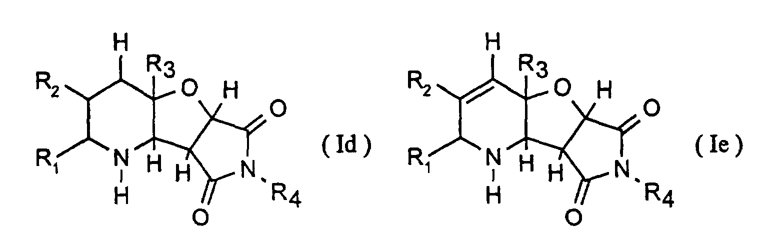 in welchen
R¹, R², R³ und R⁴ die weiter oben angegebene Bedeutung besitzen,
hydriert, oder
indem man zur selektiven Darstellung der neuen Derivate der allgemeinen Formel ( Ie ) und deren Salze die Derivate der allgemeinen Formel ( Ia ) zunächst mit Methylhalogeniden der allgemeinen Formel ( IV )
Me-Hal (IV)
in welcher
Hal für Halogen, insbesondere Fluor, Chlor, Brom oder Iod steht, gegebenenfalls in Gegenwart von Verdünnungsmitteln umsetzt, danach die gebildeten N¹-Methylammoniumhalogenide der allgemeinen Formel (V) 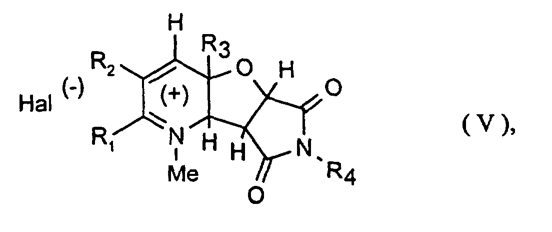 in Gegenwart eines geeigneten Verdünnungsmittels hydriert, anschließend das resultierende N¹-Methylderivat der allgemeinen Formel ( VI ) 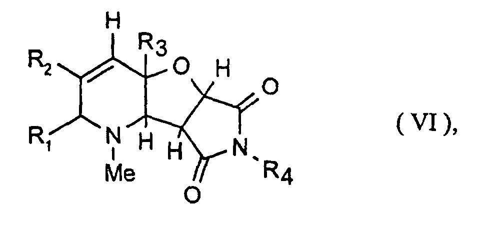 in welcher
R¹, R², R³ und R⁴ die weiter oben angegebene Bedeutung besitzen,
am N¹-Atom unter Ausbildung von ( Ie ) demethyliert, und nachfolgend
in einem zweiten Reaktionsschritt die auf diese Weise erhaltenen 1, 2, 3, 4, 4a, 5a, 8a, 8b-Oktahydro-6H-pyrrolo[3',4':4,5]füro[3,2-b]pyridin-6,8(7H)-dion- und/oder 1, 2, 4a, 5a, 8a, 8b-Hexa-hydro-6H-pyrrolo[3',4':4,5]furo[3,2-b]pyridin-6,8(7H)-dion Derivate der allgemeinen Formeln (Id) und ( Ie ) 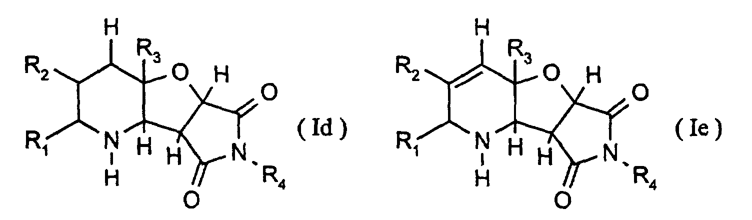 in welchen
R¹, R², R³ und R⁴ die weiter oben angegebene Bedeutung besitzen,
a) mit Verbindungen der allgemeinen Formel ( VII )
R⁵-E (VII)
in welcher
R⁵ die weiter oben angegebene Bedeutung hat, und
E für einen elektronenziehende Abgangsgruppe steht,
gegebenenfalls in Gegenwart von Verdünnungsmitteln und gegebenenfalls in Gegenwart eines basischen Reaktionshilfsmittels umsetzt, oder
b) mit Verbindungen der allgemeinen Formel ( VIII ) 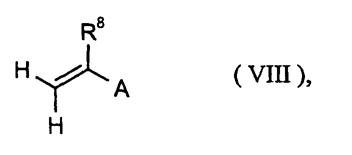 in welcher
A für eine geeignete Akzeptorgruppe, wie beispielsweise Nitro, Nitril, Carbamoyl oder R¹³-O-CO- steht, und
R⁸ und R¹³ die in Anspruch 5 angegebene Bedeutung haben, umsetzt, oder indem man
c) mit einem Epoxid der allgemeinen Formel ( IX ) 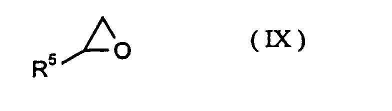 in welcher
R⁵ die unter Punkt 4 angegebene Bedeutung hat,
gegebenenfalls in Gegenwart eines Katalysators oder in Gegenwart eines basischen Reaktionshilfsmittels, gegebenenfalls in Gegenwart von Verdünnungsmitteln umsetzt, oder
d) mit Verbindungen der allgemeinen Formel (X) 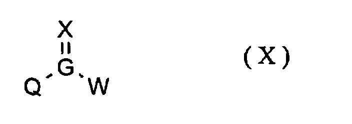 in welcher
G, Q und X die in Anspruch 5 angegebene Bedeutung haben, und
W für eine geeignete Abgangsgruppe, wie beispielsweise Halogen, Alkoxy, Alkylthio oder Aryloxy steht, gegebenenfalls in Gegenwart eines Katalysators, gegebenenfalls in Gegenwart eines Säurebindemittels und gegebenfalls in Gegenwart von Verdünnungsmitteln umsetzt, oder indem man
zur Darstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]furo[3,2-b]pyridin-6,8(7H)-dion Derivate der allgemeinen Formeln ( Ib ) und ( Ic ) und deren Salze,
in der die Gruppe 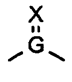 für Carboxy steht,
e) mit einem Carbonsäureanhydrid der allgemeinen Formel (XI)
(Q-C=O)₂O (XI)
in welcher
Q die unter Punkt 4 angegebene Bedeutung hat,
gegebenenfalls in Gegenwart eines Katalysators, gegebenenfalls in Gegenwart von Verdünnungsmitteln umsetzt, oder
f) mit Aminosäuederivaten der allgemeinen Formel (XII) 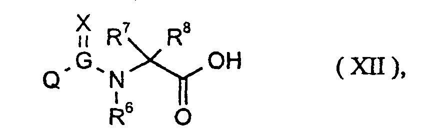 in welcher
G, Q, X , R⁶, R⁷ und R⁸ die in Anspruch 5 angegebene Bedeutung haben, bzw. deren carboxyaktivierten Derivate,
gegebenenfalls in Gegenwart eines Katalysators, gegebenenfalls in Gegenwart eines Säurebindemittels und gegebenenfalls in Gegenwart von Verdünnungsmitteln umsetzt, oder
g) mit Verbindungen der allgemeinen Formeln ( XIII ) oder ( XIV ) 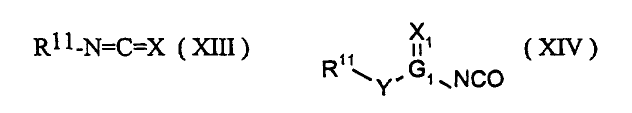 in welchen
die Reste R¹¹, G¹, X, X¹ und Y die in Anspruch 1 angegebene Bedeutung haben,
gegebenenfalls in Gegenwart eines Katalysators, gegebenenfalls in Gegenwart eines Säurebindemittels und gegebenfalls in Gegenwart von Verdünnungsmitteln umsetzt, oder indem man
zur Darstellung der neuen 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo[3',4':4,5]füro[3,2-b]pyridin-6,8(7H)-dion Derivate der allgemeinen Formeln ( Ib ) und ( Ic ) und deren Salze,
in der die Gruppe 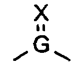 für Carboxy und Y
für Sauerstoff steht,
h) in einem dritten Reaktionsschritt mit Kohlendioxid und einem Alkalimetallcarbonat der allgemeinen Formel ( XV )
M₂CO₃ (XV)
in welcher
M für ein einwertiges Alkalimetallkation, vorzugsweise Lithium, Natrium, Kalium oder Caesium, insbesondere Kalium oder Caesium, steht,
dann in einem vierten Reaktionsschritt die resultierenden Alkalimetallsalze von Verbindungen der allgemeinen Formeln ( XVI ) und ( XVII ) 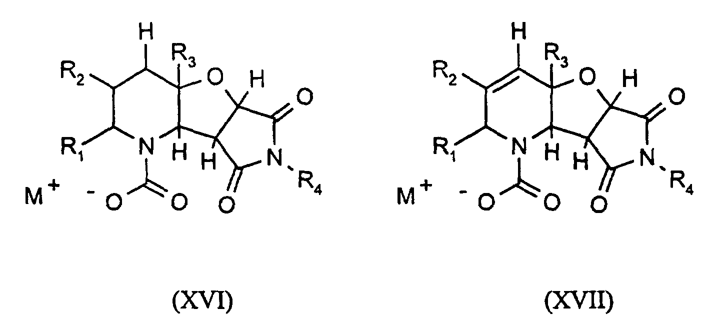
in welchen
die Reste R¹, R², R³ und R⁴ die in Anspruch 5 angegebene Bedeutung haben,
M für ein salzartig gebundenes Metallkationenäquivalent steht,
mit einem Alkylierungsmittel der Formel ( VIIa )
R¹¹-Hal (VIIa)
in welcher
R¹¹ die in Anspruch 5 angegebene Bedeutung besitzt, und
Hal für Halogen wie Fluor, Chlor, Brom oder Iod steht,
gegebenenfalls in Gegenwart eines basischen Reaktionshilfsmittels und gegebenenfalls in Gegenwart von Verdünnungsmitteln umsetzt, oder indem man
i) 1, 2, 3, 4, 4a, 5a, 8a, 8b-Oktahydro-6H-pyrrolo[3',4':4,5]furo[3,2-b]pyridin-6,8(7H)-dion- und/oder 1, 2, 4a, 5a, 8a, 8b-Hexahydro-6H-pyrrolo [3',4':4,5] furo [3,2-b]pyridin-6,8(7H)-dion Derivate der allgemeinen Formeln (If) und (Ig) 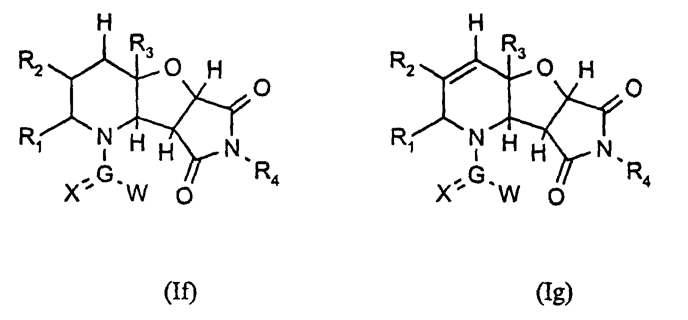 in welchen
die Reste R¹, R², R³, R⁴, G, W und X die in Anspruch 5 angegebene Bedeutung haben,
gegebenenfalls in Gegenwart eines Katalysators, gegebenenfalls in Gegenwart eines Säurebindemittels und gegebenenfalls in Gegenwart von Verdünnungsmitteln, mit Verbindungen der allgemeinen Formel ( XVIII )
R¹¹-Y-H (XVIII)
in welcher
die Reste R¹¹ und Y die in Anspruch 5 angegebene Bedeutung haben, umsetzt.

7. Endoparasitizide Mittel, **gekennzeichnet durch** einen Gehalt an mindestens einem 4a, 5a, 8a, 8b-Tetrahydro-6H-pyrrolo[3',4':4,5]furo[3,2-b]pyridin-6,8(7H)-dion Derivat der Formel (I) gemäß Anspruch 1.

8. Verfahren zur Herstellung von endoparasitiziden Mitteln, **dadurch gekennzeichnet, daß** man 4a, 5a, 8a, 8b-Tetrahydro-6H-pyrrolo[3',4':4,5]furo[3,2-b]pyridin-6,8(7H)-dion Derivate der Formel (I) gemäß Anspruch 1 mit Streckmitteln und/oder oberflächenaktiven Mitteln vermischt.

9. Verwendung von 4a, 5a, 8a, 8b-Tetrahydro-6H-pyrrolo[3',4':4,5]furo[3,2-b]pyridin-6,8(7H)-dion Derivaten der Formel (I) gemäß Anspruch 1 zur Herstellung von endoparasitiziden Mitteln.

## Claims

1. Use of 4a,5a,8a,8b-tetrahydro-6H-pyrrolo[3',4':4,5]furo[3,2-b]pyridine-6,8(7H)-dione derivatives of the general formula (I) and salts thereof, 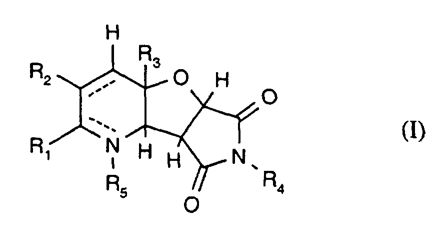 in which
R¹ represents hydrogen, straight-chain or branched alkyl, cycloalkyl, arylalkyl, aryl, heteroaryl, heteroarylalkyl, which are optionally substituted,
R² represents hydrogen, straight-chain or branched alkyl, cycloalkyl, alkoxycarbonyl, which are optionally substituted,
R¹ and R², together with the atoms to which they are bonded, represent a 5-or 6-membered ring, which can optionally be interrupted by oxygen, sulphur, sulphoxyl or sulphonyl and is optionally substituted,
R³ represents hydrogen, straight-chain or branched alkyl, cycloalkyl, alkoxycarbonyl, which are optionally substituted,
R⁴ represents hydrogen, straight-chain or branched alkyl, alkenyl, alkinyl, cycloalkyl, cycloalkylalkyl, aryl, arylalkyl, heteroaryl, heteroarylalkyl, amino, alkylamino, dialkylamino, cycloalkylamino, which are optionally substituted,
R⁵ represents hydrogen, straight-chain or branched alkyl, alkenyl, alkinyl, cycloalkyl, cycloalkylalkyl, aryl, arylalkyl, heteroaryl, heteroarylalkyl, which are optionally substituted, formyl, alkoxydicarbonyl or optionally represents a radical from the group consisting of G¹, G², G³ and G⁴ 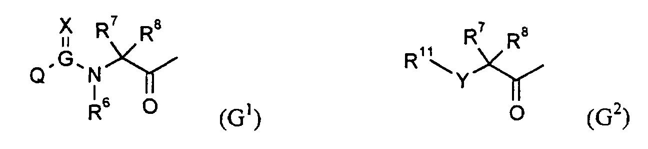 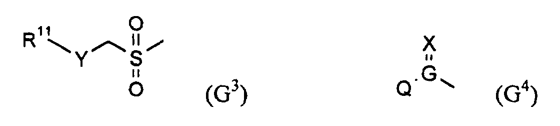 in which
R⁶ represents hydrogen, straight-chain or branched alkyl, cycloalkyl, aryl, arylalkyl, heteroaryl, heteroarylalkyl, which are optionally substituted,
R⁶ and R⁷, together with the atoms to which they are bonded, represent a 5- or 6-membered ring, which can optionally be interrupted by oxygen, sulphur, sulphoxyl or sulphonyl and is optionally substituted,
R⁷ and R⁸ independently of one another represent hydrogen, straight-chain or branched alkyl, alkenyl, cycloalkyl, cycloalkylalkyl, aryl, arylalkyl, heteroaryl, heteroarylalkyl, which are optionally substituted, or
R⁷ and R⁸ together represent a spirocyclic ring which is optionally substituted, 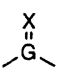 can denote carboxyl, thiocarboxyl, -C=CH-NO₂, -C=CH-CN, -C=N-R⁹, sulphoxyl, sulphonyl, -P(O)-OR¹⁰ or P(S)-OR¹⁰,
R⁹ represents hydrogen, hydroxyl, alkoxy, alkylcarbonyl, halogenoalkylcarbonyl, alkylsulphonyl, nitro or cyano, and
R¹⁰ represents hydrogen or alkyl, and
Q represents straight-chain or branched alkyl, alkenyl, alkinyl, cycloalkyl, aryl, arylalkyl, hetaryl or hetarylalkyl, which are optionally substituted, or optionally represents a radical from the group consisting of G⁵ and G⁶ 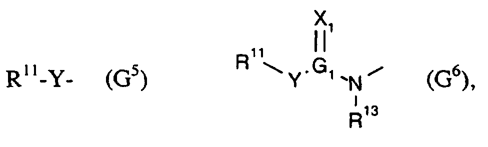 in which 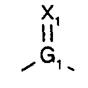 can denote carboxyl, thiocarboxyl or sulphonyl,
Y represents oxygen, sulphur or -NR¹²,
R¹¹, in the case where Y represents nitrogen, can denote a cyclic amino group linked via a nitrogen atom,
R¹¹ and R¹² independently of one another represent hydrogen, straight-chain or branched alkyl, alkenyl, alkinyl, cycloalkyl, cycloalkylalkyl, aryl, arylalkyl, heteroaryl, heteroarylalkyl, which are optionally substituted, or
R¹¹ and R¹², together with the adjacent N atom, represent a carbocyclic 5-, 6- or 7-membered ring system or represent a 7- to 10-membered bicyclic ring system, which can optionally also be interrupted by oxygen, sulphur, sulphoxyl, sulphonyl, carbonyl, -N-O, -N=, -NR¹⁴- or by quatemized nitrogen and is optionally substituted,
R¹³ represents hydrogen or alkyl,
R¹⁴ represents hydrogen, straight-chain or branched alkyl, alkenyl, alkinyl, cycloalkyl, cycloalkylalkyl, alkoxycarbonyl, alkylcarbonyl, cycloalkylcarbonyl, cyano, aryl, arylalkyl, heteroaryl, heteroarylalkyl, which are optionally substituted, and optical isomers and racemates thereof,
for the preparation of compositions for the control of endoparasites in medicine and veterinary medicine,
where optionally substituted radicals may carry one or more of the following substituents:
alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms;
hydroxyl, halogen, cyano, nitro, amino, monoalkyl- and dialkylamino having 1 to 4 carbon atoms per alkyl group, alkylcarbonyl having 2 to 4 carbon atoms, alkoxycarbonyl having 2 to 4 carbon atoms, alkylsulphinyl having 1 to 4 carbon atoms, halogenoalkylsulphinyl having 1 to 4 carbon atoms and 1 to 5 halogen atoms,
sulphonyl, alkylsulphonyl having 1 to 4 carbon atoms,
halogenoalkylsulphonyl having 1 to 4 carbon atoms and 1 to 5 halogen atoms,
arylsulphonyl having 6 to 10 aryl carbon atoms;
acyl, aryl, aryloxy, hetaryl, hetaryloxy, each of which may in turn carry an abovementioned radical;
formimino radical,
and where in the above definitions alkyl, alone or as part of a radical, has 1 to 6 carbon atoms and may be straight-chain or branched,
cycloalkyl, alone or as part of a radical, has 3 to 10 carbon atoms and may be mono-, bi- or tricyclic,
alkoxy, alone or as part of a radical, has 1 to 6 carbon atoms and may be straight-chain or branched;
arylalkyl has 6 to 10 carbon atoms in the aryl moiety and 1 to 4 carbon atoms in the alkyl moiety,
heteroaryl denotes a 5- to 7-membered ring having 1 to 3 identical or different heteroatoms selected from oxygen, sulphur and nitrogen;
alkoxycarbonyl has 1 to 6 carbon atoms and may be straight-chain or branched; alkenyl and alkinyl each have 1 to 6 carbon atoms and may be straight-chain or branched;
halogenoalkyl, alone or as part of a radical, contains 1 to 4 carbon atoms and I to 9 identical or different halogen atoms,
alkylcarbonyl has 1 to 6 carbon atoms and may be straight-chain or branched;
cycloalkylcarbonyl denotes mono-, bi- or tricyclic cycloalkylcarbonyl having 3 to 10 carbon atoms.

2. Use of compounds of the formula (I) according to Claim 1,
in which
R¹ represents hydrogen, straight-chain or branched alkyl having up to 4 carbon atoms, C₃₋₆-cycloalkyl, aryl-C₁₋₂-alkyl, aryl, heteroaryl, heteroaryl-C₁₋₂-alkyl, which are optionally substituted,
R¹ and R², together with the atoms to which they are bonded, represent a 5-or 6-membered ring, which is optionally substituted,
R² and R³ independently of one another represent hydrogen, straight-chain or branched alkyl having up to 4 carbon atoms, halogenoalkyl, hydroxyalkyl, C₁₋₄-alkanoyloxyalkyl, C₁₋₂-alkoxyalkyl, C₁₋₂-mercaptoalkyl, C₁₋₂-alkylthioalkyl, C₁₋₂alkylsulphinylalkyl, C₁₋₂-alkylsulphonylalkyl, aminoalkyl, C₁₋₆-alkylaminoalkyl, C₁₋₆-dialkylaminoalkyl, C₃₋₆-cycloalkylaminoalkyl, C₃₋₆-cycloalkyl, C₁₋₄-alkoxycarbonyl,
R⁴ represents hydrogen, straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, C₁₋₄alkoxycarbonyl-C₁₋₄-alkyl, amino-C₁₋₆-alkyl, C₁₋₆-alkylamino-C₁₋₆-alkyl, C₁₋₆-dialkylamino-C₁₋₆-alkyl, C₁₋₆-trialkylammonium-C₁₋₆-alkyl halide, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, amino, C₁-C₄-alkylamino, C₁-C₄-dialkylamino, C₃-C₇-cycloalkylamino, which are optionally substituted,
R⁵ represents hydrogen, straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, amino-C₁₋₆-alkyl, C₁₋₆-alkylamino-C₁₋₆-alkyl C₁₋₆-dialkylamino-C₁₋₆-alkyl, C₁₋₆-trialkylammonium-C₁₋₆-alkyl halide, nitroC₁₋₄-alkyl, cyano-C₁₋₄-alkyl, C₁₋₄-alkoxycarbonyl-C₁₋₄-alkyl, carbamoyl-C₁₋₄-alkyl, carboxyl-C₁₋₄-alkyl, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, formyl, C₁₋₄-alkoxydicarbonyl, or represents a radical from the group consisting of G¹, G², G³ and G⁴ 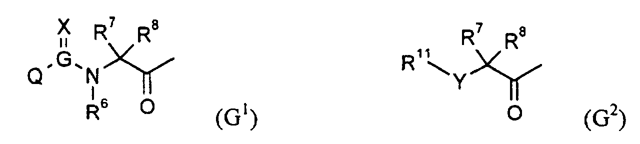 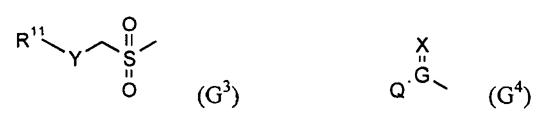 in which
R⁶ represents hydrogen, straight-chain or branched C₁₋₆-alkyl, C₃₋₆-cycloalkyl, aryl-C₁₋₂-alkyl, heteroaryl-C₁₋₂-alkyl, which are optionally substituted,
R⁶ and R⁷, together with the atoms to which they are bonded, represent a 5- or 6-membered ring, which can optionally be interrupted by oxygen, sulphur, sulphoxyl or sulphonyl and is optionally substituted,
R⁷ and R⁸ independently of one another represent hydrogen, straight-chain or branched C₁₋₆-alkyl, halogeno-C₁₋₆-alkyl, hydroxy-C₁₋₆-alkyl, C₁₋₄-alkanoyloxy-C₁₋₆-alkyl, C₁₋₂-alkoxy-C₁₋₆-alkyl, mercapto-C₁₋₆-alkyl, C₁₋₂-alkylthio-C₁₋₆-alkyl, C₁₋₂-alkylsulphinyl-C₁₋₆-alkyl, C₁₋₂-alkylsulphonyl-C₁₋₆-alkyl, carboxyl-C₁₋₆-alkyl, carbamoyl-C₁₋₆-alkyl, amino-C₁₋₆-alkyl, C₁₋₆alkylamino-C₁₋₆-alkyl, C₁₋₆-dialkylamino-C₁₋₆-alkyl, guanidino-C₁₋₆-alkyl, which radical can optionally be substituted by one or two benzyloxycarbonyl radicals or by one, two, three or four C₁₋₂-alkyl radicals, C₁₋₄-alkoxycarbonylamino-C₁₋₆-alkyl, C₂₋₆-alkenyl, C₃₋₆-cyclo-C₁₋₂-alkyl, C₃₋₆-cycloalkyl-C₁₋₂-alkyl, and represent optionally substituted aryl, aryl-C₁₋₂-alkyl, heteroaryl, heteroaryl-C₁₋₂-alkyl, or
R⁷ and R⁸ together represent a spirocyclic ring, 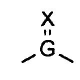 denotes carboxyl, thiocarboxyl, -C=CH-NO₂, -C=CH-CN, -C=N-R⁹, sulphoxyl, sulphonyl, -P(O)-OR¹⁰ or P(S)-OR¹⁰,
R⁹ represents hydrogen, hydroxyl, C₁₋₄-alkoxy, C₁₋₄-alkylcarbonyl, halogeno-C₁₋₄-alkylcarbonyl, C₁₋₄-alkylsulphonyl, nitro or cyano, and
R¹⁰ represents hydrogen or C₁₋₄-alkyl, and
Q represents straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, mercaptoalkyl, C₁₋₂-alkylthioalkyl, C₁₋₂-alkylsulphinylalkyl, C₁₋₂-alkylsulphonylalkyl, carboxyalkyl, carbamoylalkyl, amino-C₁₋₆-alkyl, C₁₋₆-alkylamino-C₁₋₆-alkyl, C₁₋₆-dialkylamino-C₁₋₆-alkyl, guanidino-C₁₋₆-alkyl, which radical can optionally be substituted by one or two benzyloxycarbonyl radicals, tert-butyloxycarbonyl radicals or by one, two, three or four C₁₋₂-alkyl radicals, C₁₋₄-alkoxycarbonylaminoalkyl, C₂₋₆-alkenyl, C₂₋₆-alkinyl, C₃₋₆-cycloalkyl, aryl, aryl-C₁₋₂-alkyl, heteroaryl or heteroaryl-C₁₋₂-alkyl, which are optionally substituted, or optionally represents a radical from the group consisting of G⁵ and G⁶ 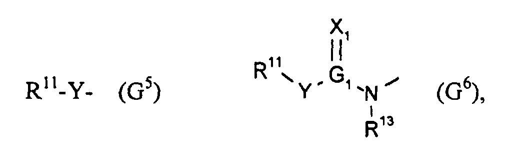 in which 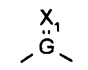 can denote carboxyl, thiocarboxyl or sulphonyl,
Y represents oxygen, sulphur or -NR¹²,
R¹¹, in the case where Y represents nitrogen, can denote a cyclic amino group linked by a nitrogen atom,
R¹¹ and R¹² independently of one another represent hydrogen, straight-chain or branched C₁₋₆-alkyl, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, which are optionally substituted, or
R¹¹ and R¹², together with the adjacent N atom, represent a carbocyclic 5-, 6- or 7- membered ring system, or represent a 7- to 10-membered bicyclic ring system, which can optionally also be interrupted by oxygen, sulphur, sulphoxyl, sulphonyl, carbonyl, -N-O, -N=, -NR¹⁴- or by quaternized nitrogen and is optionally substituted,
R¹³ represents hydrogen or C₁₋₄-alkyl,
R¹⁴ represents hydrogen, straight-chain or branched C₁₋₆-alkyl, C₂₋₆-alkenyl, C₂₋₆-alkinyl, C₃₋₈-cycloalkyl, C₃₋₈-cycloalkyl-C₁₋₂-alkyl, C₁₋₄-alkoxycarbonyl, C₁₋₄-alkylcarbonyl, C₃₋₆-cycloalkylcarbonyl, cyano, aryl, aryl-C₁₋₂-alkyl, heteroaryl, heteroaryl-C₁₋₂-alkyl, which are optionally substituted,
and optical isomers and racemates thereof, for the preparation of compositions for the control of endoparasites in medicine and veterinary medicine,
where optionally substituted radicals may carry one or more of the following substituents:
alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms;
hydroxyl, halogen, cyano, nitro, amino, monoalkyl- and dialkylamino having 1 to 4 carbon atoms per alkyl group, alkylcarbonyl having 2 to 4 carbon atoms, alkoxycarbonyl having 2 to 4 carbon atoms, alkylsulphinyl having 1 to 4 carbon atoms, halogenoalkylsulphinyl having 1 to 4 carbon atoms and 1 to 5 halogen atoms,
sulphonyl, alkylsulphonyl having 1 to 4 carbon atoms,
halogenoalkylsulphonyl having 1 to 4 carbon atoms and 1 to 5 halogen atoms,
arylsulphonyl having 6 to 10 aryl carbon atoms;
acyl, aryl, aryloxy, hetaryl, hetaryloxy, each of which may in turn carry an abovementioned radical;
formimino radical,
and where in the above definitions heteroaryl denotes a 5- to 7-membered ring having 1 to 3 identical or different heteroatoms selected from oxygen, sulphur and nitrogen.

3. 4a,5a,8a,8b-tetrahydro-6H-pyrrolo[3',4':4,5]furo[3,2-b]pyridine-6,8(7H)-dione derivatives of the general formula (Ia) and salts thereof, 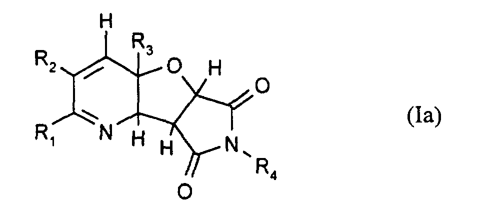 in which
R¹ represents hydrogen, straight-chain or branched alkyl having up to 4 carbon atoms, C₃₋₆-cycloalkyl, aryl-C₁₋₂-alkyl, aryl, heteroaryl, heteroaryl-C₁₋₂-alkyl, which are optionally substituted,
R² and R³ independently of one another represent hydrogen, straight-chain or branched alkyl having up to 4 carbon atoms, halogenoalkyl, hydroxyalkyl, C₁₋₄-alkanoyloxyalkyl, C₁₋₂-alkoxyalkyl, C₁₋₂-mercaptoalkyl, C₁₋₂-alkylthioalkyl, C₁₋₂-alkylsulphinylalkyl, C₁₋₂-alkylsulphonylalkyl, aminoalkyl, C₁₋₆-alkylaminoalkyl, C₁₋₆-dialkylaminoalkyl, C₃₋₆-cycloalkylaminoalkyl, C₃₋₆-cycloalkyl, C₁₋₄-alkoxycarbonyl,
R⁴ represents hydrogen, straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, C₁₋₄-alkoxycarbonyl-C₁₋₄-alkyl, amino-C₁₋₆-alkyl, C₁₋₆-alkylamino-C₁₋₆-alkyl, C₁₋₆-dialkylamino-C₁₋₆-alkyl, C₁₋₆-trialkylammonium-C₁₋₆-alkyl halide, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, amino, C₁-C₄-alkylamino, C₁-C₄-dialkylamino, C₃-C₇-cycloalkylamino, which are optionally substituted,
with the proviso in the case where
R¹ represents hydrogen, and
R² and R² represent methyl,
R⁴ does not represent methyl, n-butyl, phenyl, 2-, 3- or 4-methylphenyl, 2-, 3- or 4-chlorophenyl, 2-, 3- or 4-fluorophenyl, 2-chloro, 4-fluorophenyl, 2-fluoro, 4-chlorophenyl, 3-chloro, 4-fluorophenyl, 2-, 3- or 4-nitrophenyl, 4-methoxyphenyl or α-naphthyl,
and optical isomers and racemates thereof.

4. Process for the preparation of the novel 4a,5a,8a,8b-tetrahydro-6H-pyrrolo[3',4':4,5]furo[3,2-b]pyridine-6,8(7H)-dione derivatives of the general formula (Ia) according to Claim 3 and salts thereof, 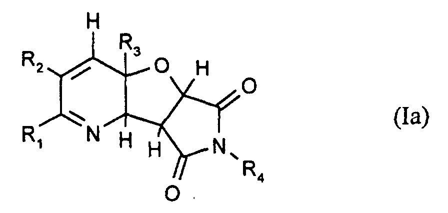 in which
R¹, R², R³ and R⁴ have the meaning given in Claim 3,
**characterized in that**
a) pyridine N-oxides of the general formula (II) 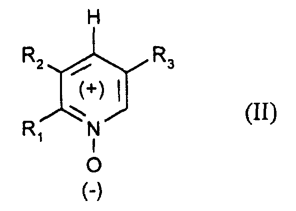 in which
R¹, R² and R³ have the meaning given in Claim 3, are reacted with maleic acid imides of the general formula (III) 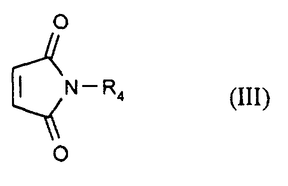 in which
R⁴ has the meaning given in Claim 3, if appropriate in the presence of a diluent.

5. Novel 1,2,3,4,4a,5a,8a,8b-octahydro-6H-pyrrolo[3',4':4,5]furo[3,2-b]pyridine-6,8(7H)-dione and 1,2,4a,5a,8a,8b-hexahydro-6H-pyrrolo[3',4':4,5]furo[3,2-b]pyridine-6,8(7H)-dione derivatives of the general formulae (Ib) and (Ic) and salts thereof, 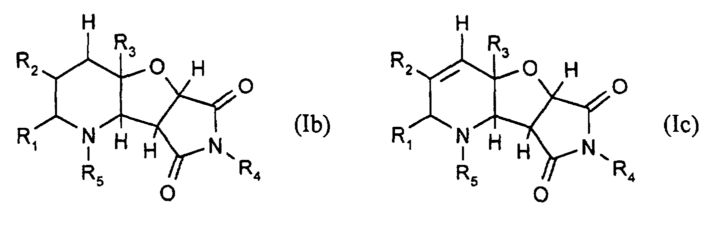 in which
R¹ represents hydrogen, straight-chain or branched alkyl having up to 4 carbon atoms, C₃₋₆-cycloalkyl, aryl-C₁₋₂-alkyl, aryl, heteroaryl, heteroaryl-C₁₋₂-alkyl, which are optionally substituted,
R² and R³ independently of one another represent hydrogen, straight-chain or branched alkyl having up to 4 carbon atoms, halogenoalkyl, hydroxyalkyl, C₁₋₄-alkanoyloxyalkyl, C₁₋₂-alkoxyalkyl, C₁₋₂-mercaptoalkyl, C₁₋₂-alkylthioalkyl, C₁₋₂alkylsulphinylalkyl, C₁₋₂-alkylsulphonylalkyl, aminoalkyl, C₁₋₆-alkylaminoalkyl, C₁₋₆-dialkylaminoalkyl, C₃₋₆-cycloalkylaminoalkyl, C₃₋₆-cycloalkyl, C₁₋₄-alkoxycarbonyl,
R⁴ represents hydrogen, straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, C₁₋₄alkoxycarbonyl-C₁₋₄-alkyl, amino-C₁₋₆-alkyl, C₁₋₆-alkylamino-C₁₋₆-alkyl, C₁₋₆-dialkylamino-C₁₋₆alkyl, C₁₋₆-trialkylammonium-C₁₋₆-alkyl halide, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, amino, C₁-C₄-alkylamino, C₁-C₄-dialkylamino, C₃-C₇-cycloalkylamino, which are optionally substituted,
R⁵ represents hydrogen, straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, amino-C₁₋₆-alkyl, C₁₋₆-alkylamino-C₁₋₆-alkyl, C₁₋₆-dialkylamino-C₁₋₆-alkyl, C₁₋₆-trialkylammonium-C₁₋₆-alkyl halide, nitro-C₁₋₄-alkyl, cyano-C₁₋₄-alkyl, C₁₋₄-alkoxycarbonyl-C₁₋₄-alkyl, carbamoyl-C₁₋₄-alkyl, carboxyl-C₁₋₄-alkyl, C₂₋₆-alkenyl, C₂₋₆-alkinyl, C₃₋₆-cycloalkyl, C₃₋₆-cycloalkyl-C₁₋₂-alkyl, aryl, aryl-C₁₋₂alkyl, heteroaryl, heteroaryl-C₁₋₂-alkyl, formyl, C₁₋₄-alkoxydicarbonyl, or represents a radical from the group consisting of G¹, G², G³ and G⁴ 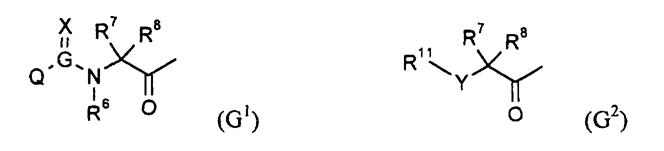 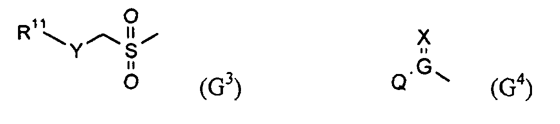 in which
R⁶ represents hydrogen, straight-chain or branched C₁₋₆-alkyl, C₃₋₆-cycloalkyl, aryl-C₁₋₂-alkyl, heteroaryl-C₁₋₂-alkyl, which are optionally substituted,
R⁶ and R⁷, together with the atoms to which they are bonded, represent a 5-or 6-membered ring, which can optionally be interrupted by oxygen, sulphur, sulphoxyl or sulphonyl and is optionally substituted,
R⁷ represents hydrogen, straight-chain or branched C₁₋₆-alkyl,C₂₋₄-alkenyl, C₃₋₆-cycloalkyl, C₃₋₆-cycloalkyl-C₁₋₂-alkyl, and represents optionally substituted aryl, aryl-C₁₋₂-alkyl, heteroaryl, heteroaryl-C₁₋₂-alkyl,
R⁸ represents hydrogen or methyl, 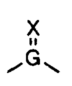 can denote carboxyl, thiocarboxyl, -C=CH-NO₂, -C=CH-CN, -C=N-R⁹, sulphoxyl, sulphonyl, -P(O)-OR¹⁰ or P(S)-OR¹⁰,
R⁹ represents hydrogen, hydroxyl, C₁₋₄-alkoxy, C₁₋₄-alkylcarbonyl, halogeno-C₁₋₄-alkylcarbonyl, C₁₋₄-alkylsulphonyl, nitro or cyano, and
R¹⁰ represents hydrogen or C₁₋₄-alkyl, and
Q represents straight-chain or branched C₁₋₆-alkyl, halogeno-C₁₋₆-alkyl, hydroxy-C₁₋₆-alkyl, C₁₋₄-alkanoyloxyalkyl, C₁₋₂-alkoxyalkyl, mercaptoalkyl, C₁₋₂-alkylthioalkyl, C₁₋₂-alkylsulphinylalkyl, C₁₋₂-alkylsulphonylalkyl, carboxyalkyl, carbamoylalkyl, amino-C₁₋₆-alkyl, C₁₋₆-alkylamino-C₁₋₆-alkyl, C₁₋₆-dialkylamino-C₁₋₆-alkyl, guanidino-C₁₋₆-alkyl, which radical can optionally be substituted by one or two benzyloxycarbonyl radicals, tert-butyloxycarbonyl radicals or by one, two, three or four C₁₋₂-alkyl radicals, C₁₋₄-alkoxycarbonylaminoalkyl, C₂₋₆-alkenyl, C₂₋₆-alkinyl, C₃₋₆-cycloalkyl, aryl, aryl-C₁₋₂-alkyl, heteroaryl or heteroaryl-C₁₋₂-alkyl, which are optionally substituted, or optionally represents a radical from the group consisting of G⁵ and G⁶ 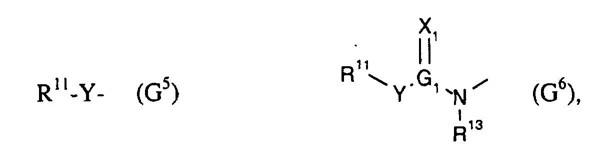 in which 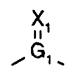 can denote carboxyl, thiocarboxyl or sulphonyl,
Y represents oxygen, sulphur or -NR¹²,
R¹¹, in the case where Y represents nitrogen, can denote a cyclic amino group linked by a nitrogen atom,
R¹¹ and R¹² independently of one another represent hydrogen, straight-chain or branched C₁₋₆-alkyl, C₂₋₆-alkenyl, C₂₋₆-alkinyl, C₃₋₆-cycloalkyl, C₃₋₆-cycloalkyl-C₁₋₂-alkyl, aryl, aryl-C₁₋₂-alkyl, heteroaryl, heteroaryl-C₁₋₂-alkyl, which are optionally substituted, or
R¹¹ and R¹², together with the adjacent N atom, represent a carbocyclic 5-, 6- or 7-membered ring system, or represent a 7- to 10-membered bicyclic ring system, which can optionally also be interrupted by oxygen, sulphur, sulphoxyl, sulphonyl, carbonyl, -N-O, -N=, -NR¹⁴- or by quatemized nitrogen and is optionally substituted,
R¹³ represents hydrogen or C₁₋₄-alkyl,
R¹⁴ represents hydrogen, straight-chain or branched C₁₋₆-alkyl, C₂₋₆-alkenyl, C₂₋₆-alkinyl, C₃₋₈-cycloalkyl, C₃₋₈-cycloalkyl-C₁₋₂-alkyl, C₁₋₄-alkoxycarbonyl, C₁₋₄-alkylcarbonyl, C₃₋₆-cycloalkylcarbonyl, cyano, aryl, aryl-C₁₋₂-alkyl, heteroaryl, heteroaryl-C₁₋₂-alkyl, which are optionally substituted,
and optical isomers and racemates thereof.

6. Process for the preparation of the novel 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo[3',4':4,5]furo[3,2-b]pyridine-6,8(7H)-dione derivatives of the general formulae (Ib) and (Ic) according to Claim 5 and salts thereof, 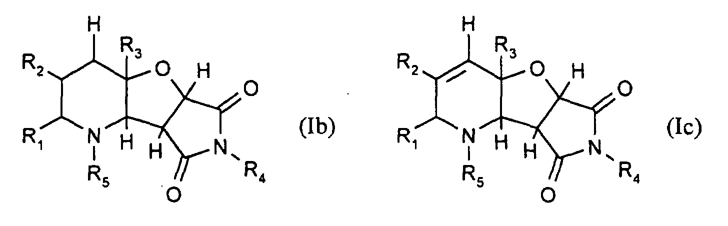 in which
R¹, R², R³, R⁴ and R⁵ have the meaning given in Claim 5, **characterized in that**
the 4a,5a,8a,8b-tetrahydro-6H-pyrrolo[3',4':4,5]furo[3,2-b] pyridine-6,8(7H)-dione derivatives, obtainable, for example, according to process 3, of the general formula (Ia) 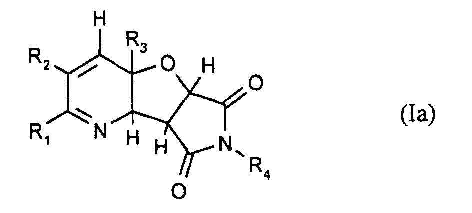 in which
R¹, R², R³ and R⁴ have the meaning given in Claim 3,
are hydrogenated in a first reaction step in the presence of suitable catalysts to 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo[3',4':4,5]furo[3,2-b]pyridine-6,8(7H)-dione derivatives of the general formulae (Id) and (Ie) 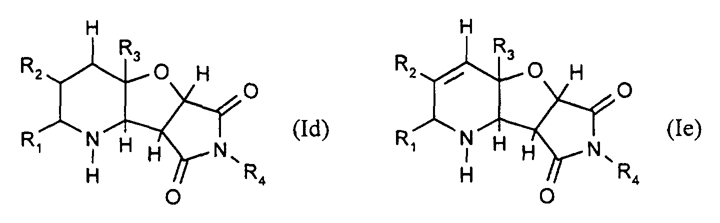 in which
R¹, R², R³ and R⁴ have the meaning given above, or
by first of all, for the selective preparation of the novel derivatives of the general formula (Ie) and their salts, reacting the derivatives of the general formula (Ia) with methyl halides of the general formula (IV)
Me-Hal (IV)
in which
Hal represents halogen, especially fluorine, chlorine, bromine or iodine, optionally in the presence of diluents, then the N¹-methylammonium halides of the general formula (V) 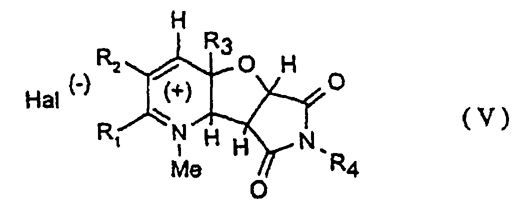 that are formed are hydrogenated in the presence of a suitable diluent, then the resulting N¹-methyl derivative of the general formula (VI) 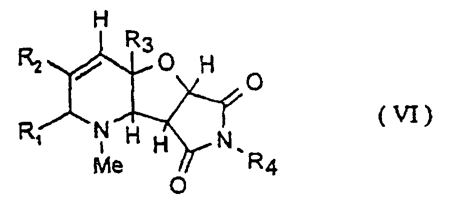 in which
R¹, R², R³ and R⁴ have the meaning given above
are demethylated on the N¹ atom to form (Ie), and subsequently
in a second reaction step the resultant 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo[3',4':4,5]furo[3,2-b]pyridine-6,8(7H)-dione derivatives of the general formulae (Id) and (Ie) 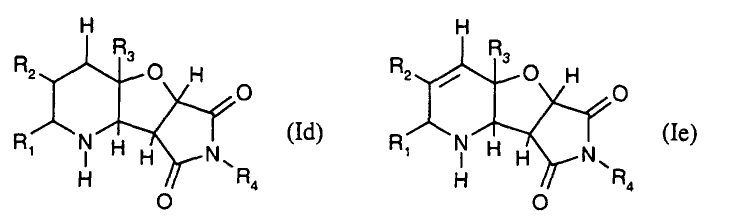 in which
R¹, R², R³ and R⁴ have the meaning given above,
a) are reacted with compounds of the general formula (VII)
R⁵-E (VII)
in which
R⁵ has the meaning given above, and
E represents an electron-withdrawing leaving group,
if appropriate in the presence of diluents and if appropriate in the presence of a basic reaction auxiliary, or
b) with compounds of the general formula (VIII) 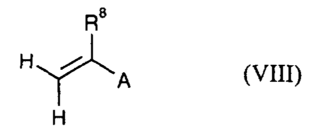 in which
A represents a suitable acceptor group, such as, for example, nitro, nitrile, carbamoyl or R¹³-O-CO-, and
R⁸ and R¹³ have the meaning given in Claim 5, or **in that**
c) are reacted with an epoxide of the general formula (IX) 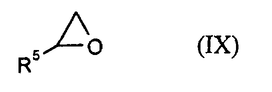 in which
R⁵ has the meaning given in Claim 5,
if appropriate in the presence of a catalyst or in the presence of a basic reaction auxiliary, if appropriate in the presence of diluents, or
d) are reacted with compounds of the general formula (X) 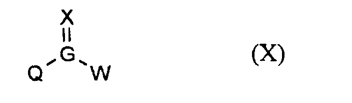 in which
G, Q and X have the meaning given in Claim 5, and
W represents a suitable leaving group, such as, for example, halogen, alkoxy, alkylthio or aryloxy, if appropriate in the presence of a catalyst, if appropriate in the presence of an acid-binding agent and if appropriate in the presence of diluents, or **in that**
to prepare the novel 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo[3',4':4,5]furo[3,2-b]pyridine-6,8-(7H)-dione derivatives of the general formulae (Ib) and (Ic) and salts thereof, in which the group 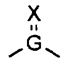 represents carboxyl,
e) are reacted with a carboxylic acid anhydride of the general formula (XI)
(Q-C=O)₂O (XI)
in which
Q has the meaning given in Claim 5,
if appropriate in the presence of a catalyst, if appropriate in the presence of diluents, or
f) are reacted with amino acid derivatives of the general formula (XII) 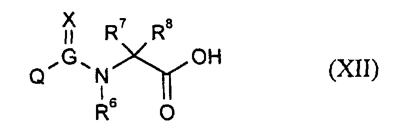 in which
G, Q, X, R⁶, R⁷ and R⁸ have the meaning given in Claim 5, or carboxyl-activated derivatives thereof,
if appropriate in the presence of a catalyst, if appropriate in the presence of an acid-binding agent and if appropriate in the presence of diluents, or
g) are reacted with compounds of the general formulae (XIII) or (XIV) 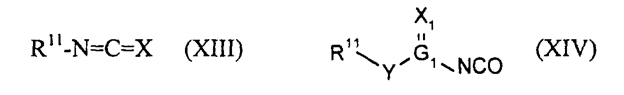 in which
the radicals R¹¹, G¹, X, X¹ and Y have the meaning given in Claim 1,
if appropriate in the presence of a catalyst, if appropriate in the presence of an acid-binding agent and if appropriate in the presence of diluents, or **in that**
to prepare the novel 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo[3',4':4,5]furo[3,2-b]pyridine-6,8(7H)-dione derivatives of the general formulae (Ib) and (Ic) and salts thereof,
in which the group 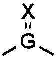 represents carboxyl and Y represents oxygen,
h) in a third reaction step are reacted with carbon dioxide and an alkali metal carbonate of the general formula (XV)
M₂CO₃ (XV)
in which
M represents a monovalent alkali metal cation, preferably lithium, sodium, potassium or caesium, in particular potassium or caesium,
and then, in a fourth reaction step, the resulting alkali metal salts of compounds of the general formulae (XVI) and (XVII) 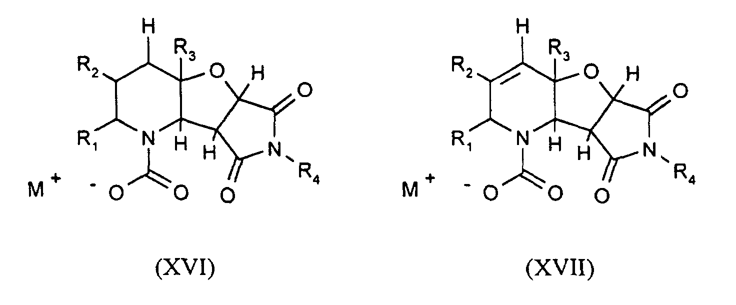
in which
the radicals R¹, R², R³ and R⁴ have the meaning given in Claim 5,
M represents one metal cation equivalent bonded in salt form,
are reacted with an alkylating agent of the formula (VIIa)
R¹¹-Hal (VIIa)
in which
R¹¹ has the meaning given under Claim 5, and
Hal represents halogen, such as fluorine, chlorine, bromine or iodine,
if appropriate in the presence of a basic reaction auxiliary and if appropriate in the presence of diluents, or **in that**
i) 1,2,3,4,4a,5a,8a,8b-octahydro-6H-pyrrolo[3',4':4,5]furo[3,2-b]pyridine-6,8(7H)-dione and/or 1,2,4a,5a,8a,8b-hexahydro-6H-pyrrolo-[3',4,':4,5]furo[3,2-b]pyridine-6,8(7H)-dione derivatives of the general formulae (If) and (Ig) 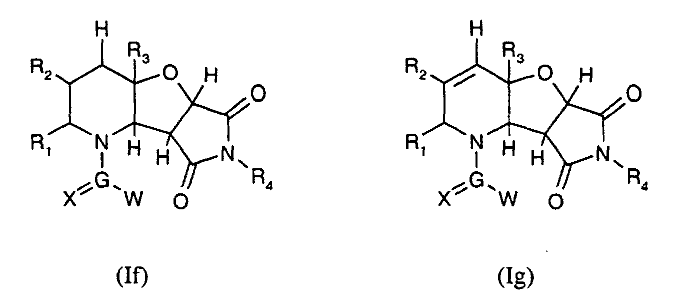 in which
the radicals R¹, R², R³, R⁴, G, W and X have the meaning given in Claim 5,
if appropriate in the presence of a catalyst, if appropriate in the presence of an acid-binding agent and if appropriate in the presence of diluents, are reacted with compounds of the general formula (XVIII)
R¹¹-Y-H (XVIII)
in which
the radicals R¹¹ and Y have the meaning given in Claim 5.

7. Endoparasiticidal composition, **characterized by** a content of at least one 4a,5a,8a,8b-tetrahydro-6H-pyrrolo[3',4':4,5]furo[3,2-b]pyridine-6,8(7H)-dione derivative of the formula (I) according to Claim 1.

8. Process for the preparation of endoparasiticidal compositions, **characterized in that** 4a,5a,8a,8b-tetrahydro-6H-pyrrolo[3',4':4,5]-furo[3,2-b]pyridine-6,8(7H)-dione derivatives of the formula (I) according to Claim 1 are mixed with extenders and/or surface-active compositions.

9. Use of 4a,5a,8a,8b-tetrahydro-6H-pyrrolo[3',4':4,5]furo[3,2-b]pyridine-6,8(7H)-dione derivatives of the formula (I) according to Claim 1 for the preparation of endoparasiticidal compositions.

## Revendications

1. Utilisation de dérivés de 4a,5a,8a,8b-tétrahydro-6H-pyrrolo[3',4':4,5]furo[3,2-b]pyridine-6,8(7H)-dione de formule générale (I) et de ses sels, 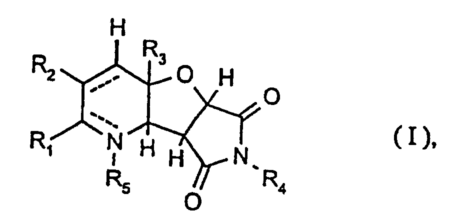 formule dans laquelle
R¹ représente l'hydrogène, un groupe alkyle linéaire ou ramifié, cycloalkyle, arylalkyle, aryle, hétéroaryle, hétéroarylalkyle, ces groupes étant éventuellement substitués,
R² représente l'hydrogène, un groupe alkyle linéaire ou ramifié, cycloalkyle, alkoxycarbonyle, ces groupes étant éventuellement substitués,
R¹ et R² forment conjointement avec les atomes auxquels ils sont liés un noyau pentagonal ou hexagonal qui peut éventuellement être interrompu par de l'oxygène, du soufre, un groupe sulfoxyle ou un groupe sulfonyle et qui est éventuellement substitué,
R³ représente l'hydrogène, un groupe alkyle linéaire ou ramifié, cycloalkyle, alkoxycarbonyle, ces groupes étant éventuellement substitués,
R⁴ représente l'hydrogène, un groupe alkyle linéaire ou ramifié, alcényle, alcynyle, cycloalkyle, cycloalkylalkyle, aryle, arylalkyle, hétéroaryle, hétéroarylalkyle, amino, alkylamino, dialkylamino, cycloalkylamino, ces groupes étant éventuellement substitués,
R⁵ représente l'hydrogène, un groupe alkyle linéaire ou ramifié, alcényle, alcynyle, cycloalkyle, cycloalkylalkyle, aryle, arylalkyle, hétéroaryle, hétéroarylalkyle, ces groupes étant éventuellement substitués, formyle, alkoxydicarbonyle ou représente éventuellement un reste du groupe G¹, G², G³ ou G⁴, 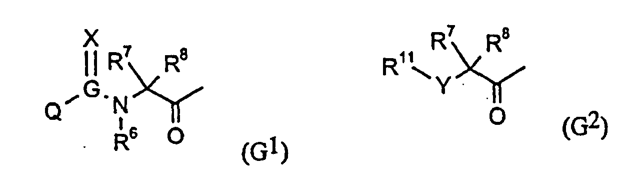 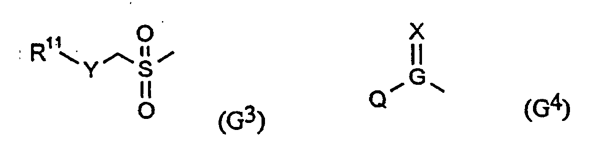 où
R⁶ représente l'hydrogène, un groupe alkyle linéaire ou ramifié, cycloalkyle, aryle, arylalkyle, hétéroaryle, hétéroarylalkyle, ces groupes étant éventuellement substitués,
R⁶ et R⁷ forment conjointement avec les atomes auxquels ils sont liés un noyau pentagonal ou hexagonal qui peut être interrompu, le cas échéant par de l'oxygène, du soufre, un groupe sulfoxyle ou un groupe sulfonyle et qui est éventuellement substitué,
R⁷ et R⁸ représentent, indépendamment l'un de l'autre, l'hydrogène, un groupe alkyle linéaire ou ramifié, alcényle, cycloalkyle, cycloalkylalkyle, aryle, arylalkyle, hétéroaryle, hétéroarylalkyle, ces groupes étant éventuellement substitués, ou bien
R⁷ et R⁸ forment ensemble un noyau spirocyclique qui est éventuellement substitué, 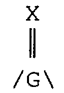 peut représenter un groupe carboxy, thiocarboxy, -C=CH-NO₂, -C=CH-CN, -C=N-R⁹, sulfoxyle, sulfonyle, -P(O)-OR¹⁰ ou P(S)-OR¹⁰,
R⁹ représente l'hydrogène, un groupe hydroxy, alkoxy, alkylcarbonyle, halogénalkylcarbonyle, alkylsulfonyle, nitro ou cyano, et
R¹⁰ représente l'hydrogène ou un groupe alkyle, et
Q représente un groupe alkyle linéaire ou ramifié, alcényle, alcynyle, cycloalkyle, aryle, arylalkyle, hétaryle ou hétarylalkyle, ces groupes étant éventuellement substitués, ou, le cas échéant, un reste du groupe G⁵ ou G⁶ 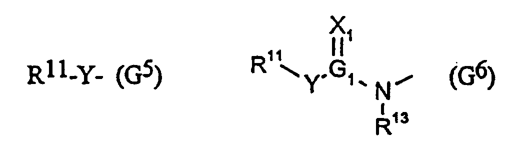 où 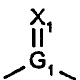 peut représenter un groupe carboxy, thiocarboxy ou sulfonyle,
Y représente l'oxygène, le soufre ou -NR¹²,
R¹¹ au cas où Y est l'azote, peut représenter un groupe amino cyclique lié par l'intermédiaire d'un atome d'azote,
R¹¹ et R¹² représentent, indépendamment l'un de l'autre, l'hydrogène, un groupe alkyle linéaire ou ramifié, alcényle, alcynyle, cycloalkyle, cycloalkylalkyle, aryle, arylalkyle, hétéroaryle, hétéroarylalkyle, ces groupes étant éventuellement substitués, ou bien
R¹¹ et R¹² forment conjointement avec l'atome contigu d'azote un système carbocyclique de 5, 6 ou 7 atomes ou un système bicyclique de 7 à 10 atomes, qui peut aussi être interrompu, le cas échéant par de l'oxygène, du soufre, un groupe sulfoxyle, sulfonyle, carbonyle, -N-O, -N=, -NR¹⁴- ou par de l'azote quaternisé et qui est éventuellement substitué,
R¹³ représente l'hydrogène ou un groupe alkyle,
R¹⁴ représente l'hydrogène, un groupe alkyle linéaire ou ramifié, alcényle, alcynyle, cycloalkyle, cycloalkylalkyle, alkoxycarbonyle, alkylcarbonyle, cycloalkylcarbonyle, cyano, aryle, arylalkyle, hétéroaryle, hétéroarylalkyle, ces groupes étant éventuellement substitués, ainsi que de leurs isomères optiques et leurs racémates,
pour la préparation de compositions destinées à combattre des endoparasites en médecine humaine et en médecine vétérinaire,
les restes éventuellement substitués pouvant porter un ou plusieurs des substituants suivants :
alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes ;
hydroxy, halogéno, cyano, nitro, amino, monoalkyl- et dialkylamino ayant 1 à 4 atomes de carbone par groupe alkyle, alkylcarbonyle ayant 2 à 4 atomes de carbone, alkoxycarbonyle ayant 2 à 4 atomes de carbone, alkylsulfinyle ayant 1 à 4 atomes de carbone, halogénalkylsulfinyle ayant 1 à 4 atomes de carbone et 1 à 5 atomes d'halogènes,
sulfonyle, alkylsulfonyle ayant 1 à 4 atomes de carbone, halogénalkylsulfonyle ayant 1 à 4 atomes de carbone et 1 à 5 atomes d'halogènes, arylsulfonyle ayant 6 à 10 atomes de carbone d'aryle ;
acyle, aryle, aryloxy, hétaryle, hétaryloxy, pouvant eux-mêmes porter l'un des restes mentionnés ci-dessus ;
reste formimino,
et dans les définitions indiquées ci-dessus, le terme alkyle individuellement ou comme constituant d'un reste présente 1 à 6 atomes de carbone et peut être linéaire ou ramifié,
cycloalkyle individuellement ou comme constituant d'un reste présente 3 à 10 atomes de carbone et peut être monocyclique, bicyclique ou tricyclique,
alkoxy individuellement ou comme constituant d'un reste présente 1 à 6 atomes de carbone et peut être linéaire ou ramifié ;
arylalkyle comprend 6 à 10 atomes de carbone dans la partie aryle et 1 à 4 atomes de carbone dans la partie alkyle,
hétéroaryle désigne un noyau pentagonal à heptagonal ayant 1 à 3 hétéroatomes identiques ou différents choisis entre oxygène, soufre et azote ;
alkoxycarbonyle présente 1 à 6 atomes de carbone et peut être linéaire ou ramifié ; alcényle et alcynyle ont chacun 1 à 6 atomes de carbone et peuvent être linéaires ou ramifiés ;
halogénalkyle individuellement ou comme constituant d'un reste contient 1 à 4 atomes de carbone et 1 à 9 atomes d'halogènes identiques ou différents,
alkylcarbonyle présente 1 à 6 atomes de carbone et peut être linéaire ou ramifié ;
cycloalkylcarbonyle désigne un groupe cycloalkylcarbonyle monocyclique, bicyclique ou tricyclique ayant 3 à 10 atomes de carbone.

2. Utilisation de composés de formule (I) suivant la revendication 1
dans laquelle
R¹ représente l'hydrogène, un groupe alkyle linéaire ou ramifié ayant jusqu'à 4 atomes de carbone, cycloalkyle en C₃ à C₆, aryl-(alkyle en C₁ ou C₂), aryle, hétéroaryle, hétéroaryl-(alkyle en C₁ ou C₂), ces groupes étant éventuellement substitués,
R¹ et R² forment conjointement avec les atomes auxquels ils sont liés un noyau pentagonal ou hexagonal qui est éventuellement substitué,
R² et R³ représentent, indépendamment l'un de l'autre, l'hydrogène, un groupe linéaire ou ramifié, alkyle ayant jusqu'à 4 atomes de carbone, halogénalkyle, hydroxyalkyle, (alcanoyloxy en C₁ à C₄)-alkyle, (alkoxy en C₁ ou C₂)-alkyle, mercapto-alkyle en C₁ ou C₂, alkylthioalkyle en C₁ ou C₂, (alkylsulfinyle en C₁ ou C₂)-alkyle, (alkylsulfonyle en C₁ ou C₂)-alkyle, amino-alkyle, (alkylamino en C₁ à C₆)-alkyle, di-(alkyle en C₁ à C₆)amino-alkyle, (cycloalkyle en C₃ à C₆)amino-alkyle, cycloalkyle en C₃ à C₆, (alkoxy en C₁ à C₄)carbonyle,
R⁴ représente l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄)alkyle, (alkoxy en C₁ ou C₂)alkyle, (alkoxy en C₁ à C₄)carbonyl(alkyle en C₁ à C₄), amino-alkyle en C₁ à C₆, (alkylamino en C₁ à C₆)-alkyle en C₁ à C₆, di-(alkyle en C₁ à C₆)amino-alkyle en C₁ à C₆, halogénure de tri(alkyle en C₁ à C₆)ammonium-(alkyle en C₁ à C₆), alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), amino, alkylamino en C₁ à C₄, di-(alkyle en C₁ à C₄)amino, (cycloalkyle en C₃ à C₇)amino, ces groupes étant éventuellement substitués,
R⁵ représente l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄) alkyle, (alkoxy en C₁ ou C₂)alkyle, amino-alkyle en C₁ à C₆, (alkylamino en C₁ à C₆)-(alkyle en C₁ à C₆), di-(alkyle en C₁ à C₆)amino-(alkyle en C₁ à C₆), halogénure de tri-(alkyle en C₁ à C₆)ammonium-(alkyle en C₁ à C₆), nitro-alkyle en C₁ à C₄, cyano-alkyle en C₁ à C₄, (alkoxy en C₁ à C₄)carbonyl-(alkyle en C₁ à C₄), carbamoyl-(alkyle en C₁ à C₄), carboxy-(alkyle en C₁ à C₄), alcényle en C₂ à C₆, alcynyle en C₂ à C₆, cycloalkyle en C₃ à C₆, (cycloalkyle en C₃ à C₆)(alkyle en C₁ ou C₂), aryle, aryl-(alkyle en C₁ ou C₂), hétéroaryle, hétéroaryl-(alkyle en C₁ ou C₂), formylé, (alkoxy en C₁ à C₄)dicarbonyle ou un reste des groupes G¹, G², G³ ou G⁴ 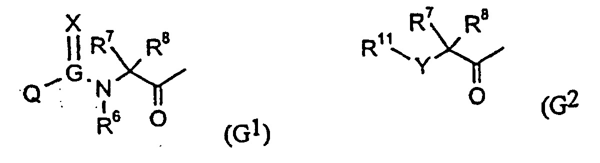 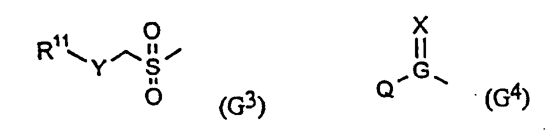 où
R⁶ représente l'hydrogène, un groupe alkyle en C₁ à C₆ linéaire ou ramifié, cycloalkyle en C₃ à C₆, aryl-(alkyle en C₁ ou C₂), hétéroaryl-(alkyle en C₁ ou C₂), ces groupes étant éventuellement substitués,
R⁶ et R⁷ forment conjointement avec les atomes auxquels ils sont liés un noyau pentagonal ou hexagonal qui peut éventuellement être interrompu par de l'oxygène, du soufre, un groupe sulfoxyle ou sulfonyle et qui est éventuellement substitué,
R⁷ et R⁸ représentent, indépendamment l'un de l'autre, l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄)-(alkyle en C₁ à C₆), (alkoxy en C₁ ou C₂)-(alkyle en C₁ à C₆), mercapto-alkyle en C₁ à C₆, (alkylthio en C₁ ou C₂)-(alkyle en C₁ à C₆), (alkylsulfinyle en C₁ ou C₂)-(alkyle en C₁ à C₆), (alkylsulfonyle en C₁ ou C₂)-(alkyle en C₁ à C₆), carboxy-(alkyle en C₁ à C₆), carbamoyl-(alkyle en C₁ à C₆), amino-(alkyle en C₁ à C₆), (alkylamino en C₁ à C₆)-(alkyle en C₁ à C₆), di-(alkyle en C₁ à C₆)amino-(alkyle en C₁ à C₆), guanidino-(alkyle en C₁ à C₆) qui peut être substitué, le cas échéant par un ou deux restes benzyloxycarbonyle ou par un, deux, trois ou quatre restes alkyle en C₁ ou C₂, (alkoxy en C₁ à C₄)-carbonylamino-(alkyle en C₁ à C₆), alcényle en C₂ à C₆, (cycloalkyle en C₃ à C₆)-(alkyle en C₁ ou C₂) ainsi qu'un groupe aryle éventuellement substitué, aryl-(alkyle en C₁ ou C₂), hétéroaryle, hétéroaryl-(alkyle en C₁ ou C₂), ou bien
R⁷ et R⁸ forment ensemble un noyau spirocyclique, 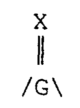 désigne un groupe carboxy, thiocarboxy, -C=CH-NO₂, -C=CH-CN, -C=N-R⁹, sulfoxyle, sulfonyle, -P(O)-OR¹⁰ ou P(S)-OR¹⁰,
R⁹ représente l'hydrogène, un groupe hydroxy, alkoxy en C₁ à C₄, (alkyle en C₁ à C₄)-carbonyle, (halogénalkyle en C₁ à C₄)-carbonyle, alkylsulfonyle en C₁ à C₄), nitro ou cyano, et
R¹⁰ représente l'hydrogène ou un groupe alkyle en C₁ à C₄, et
Q représente un groupe, alkyle linéaire ou ramifié, alkyle en C₁ à C₆, halogénalkyle en C₁ à C₆, hydroxyalkyle en C₁ à C₆, (alcanoyloxy en C₁ à C₄)-alkyle, (alkoxy en C₁ ou C₂)-alkyle, mercapto-alkyle, (alkylthio en C₁ ou C₂)-alkyle, (alkylsulfinyle en C₁ ou C₂)-alkyle, (alkylsulfonyle en C₁ ou C₂)-alkyle, carboxyalkyle, carbamoylalkyle, amino-(alkyle en C₁ à C₆), (alkylamino en C₁ à C₆)-(alkyle en C₁ à C₆), di-(alkyle en C₁ à C₆)amino-(alkyle en C₁ à C₆), guanidino-(alkyle en C₁ à C₆), qui peut être subsitué, le cas échéant par un ou deux restes benzyloxycarbonyle, tertio-butyloxycarbonyle ou par un, deux, trois ou quatre restes alkyle en C₁ ou C₂, (alkoxy en C₁ à C₄)carbonylamino-alkyle, alcényle en C₂ à C₆, alcynyle en C₂ à C₆, cycloalkyle en C₃ à C₆, aryle, aryl-(alkyle en C₁ ou C₂), hétéroaryle ou hétéroaryl-(alkyle en C₁ ou C₂), ces groupes pouvant être substitués, ou éventuellement un reste du groupe G⁵ ou G⁶ 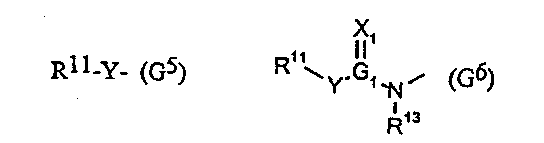 où 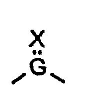 peut représenter un groupe carboxy, thiocarboxy ou sulfonyle,
Y est l'oxygène, le soufre ou un groupe -NR¹²,
R¹¹, au cas où Y est l'azote, peut représenter un groupe amino cyclique lié par l'intermédiaire d'un atome d'azote,
R¹¹ et R¹² représentent, indépendamment l'un de l'autre, l'hydrogène, un groupe linéaire ou ramifié alkyle en C₁ à C₆, alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), ces groupes pouvant être substitués, ou bien
R¹¹ et R¹² forment conjointement avec l'atome d'azote contigu un noyau carbocyclique pentagonal, hexagonal ou heptagonal ou un noyau bicyclique de 7 à 10 atomes, qui peut éventuellement être interrompu également par de l'azote, du soufre, un groupe sulfoxyle, sulfonyle, carbonyle, -N-O, -N=, -NR¹⁴- ou par de l'azote quaternisé et qui est éventuellement substitué,
R¹³ représente l'hydrogène ou un groupe alkyle en C₁ à C₄,
R¹⁴ représente l'hydrogène, un groupe linéaire ou ramifié alkyle en C₁ à C₆, alcényle en C₂ à C₆, alcynyle en C₂ à C₆, cycloalkyle en C₃ à C₈, (cycloalkyle en C₃ à C₈)-(alkyle en C₁ ou C₂), (alkoxy en C₁ à C₄)-carbonyle, (alkyle en C₁ à C₄)carbonyle, (cycloalkyle en C₃ à C₆)carbonyle, cyano, aryle, aryl-(alkyle en C₁ ou C₂), hétéroaryle, hétéroaryl-(alkyle en C₁ ou C₂), ces groupes pouvant être substitués,
ainsi que de leurs isomères optiques et leurs racémates, pour la préparation de compositions destinées à combattre des endoparasites en médecine humaine et en médecine vétérinaire,
les restes éventuellement substitués pouvant porter un ou plusieurs des substituants suivants :
alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes ;
hydroxy, halogéno, cyano, nitro, amino, monoalkyl- et dialkylamino ayant 1 à 4 atomes de carbone par groupe alkyle, alkylcarbonyle ayant 2 à 4 atomes de carbone, alkoxycarbonyle ayant 2 à 4 atomes de carbone, alkylsulfinyle ayant 1 à 4 atomes de carbone, halogénalkylsulfinyle ayant 1 à 4 atomes de carbone et 1 à 5 atomes d'halogènes,
sulfonyle, alkylsulfonyle ayant 1 à 4 atomes de carbone, halogénalkylsulfonyle ayant 1 à 4 atomes de carbone et 1 à 5 atomes d'halogènes, arylsulfonyle ayant 6 à 10 atomes de carbone d'aryle ;
acyle, aryle, aryloxy, hétaryle, hétaryloxy, ces groupes pouvant eux-mêmes porter un ou plusieurs des restes mentionnés ci-dessus ;
reste formimino,
et dans les définitions indiquées ci-dessus, le terme hétéroaryle désigne un noyau pentagonal à heptagonal ayant 1 à 3 hétéroatomes identiques ou différents choisis entre oxygène, soufre et azote.

3. Dérivés de 4a,5a,8a,8b-tétrahydro-6H-pyrrolo[3',4':4,5]furo[3,2-b]pyridine-6,8(7H)-dione de formule générale (Ia) et leurs sels 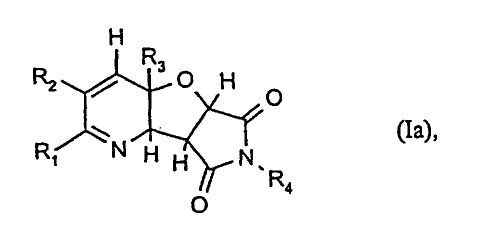 formule dans laquelle
R¹ représente l'hydrogène, un groupe alkyle linéaire ou ramifié ayant jusqu'à 4 atomes de carbone, cycloalkyle en C₃ à C₆, aryl-(alkyle en C₁ ou C₂), aryle, hétéroaryle, hétéroaryl-(alkyle en C₁ ou C₂), ces groupes étant éventuellement substitués,
R² et R³ représentent, indépendamment l'un de l'autre, l'hydrogène, un groupe linéaire ou ramifié alkyle ayant jusqu'à 4 atomes de carbone, halogénalkyle, hydroxyalkyle, (alcanoyloxy en C₁ à C₄)alkyle, (alkoxy en C₁ ou C₂)alkyle, mercapto-alkyle en C₁ ou C₂, (alkylthio en C₁ ou C₂)alkyle, (alkylsulfinyle en C₁ ou C₂)alkyle, (alkylsulfonyle en C₁ ou C₂)alkyle, amino-alkyle, (alkylamino en C₁ à C₆)-alkyle, di-(alkyle en C₁ à C₆)amino-alkyle, (cycloalkyle en C₃ à C₆)aminoalkyle, cycloalkyle en C₃ à C₆, (alkoxy en C₁ à C₄)carbonyle,
R⁴ représente l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄)-alkyle, (alkoxy en C₁ ou C₂)alkyle, (alkoxy en C₁ à C₄)carbonyl-(alkyle en C₁ à C₄), amino-alkyle en C₁ à C₆, (alkylamino en C₁ à C₆)-(alkyle en C₁ à C₆), di-(alkyle en C₁ à C₆)amino-(alkyle en C₁ à C₆), tri-(alkyle en C₁ à C₆)ammonium-(alkyle en C₁ à C₆), alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), amino, alkylamino en C₁ à C₄, di-(alkyle en C₁ à C₄)amino, cycloalkylamino en C₃ à C₇, ces groupes étant éventuellement substitués,
sous réserve que lorsque
R¹ est l'hydrogène, et
R² et R³ représentent un groupe méthyle,
R⁴ ne soit pas un groupe méthyle, n-butyle, phényle, 2-, 3- ou 4-méthylphényle, 2-, 3- ou 4-chlorophényle, 2-, 3- ou 4-fluorophényle, 2-chloro-, 4-fluorophényle, 2-fluoro, 4-chlorophényle, 3-chloro, 4-fluorophényle, 2-, 3- ou 4-nitrophényle, 4-méthoxyphényle ou α-naphtyle,
ainsi que leurs isomères optiques et leurs racémates.

4. Procédé de production des nouveaux dérivés de 4a,5a,8a,8b-tétrahydro-6H-pyrrolo[3',4':4,5]furo[3,2-b]pyridine-6,8(7H)-dione de formule générale (Ia) suivant la revendication 3 et de leurs sels, 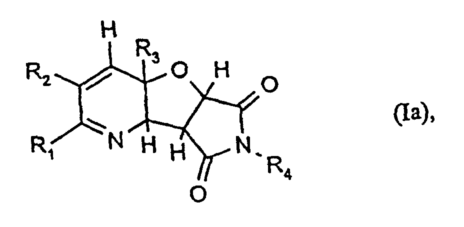 dans laquelle
R¹, R², R³ et R⁴ ont la définition indiquée dans la revendication 3,
**caractérisé en ce que**
a) on fait réagir des N-oxydes de pyridine de formule générale (II) 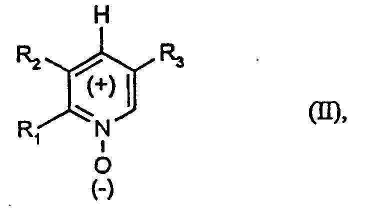
dans laquelle
R¹, R² et R³ ont la définition indiquée dans la revendication 3, avec des imides d'acide maléique de formule générale (III) 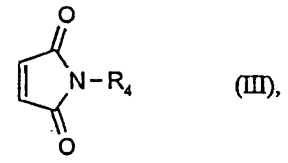 dans laquelle
R⁴ a la définition indiquée dans la revendication 3, le cas échéant en présence d'un diluant.

5. Nouveaux dérivés de 1,2,3,4,4a,5a,8a,8b-octahydro-6H-pyrrolo[3',4':4,5]furo[3,2-b]pyridine-6,8(7H)-dione et de 1,2,4a,5a,8a,8b-hexahydro-6H- pyrrolo[3',4':4,5]furo[3,2-b]pyridine-6,8(7H)-dione de formules générales (Ib) et (Ic) ainsi que leurs sels, 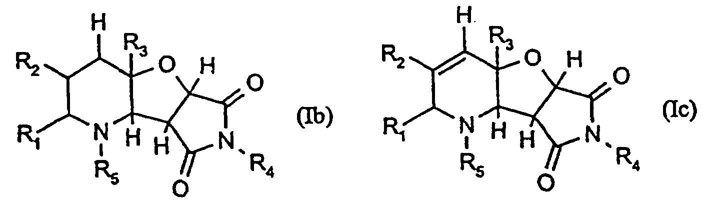 formules dans lesquelles
R¹ représente l'hydrogène, un groupe alkyle linéaire ou ramifié ayant jusqu'à 4 atomes de carbone, un groupe cycloalkyle en C₃ à C₆, aryl-(alkyle en C₁ ou C₂), aryle, hétéroaryle, hétéroaryl-(alkyle en C₁ ou C₂), ces groupes étant éventuellement substitués,
R² et R³ représentent, indépendamment l'un de l'autre, l'hydrogène, un groupe linéaire ou ramifié alkyle ayant jusqu'à 4 atomes de carbone, halogénalkyle, hydroxyalkyle, (alcanoyloxy en C₁ à C₄)-alkyle, (alkoxy en C₁ ou C₂)-alkyle, mercapto-alkyle en C₁ ou C₂, (alkylthio en C₁ ou C₂)-alkyle, (alkylsulfinyle en C₁ ou C₂)-alkyle, (alkylsulfonyle en C₁ ou C₂)-alkyle, amino-alkyle, (alkylamino en C₁ à C₆)-alkyle, di-(alkyle en C₁ à C₆)-amino-alkyle, (cycloalkyle en C₃ à C₆)amino-alkyle, cycloalkyle en C₃ à C₆, (alkoxy en C₁ à C₄)carbonyle,
R⁴ représente l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄)-alkyle, (alkoxy en C₁ ou C₂)-alkyle, (alkoxy en C₁ à C₄)-carbonyl-(alkyle en C₁ à C₄), amino-alkyle en C₁ à C₆, (alkylamino en C₁ à C₆)-(alkyle en C₁ à C₆), di-(alkyle en C₁ à C₆)amino-(alkyle en C₁ à C₆), halogénure de tri-(alkyle en C₁ à C₆)ammonium-(alkyle en C₁ à C₆), alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), amino, alkylamino en C₁ à C₄, di-(alkyle en C₁ à C₄)amino, cycloalkylamino en C₃ à C₇, ces groupes étant éventuellement substitués,
R⁵ représente l'hydrogène, un groupe linéaire ou ramifié alkyle en C₁ à C₆, halogénalkyle en C₁ à C₆, hydroxyalkyle en C₁ à C₆, (alcanoyloxy en C₁ à C₄)-alkyle, (alkoxy en C₁ ou C₂)-alkyle, amino-alkyle en C₁ à C₆, (alkylamino en C₁ à C₆)-(alkyle en C₁ à C₆), di-(alkyle en C₁ à C₆)amino-(alkyle en C₁ à C₆), halogénure de tri-(alkyle en C₁ à C₆)ammonium-(alkyle en C₁ à C₆), nitro-alkyle en C₁ à C₄, cyano-alkyle en C₁ à C₄, (alkoxy en C₁ à C₄)carbonyl-(alkyle en C₁ à C₄), carbamoyl-(alkyle en C₁ à C₄), carboxy-(alkyle en C₁ à C₄), alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), formyle, (alkoxy en C₁ à C₄)-dicarbonyle ou un reste du groupe G¹, G², G³ ou G⁴ 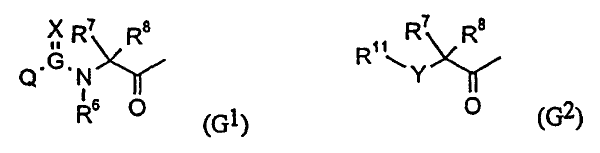 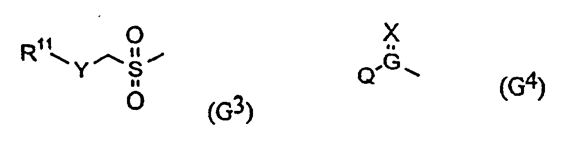 où
R⁶ représente l'hydrogène, un groupe alkyle en C₁ à C₄ linéaire ou ramifié, cycloalkyle en C₃ à C₆, aryl-(alkyle en C₁ à C₂) ou hétéroaryl-(alkyle en C₁ ou C₂), ces groupes étant éventuellement substitués,
R⁶ et R⁷ forment conjointement avec les atomes auxquels ils sont liés un noyau pentagonal ou hexagonal qui peut être interrompu éventuellement par de l'oxygène, du soufre, un groupe sulfoxyle ou sulfonyle et qui est éventuellement substitué,
R⁷ représente l'hydrogène, un groupe linéaire ou ramifié alkyle en C₁ à C₆, alcényle en C₂ à C₄, cycloalkyle en C₃ à C₆), (cycloalkyle en C₃ à C₆)-(alkyle en C₁ ou C₂) ainsi qu'un groupe éventuellement substitué aryle, aryl-(alkyle en C₁ ou C₂), hétéroaryle, hétéroaryl-(alkyle en C₁ ou C₂), ou bien
R⁸ représente l'hydrogène ou un groupe méthyle, 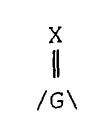 peut représenter un groupe carboxy, thiocarboxy, -C=CH-NO₂, -C=CH-CN, -C=N-R⁹, sulfoxyle, sulfonyle, -P(O)-OR¹⁰ ou P(S)-OR¹⁰,
R⁹ représente l'hydrogène, un groupe hydroxy, alkoxy en C₁ à C₄, (alkyle en C₁ à C₄)-carbonyle, (halogénalkyle en C₁ à C₄)-carbonyle, alkylsulfonyle en C₁ à C₄, nitro ou cyano, et
R¹⁰ représente l'hydrogène ou un groupe alkyle en C₁ à C₄, et
Q représente un groupe alkyle linéaire ou ramifié en C₁ à C₆, halogénalkyle en C₁ à C₆, hydroxyalkyle en C₁ à C₆, (alcanoyloxy en C₁ à C₄)-alkyle, (alkoxy en C₁ ou C₂)-alkyle, mercapto-alkyle, (alkylthio en C₁ ou C₂)-alkyle, (alkylsulfinyle en C₁ ou C₂)-alkyle, (alkylsulfonyle en C₁ ou C₂)-alkyle, carboxyalkyle, carbamoylalkyle, amino-(alkyle en C₁ à C₆), (alkylamino en C₁ à C₆)-(alkyle en C₁ à C₆), di-(alkyle en C₁ à C₆)amino-(alkyle en C₁ à C₆), guanidino-(alkyle en C₁ à C₆), qui peut être substitué, le cas échéant par un ou deux restes benzyloxycarbonyle, tertio-butyloxycarbonyle ou par un, deux, trois ou quatre restes alkyle en C₁ ou C₂, (alkoxy en C₁ à C₄)carbonylamino-alkyle, alcényle en C₂ à C₆, alcynyle en C₂ à C₆, cycloalkyle en C₃ à C₆, aryle, aryl-(alkyle en C₁ ou C₂), hétéroaryle ou hétaryl-(alkyle en C₁ ou C₂), ces groupes pouvant être substitués, ou, le cas échéant, un reste du groupe G⁵ ou G⁶ 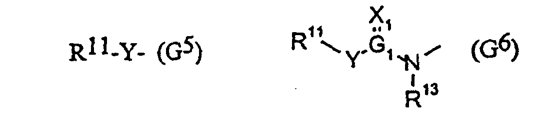 où 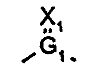 peut représenter un groupe carboxy, thiocarboxy ou sulfonyle,
Y est l'oxygène, le soufre ou un groupe -NR¹²,
R¹¹, au cas où Y est l'azote, peut représenter un groupe amino cyclique lié par l'intermédiaire d'un atome d'azote,
R¹¹ et R¹² représentent, indépendamment l'un de l'autre, l'hydrogène, un groupe linéaire ou ramifié alkyle en C₁ à C₆, alcényle en C₂ à C₆, alcynyle en C₂ à C₆, cycloalkyle en C₃ à C₆, (cycloalkyle en C₃ à C₆)-(alkyle en C₁ ou C₂), aryle, aryl-(alkyle en C₁ ou C₂), hétéroaryle, hétéroaryl-(alkyle en C₁ ou C₂), ces groupes étant éventuellement substitués, ou bien
R¹¹ et R¹² forment conjointement avec l'atome contigu d'azote un noyau pentagonal, hexagonal ou heptagonal carbocyclique ou un noyau bicyclique de 7 à 10 atomes, qui peut être interrompu, le cas échéant par de l'oxygène, du soufre, un groupe sulfoxyle, sulfonyle, carbonyle, -N-O, -N=, -NR¹⁴- ou par de l'azote quaternisé et qui est éventuellement substitué,
R¹³ représente l'hydrogène ou un groupe alkyle en C₁ à C₄,
R¹⁴ représente l'hydrogène, un groupe linéaire ou ramifié alkyle en C₁ à C₆, alcényle en C₂ à C₆, alcynyle en C₂ à C₆, cycloalkyle en C₃ à C₈, (cycloalkyle en C₃ à C₈)-(alkyle en C₁ ou C₂), (alkoxy en C₁ à C₄)-carbonyle, (alkyle en C₁ à C₄)-carbonyle, (cycloalkyle en C₃ à C₆)-carbonyle, cyano, aryle, aryl-(alkyle en C₁ ou C₂), hétéroaryle, hétéroaryl-(alkyle en C₁ ou C₂), ces groupes étant éventuellement substitués,
ainsi que de leurs isomères optiques et leurs racémates.

6. Procédé de production des nouveaux dérivés de 1,2,3,4,4a,5a,8a,8b-octahydro-6H-pyrrolo[3',4':4,5]furo[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione de formules générales (Ib) et (Ic) suivant la revendication 5 ainsi que de leurs sels, 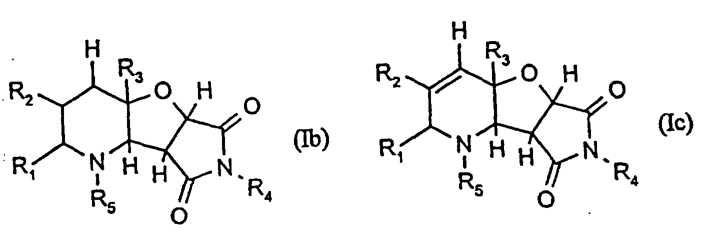 où
R¹, R², R³, R⁴ et R⁵ ont la définition indiquée dans la revendication 5,
**caractérisé en ce que** dans une première étape réactionnelle, on hydrogène les dérivés de 4a,5a,8a,8b-tétrahydro-6H-pyrrolo[3',4':4,5]furo[3,2-b]pyridine-6,8(7H)-dione obtenus par exemple selon le procédé 3, de formule générale (Ia) 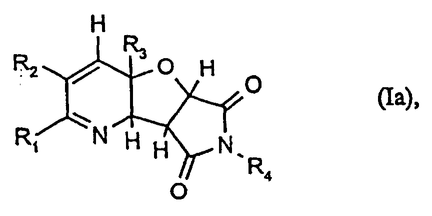 dans laquelle
R¹, R², R³ et R⁴ ont la définition indiquée dans la revendication 3,
en présence de catalyseurs appropriés pour former des dérivés de 1,2,3,4,4a,5a,8a,8b-octahydro-6H-pyrrolo[3',4':4,5]furo[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione de formules générales (Id) et (Ie) 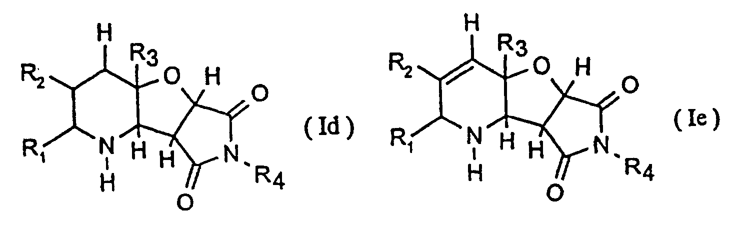 formules dans lesquelles
R¹, R², R³ et R⁴ ont la définition indiquée ci-dessus,
ou bien
en vue de la préparation sélective des dérivés nouveaux de formule générale (Ie) et de leurs sels, on fait réagir les dérivés de formule générale (Ia) tout d'abord avec des halogénures de méthyle de formule générale (IV)
Me-Hal (IV)
dans laquelle
Hal représente un halogène, en particulier le fluor, le chlore, le brome ou l'iode, éventuellement en présence de diluants, puis on hydrogène les halogénures de N¹-méthylammonium formés, de formule générale (V) 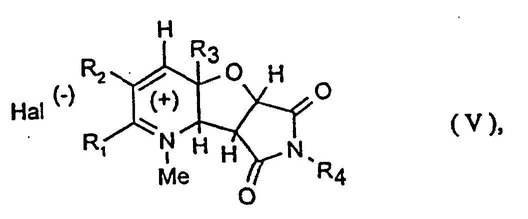 en présence d'un diluant approprié, après quoi on déméthyle le dérivé N¹-méthylique résultant de formule générale (VI) 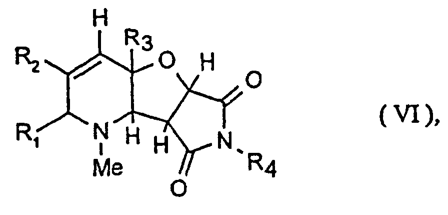 dans laquelle
R¹, R², R³ et R⁴ ont la définition indiquée ci-dessus,
sur l'atome N¹ avec formation de (Ie), puis on fait réagir dans une deuxième étape réactionnelle les dérivés de 1,2,3,4,4a,5a,8a,8b-octahydro-6H-pyrrolo[3',4':4,5]furo[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione ainsi obtenus de formules générales (Id) et (Ie) 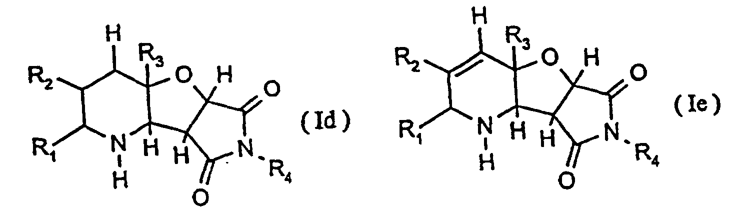 dans lesquelles
R¹, R², R³ et R⁴ ont la définition indiquée ci-dessus,
a) avec des composés de formule générale (VII)
R⁵-E (VII)
dans laquelle
R⁵ a la définition indiquée ci-dessus et
E représente un groupe partant attirant les électrons,
le cas échéant en présence de diluants et en présence éventuelle d'auxiliaires basiques de réaction, ou bien
b) avec des composés de formule générale (VIII) 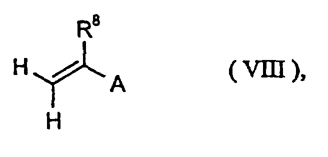 dans laquelle
A est un groupe accepteur approprié tel que par exemple nitro, nitrile, carbamoyle ou R¹³-O-CO-, et
R⁸ et R¹³ ont la définition indiquée dans la revendication 5, ou bien
c) avec un époxyde de formule générale (IX) 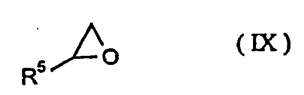 dans laquelle
R⁵ a la définition indiquée au point 4,
le cas échéant en présence d'un catalyseur ou en présence d'un auxiliaire basique de réaction, le cas échéant en présence de diluants, ou bien
d) avec des composés de formule générale (X) 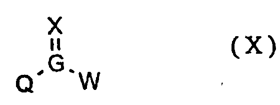 dans laquelle
G, Q et X ont la définition indiquée dans la revendication 5, et
W représente un groupe partant approprié tel que, par exemple, un halogène, un groupe alkoxy, alkylthio ou aryloxy, éventuellement en présence d'un catalyseur, en présence éventuelle d'un accepteur d'acide et, le cas échéant en présence de diluants, ou bien
en vue de la préparation des dérivés nouveaux de 1,2,3,4,4a,5a,8a,8b-octahydro-6H-pyrrolo[3',4':4,5]furo[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione de formules générales (Ib) et (Ic) et de leurs sels,
où le groupe 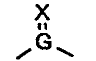 est un groupe carboxy,
e) avec un anhydride d'acide carboxylique de formule générale (XI)
(Q-C=O)₂O (XI)
dans laquelle
Q a la définition indiquée au point 4,
le cas échéant en présence d'un catalyseur et en présence éventuelle de diluants, ou bien
f) avec des dérivés d'amino-acides de formule générale (XII) 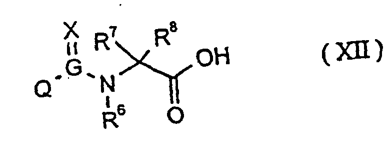 dans laquelle
G, Q, X, R⁶, R⁷ et R⁸ ont la définition indiquée dans la revendication 5, ou leurs dérivés activés sur le groupe carboxy,
le cas échéant en présence d'un catalyseur, en présence éventuelle d'un accepteur d'acide et, le cas échéant en présence de diluants, ou bien
g) avec des composés de formule générale (XIII) ou (XIV) 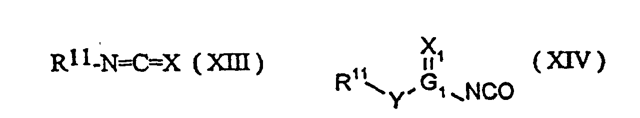 où
les restes R¹¹, G¹, X, X¹ et Y ont la définition indiquée dans la revendication 1,
le cas échéant en présence d'un catalyseur, en présence éventuelle d'un accepteur d'acide et, le cas échéant en présence de diluants, ou bien
pour la préparation des dérivés nouveaux de 1,2,3,4,4a,5a,8a,8b-octahydro-6H-pyrrolo[3',4':4,5]furo[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione de formules générales (Ib) et (Ic) et de leurs sels,
où le groupe 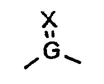 représente un groupe carboxy, et Y représente l'oxygène,
h) dans une troisième étape réactionnelle, avec le dioxyde de carbone et un carbonate de métal alcalin de formule générale (XV)
M₂CO₃ (XV)
dans laquelle
M est un cation de métal alcalin monovalent, de préférence le cation lithium, sodium, potassium ou césium, en particulier potassium ou césium,
puis dans une quatrième étape réactionnelle, on fait réagir les sels de métal alcalin résultants de composés de formules générales (XVI) et (XVII) 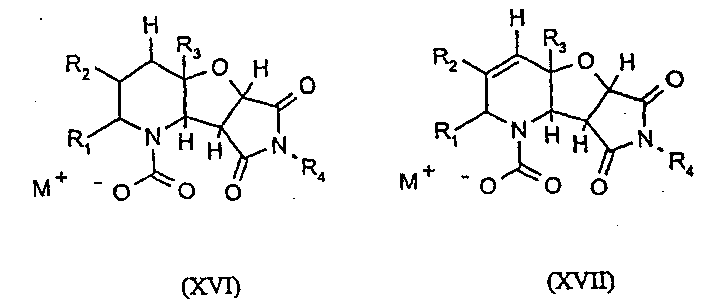
dans lesquelles
les restes R¹, R², R³ et R⁴ ont la définition indiquée dans la revendication 5,
M représente un équivalent de cation métallique lié à la manière d'un sel,
avec un agent alkylant de formule (VIIa)
R¹¹-Hal (VIIa)
dans laquelle
R¹¹ a la définition indiquée dans la revendication 5 et
Hal représente un halogène tel que le fluor, le chlore, le brome ou l'iode,
éventuellement en présence d'un auxiliaire basique de réaction et en présence éventuelle de diluants, ou bien
i) on fait réagir des dérivés de 1,2,3,4,4a,5a,8a,8b-octahydro-6H-pyrrolo[3',4':4,5]furo-[3,2-b]pyridine-6,8(7H)-dione et/ou de 1,2,4a,5a,8a,8b-hexahydro-6H-pyrrolo[3',4':4,5]furo[3,2-b]pyridine-6,8(7H)-dione de formules générales (If) et (Ig) 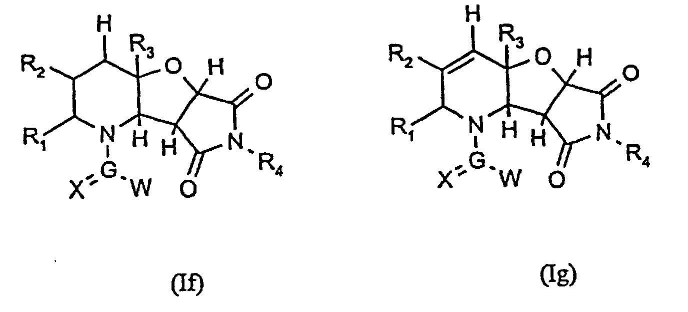 dans lesquelles
les restes R¹, R², R³, R⁴, G, W et X ont la définition indiquée dans la revendication 5,
le cas échéant en présence d'un catalyseur, en présence éventuelle d'un accepteur d'acide et, le cas échéant en présence de diluants, avec des composés de formule générale (XVIII)
R¹¹-Y-H (XVIII)
dans laquelle
les restes R¹¹ et Y ont la définition indiquée dans la revendication 5.

7. Compositions endoparasiticides, **caractérisées par** une teneur en au moins un dérivé de 4a,5a,8a,8b-tétrahydro-6H-pyrrolo[3',4':4,5]furo[3,2-b]pyridine-6,8(7H)-dione de formule (I) suivant la revendication 1.

8. Procédé de préparation de compositions endoparasiticides, **caractérisé en ce qu'**on mélange des dérivés de 4a,5a,8a,8b-tétrahydro-6H-pyrrolo[3',4':4,5]furo[3,2-b]pyridine-6,8(7H)-dione de formule (I) suivant la revendication 1 avec des diluants et/ou des agents tensioactifs.

9. Utilisation de dérivés de 4a,5a,8a,8b-tétrahydro-6H-pyrrolo[3',4':4,5]furo[3,2-b]pyridine-6,8(7H)-dione de formule (I) suivant la revendication 1 pour la préparation de compositions endoparasiticides.
